# EUROPEAN PATENT APPLICATION

(11) **EP 4 047 088 A1**
(43) Date of publication of application: **24.08.2022**
(21) Application number: 21158484.2
(22) Date of filing: 22.02.2021
(51) Int. Cl.: C12N 9/28, C12N 15/63

(54) **AMYLASE VARIANTS**

(71) Applicant: BASF SE, 67056 Ludwigshafen am Rhein (DE); Henkel AG & Co. KGaA, 40589 Düsseldorf (DE)
(72) Inventor: Nielsen, Jasper, San Diego, CA 92121 (US); Pop. Christina, San Diego, CA 92121 (US); Jenewein, Stefan, 67056 Ludwigshafen (DE); Lyon, Jonathan D, San Diego, CA 92121 (US); Miller, Mark, San Diego, CA 92121 (US); Logue, Amanda Rae, San Diego, CA 92121 (US); Kline, Katie, San Diego, CA 92121 (US); Hoang, Cindy, San Diego, CA 92121 (US); Wieland, Susanne, 41541 Dormagen-Zons (DE)
(74) Representative: BASF IP Association

(57) **Abstract**

In the present invention new amylase enzymes are provided. More specifically, genetically engineered amylase enzymes, compositions comprising the enzymes, and methods of making and using the enzymes or compositions comprising the enzymes are provided.

SEQUENCE LISTING

<110> BASF SE
      Henkel AG & Co. KGAA

<120> Amylase Variants

<130> 210083EP02

<160> 14

<170> BiSSAP 1.3.6

<210> 1
<211> 483
<212> PRT
<213> Artificial Sequence

<220>
<223> Artificial Sequence

<400> 1
His His Asn Gly Thr Asn Gly Thr Met Met Gln Tyr Phe Glu Trp Tyr
1               5                   10                  15
Leu Pro Asn Asp Gly Asn His Trp Asn Arg Leu Arg Ser Asp Ala Ser
            20                  25                  30
Asn Leu Lys Asp Lys Gly Ile Thr Ala Val Trp Ile Pro Pro Ala Trp
        35                  40                  45
Lys Gly Ala Ser Gln Asn Asp Val Gly Tyr Gly Ala Tyr Asp Leu Tyr
    50                  55                  60
Asp Leu Gly Glu Phe Asn Gln Lys Gly Thr Val Arg Thr Lys Tyr Gly
65                  70                  75                  80
Thr Arg Asn Gln Leu Gln Ala Ala Val Thr Ala Leu Lys Ser Asn Gly
                85                  90                  95
Ile Gln Val Tyr Gly Asp Val Val Met Asn His Lys Gly Gly Ala Asp
            100                 105                 110
Ala Thr Glu Trp Val Arg Ala Val Glu Val Asn Pro Ser Asn Arg Asn
        115                 120                 125
Gln Glu Val Ser Gly Asp Tyr Thr Ile Glu Ala Trp Thr Lys Phe Asp
    130                 135                 140
Phe Pro Gly Arg Gly Asn Thr His Ser Asn Phe Lys Trp Arg Trp Tyr
145                 150                 155                 160
His Phe Asp Gly Val Asp Trp Asp Gln Ser Arg Gln Leu Gln Asn Arg
                165                 170                 175
Ile Tyr Lys Phe Arg Gly Lys Gly Trp Asp Trp Glu Val Asp Thr Glu
            180                 185                 190
Asn Gly Asn Tyr Asp Tyr Leu Met Tyr Ala Asp Ile Asp Met Asp His
        195                 200                 205
Pro Glu Val Val Asn Glu Leu Arg Asn Trp Gly Val Trp Tyr Thr Asn
    210                 215                 220
Thr Leu Gly Leu Asp Gly Phe Arg Ile Asp Ala Val Lys His Ile Lys
225                 230                 235                 240
Tyr Ser Phe Thr Arg Asp Trp Leu Thr His Val Arg Asn Thr Thr Gly
                245                 250                 255
Lys Asn Met Phe Ala Val Ala Glu Phe Trp Lys Asn Asp Ile Gly Ala
            260                 265                 270
Ile Glu Asn Tyr Leu Ser Lys Thr Asn Trp Asn His Ser Val Phe Asp
        275                 280                 285
Val Pro Leu His Tyr Asn Leu Tyr Asn Ala Ser Arg Ser Gly Gly Asn
    290                 295                 300
Tyr Asp Met Arg Gln Ile Phe Asn Gly Thr Val Val Gln Arg His Pro
305                 310                 315                 320

Thr His Ala Val Thr Phe Val Asp Asn His Asp Ser Gln Pro Glu Glu
                325                 330                 335
Ala Leu Glu Ser Phe Val Glu Glu Trp Phe Lys Pro Leu Ala Tyr Ala
            340                 345                 350
Leu Thr Leu Thr Arg Asp Gln Gly Tyr Pro Ser Val Phe Tyr Gly Asp
        355                 360                 365
Tyr Tyr Gly Ile Pro Thr His Gly Val Pro Ala Met Lys Ser Lys Ile
    370                 375                 380
Asp Pro Ile Leu Glu Ala Arg Gln Lys Tyr Ala Tyr Gly Thr Gln Arg
385                 390                 395                 400
Asp Tyr Leu Asp His Pro Asp Val Ile Gly Trp Thr Arg Glu Gly Asp
                405                 410                 415
Gly Val His Ala Asp Ser Gly Leu Ala Thr Leu Met Ser Asp Gly Pro
            420                 425                 430
Gly Gly Ser Lys Trp Met Glu Val Gly Lys Asn Asn Ala Gly Glu Val
        435                 440                 445
Trp Tyr Asp Ile Thr Gly Asn Gln Thr Asn Thr Val Thr Ile Asn Lys
    450                 455                 460
Asp Gly Trp Gly Gln Phe His Val Ser Gly Gly Ser Val Ser Ile Tyr
465                 470                 475                 480
Val Gln Gln

<210> 2
<211> 1449
<212> DNA
<213> Artificial Sequence

<220>
<223> Artificial Sequence

<400> 2
catcacaatg gcacaaacgg taccatgatg caatattttg agtggtacct cccaaatgac      60

ggaaatcatt ggaacagatt gcgctctgat gcaagcaacc ttaaagataa gggcataaca     120

gctgtttgga ttccgccagc atggaaaggc gccagtcaaa acgacgtcgg atatggagcg     180

tacgatctgt atgatttagg cgagtttaat cagaaaggca cagtaagaac gaaatatgga     240

accaggaatc aacttcaggc ggctgtgacg gccctcaagt ctaatggcat tcaggtgtat     300

ggcgacgttg tgatgaacca caaaggagga gctgatgcga cagaatgggt tagagcagtc     360

gaagtaaatc ctagcaatcg aaaccaggag gtgagcggtg attacacaat cgaggcatgg     420

accaaattcg actttcctgg acgtggcaac acacatagta attttaagtg gagatggtat     480

cacttcgatg gcgtagactg ggatcaatct agacagttac agaaccggat ctacaaattt     540

cgcggaaaag gctgggattg ggaagttgat acggagaacg gcaattatga ttacttgatg     600

tacgcagata tcgacatgga tcatccggaa gtggtcaatg agttacgtaa ttggggcgtc     660

tggtacacta acacactggg ccttgacgga ttccgcattg atgcggttaa acatatcaaa     720

tattcgttta cgcgcgactg gttaactcat gtccggaata caacaggcaa gaatatgttt     780

gctgttgcag aattttggaa aaacgatatc ggcgcgattg aaaattatct ttcaaagact     840

aactggaatc attccgtatt cgacgtgcct ttgcactaca acctgtataa tgcgtcgcgc     900

tccggcggta actatgacat gcgacaaatc ttcaacggca cagtcgttca gcgtcacccc 960

acacacgctg ttacatttgt agacaaccat gacagccaac cggaggaagc tcttgaaagc 1020

ttcgtagaag aatggtttaa gcctctggcc tatgcgctga cgcttacgcg ggatcaagga 1080

tacccgagcg tgttttatgg agattactat ggcattccga cgcatggcgt gccagccatg 1140

aaatcaaaaa ttgatccgat actcgaagcc agacaaaaat atgcatatgg tactcaacgg 1200

gattatttgg accatccgga tgtcattggt tggacccgtg aaggagatgg agtacatgct 1260

gactctggct tagccacact gatgtccgat ggtcctggag gctcaaaatg gatggaagtg 1320

ggtaagaaca atgcaggtga agtttggtat gatattaccg gcaatcagac gaatacggtt 1380

actattaata aagatggttg gggacaattt catgtctcag gcggctctgt ctcaatctat 1440

gttcagcag 1449

<210> 3
<211> 485
<212> PRT
<213> Artificial Sequence

<220>
<223> Artificial Sequence

<400> 3
His His Asn Gly Thr Asn Gly Thr Met Met Gln Tyr Phe Glu Trp Tyr
1               5                   10                  15
Leu Pro Asn Asp Gly Asn His Trp Asn Arg Leu Arg Ser Asp Ala Ser
            20                  25                  30
Asn Leu Lys Asp Lys Gly Ile Thr Ala Val Trp Ile Pro Pro Ala Trp
        35                  40                  45
Lys Gly Ala Ser Gln Asn Asp Val Gly Tyr Gly Ala Tyr Asp Leu Tyr
    50                  55                  60
Asp Leu Gly Glu Phe Asn Gln Lys Gly Thr Val Arg Thr Lys Tyr Gly
65                  70                  75                  80
Thr Arg Asn Gln Leu Gln Ala Ala Val Thr Ala Leu Lys Ser Asn Gly
                85                  90                  95
Ile Gln Val Tyr Gly Asp Val Val Met Asn His Lys Gly Gly Ala Asp
            100                 105                 110
Ala Thr Glu Trp Val Arg Ala Val Glu Val Asn Pro Ser Asn Arg Asn
        115                 120                 125
Gln Glu Val Ser Gly Asp Tyr Thr Ile Glu Ala Trp Thr Lys Phe Asp
    130                 135                 140
Phe Pro Gly Arg Gly Asn Thr His Ser Asn Phe Lys Trp Arg Trp Tyr
145                 150                 155                 160
His Phe Asp Gly Val Asp Trp Asp Gln Ser Arg Gln Leu Gln Asn Arg
                165                 170                 175
Ile Tyr Lys Phe Arg Gly Asp Gly Lys Gly Trp Asp Trp Glu Val Asp
            180                 185                 190
Thr Glu Asn Gly Asn Tyr Asp Tyr Leu Met Tyr Ala Asp Ile Asp Met
        195                 200                 205
Asp His Pro Glu Val Val Asn Glu Leu Arg Asn Trp Gly Val Trp Tyr
    210                 215                 220
Thr Asn Thr Leu Gly Leu Asp Gly Phe Arg Ile Asp Ala Val Lys His
225                 230                 235                 240
Ile Lys Tyr Ser Phe Thr Arg Asp Trp Leu Thr His Val Arg Asn Thr
                245                 250                 255

Thr Gly Lys Asn Met Phe Ala Val Ala Glu Phe Trp Lys Asn Asp Ile
            260                 265                 270
Gly Ala Ile Glu Asn Tyr Leu Ser Lys Thr Asn Trp Asn His Ser Val
        275                 280                 285
Phe Asp Val Pro Leu His Tyr Asn Leu Tyr Asn Ala Ser Arg Ser Gly
    290                 295                 300
Gly Asn Tyr Asp Met Arg Gln Ile Phe Asn Gly Thr Val Val Gln Arg
305                 310                 315                 320
His Pro Thr His Ala Val Thr Phe Val Asp Asn His Asp Ser Gln Pro
                325                 330                 335
Glu Glu Ala Leu Glu Ser Phe Val Glu Glu Trp Phe Lys Pro Leu Ala
            340                 345                 350
Tyr Ala Leu Thr Leu Thr Arg Asp Gln Gly Tyr Pro Ser Val Phe Tyr
        355                 360                 365
Gly Asp Tyr Tyr Gly Ile Pro Thr His Gly Val Pro Ala Met Lys Ser
    370                 375                 380
Lys Ile Asp Pro Ile Leu Glu Ala Arg Gln Lys Tyr Ala Tyr Gly Lys
385                 390                 395                 400
Gln Asn Asp Tyr Leu Asp His His Asn Met Ile Gly Trp Thr Arg Glu
                405                 410                 415
Gly Asn Thr Ala His Pro Asn Ser Gly Leu Ala Thr Ile Met Ser Asp
            420                 425                 430
Gly Pro Gly Gly Asn Lys Trp Met Tyr Val Gly Arg Asn Lys Ala Gly
        435                 440                 445
Gln Val Trp Arg Asp Ile Thr Gly Asn Arg Ser Gly Thr Val Thr Ile
    450                 455                 460
Asn Ala Asp Gly Trp Gly Asn Phe Ser Val Asn Gly Gly Ser Val Ser
465                 470                 475                 480
Ile Trp Val Asn Asn
                485

<210> 4
<211> 483
<212> PRT
<213> Artificial Sequence

<220>
<223> Artificial Sequence

<400> 4
His His Asn Gly Thr Asn Gly Thr Met Met Gln Tyr Phe Glu Trp Tyr
1               5                   10                  15
Leu Pro Asn Asp Gly Asn His Trp Asn Arg Leu Arg Ser Asp Ala Ser
            20                  25                  30
Asn Leu Lys Asp Lys Gly Ile Thr Ala Val Trp Ile Pro Pro Ala Trp
        35                  40                  45
Lys Gly Ala Ser Gln Asn Asp Val Gly Tyr Gly Ala Tyr Asp Leu Tyr
    50                  55                  60
Asp Leu Gly Glu Phe Asn Gln Lys Gly Thr Val Arg Thr Lys Tyr Gly
65                  70                  75                  80
Thr Arg Asn Gln Leu Gln Ala Ala Val Thr Ala Leu Lys Ser Asn Gly
                85                  90                  95
Ile Gln Val Tyr Gly Asp Val Val Met Asn His Lys Gly Gly Ala Asp
            100                 105                 110
Ala Thr Glu Trp Val Arg Ala Val Glu Val Asn Pro Ser Asn Arg Asn
        115                 120                 125
Gln Glu Val Ser Gly Asp Tyr Thr Ile Glu Ala Trp Thr Lys Phe Asp
    130                 135                 140
Phe Pro Gly Arg Gly Asn Thr His Ser Asn Phe Lys Trp Arg Trp Tyr
145                 150                 155                 160
His Phe Asp Gly Val Asp Trp Asp Gln Ser Arg Gln Leu Gln Asn Arg
                165                 170                 175

Ile Tyr Lys Phe Arg Gly Lys Gly Trp Asp Trp Glu Val Asp Thr Glu
            180                 185                 190
Asn Gly Asn Tyr Asp Tyr Leu Met Tyr Ala Asp Ile Asp Met Asp His
        195                 200                 205
Pro Glu Val Val Asn Glu Leu Arg Asn Trp Gly Val Trp Tyr Thr Asn
    210                 215                 220
Thr Leu Gly Leu Asp Gly Phe Arg Ile Asp Ala Val Lys His Ile Lys
225                 230                 235                 240
Tyr Ser Phe Thr Arg Asp Trp Leu Thr His Val Arg Asn Thr Thr Gly
                245                 250                 255
Lys Asn Met Phe Ala Val Ala Glu Phe Trp Lys Asn Asp Ile Gly Ala
            260                 265                 270
Ile Glu Asn Tyr Leu Ser Lys Thr Asn Trp Asn His Ser Val Phe Asp
        275                 280                 285
Val Pro Leu His Tyr Asn Leu Tyr Asn Ala Ser Arg Ser Gly Gly Asn
    290                 295                 300
Tyr Asp Met Arg Gln Ile Phe Asn Gly Thr Val Val Gln Arg His Pro
305                 310                 315                 320
Thr His Ala Val Thr Phe Val Asp Asn His Asp Ser Gln Pro Glu Glu
                325                 330                 335
Ala Leu Glu Ser Phe Val Glu Glu Trp Phe Lys Pro Leu Ala Tyr Ala
            340                 345                 350
Leu Thr Leu Thr Arg Asp Gln Gly Tyr Pro Ser Val Phe Tyr Gly Asp
        355                 360                 365
Tyr Tyr Gly Ile Pro Thr His Gly Val Pro Ala Met Lys Ser Lys Ile
    370                 375                 380
Asp Pro Ile Leu Glu Ala Arg Gln Lys Tyr Ala Tyr Gly Lys Gln Asn
385                 390                 395                 400
Asp Tyr Leu Asp His His Asn Met Ile Gly Trp Thr Arg Glu Gly Asn
                405                 410                 415
Thr Ala His Pro Asn Ser Gly Leu Ala Thr Ile Met Ser Asp Gly Pro
            420                 425                 430
Gly Gly Asn Lys Trp Met Tyr Val Gly Arg Asn Lys Ala Gly Gln Val
        435                 440                 445
Trp Arg Asp Ile Thr Gly Asn Arg Ser Gly Thr Val Thr Ile Asn Ala
    450                 455                 460
Asp Gly Trp Gly Asn Phe Ser Val Asn Gly Gly Ser Val Ser Ile Trp
465                 470                 475                 480
Val Asn Asn

<210> 5
<211> 486
<212> PRT
<213> Artificial Sequence

<220>
<223> Artificial Sequence

<400> 5
Ala Thr Ile Asn Asn Gly Thr Leu Met Gln Tyr Phe Glu Trp Tyr Ala
1               5                   10                  15
Pro Asn Asp Gly Asn His Trp Lys Arg Leu His Thr Asp Ala Gly Asn
            20                  25                  30
Leu Ala Gln Lys Gly Ile Thr Ser Val Trp Ile Pro Pro Ala Tyr Lys
        35                  40                  45
Gly Thr Thr Gln Asn Asp Val Gly Tyr Gly Ala Tyr Asp Leu Tyr Asp
    50                  55                  60
Leu Gly Glu Phe Asn Gln Lys Gly Thr Val Arg Thr Lys Tyr Gly Thr
65                  70                  75                  80
Lys Ala Gln Leu Lys Ser Ala Ile Glu Ala Leu His Lys Gln Asn Ile
                85                  90                  95

Asn Val Tyr Gly Asp Val Val Met Asn His Lys Gly Gly Ala Asp Tyr
            100                 105                 110
Thr Glu Thr Val Thr Ala Val Glu Val Asp Arg Asn Asn Arg Asn Ile
        115                 120                 125
Glu Val Ser Gly Asp Tyr Glu Ile Asn Ala Trp Thr Gly Phe Asn Phe
    130                 135                 140
Pro Gly Arg Gly Asp Thr His Ser Asn Phe Lys Trp Lys Trp Tyr His
145                 150                 155                 160
Phe Asp Gly Thr Asp Trp Asp Glu Gly Arg Lys Leu Asn Arg Ile Tyr
                165                 170                 175
Lys Phe Arg Gly Ile Gly Lys Ala Trp Asp Trp Glu Val Ser Ser Glu
            180                 185                 190
Asn Gly Asn Tyr Asp Tyr Leu Met Tyr Ala Asp Leu Asp Phe Asp His
        195                 200                 205
Pro Asp Val Ala Asn Glu Met Lys Asn Trp Gly Thr Trp Tyr Ala Asn
    210                 215                 220
Glu Leu Asn Leu Asp Gly Phe Arg Leu Asp Ala Val Lys His Ile Asp
225                 230                 235                 240
His Glu Tyr Leu Arg Asp Trp Val Asn His Val Arg Gln Gln Thr Gly
                245                 250                 255
Lys Glu Met Phe Thr Val Ala Glu Tyr Trp Gln Asn Asp Ile Gln Thr
            260                 265                 270
Leu Asn Asn Tyr Leu Ala Lys Val Asn Tyr Asn Gln Ser Val Phe Asp
        275                 280                 285
Ala Pro Leu His Tyr Asn Phe His Tyr Ala Ser Lys Gly Asn Gly Asn
    290                 295                 300
Tyr Asp Met Arg Asn Ile Leu Asn Gly Thr Val Met Gln Asn His Pro
305                 310                 315                 320
Ala Leu Ala Val Thr Leu Val Glu Asn His Asp Ser Gln Pro Gly Gln
                325                 330                 335
Ser Leu Glu Ser Val Val Ser Pro Trp Phe Lys Pro Leu Ala Tyr Ala
            340                 345                 350
Phe Ile Leu Thr Arg Ala Glu Gly Tyr Pro Ser Val Phe Tyr Gly Asp
        355                 360                 365
Tyr Tyr Gly Thr Ser Gly Asn Ser Ser Tyr Glu Ile Pro Ala Leu Lys
    370                 375                 380
Asp Lys Ile Asp Pro Ile Leu Met Ala Arg Lys Asn Phe Ala Tyr Gly
385                 390                 395                 400
Thr Gln Arg Asp Tyr Leu Asp His Pro Asp Val Ile Gly Trp Thr Arg
                405                 410                 415
Glu Gly Asp Gly Val His Ala Asp Ser Gly Leu Ala Thr Leu Ile Ser
            420                 425                 430
Asp Gly Pro Gly Gly Ser Lys Trp Met Glu Val Gly Lys Asn Asn Ala
        435                 440                 445
Gly Glu Val Trp Tyr Asp Met Thr Gly Asn Gln Thr Asn Thr Val Thr
    450                 455                 460
Ile Asn Lys Asp Gly Trp Gly Gln Phe His Val Ser Gly Gly Ser Val
465                 470                 475                 480
Ser Ile Tyr Val Gln Gln
                485

<210> 6
<211> 399
<212> PRT
<213> Artificial Sequence

<220>
<223> Artificial Sequence

<400> 6
His His Asn Gly Thr Asn Gly Thr Met Met Gln Tyr Phe Glu Trp Tyr
1               5                   10                  15

Leu Pro Asn Asp Gly Asn His Trp Asn Arg Leu Arg Ser Asp Ala Ser
                20                  25                  30
Asn Leu Lys Asp Lys Gly Ile Thr Ala Val Trp Ile Pro Pro Ala Trp
            35                  40                  45
Lys Gly Ala Ser Gln Asn Asp Val Gly Tyr Gly Ala Tyr Asp Leu Tyr
    50                  55                  60
Asp Leu Gly Glu Phe Asn Gln Lys Gly Thr Val Arg Thr Lys Tyr Gly
65                  70                  75                  80
Thr Arg Asn Gln Leu Gln Ala Ala Val Thr Ala Leu Lys Ser Asn Gly
                85                  90                  95
Ile Gln Val Tyr Gly Asp Val Val Met Asn His Lys Gly Gly Ala Asp
            100                 105                 110
Ala Thr Glu Trp Val Arg Ala Val Glu Val Asn Pro Ser Asn Arg Asn
        115                 120                 125
Gln Glu Val Ser Gly Asp Tyr Thr Ile Glu Ala Trp Thr Lys Phe Asp
    130                 135                 140
Phe Pro Gly Arg Gly Asn Thr His Ser Asn Phe Lys Trp Arg Trp Tyr
145                 150                 155                 160
His Phe Asp Gly Val Asp Trp Asp Gln Ser Arg Gln Leu Gln Asn Arg
                165                 170                 175
Ile Tyr Lys Phe Arg Gly Asp Gly Lys Gly Trp Asp Trp Glu Val Asp
            180                 185                 190
Thr Glu Asn Gly Asn Tyr Asp Tyr Leu Met Tyr Ala Asp Ile Asp Met
        195                 200                 205
Asp His Pro Glu Val Val Asn Glu Leu Arg Asn Trp Gly Val Trp Tyr
    210                 215                 220
Thr Asn Thr Leu Gly Leu Asp Gly Phe Arg Ile Asp Ala Val Lys His
225                 230                 235                 240
Ile Lys Tyr Ser Phe Thr Arg Asp Trp Leu Thr His Val Arg Asn Thr
                245                 250                 255
Thr Gly Lys Asn Met Phe Ala Val Ala Glu Phe Trp Lys Asn Asp Ile
            260                 265                 270
Gly Ala Ile Glu Asn Tyr Leu Ser Lys Thr Asn Trp Asn His Ser Val
        275                 280                 285
Phe Asp Val Pro Leu His Tyr Asn Leu Tyr Asn Ala Ser Arg Ser Gly
    290                 295                 300
Gly Asn Tyr Asp Met Arg Gln Ile Phe Asn Gly Thr Val Val Gln Arg
305                 310                 315                 320
His Pro Thr His Ala Val Thr Phe Val Asp Asn His Asp Ser Gln Pro
                325                 330                 335
Glu Glu Ala Leu Glu Ser Phe Val Glu Glu Trp Phe Lys Pro Leu Ala
            340                 345                 350
Tyr Ala Leu Thr Leu Thr Arg Asp Gln Gly Tyr Pro Ser Val Phe Tyr
        355                 360                 365
Gly Asp Tyr Tyr Gly Ile Pro Thr His Gly Val Pro Ala Met Lys Ser
    370                 375                 380
Lys Ile Asp Pro Ile Leu Glu Ala Arg Gln Lys Tyr Ala Tyr Gly
385                 390                 395

<210> 7
<211> 86
<212> PRT
<213> Artificial Sequence

<220>
<223> Artificial Sequence

<400> 7
Lys Gln Asn Asp Tyr Leu Asp His His Asn Met Ile Gly Trp Thr Arg
1               5                   10                  15
Glu Gly Asn Thr Ala His Pro Asn Ser Gly Leu Ala Thr Ile Met Ser
                20                  25                  30

Asp Gly Pro Gly Gly Asn Lys Trp Met Tyr Val Gly Arg Asn Lys Ala
            35                  40                  45
Gly Gln Val Trp Arg Asp Ile Thr Gly Asn Arg Ser Gly Thr Val Thr
    50                  55                  60
Ile Asn Ala Asp Gly Trp Gly Asn Phe Ser Val Asn Gly Gly Ser Val
65                  70                  75                  80
Ser Ile Trp Val Asn Asn
                85

<210> 8
<211> 86
<212> PRT
<213> Artificial Sequence

<220>
<223> Artificial Sequence

<400> 8
Thr Gln Arg Asp Tyr Leu Asp His Pro Asp Val Ile Gly Trp Thr Arg
1               5                   10                  15
Glu Gly Asp Gly Val His Ala Asp Ser Gly Leu Ala Thr Leu Ile Ser
            20                  25                  30
Asp Gly Pro Gly Gly Ser Lys Trp Met Glu Val Gly Lys Asn Asn Ala
            35                  40                  45
Gly Glu Val Trp Tyr Asp Met Thr Gly Asn Gln Thr Asn Thr Val Thr
    50                  55                  60
Ile Asn Lys Asp Gly Trp Gly Gln Phe His Val Ser Gly Gly Ser Val
65                  70                  75                  80
Ser Ile Tyr Val Gln Gln
                85

<210> 9
<211> 400
<212> PRT
<213> Artificial Sequence

<220>
<223> Artificial Sequence

<400> 9
Ala Thr Ile Asn Asn Gly Thr Leu Met Gln Tyr Phe Glu Trp Tyr Ala
1               5                   10                  15
Pro Asn Asp Gly Asn His Trp Lys Arg Leu His Thr Asp Ala Gly Asn
            20                  25                  30
Leu Ala Gln Lys Gly Ile Thr Ser Val Trp Ile Pro Pro Ala Tyr Lys
            35                  40                  45
Gly Thr Thr Gln Asn Asp Val Gly Tyr Gly Ala Tyr Asp Leu Tyr Asp
    50                  55                  60
Leu Gly Glu Phe Asn Gln Lys Gly Thr Val Arg Thr Lys Tyr Gly Thr
65                  70                  75                  80
Lys Ala Gln Leu Lys Ser Ala Ile Glu Ala Leu His Lys Gln Asn Ile
                85                  90                  95
Asn Val Tyr Gly Asp Val Val Met Asn His Lys Gly Gly Ala Asp Tyr
            100                 105                 110
Thr Glu Thr Val Thr Ala Val Glu Val Asp Arg Asn Asn Arg Asn Ile
        115                 120                 125
Glu Val Ser Gly Asp Tyr Glu Ile Asn Ala Trp Thr Gly Phe Asn Phe
    130                 135                 140
Pro Gly Arg Gly Asp Thr His Ser Asn Phe Lys Trp Lys Trp Tyr His
145                 150                 155                 160
Phe Asp Gly Thr Asp Trp Asp Glu Gly Arg Lys Leu Asn Arg Ile Tyr

165                 170                 175
Lys Phe Arg Gly Ile Gly Lys Ala Trp Asp Trp Glu Val Ser Ser Glu
            180                 185                 190
Asn Gly Asn Tyr Asp Tyr Leu Met Tyr Ala Asp Leu Asp Phe Asp His
        195                 200                 205
Pro Asp Val Ala Asn Glu Met Lys Asn Trp Gly Thr Trp Tyr Ala Asn
    210                 215                 220
Glu Leu Asn Leu Asp Gly Phe Arg Leu Asp Ala Val Lys His Ile Asp
225                 230                 235                 240
His Glu Tyr Leu Arg Asp Trp Val Asn His Val Arg Gln Gln Thr Gly
                245                 250                 255
Lys Glu Met Phe Thr Val Ala Glu Tyr Trp Gln Asn Asp Ile Gln Thr
            260                 265                 270
Leu Asn Asn Tyr Leu Ala Lys Val Asn Tyr Asn Gln Ser Val Phe Asp
        275                 280                 285
Ala Pro Leu His Tyr Asn Phe His Tyr Ala Ser Lys Gly Asn Gly Asn
    290                 295                 300
Tyr Asp Met Arg Asn Ile Leu Asn Gly Thr Val Met Gln Asn His Pro
305                 310                 315                 320
Ala Leu Ala Val Thr Leu Val Glu Asn His Asp Ser Gln Pro Gly Gln
                325                 330                 335
Ser Leu Glu Ser Val Val Ser Pro Trp Phe Lys Pro Leu Ala Tyr Ala
            340                 345                 350
Phe Ile Leu Thr Arg Ala Glu Gly Tyr Pro Ser Val Phe Tyr Gly Asp
        355                 360                 365
Tyr Tyr Gly Thr Ser Gly Asn Ser Ser Tyr Glu Ile Pro Ala Leu Lys
    370                 375                 380
Asp Lys Ile Asp Pro Ile Leu Met Ala Arg Lys Asn Phe Ala Tyr Gly
385                 390                 395                 400

<210> 10
<211> 269
<212> PRT
<213> Bacillus lentus

<400> 10
Ala Gln Ser Val Pro Trp Gly Ile Ser Arg Val Gln Ala Pro Ala Ala
1               5                   10                  15
His Asn Arg Gly Leu Thr Gly Ser Gly Val Lys Val Ala Val Leu Asp
            20                  25                  30
Thr Gly Ile Ser Thr His Pro Asp Leu Asn Ile Arg Gly Gly Ala Ser
        35                  40                  45
Phe Val Pro Gly Glu Pro Ser Thr Gln Asp Gly Asn Gly His Gly Thr
    50                  55                  60
His Val Ala Gly Thr Ile Ala Ala Leu Asn Asn Ser Ile Gly Val Leu
65                  70                  75                  80
Gly Val Ala Pro Ser Ala Glu Leu Tyr Ala Val Lys Val Leu Gly Ala
                85                  90                  95
Asp Gly Arg Gly Ala Ile Ser Ser Ile Ala Gln Gly Leu Glu Trp Ala
            100                 105                 110
Gly Asn Asn Gly Met His Val Ala Asn Leu Ser Leu Gly Ser Pro Ser
        115                 120                 125
Pro Ser Ala Thr Leu Glu Gln Ala Val Asn Ser Ala Thr Ser Arg Gly
    130                 135                 140
Val Leu Val Val Ala Ala Ser Gly Asn Ser Gly Ala Ser Ser Ile Ser
145                 150                 155                 160
Tyr Pro Ala Arg Tyr Ala Asn Ala Met Ala Val Gly Ala Thr Asp Gln
                165                 170                 175
Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205
Ala Ser Leu Asn Gly Thr Ser Met Ala Thr Pro His Val Ala Gly Ala
    210                 215                 220
Ala Ala Leu Val Lys Gln Lys Asn Pro Ser Trp Ser Asn Val Gln Ile
225                 230                 235                 240
Arg Asn His Leu Lys Asn Thr Ala Thr Ser Leu Gly Ser Thr Asn Leu
                245                 250                 255
Tyr Gly Ser Gly Leu Val Asn Ala Glu Ala Ala Thr Arg
            260                 265

<210> 11
<211> 275
<212> PRT
<213> Bacillus amyloliquefaciens

<400> 11

<210> 12
<211> 269
<212> PRT
<213> Bacillus gibsonii

<400> 12
Gln Gln Thr Val Pro Trp Gly Ile Thr Arg Val Gln Ala Pro Thr Val

1                   5                   10                  15
His Asn Arg Gly Ile Thr Gly Ser Gly Val Lys Val Ala Ile Leu Asp
                20                  25                  30
Thr Gly Ile Ala Gln His Ser Asp Leu Thr Ile Arg Gly Gly Ala Ser
            35                  40                  45
Phe Val Pro Gly Glu Ser Thr Thr Ala Asp Leu Asn Gly His Gly Thr
    50                  55                  60
His Val Ala Gly Thr Val Ala Ala Leu Asn Asn Ser Ile Gly Val Ile
65                  70                  75                  80
Gly Val Ala Pro Ser Ala Asp Leu Tyr Ala Val Lys Val Leu Gly Ala
                85                  90                  95
Asn Gly Arg Gly Ser Val Ser Gly Ile Ala Gln Gly Leu Glu Trp Ala
                100                 105                 110
Ala Thr Asn Asn Met His Ile Ala Asn Met Ser Leu Gly Ser Asp Ala
            115                 120                 125
Pro Ser Thr Thr Leu Glu Arg Ala Val Asn Tyr Ala Thr Ser Arg Gly
    130                 135                 140
Val Leu Val Ile Ala Ala Thr Gly Asn Asn Gly Thr Gly Ser Ile Gly
145                 150                 155                 160
Tyr Pro Ala Arg Tyr Ala Asn Ala Met Ala Val Gly Ala Thr Asp Gln
                165                 170                 175
Asn Asn Arg Arg Ala Ser Phe Ser Gln Tyr Gly Thr Gly Ile Asp Ile
                180                 185                 190
Val Ala Pro Gly Val Gly Ile Gln Ser Thr Tyr Leu Asn Asn Ser Tyr
            195                 200                 205
Ala Ser Met Pro Gly Thr Ser Met Ala Thr Pro His Val Ala Gly Val
    210                 215                 220
Ala Ala Leu Val Lys Gln Lys Asn Pro Ser Trp Asn Ala Thr Gln Ile
225                 230                 235                 240
Arg Asn His Leu Lys Asn Thr Ala Thr Asn Leu Gly Asn Ser Ser Gln
                245                 250                 255
Phe Gly Ser Gly Leu Val Asn Ala Asp Ala Ala Thr Arg
                260                 265

<210> 13
<211> 275
<212> PRT
<213> Bacillus pumilus

<400> 13
Ala Gln Thr Val Pro Tyr Gly Ile Pro Gln Ile Lys Ala Pro Ala Val
1                   5                   10                  15
His Ala Gln Gly Tyr Lys Gly Ala Asn Val Lys Val Ala Val Leu Asp
                20                  25                  30
Thr Gly Ile His Ala Ala His Pro Asp Leu Asn Val Ala Gly Gly Ala
            35                  40                  45
Ser Phe Val Pro Ser Glu Pro Asn Ala Thr Gln Asp Phe Gln Ser His
    50                  55                  60
Gly Thr His Val Ala Gly Thr Ile Ala Ala Leu Asp Asn Thr Ile Gly
65                  70                  75                  80
Val Leu Gly Val Ala Pro Ser Ala Ser Leu Tyr Ala Val Lys Val Leu
                85                  90                  95
Asp Arg Asn Gly Asp Gly Gln Tyr Ser Trp Ile Ile Ser Gly Ile Glu
                100                 105                 110
Trp Ala Val Ala Asn Asn Met Asp Val Ile Asn Met Ser Leu Gly Gly
            115                 120                 125
Pro Asn Gly Ser Thr Ala Leu Lys Asn Ala Val Asp Thr Ala Asn Asn
    130                 135                 140
Arg Gly Val Val Val Val Ala Ala Ala Gly Asn Ser Gly Ser Phe Gly
145                 150                 155                 160
Ser Thr Ser Thr Val Gly Tyr Pro Ala Lys Tyr Asp Ser Thr Ile Ala
                165                 170                 175

Val Ala Asn Val Asn Ser Asn Asn Val Arg Asn Ser Ser Ser Ser Ala
                180                 185                 190
Gly Pro Glu Leu Asp Val Ser Ala Pro Gly Thr Ser Ile Leu Ser Thr
            195                 200                 205
Val Pro Ser Ser Gly Tyr Thr Ser Tyr Thr Gly Thr Ser Met Ala Ser
    210                 215                 220
Pro His Val Ala Gly Ala Ala Ala Leu Ile Leu Ser Lys Tyr Pro Asn
225                 230                 235                 240
Leu Ser Thr Ser Gln Val Arg Gln Arg Leu Glu Asn Thr Ala Thr Pro
                245                 250                 255
Leu Gly Asp Ser Phe Tyr Tyr Gly Lys Gly Leu Ile Asn Val Gln Ala
                260                 265                 270
Ala Ser Asn
            275

<210> 14
<211> 311
<212> PRT
<213> Artificial sequence

<220>
<223> Artificial sequence

<400> 14

## Description

### Field of the invention

In the present invention new amylase enzymes are provided. More specifically, genetically engineered amylase enzymes, compositions comprising the enzymes, and methods of making and using the enzymes or compositions comprising the enzymes are provided.

### Background of the invention

Enzymes are increasingly used in various application as sustainable alternative to petrochemistry. Enzymes are biodegradable and can be catalytically active already at lower temperatures, which results in reduction of energy consumption. In particular, in the detergent industry enzymes are implemented in washing formulations to improve cleaning efficiency and reducing energy consumption in a washing step.

Amylases are enzymes capable of hydrolyzing starch. Thus, amylases have been employed in the removal of starch stains and have been added to cleaning compositions for this purpose. In these detergent applications the amylases shall be stable at elevated temperatures and/or within denaturing conditions of the detergents and wash liquor. Thus, the need exists for new amylase enzymes with improved properties, in particular, with improved stability and improved performance.

### Brief summary of the invention

The present invention is directed to an alpha-amylase variant of a parent alpha-amylase, wherein said variant comprises
(i) amino acid alternation, preferably insertion, deletion, substitution, or combinations thereof, preferably substitution, at one or more positions corresponding to positions selected from the group consisting of 1, 2, 3, 4, 5, 6, 7, 8, 10, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 45, 47, 48, 51, 54, 59, 60, 63, 70, 71, 72, 73, 75, 76, 81, 82, 83, 86, 87, 89, 90, 91, 92, 93, 94, 95, 96, 97, 98, 99, 100, 103, 106, 108, 109, 113, 114, 115, 116, 117, 119, 120, 123, 125, 126, 128, 129, 131, 132, 133, 134, 135, 136, 139, 141, 142, 143, 144, 145, 146, 147, 149, 150, 151, 154, 155, 156, 158, 160, 161, 162, 163, 164, 165, 169, 170, 172, 173, 174, 175, 176, 177, 178, 180, 182, 184, 185, 187, 188, 189, 191, 192, 193, 203, 208, 210, 211, 212, 213, 214, 215, 216, 218, 219, 221, 222, 223, 224, 225, 226, 227, 230, 231, 233, 234, 235, 236, 237, 238, 240, 242, 243, 245, 249, 250, 251, 252, 253, 255, 256, 257, 258, 259, 260, 261, 262, 263, 264, 265, 268, 269, 270, 271, 272, 273, 274, 275, 276, 277, 279, 280, 281, 282, 284, 285, 286, 287, 288, 289, 290, 291, 292, 293, 294, 295, 296, 297, 298, 299, 301, 302, 303, 304, 306, 307, 308, 309, 310, 311, 312, 313, 314, 315, 317, 318, 319, 320, 321, 322, 323, 324, 326, 327, 333, 334, 336, 337, 338, 341, 342, 343, 344, 345, 346, 348, 350, 351, 352, 353, 354, 355, 356, 357, 358, 359, 360, 362, 363, 364, 365, 366, 367, 368, 370, 372, 375, 376, 378, 379, 381, 382, 383, 384, 385, 387, 388, 389, 390, 391, 392, 393, 394, 395, 396, 397, 398, 399, 400, 401, 403, 405, 406, 407, 408, 410, 413, 414, 415, 416, 418, 421, 424, 426, 427, 429, 431, 432, 434, 436, 438, 439, 440, 442, 443, 445, 446, 447, 448, 449, 451, 453, 455, 456, 457, 458, 459, 460, 461, 462, 463, 465, 467, 468, 470, 471, 474, 478, 480, and 482 according to the numbering of the amino acid sequence set forth in SEQ ID NO: 3,
(ii) said variant has at least 80% but less than 100% sequence identity with the amino acid sequence set forth in SEQ ID NO: 1, and
(iii) said variant has alpha-amylase activity.

Furthermore, the present invention is directed to a polynucleotide encoding said alpha-amylase variant and a nucleic acid construct or an expression vector comprising said polynucleotide as well as a host cell comprising the polynucleotide, the nucleic acid construct, or the expression vector.

Moreover, the present invention is directed to a composition comprising the alpha-amylase variant and at least one additional component, preferably a detergent composition (e.g., laundry detergent composition or an Automatic Dish Wash (ADW) detergent composition) as well as the use of the alpha-amylase variant in such composition.

The present invention is also directed to a method of producing an alpha-amylase variant, comprising cultivating a host cell under conditions suitable for expression of said alpha-amylase variant; and recovering said alpha-amylase variant.

### Detailed description of the invention

The present invention may be understood more readily by reference to the following detailed description of the embodiments of the invention and the examples included herein.

Although the present invention will be described with respect to particular embodiments, this description is not to be construed in a limiting sense.

### Definitions

Unless otherwise noted, the terms used herein are to be understood according to conventional usage by those of ordinary skill in the relevant art.

Before describing in detail exemplary embodiments of the present invention, definitions important for understanding the present invention are given. Unless stated otherwise or apparent from the nature of the definition, the definitions apply to all compounds, methods and uses described herein.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %. Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

Throughout this application, various publications are referenced. The disclosures of all of these publications and those references cited within those publications in their entireties are hereby incorporated by reference into this application in order to more fully describe the state of the art to which this invention pertains.

It is to be understood that the term "comprising" is not limiting. For the purposes of the present invention the term "consisting of" is considered to be a preferred embodiment of the term "comprising". If hereinafter a group is defined to comprise at least a certain number of embodiments, this is meant to also encompass a group which preferably consists of these embodiments only. "Parent" sequence (also called "parent enzyme" or "parent protein") is the starting sequences for introduction of changes (e.g. by introducing one or more amino acid substitutions) of the sequence resulting in "variants" of the parent sequences. Thus, the term "enzyme variant" or "sequence variant" or "protein variant" are used in reference to parent enzymes that are the origin for the respective variant enzymes. Therefore, parent enzymes include wild type enzymes and variants of wild-type enzymes which are used for development of further variants. Variant enzymes differ from parent enzymes in their amino acid sequence to a certain extent.

In describing the variants of the present invention, the abbreviations for single amino acids used according to the accepted IUPAC single letter or three letter amino acid abbreviation is used. "Amino acid alteration" as used herein refers to amino acid substitution, deletion, or insertion. "Substitutions" are described by providing the original amino acid followed by the number of the position within the amino acid sequence, followed by the substituted amino acid. For example, the substitution of histidine at position 120 with alanine is designated as "His120Ala" or "H120A". Substitutions can also be described by merely naming the resulting amino acid in the variant without specifying the amino acid of the parent at this position, e.g., "X120A" or "120A" or "Xaa120AIa" or "120Ala".

"Deletions" are described by providing the original amino acid followed by the number of the position within the amino acid sequence, followed by *. Accordingly, the deletion of glycine at position 150 is designated as "Gly150*" or G150*". Alternatively, deletions are indicated by e.g. "deletion of D183 and G184".

"Insertions" are described by providing the original amino acid followed by the number of the position within the amino acid sequence, followed by the original amino acid and the additional amino acid. For example, an insertion at position 180 of lysine next to glycine is designated as "Gly180GlyLys" or "G180GK". When more than one amino acid residue is inserted, such as e.g. a Lys and Ala after Gly180 this may be indicated as: Gly180GlyLysAla or G195GKA.

In cases where a substitution and an insertion occur at the same position, this may be indicated as S99SD+S99A or in short S99AD. In cases where an amino acid residue identical to the existing amino acid residue is inserted, it is clear that degeneracy in the nomenclature arises. If for example a glycine is inserted after the glycine in the above example this would be indicated by G180GG. Variants comprising multiple alterations are separated by "+", e.g., "Arg170Tyr+Gly195Glu" or "R170Y+G195E" representing a substitution of arginine and glycine at positions 170 and 195 with tyrosine and glutamic acid, respectively. Alternatively, multiple alterations may be separated by space or a comma, e.g., R170Y G195E or R170Y, G195E respectively. Where different alternative alterations can be introduced at a position, the different alterations are separated by a comma, e.g., "Arg170Tyr, Glu" and R170T, E, respectively, represents a substitution of arginine at position 170 with tyrosine or glutamic acid. Alternative substitutions at a particular position can also be indicated as X120A,G,H, 120A,G,H, X120A/G/H, or 120A/G/H. Alternatively, different alterations or optional substitutions may be indicated in brackets, e.g., Arg170[Tyr, Gly] or Arg170{Tyr, Gly} or in short R170 [Y, G] or R170 {Y, G}.

A "synthetic" or "artificial" compound is produced by *in vitro* chemical and/or enzymatic synthesis. The term "native" (or naturally-occurring or wildtype or endogenous) cell or organism or polynucleotide or polypeptide refers to the cell or organism or polynucleotide or polypeptide as found in nature (i.e., without there being any human intervention). For the purposes of the invention, "recombinant" (or transgenic) with regard to a cell or an organism means that the cell or organism contains a heterologous polynucleotide which is introduced by man by gene technology and with regard to a polynucleotide includes all those constructions brought about by man by gene technology / recombinant DNA techniques in which either
(a) the sequence of the polynucleotide or a part thereof, or
(b) one or more genetic control sequences which are operably linked with the polynucleotide, including but not limited thereto a promoter, or
(c) both a) and b)
are not located in their wildtype genetic environment or have been modified by man.

The term "heterologous" (or exogenous or foreign or recombinant or non-native) polypeptide is defined herein as a polypeptide that is not native to the host cell, a polypeptide native to the host cell in which structural modifications, e.g., deletions, substitutions, and/or insertions, have been made by recombinant DNA techniques to alter the native polypeptide, or a polypeptide native to the host cell whose expression is quantitatively altered or whose expression is directed from a genomic location different from the native host cell as a result of manipulation of the DNA of the host cell by recombinant DNA techniques, e.g., a stronger promoter. Similarly, the term "heterologous" (or exogenous or foreign or recombinant or non-native) polynucleotide refers to a polynucleotide that is not native to the host cell, a polynucleotide native to the host cell in which structural modifications, e.g., deletions, substitutions, and/or insertions, have been made by recombinant DNA techniques to alter the native polynucleotide, or a polynucleotide native to the host cell whose expression is quantitatively altered as a result of manipulation of the regulatory elements of the polynucleotide by recombinant DNA techniques, e.g., a stronger promoter, or a polynucleotide native to the host cell, but integrated not within its natural genetic environment as a result of genetic manipulation by recombinant DNA techniques. With respect to two or more polynucleotide sequences or two or more amino acid sequences, the term "heterologous" is used to characterize that the two or more polynucleotide sequences or two or more amino acid sequences are naturally not occurring in the specific combination with each other.

Variant polynucleotide and variant polypeptide sequences may be defined by their sequence identity when compared to a parent sequence. Sequence identity usually is provided as "% sequence identity" or "% identity". For calculation of sequence identities, in a first step a sequence alignment is produced. According to this invention, a pairwise global alignment is produced, meaning that two sequences are aligned over their complete length, which is usually produced by using a mathematical approach, called alignment algorithm.

According to the invention, the alignment is generated by using the algorithm of Needleman and Wunsch (J. Mol. Biol. (1979) 48, p. 443-453). Preferably, the program "NEEDLE" (The European Molecular Biology Open Software Suite (EMBOSS)) is used for the purposes of the current invention, with using the programs default parameter (polynucleotides: gap open=10.0, gap extend=0.5 and matrix=EDNAFULL; polypeptides: gap open=10.0, gap extend=0.5 and matrix=EBLOSUM62). After aligning two sequences, in a second step, an identity value is determined from the alignment produced. For this purpose, the %-identity is calculated by dividing the number of identical residues by the length of the alignment region which is showing the respective sequence of the present invention over its complete length multiplied with 100: %-identity = (identical residues / length of the alignment region which is showing the respective sequence of the present invention over its complete length) *100.

For calculating the percent identity of two nucleic acid sequences the same applies as for the calculation of percent identity of two amino acid sequences with some specifications. For nucleic acid sequences encoding for a protein the pairwise alignment shall be made over the complete length of the coding region of the sequence of this invention from start to stop codon excluding introns. Introns present in the other sequence, to which the sequence of this invention is compared, may also be removed for the pairwise alignment. Percent identity is then calculated by %-identity = (identical residues / length of the alignment region which is showing the sequence of the invention from start to stop codon excluding introns over their complete length) *100. After aligning two sequences, in a second step, an identity value is determined from the alignment produced.

Moreover, the preferred alignment program for nucleic acid sequences implementing the Needleman and Wunsch algorithm (J. Mol. Biol. (1979) 48, p. 443-453) is "NEEDLE" (The European Molecular Biology Open Software Suite (EMBOSS)) with the programs default parameters (gapopen=10.0, gapextend=0.5 and matrix=EDNAFULL).

Sequences, having identical or similar regions with a sequence of this invention, and which shall be compared with a sequence of this invention to determine % identity, can easily be identified by various ways that are within the skill in the art, for instance, using publicly available computer methods and programs such as BLAST, BLAST-2, available for example at NCBI.

Variant polypeptides may be defined by their sequence similarity when compared to a parent sequence. Sequence similarity usually is provided as "% sequence similarity" or "%-similarity". % sequence similarity takes into account that defined sets of amino acids share similar properties, e.g. by their size, by their hydrophobicity, by their charge, or by other characteristics. Herein, the exchange of one amino acid with a similar amino acid may be called "conservative mutation". Similar amino acids according to the invention are defined as follows, which shall also apply for determination of %-similarity according to this invention, which is also in accordance with the BLOSUM62 matrix as for example used by program "NEEDLE", which is one of the most used amino acids similarity matrix for database searching and sequence alignments:
Amino acid A is similar to amino acids S
Amino acid D is similar to amino acids E; N
Amino acid E is similar to amino acids D; K; Q
Amino acid F is similar to amino acids W; Y
Amino acid H is similar to amino acids N; Y
Amino acid I is similar to amino acids L; M; V
Amino acid K is similar to amino acids E; Q; R
Amino acid L is similar to amino acids I; M; V
Amino acid M is similar to amino acids I; L; V
Amino acid N is similar to amino acids D; H; S
Amino acid Q is similar to amino acids E; K; R
Amino acid R is similar to amino acids K; Q
Amino acid S is similar to amino acids A; N; T
Amino acid T is similar to amino acids S
Amino acid V is similar to amino acids I; L; M
Amino acid W is similar to amino acids F; Y
Amino acid Y is similar to amino acids F; H; W

Conservative amino acid substitutions may occur over the full length of the sequence of a polypeptide sequence of a functional protein such as an enzyme. In one embodiment, such mutations are not pertaining the functional domains of an enzyme. In one embodiment, conservative mutations are not pertaining the catalytic centers of an enzyme. For calculation of sequence similarity, in a first step a sequence alignment is produced as described above. After aligning two sequences, in a second step, a similarity value is determined from the alignment produced. For this purpose, the %-similarity is calculated by dividing the number of identical residues plus the number of similar residues by the length of the alignment region which is showing the sequence of the invention over its complete length multiplied with 100: %-similarity = [(identical residues + similar residues) / length of the alignment region which is showing the sequence of the invention over its complete length] *100.

For nucleic acids, similar sequences can also be determined by hybridization using respective stringency conditions. The term "high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 65°C. The term "very high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 70°C.

The term "A and B domain" of an amylase as used herein means these two domains taken as one unit, whereas the C domain is another unit of the alpha-amylases. Thus, the amino acid sequence of the "A and B domain" is understood as one consecutive sequence or one part of a sequence of an alpha-amylase comprising an "A and B domain" and other, additional domains (such as the C domain).

A "Fragment" or "subsequence" as used herein are a portion of a polynucleotide or an amino acid sequence. The term "functional fragment" refers to any nucleic acid or amino acid sequence which comprises merely a part of the full-length amino acid sequence, respectively, but still has the same or similar activity and/or function. Preferably, the functional fragment is at least 75% identical, at least 76% identical, at least 77% identical, at least 78% identical, at least 79% identical, at least 80% identical, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 98.5 %, at least 99%, or at least 99.5% identical to the original full length amino acid sequence. The functional fragment comprises contiguous nucleic acids or amino acids compared to the original nucleic acid or original amino acid sequence, respectively.

"Genetic construct" or "expression cassette" as used herein, is a nucleic acid molecule composed of at least one sequence of interest to be expressed, operably linked to one or more control sequences (at least to a promoter) as described herein.

The term "vector" as used herein comprises any kind of construct suitable to carry foreign polynucleotide sequences for transfer to another cell, or for stable or transient expression within a given cell. The term "vector" as used herein encompasses any kind of cloning vehicles, such as but not limited to plasmids, phagemids, viral vectors (e.g., phages), bacteriophage, baculoviruses, cosmids, fosmids, artificial chromosomes, or and any other vectors specific for specific hosts of interest. Foreign polynucleotide sequences usually comprise a coding sequence which may be referred to herein as "gene of interest". The gene of interest may comprise introns and exons, depending on the kind of origin or destination of host cell.

The term "introduction" or "transformation" as referred to herein encompasses the transfer of an exogenous polynucleotide into a host cell, irrespective of the method used for transfer. That is, the term "transformation" as used herein is independent from vector, shuttle system, or host cell, and it not only relates to the polynucleotide transfer method of transformation as known in the art (cf., for example, Sambrook, J. et al. (1989) Molecular Cloning: A Laboratory Manual, 2nd Ed., Cold Spring Harbor Laboratory Press, Cold Spring Harbor, NY), but it encompasses any further kind polynucleotide transfer methods such as, but not limited to, transduction or transfection.

A polynucleotide encoding a polypeptide may be "expressed". The term "expression" or "gene expression" means the transcription of a specific gene or specific genes or specific nucleic acid construct. The term "expression" or "gene expression" means the transcription of a gene or genes or genetic construct into structural RNA (e.g., rRNA, tRNA) or mRNA with or without subsequent translation of the latter into a protein. The process includes transcription of DNA and processing of the resulting mRNA product.

Recombinant cells may exhibit "increased" or "decreased" expression when compared to the respective wild-type cell. The term "increased expression", "enhanced expression" or "overexpression" as used herein means any form of expression that is additional to the original wild-type expression level (which can be absence of expression or immeasurable expression as well). Reference herein to "increased expression", "enhanced expression" or "overexpression" is taken to mean an increase in gene expression and/or, as far as referring to polypeptides, increased polypeptide levels and/or increased polypeptide activity, relative to control organisms. The increase in expression may be in increasing order of preference at least 5%, at least 10%, at least 20%, at least 30%, at least 40%, at least 50%, at least 60%, at least 70%, at least 80%, at least 85%, at least 90%, or 100% or even more compared to that of the control organism.

The term "purification" or "purifying" refers to a process in which at least one component, e.g., a protein of interest, is separated from at least another component, e.g., a particulate matter of a fermentation broth, and transferred into a different compartment or phase, wherein the different compartments or phases do not necessarily need to be separated by a physical barrier. Examples of such different compartments are two compartments separated by a filtration membrane or cloth, i.e., filtrate and retentate; examples of such different phases are pellet and supernatant or cake and filtrate, respectively. The resulting solution after purifying the enzyme of interest from the fermentation broth is called herein "purified enzyme solution".

"Protein formulation" means any non-complex formulation comprising a small number of ingredients, wherein the ingredients serve the purpose of stabilizing the proteins comprised in the protein formulation and/or the stabilization of the protein formulation itself.

"Enzyme properties" include, but are not limited to catalytic activity, substrate/cofactor specificity, product specificity, stability in the course of time, thermostability, pH stability, and chemical stability. "Enzymatic activity" or "catalytic activity" means the catalytic effect exerted by an enzyme, expressed as units per milligram of enzyme (specific activity) or molecules of substrate transformed per minute per molecule of enzyme (molecular activity). Enzymatic activity can be specified by the enzymes actual function, e.g., proteases exerting proteolytic activity by catalyzing hydrolytic cleavage of peptide bonds, lipases exerting lipolytic activity by hydrolytic cleavage of ester bonds, amylases activity involves hydrolysis of glycosidic linkages in polysaccharides, etc.

The term "enzyme stability" according to the current invention relates to the retention of enzymatic activity as a function of time during storage or operation. Retention of enzymatic activity as a function of time during storage is called "storage stability" and is preferred within the context of the invention.

To determine and quantify changes in catalytic activity of enzymes stored or used under certain conditions over time, the "initial enzymatic activity" is measured under defined conditions at time zero (100%) and at a certain point in time later (x%). By comparison of the values measured, a potential loss of enzymatic activity can be determined in its extent. The extent of enzymatic activity loss determines an enzyme's stability or non-stability. "Half-life of enzymatic activity" is a measure for time required for the decaying of enzymatic activity to fall to one half (50%) of its initial value. "Half-life of enzymatic activity" can be expresses with respect to the challenging factor under investigation, e.g., for enzyme thermostability the T₅₀ may indicate the temperature at which 50% residual enzymatic activity is still present after thermal inactivation for a certain time when compared with a reference sample which has not undergone thermal treatment.

A "pH stability" or "pH-dependent activity", refers to the ability of an enzyme to exert enzymatic activity after exposure to certain pH value.

The terms "thermal stability", "thermostability" or "temperature-dependent activity" refer to the ability of an enzyme to exert catalytic activity or wash performance after exposure to elevated temperatures, preferably, at a temperature of 40°C for 14 days, preferably in a detergent composition (preferably, in ES1-C detergent), or at 92°C for at least 10min.

The terms "detergent stability" or "stability under storage in a detergent composition" refer to the ability of an enzyme to exert catalytic activity or wash performance after storage in a detergent composition, preferably, at a temperature of 40°C or 50°C for 14 days in a detergent composition (preferably, in ES1-C detergent).

"Improvement Factor" (IF) is the degree of improvement of an enzyme variant over the respective parent enzyme in a certain property. An improvement of the enzyme variant over the respective parent enzyme is characterized by an Improvement Factor (IF) of >1.0. The improvement factor can alternatively be expressed in percentages, e.g., and IF of 1.1 equals 110%.

As used herein, "wash performance" (also called herein "cleaning performance") of an enzyme refers to the contribution of the enzyme to the cleaning performance of a detergent composition, i.e. the cleaning performance added to the detergent composition by the performance of the enzyme. The term "wash performance" is used herein similarly for laundry and hard surface cleaning. Wash performance is compared under relevant washing conditions. The term "relevant washing conditions" is used herein to indicate the conditions, particularly washing temperature, time, washing mechanics, sud concentration, type of detergent and water hardness, actually used in households in a detergent market segment. The term "improved wash performance" is used to indicate that a better end result is obtained in stain removal under relevant washing conditions, or that less enzyme, on weight basis, is needed to obtain the same end result relative to the corresponding control conditions.

As used herein, the term "specific performance" refers to the cleaning and removal of specific stains or soils per unit of active enzyme. In some embodiments, the specific performance is determined using stains or sails such as egg, egg yolk, milk, grass, minced meat blood, chocolate sauce, baby food, sebum, etc.

"Detergent composition" or "detergent formulation" or "cleaning formulation" or "detergent solution" means compositions designated for cleaning soiled material. Detergent compositions and / or detergent solutions according to the invention include detergent compositions and / or detergent solutions for different applications such as laundry and hard surface cleaning. The term "detergent component" is defined herein to mean the types of chemicals, which can be used in detergent compositions and / or detergent solutions.

Detergent formulations of the invention may comprise one or more surfactant(s). "Surfactant" (synonymously used herein with "surface active agent") means an organic chemical that, when added to a liquid, changes the properties of that liquid at an interface. According to its ionic charge, a surfactant is called non-ionic, anionic, cationic, or amphoteric.

The term "effective amount of a detergent component" includes amounts of certain components to provide effective stain removal and effective cleaning conditions (e.g. pH, quantity of foaming), amounts of certain components to effectively provide optical benefits (e.g. optical brightening, dye transfer inhibition), and amounts of certain components to effectively aid the processing (maintain physical characteristics during processing, storage and use; e.g. rheology modifiers, hydrotropes, desiccants).

The term "laundry" or "laundering" relates to both household laundering and industrial laundering and means the process of treating textiles and/or fabrics with a solution containing a detergent composition of the present invention. The laundering process may be carried out by using technical devices such as a household or an industrial washing machine. Alternatively, the laundering process may be done by hand.

The term "textile" means any textile material including yarns (thread made of natural or synthetic fibers used for knitting or weaving), yarn intermediates, fibers, non-woven materials, natural materials, synthetic materials, as well as fabrics made of these materials such as garments, cloths and other articles. The terms "fabric" (a textile made by weaving, knitting or felting fibers) or "garment" (any article of clothing made of textile) as used herein, mean to include the broader term textile as well.

The term "hard surface cleaning" is defined herein as cleaning of hard surfaces wherein hard surfaces may include any hard surfaces in the household, such as floors, furnishing, walls, sanitary ceramics, glass, metallic surfaces including cutlery or dishes. A particular form of hard surface cleaning is dishwashing, particularly automatic dishwashing (ADW).

The term "dish wash" refers to all forms of washing dishes, e.g. by hand or automatic dish wash. Washing dishes includes, but is not limited to, the cleaning of all forms of crockery such as plates, cups, glasses, bowls, all forms of cutlery such as spoons, knives, forks and serving utensils as well as ceramics, plastics such as melamine, metals, china, glass and acrylics.

Cleaning performance is evaluated under relevant cleaning conditions. The term "relevant cleaning conditions" herein refers to the conditions, particularly cleaning temperature, time, cleaning mechanics, suds concentration, type of detergent and water hardness, actually used in laundry machines, automatic dish washers or in manual cleaning processes.

In the technical field of the present invention, usually the term "stains" is used with reference to laundry, e.g., cleaning for textiles, fabric, or fibers, whereas the term "soils" is usually used with reference to hard surface cleaning, e.g., cleaning of dishes and cutlery. However, herein the terms "stain" and "soil" shall be used interchangeably.

A "sequestering builder" as used herein is different from a precipitating builder in that no significant amount of precipitate is formed when the builder is used in an amount sufficient to combine with all of the calcium ions in an aqueous solution with 7 °dH hardness (German hardness) initially at neutral pH. A "strong builder" is classified as high efficiency chelators that can bind the divalent cations such as Ca2+ strongly with a logarithmic stability constant (Log K_{Ca}) of the cation/chelator complex of above 4, particular above 5, above 6 or above 7. The stability constants are determined at an ionic strength of 0.1 M and at a temperature of 25°C. A "strong sequestering builder" combines both of the above-mentioned properties.

### Detailed description

In the present invention new amylase enzymes are provided. More specifically, variants of a parent alpha-amylase, methods of making the variant alpha-amylases, compositions comprising the alpha-amylase variants, and methods of using the variant alpha-amylases or compositions comprising the variant alpha-amylases are provided.

### Alpha-Amylase Variant

The present invention is directed to an alpha-amylase variant of a parent alpha-amylase, wherein said variant comprises
(i) an amino acid alteration, preferably insertion, deletion, substitution, or combinations thereof, preferably substitution, at one or more positions corresponding to positions selected from the group consisting of 1, 2, 3, 4, 5, 6, 7, 8, 10, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 45, 47, 48, 51, 54, 59, 60, 63, 70, 71, 72, 73, 75, 76, 81, 82, 83, 86, 87, 89, 90, 91, 92, 93, 94, 95, 96, 97, 98, 99, 100, 103, 106, 108, 109, 113, 114, 115, 116, 117, 119, 120, 123, 125, 126, 128, 129, 131, 132, 133, 134, 135, 136, 139, 141, 142, 143, 144, 145, 146, 147, 149, 150, 151, 154, 155, 156, 158, 160, 161, 162, 163, 164, 165, 169, 170, 172, 173, 174, 175, 176, 177, 178, 180, 182, 184, 185, 187, 188, 189, 191, 192, 193, 203, 208, 210, 211, 212, 213, 214, 215, 216, 218, 219, 221, 222, 223, 224, 225, 226, 227, 230, 231, 233, 234, 235, 236, 237, 238, 240, 242, 243, 245, 249, 250, 251, 252, 253, 255, 256, 257, 258, 259, 260, 261, 262, 263, 264, 265, 268, 269, 270, 271, 272, 273, 274, 275, 276, 277, 279, 280, 281, 282, 284, 285, 286, 287, 288, 289, 290, 291, 292, 293, 294, 295, 296, 297, 298, 299, 301, 302, 303, 304, 306, 307, 308, 309, 310, 311, 312, 313, 314, 315, 317, 318, 319, 320, 321, 322, 323, 324, 326, 327, 333, 334, 336, 337, 338, 341, 342, 343, 344, 345, 346, 348, 350, 351, 352, 353, 354, 355, 356, 357, 358, 359, 360, 362, 363, 364, 365, 366, 367, 368, 370, 372, 375, 376, 378, 379, 381, 382, 383, 384, 385, 387, 388, 389, 390, 391, 392, 393, 394, 395, 396, 397, 398, 399, 400, 401, 403, 405, 406, 407, 408, 410, 413, 414, 415, 416, 418, 421, 424, 426, 427, 429, 431, 432, 434, 436, 438, 439, 440, 442, 443, 445, 446, 447, 448, 449, 451, 453, 455, 456, 457, 458, 459, 460, 461, 462, 463, 465, 467, 468, 470, 471, 474, 478, 480, and 482 according to the numbering of the amino acid sequence set forth in SEQ ID NO: 3
(ii) said variant has at least 60% but less than 100% sequence identity with the amino acid sequence set forth in SEQ ID NO: 1, and
(iii) said variant has alpha-amylase activity.
The alpha-amylase variant of the present invention is a non-naturally occurring amylase. Preferably, the alpha-amylase variant of the present invention is an isolated, synthetic, and/or recombinant alpha-amylase variant.
Amylases according to the invention have "amylolytic activity" or "amylase activity". "Amylolytic activity" or "amylase activity" describes the capability for the hydrolysis of glucosidic linkages in polysaccharides. Alpha-amylase activity may be determined by assays for measurement of alpha-amylase activity which are known to those skilled in the art. Examples for assays measuring alpha-amylase activity are the Phadebas assay or the EPS assay ("Infinity reagent"). In the Phadebas assay alpha-amylase activity is determined by employing Phadebas tablets as substrate (Phadebas Amylase Test, supplied by Magle Life Science). Starch is hydrolyzed by the alpha-amylase giving soluble blue fragments. The absorbance of the resulting blue solution, measured spectrophotometrically at 620 nm, is a function of the alpha-amylase activity. The measured absorbance is directly proportional to the specific activity (activity/mg of pure alpha-amylase protein) of the alpha-amylase in question under the given set of conditions.

Alternatively, alpha-amylase activity can also be determined by a method employing the Ethyliden-4-nitrophenyl-alpha-D-maltoheptaosid (EPS). D-maltoheptaoside is a blocked oligosaccharide which can be cleaved by an endo-amylase. Following the cleavage, the alpha-glucosidase included in the kit to digest the substrate to liberate a free PNP molecule which has a yellow color and thus can be measured by visible spectophotometry at 405nm. Kits containing EPS substrate and alpha-glucosidase is manufactured for example by Roche Costum Biotech (cat. No. 10880078103). The slope of the time dependent absorption-curve is directly proportional to the specific activity (activity per mg enzyme) of the alpha-amylase in question under the given set of conditions.
In one embodiment, the alpha-amylase variant of the present invention exhibits at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, at least 160%, at least 170%, at least 180%, at least 190%, or at least 200% of the amylolytic activity of the parent amylase. In one embodiment, the alpha-amylase variant of the present invention exhibits the same or an increased amylolytic activity compared to the parent amylase. Preferably, the alpha-amylase variant of the present invention exhibits an increased amylolytic activity compared to the parent amylase
Preferably, the parent alpha-amylase for the alpha-amylase variant of the present invention is an amylase according to SEQ ID NO: 1 or SEQ ID NO: 3 or any alpha-amylase having at least 60% sequence identity to SEQ IDNO: 1 or SEQ ID NO: 3, most preferably the parent alpha-amylase for the alpha-amylase variant is an amylase according to according to SEQ ID NO: 1.

The present invention is directed to an alpha-amylase variant of a parent alpha-amylase having alpha-amylase activity, wherein said variant comprises compared to the parent alpha-amylase amino acid alteration, preferably insertion, deletion, substitution, or combination thereof, most preferably substitution, at one or more positions corresponding to positions selected from the group consisting of 1, 2, 3, 4, 5, 6, 7, 8, 10, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 45, 47, 48, 51, 54, 59, 60, 63, 70, 71, 72, 73, 75, 76, 81, 82, 83, 86, 87, 89, 90, 91, 92, 93, 94, 95, 96, 97, 98, 99, 100, 103, 106,108,109, 113, 114, 115, 116, 117, 119, 120, 123, 125, 126, 128, 129, 131, 132, 133, 134, 135, 136, 139, 141, 142, 143, 144, 145, 146, 147, 149, 150, 151, 154, 155, 156, 158, 160, 161, 162, 163, 164, 165, 169, 170, 172, 173, 174, 175, 176, 177, 178, 180, 182, 184, 185, 187, 188, 189, 191, 192, 193, 203, 208, 210, 211, 212, 213, 214, 215, 216, 218, 219, 221, 222, 223, 224, 225, 226, 227, 230, 231, 233, 234, 235, 236, 237, 238, 240, 242, 243, 245, 249, 250, 251, 252, 253, 255, 256, 257, 258, 259, 260, 261, 262, 263, 264, 265, 268, 269, 270, 271, 272, 273, 274, 275, 276, 277, 279, 280, 281, 282, 284, 285, 286, 287, 288, 289, 290, 291, 292, 293, 294, 295, 296, 297, 298, 299, 301, 302, 303, 304, 306, 307, 308, 309, 310, 311, 312, 313, 314, 315, 317, 318, 319, 320, 321, 322, 323, 324, 326, 327, 333, 334, 336, 337, 338, 341, 342, 343, 344, 345, 346, 348, 350, 351, 352, 353, 354, 355, 356, 357, 358, 359, 360, 362, 363, 364, 365, 366, 367, 368, 370, 372, 375, 376, 378, 379, 381, 382, 383, 384, 385, 387, 388, 389, 390, 391, 392, 393, 394, 395, 396, 397, 398, 399, 400, 401, 403, 405, 406, 407, 408, 410, 413, 414, 415, 416, 418, 421, 424, 426, 427, 429, 431, 432, 434, 436, 438, 439, 440, 442, 443, 445, 446, 447, 448, 449, 451, 453, 455, 456, 457, 458, 459, 460, 461, 462, 463, 465, 467, 468, 470, 471, 474, 478, 480, and 482 according to the numbering of the amino acid sequence set forth in SEQ ID NO: 3. Preferably, the parent alpha-amylase for the alpha-amylase variant of the present invention is an amylase according to SEQ ID NO: 1 or SEQ ID NO: 3 or any alpha-amylase having at least 60% sequence identity to SEQ IDNO: 1 or SEQ ID NO: 3, most preferably the parent alpha-amylase for the alpha-amylase variant is an amylase according to according to SEQ ID NO: 1.
In a preferred embodiment, the resulting amino acid residue of the amino acid substitution does not equal to an amino acid residue in SEQ ID NO: 3 or 5 at the corresponding position.
Particularly preferred, the alpha-amylase variant having alpha-amylase activity comprises amino acid alterations, preferably insertion, deletion, substitution, or combination thereof, compared to the parent alpha-amylase at one or more amino acid positions selected from the group consisting of 4, 7, 25, 37, 70, 100, 118, 135, 160, 176, 193, 210, 251, 281, 258, 323, 361, 363, 368, 405, 434, 441, 459, 460, 451, and 482 according to the numbering of the amino acid sequence set forth in SEQ ID NO: 3 and wherein said variant has alpha-amylase activity. More particularly preferred, the alpha-amylase variant having alpha-amylase activity comprises amino acid alterations, preferably insertion, deletion, substitution, or combination thereof, compared to the parent alpha-amylase at one or more amino acid positions selected from the group consisting of 25, 100, 135, 176, 193, and 460 according to the numbering of the amino acid sequence set forth in SEQ ID NO: 3 and wherein said variant has alpha-amylase activity.
Preferably, the present invention is directed to an alpha-amylase variant of a parent alpha-amylase having alpha-amylase activity, wherein said variant comprises compared to the parent alpha-amylase amino acid alteration, preferably insertion, deletion, substitution, or combination thereof, most preferably substitution, at one or more positions corresponding to positions selected from the group consisting of 4, 25, 100, 135, 135, 135, 135, 160, 176, 193, 251, 258, 276, 299, 323, 363, 363, 382, 405, 460, and 482 according to the numbering of the amino acid sequence set forth in SEQ ID NO: 3.
Preferably, the present invention is directed to an alpha-amylase variant of a parent alpha-amylase having alpha-amylase activity, wherein said variant comprises compared to the parent alpha-amylase amino acid substitution at one or more positions corresponding to positions selected from the group consisting of 1, 2, 3, 4, 5, 6, 7, 8, 10, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 45, 47, 48, 51, 54, 59, 60, 63, 70, 71, 72, 73, 75, 76, 81, 82, 83, 86, 87, 89, 90, 91, 92, 93, 94, 95, 96, 97, 98, 99, 100, 103, 106, 108, 109, 113, 114, 115, 116, 117, 119, 120, 123, 125, 126, 128, 129, 131, 132, 133, 134, 135, 136, 139, 141, 142, 143, 144, 145, 146, 147, 149, 150, 151, 154, 155, 156, 158, 160, 161, 162, 163, 164, 165, 169, 170, 172, 173, 174, 175, 176, 177, 178, 180, 182, 184, 185, 187, 188, 189, 191, 192, 193, 203, 208, 210, 211, 212, 213, 214, 215, 216, 218, 219, 221, 222, 223, 224, 225, 226, 227, 230, 231, 233, 234, 235, 236, 237, 238, 240, 242, 243, 245, 249, 250, 251, 252, 253, 255, 256, 257, 258, 259, 260, 261, 262, 263, 264, 265, 268, 269, 270, 271, 272, 273, 274, 275, 276, 277, 279, 280, 281, 282, 284, 285, 286, 287, 288, 289, 290, 291, 292, 293, 294, 295, 296, 297, 298, 299, 301, 302, 303, 304, 306, 307, 308, 309, 310, 311, 312, 313, 314, 315, 317, 318, 319, 320, 321, 322, 323, 324, 326, 327, 333, 334, 336, 337, 338, 341, 342, 343, 344, 345, 346, 348, 350, 351, 352, 353, 354, 355, 356, 357, 358, 359, 360, 362, 363, 364, 365, 366, 367, 368, 370, 372, 375, 376, 378, 379, 381, 382, 383, 384, 385, 387, 388, 389, 390, 391, 392, 393, 394, 395, 396, 397, 398, 399, 400, 401, 403, 405, 406, 407, 408, 410, 413, 414, 415, 416, 418, 421, 424, 426, 427, 429, 431, 432, 434, 436, 438, 439, 440, 442, 443, 445, 446, 447, 448, 449, 451, 453, 455, 456, 457, 458, 459, 460, 461, 462, 463, 465, 467, 468, 470, 471, 474, 478, 480, and 482 according to the numbering of the amino acid sequence set forth in SEQ ID NO: 3.
Particularly preferred, the alpha-amylase variant having alpha-amylase activity comprises amino acid substitutions compared to the parent alpha-amylase at one or more amino acid positions selected from the group consisting of 4, 7, 25, 37, 70, 100, 118, 135, 160, 176, 193, 210, 251, 281, 258, 323, 361, 363, 368, 405, 434, 441, 459, 460, 451, and 482 according to the numbering of the amino acid sequence set forth in SEQ ID NO: 3 and wherein said variant has alpha-amylase activity. More particularly preferred, the alpha-amylase variant having alpha-amylase activity comprises amino acid substitutions compared to the parent alpha-amylase at one or more amino acid positions selected from the group consisting of 25, 100, 135, 176, 193, and 460 according to the numbering of the amino acid sequence set forth in SEQ ID NO: 3 and wherein said variant has alpha-amylase activity.
Preferably, the present invention is directed to an alpha-amylase variant of a parent alpha-amylase having alpha-amylase activity, wherein said variant comprises compared to the parent alpha-amylase amino acid substitution at one or more positions corresponding to positions selected from the group consisting of 4, 25, 100, 135, 135, 135, 135, 160, 176, 193, 251, 258, 276, 299, 323, 363, 363, 382, 405, 460, and 482 according to the numbering of the amino acid sequence set forth in SEQ ID NO: 3.

In an alternative embodiment, the present invention is directed to an alpha-amylase variant comprising compared to a parent sequence one or more amino acid substitutions selected from the group consisting of X9D, X9F, X9K, X9N, X9P, X9Q, X9S, X9T, X9Y, X179G, X181D, X181F, X181H, X181I, X181N, X181Q, X181S, X181T, X181V, X181W, X181Y X186A, X186C, X186D, X186F, X186H, X186I, X186K, X186L, X186M, X186R, X186V, X186W, X186Y, X190H, X195H, X195K, X195L, X195W, X195Y, X206C, X206H, X206M, X206Y, X244A, X244C, X244D, X244E, X244F, X244G, X244H, X244M, X244N, X244V, X402T X419C, X420D, X420E, X420G, X420H, X420K, X420L, X420Q, X422C, X422N, X422H, X423F, X423M, X423Q, X423S, X428C, X428D, X428E, X428G, X428I, X428K, X428L, X428M, X428N, X428R, X428S, X428V, X428W, X428Y, X430A, X430C, X430D, X430E, X430F, X430G, X430L, X430P, X430Q, X430S, X430T, X430V, X435K, X435P, X435S, X435A, X435D, X437L, X437T, X437W, X441C, X441K, X441L, X441M, X441S, X444H, X444M, X444N, X444R, X444T, X450C, X450D, X450E, X450H, X450L, X450M, X450P, X450Q, X450R, X450T, X452A, X452C, X452E, X452F, X452I, X452K, X452M, X452N, X452T, X454A, X454E, X454K, X454L, X454P, X454S, X454T, X466E, X466W, X469F, X469L, X469Y, X475A, X475K, X475E, X475L, X479I, X479K, X479M, X483F, X483L, X483Q, and X483R according to the numbering of the amino acid sequence set forth in SEQ ID NO: 3 and wherein said variant has alpha-amylase activity, preferably wherein the parent alpha-amylase for the alpha-amylase variant of the present invention is an amylase according to SEQ ID NO: 1 or SEQ ID NO: 3 or any alpha-amylase having at least 60% sequence identity to SEQ IDNO: 1 or SEQ ID NO: 3, most preferably the parent alpha-amylase for the alpha-amylase variant is an amylase according to according to SEQ ID NO: 1. Preferably, in this alternative embodiment, the amino acid residue at the above cited positions (i.e., X) corresponds to the amino acid residue shown in SEQ ID NO: 1 or 3, preferably in SEQ ID NO: 1, at the respective position (according to the numbering of SEQ ID NO: 3). Preferably, the present invention is directed to an alpha-amylase variant comprising compared to a parent sequence one or more amino acid substitutions selected from the group consisting of X181D, X181F, X181H, X181I, X181N, X181Q, X181S, X181T, X181V, X181W, X181Y X186A, X186C, X186D, X186F, X186H, X186I, X186K, X186L, X186M, X186R, X186V, X186W, X186Y, X195H, X195K, X195L, X195W, X195Y, X206C, X206H, X206M, X206Y, X441C, X441K, X441L, X441M, and X441S according to the numbering of the amino acid sequence set forth in SEQ ID NO: 3 and wherein said variant has alpha-amylase activity, preferably wherein the parent alpha-amylase for the alpha-amylase variant of the present invention is an amylase according to SEQ ID NO: 1 or SEQ ID NO: 3 or any alpha-amylase having at least 60% sequence identity to SEQ IDNO: 1 or SEQ ID NO: 3, most preferably the parent alpha-amylase for the alpha-amylase variant is an amylase according to according to SEQ ID NO: 1. Preferably, the amino acid residue at the above cited positions (i.e., X) corresponds to the amino acid residue shown in SEQ ID NO: 1 or 3, preferably in SEQ ID NO: 1, at the respective position (according to the numbering of SEQ ID NO: 3).
Particularly preferred, the present invention is directed to an alpha-amylase variant of a parent alpha-amylase having alpha-amylase activity, wherein said variant comprises compared to the parent alpha-amylase amino acid substitution at one or more positions corresponding to positions selected from the group consisting of 4, 25, 100, 135, 135, 135, 135, 160, 176, 193, 251, 258, 276, 299, 323, 363, 363, 382, 405, 460, and 482 according to the numbering of the amino acid sequence set forth in SEQ ID NO: 3.
Preferably, the present invention is directed to an alpha-amylase variant of a parent alpha-amylase having alpha-amylase activity, wherein said variant comprises compared to the parent alpha-amylase amino acid substitution at one or more positions corresponding to positions selected from the group consisting of H1, H2, N3, G4, T5, N6, G7, T8, M10, Y12, F13, E14, W15, Y16, L17, P18, N19, D20, G21, N22, H23, W24, N25, R26, L27, R28, S29, D30, A31, S32, N33, L34, K35, D36, K37, G38, I39, T40, A41, V42, P45, A47, W48, A51, G59, A60, L63, N70, Q71, K72, G73, V75, R76, T81, R82, N83, Q86, V89, T90, A91, L92, K93, S94, N95, G96, I97, Q98, V99, Y100, V103, N106, K108, G109, A113, T114, E115, W116, V117, A119, S125, N126, N128, Q129, V131, S132, G133, D134, Y135, T136, A139, T141, K142, F143, D144, F145, P146, G147, G149, N150, T151, N154, F155, K156, Y160, H161, F162, G164, V165, Q169, S170, Q172, L173, Q174, N175, R176, G184, D188, V191, Y203, H210, P211, E212, V213, V214, N215, E216, R218, N219, G221, V222, W223, Y224, T225, N226, T227, L230, D231, F233, R234, I235, D236, A237, V238, H240, K242, Y243, F245, W249, L250, T251, H252, V253, N255, T256, T257, K259, M261, F262, A263, V264, A265, W268, N270, I272, G273, A274, E276, L279, S280, W284, N285, H286, S287, V288, F289, D290, V291, P292, L293, H294, Y295, N296, L297, N299, S301, R302, S303, G304, N306, Y307, D308, M309, R310, Q311, 1312, F313, N314, G315, V318, Q319, R320, H321, T323, H324, V326, T327, D333, P336, E337, E338, E341, S342, F343, V344, E345, E346, F348, P350, L351, A352, Y353, A354, L355, L357, T358, R359, D360, G362, Y363, P364, V366, F367, Y368, D370, Y372, P375, T376, G378, V379, A381, M382, K383, S384, K385, D387, P388, I389, L390, E391, A392, R393, Q394, K395, Y396, A397, Y398, G399, T400, Q401, D403, L405, D406, H407, P408, V410, W413, T414, R415, E416, D418, H421, S424, L426, A427, L429, S431, G436, K438, W439, M440, V442, G443, N445, N446, A447, G448, E449, W451, D453, T455, G456, N457, Q458, T459, N460, T461, V462, T463, N465, D467, G468, G470, V474, S478, S480, Y482, A119, D163, D271, E439, F180, G182, G258, I177, I275, K185, K269, K281, N224, N260, N277, P322, P434, R118, S365, T193, T282, T356, V120, V317, W187, W189, Y178, and Y298 according to the numbering of the amino acid sequence set forth in SEQ ID NO: 3.

Particularly preferred, the alpha-amylase variant having alpha-amylase activity comprises amino acid substitution compared to the parent alpha-amylase at one or more amino acid positions selected from the group consisting of G4, N25, K37, N70, Y100, R118, Y135, Y160, R176, T193, H210, T251, K281, G258, T323, Q361, Y363, Y368, L405, P434, E441, T459, N460, T451, and Y482 according to the numbering of the amino acid sequence set forth in SEQ ID NO: 3 and wherein said variant has alpha-amylase activity.
Particularly preferred, the alpha-amylase variant having alpha-amylase activity comprises amino acid substitution compared to the parent alpha-amylase at one or more amino acid positions selected from the group consisting of N25, Y100, Y135, R176, T193, and N460 according to the numbering of the amino acid sequence set forth in SEQ ID NO: 3 and wherein said variant has alpha-amylase activity.
Particularly preferred, the present invention is directed to an alpha-amylase variant of a parent alpha-amylase having alpha-amylase activity, wherein said variant comprises compared to the parent alpha-amylase amino acid substitution at one or more positions corresponding to positions selected from the group consisting of G4, N25, Y100, Y135, Y160, R176, T193, T251, G258, E276, N299, T323, Y363, Y363, M382, L405, N460, and Y482 according to the numbering of the amino acid sequence set forth in SEQ ID NO: 3.
Preferably, the alpha-amylase variant having alpha-amylase activity comprises compared to the parent alpha-amylase one or more amino acid substitutions selected from the group of amino acid substitutions consisting of X100D, X100F, X100K, X100L, X103A, X106D, X108A, X109A, X10A, X10C, X10E, X10F, X10L, X10W, X10Y, X113F, X113H, X113M, X113R, X113V, X113W, X113Y, X114A, X114C, X114D, X114E, X114F, X114G, X114H, X114I, X114K, X114L, X114M, X114N, X114Q, X114R, X114S, X114V, X114W, X114Y, X115C, X115D, X115K, X115M, X115N, X115Q, X115R, X115S, X115T, X115V, X116I, X116K, X116L, X116M, X116R, X116T, X117A, X117C, X117D, X117F, X117I, X117N, X117P, X117R, X117W, X117Y, X118A, X118D, X118E, X119H, X119P, X119R, X119S, X119Y, X120E, X120G, X120M, X120S, X120W, X120Y, X123D, X123S, X125A, X125D, X125E, X125F, X125G, X125H, X125K, X125L, X125M, X125N, X125Q, X125R, X125T, X125V, X125W, X125Y, X126D, X128C, X128E, X128L, X128M, X128W, X128Y, X129E, X12N, X12S, X131C, X131I, X131L, X131Q, X131W, X132T, X133A, X133C, X133D, X133E, X133H, X133K, X133N, X133P, X133Q, X133S, X134C, X134E, X134F, X134I, X134L, X134M, X134P, X134T, X134V, X134W, X134Y, X135D, X135E, X135G, X135M, X135N, X135P, X135S, X135T, X135W, X136A, X136C, X136D, X136E, X136F, X136H, X136L, X136M, X136N, X136P, X136W, X139C, X139S, X13A, X13Y, X141V, X142C, X142E, X142F, X142L, X142M, X142Q, X142R, X142W, X142Y, X143F, X144C, X144E, X144G, X144K, X144N, X144Q, X144R, X144S, X144T, X144V, X144Y, X145A, X146C, X146D, X146E, X146F, X146G, X146H, X146K, X146L, X146S, X146T, X146W, X147M, X149E, X14N, X150C, X150E, X150Q, X151C, X151E, X154A, X154Y, X155Y, X156E, X156V, X158H, X158N, X158Y, X15R, X160D, X160E, X160W, X161T, X162V, X163A, X163Q, X163T, X164V, X165S, X165T, X165W, X169A, X169D, X169E, X169S, X169V, X16F, X16R, X170C, X170F, X172A, X172C, X172D, X172K, X172N, X173I, X173Y, X174D, X174E, X174G, X174H, X174M, X174N, X174P, X174S, X174T, X175A, X175G, X175H, X175Q, X176K, X176S, X176T, X177A, X177G, X177K, X177N, X177P, X177R, X177S, X177W, X178F, X17K, X17V, X180M, X180N, X180T, X180W, X182D, X182E, X182N, X182Q, X182S, X184G, X185E, X185M, X185N, X187A, X187D, X187M, X187V, X188H, X188T, X188V, X189I, X18F, X18I, X18K, X18M, X18R, X18T, X191F, X191H, X191K, X191M, X191W, X191Y, X192A, X192T, X193D, X193E, X193G, X193V, X19A, X19C, X19D, X19F, X19G, X19H, X19I, X19K, X19L, X19M, X19P, X19Q, X19S, X19T, X19Y, X1R, X1V, X203E, X203R, X208F, X208I, X208Y, X20A, X20C, X20D, X20E, X20F, X20G, X20H, X20K, X20L, X20M, X20N, X20P, X20Q, X20S, X20T, X20V, X20W, X20Y, X210A, X210C, X210D, X210E, X210F, X210M, X210N, X210Q, X210S, X210Y, X211A, X211C, X211D, X211E, X211G, X211H, X211L, X211N, X211Q, X211S, X211T, X211V, X212C, X212D, X212I, X212L, X212W, X213A, X213C, X213M, X213R, X213S, X214E, X214P, X215A, X215D, X215E, X215H, X215W, X216G, X216H, X218A, X218C, X218D, X218E, X218F, X218G, X218H, X218I, X218K, X218L, X218N, X218Q, X218S, X218T, X218V, X218W, X218Y, X219A, X219C, X219D, X219E, X219F, X219G, X219H, X219K, X219M, X219Q, X219R, X219S, X219T, X219W, X219Y, X21E, X21S, X221F, X221N, X222D, X222K, X222S, X222T, X223C, X223L, X223V, X223Y, X224F, X224V, X225A, X225C, X225E, X225F, X225H, X225I, X225N, X225R, X225S, X225Y, X226A, X226C, X226D, X226E, X226G, X226H, X226I, X226L, X226M, X226Q, X226R, X226S, X226T, X226V, X226W, X226Y, X227C, X227F, X227G, X227H, X227I, X227K, X227L, X227M, X227R, X227T, X227V, X227W, X227Y, X22A, X22D, X22E, X22F, X22G, X22K, X22L, X22M, X22Q, X22R, X22T, X22W, X22Y, X230A, X230F, X231C, X231D, X231N, X233C, X233H, X233I, X233M, X233T, X233W, X233Y, X234C, X235L, X235M, X235V, X236Y, X237C, X237I, X238A, X238F, X238T, X23N, X23Q, X23W, X240M, X242M, X242N, X243D, X243F, X245E, X245H, X245M, X249D, X249I, X24G, X250V, X251A, X251E, X251F, X251L, X251M, X251S, X251T, X252C, X252I, X252S, X253C, X253G, X253Y, X255D, X255F, X255I, X255T, X255V, X256C, X257L, X257V, X257Y, X258F, X258Q, X258R, X259L, X25A, X25C, X25D, X25F, X25G, X25H, X25K, X25L, X25M, X25Q, X25S, X25W, X25Y, X260H, X261R, X262C, X262D, X262E, X262H, X262P, X262Y, X263I, X264H, X264T, X264W, X265S, X268F, X268G, X269M, X26A, X26D, X26E, X26F, X26L, X26M, X26P, X26S, X26V, X26Y, X270A, X270Q, X270Y, X271S, X272F, X272G, X272L, X272S, X273A, X273C, X273D, X273E, X273F, X273H, X273I, X273L, X273M, X273P, X273Q, X273R, X273V, X273W, X273Y, X274F, X274S, X275V, X276D, X276K, X276L, X276N, X276R, X276Y, X277D, X277E, X277T, X279A, X279P, X27A, X27D, X27F, X27G, X27H, X27I, X27Q, X27R, X27T, X27V, X280D, X280F, X280G, X280H, X280I, X280K, X280N, X280R, X280V, X280Y, X281A, X281D, X281E, X281H, X284A, X284F, X284H, X284L, X284M, X284N, X284Y, X285G, X285L, X285N, X285P, X286Q, X287A, X287D, X287E, X287H, X287T, X288A, X288K, X288P, X288Y, X289F, X289G, X289R, X289T, X28C, X28D, X28E, X28F, X28G, X28I, X28K, X28N, X28Q, X28S, X28T, X28V, X290D, X290M, X290N, X290Q, X290W, X291D, X291K, X291T, X291Y, X292C, X292D, X292F, X292I, X292L, X292T, X292W, X292Y, X293D, X293E, X293F, X293K, X293R, X294G, X294T, X295F, X296A, X296C, X296L, X296Y, X297E, X297F, X297H, X297K, X297M, X297S, X297V, X299G, X299I, X299K, X299L, X299S, X299Y, X29D, X29E, X29F, X29G, X29H, X29I, X29K, X29L, X29N, X29P, X29Q, X29V, X29W, X29Y, X2I, X2S, X301F, X302H, X302I, X302Q, X302V, X302Y, X303E, X303H, X303I, X303K, X303L, X303M, X303N, X303P, X303R, X303T, X304A, X304D, X304E, X304H, X304K, X304M, X304N, X304P, X304R, X304T, X304W, X304Y, X306A, X306D, X306E, X306G, X306H, X306I, X306M, X306Q, X306R, X306S, X306T, X306V, X306W, X306Y, X307F, X307M, X308S, X309H, X309L, X309Q, X30A, X30E, X30F, X30G, X30H, X30I, X30K, X30L, X30M, X30Q, X30T, X30W, X30Y, X310A, X310Q, X311A, X311E, X311G, X311H, X311K, X311N, X311R, X311T, X311Y, X312L, X312M, X313V, X314C, X314E, X314K, X314Q, X315A, X315C, X315E, X315H, X315K, X315T, X318I, X318S, X318T, X319A, X319D, X319H, X319I, X319K, X319M, X319N, X319P, X319S, X319T, X319W, X31N, X31Q, X31S, X31T, X31V, X31W, X320A, X320C, X320D, X320E, X320G, X320H, X320K, X320L, X320N, X320Q, X320S, X320Y, X321A, X321E, X321K, X321N, X321T, X321V, X321W, X323A, X323G, X323K, X323L, X323V, X324K, X324L, X324M, X324W, X324Y, X326G, X326N, X326S, X326Y, X327C, X327L, X327M, X32A, X32D, X32E, X32F, X32H, X32I, X32L, X32M, X32N, X32P, X32Q, X32T, X32W, X333I, X334T, X336K, X337A, X337C, X337F, X337G, X337I, X337K, X337L, X337M, X337N, X337Q, X337R, X337S, X337T, X337V, X337Y, X338G, X338S, X338T, X33D, X33E, X33H, X33K, X33M, X33Q, X33R, X33Y, X341V, X342P, X343L, X343T, X343W, X343Y, X344I, X344Q, X344V, X345D, X345G, X345M, X345N, X345Q, X345S, X345T, X346A, X346C, X346D, X346G, X346H, X346N, X346Q, X348T, X34H, X34I, X34V, X350H, X350K, X350P, X351A, X351M, X352S, X353H, X354I, X354N, X354T, X354Y, X355I, X355M, X356I, X356V, X357A, X358I, X358L, X358N, X358P, X358V, X359E, X35A, X35C, X35D, X35G, X35H, X35I, X35L, X35M, X35N, X35P, X35Q, X35R, X35S, X35T, X35V, X35Y, X360A, X360F, X360G, X360I, X360L, X360N, X360Q, X360R, X360S, X360T, X360V, X360Y, X362F, X362K, X362M, X362N, X362T, X362V, X362Y, X363A, X363C, X363D, X363E, X363G, X363H, X363K, X363L, X363M, X363P, X363Q, X363R, X363S, X363T, X363V, X363W, X363Y, X364A, X364C, X364G, X364K, X364L, X364N, X364S, X364T, X364V, X366I, X366L, X366T, X367E, X367S, X368A, X368F, X368L, X368N, X36A, X36E, X36G, X36I, X36K, X36M, X36N, X36P, X36Q, X36R, X36S, X36T, X36V, X370E, X370I, X372A, X372C, X372E, X372F, X372H, X372M, X372N, X372Q, X375A, X375D, X375E, X375I, X375K, X375Q, X375R, X375T, X375W, X375Y, X376G, X376I, X376K, X376L, X376M, X376Q, X376R, X376S, X376V, X377Q, X378C, X378D, X378E, X378R, X379A, X379L, X379S, X37A, X37G, X37M, X37P, X37T, X37V, X37W, X381E, X381V, X382A, X382H, X382K, X382L, X382N, X382Q, X382S, X383C, X383D, X383E, X383H, X383I, X383M, X383N, X383Q, X383R, X383S, X383V, X383Y, X384A, X384C, X384D, X384E, X384F, X384I, X384L, X384M, X384N, X384Q, X384R, X384T, X384V, X384W, X384Y, X385A, X385C, X385D, X385E, X385F, X385G, X385H, X385I, X385L, X385M, X385N, X385P, X385Q, X385R, X385S, X385T, X385V, X385W, X385Y, X387C, X387E, X387N, X388E, X388F, X388H, X388I, X388M, X388R, X388V, X389G, X389H, X389K, X38N, X390D, X390F, X390M, X390N, X390P, X390R, X391A, X391F, X391G, X391K, X391M, X391N, X391Q, X391S, X391T, X391Y, X392C, X392V, X393E, X393H, X393P, X393S, X393V, X394A, X394C, X394E, X394H, X394I, X394L, X394M, X394N, X394R, X394S, X395A, X395H, X395M, X395V, X396H, X396P, X397D, X397H, X397P, X397S, X398M, X399P, X39E, X39K, X3A, X3F, X3G, X3I, X3K, X3L, X3Q, X3V, X400A, X400D, X400E, X400G, X400H, X400I, X400K, X400L, X400M, X400N, X400P, X400Q, X400R, X400S, X400V, X400W, X401I, X401K, X401M, X401T, X403N, X405C, X405H, X405M, X405T, X405V, X406P, X407D, X407R, X407S, X408E, X408I, X408Q, X40I, X40S, X410H, X410I, X410K, X410L, X410P, X410R, X410Y, X413S, X414C, X414E, X414S, X415E, X415I, X416S, X418C, X418N, X418P, X41C, X41D, X41E, X41G, X41Q, X41S, X41T, X421N, X421P, X424A, X426D, X426W, X427C, X427F, X427G, X427K, X427Q, X427R, X427S, X427T, X427V, X429A, X429D, X429E, X429F, X429G, X429I, X429M, X429N, X429P, X429Q, X429S, X429T, X429V, X429W, X42C, X42I, X42Q, X42V, X431I, X434S, X436E, X438F, X438P, X438Y, X439C, X439P, X440V, X442Q, X443H, X443T, X445A, X445C, X445D, X445F, X445G, X445H, X445K, X445M, X445Q, X445R, X445S, X445T, X445V, X446F, X446I, X446L, X446P, X446Q, X446R, X446V, X446W, X447V, X448D, X448E, X448H, X448N, X449A, X449F, X449G, X449K, X449L, X449M, X449N, X449P, X449Q, X449R, X449S, X449V, X449W, X449Y, X451L, X453G, X453I, X453N, X453P, X453Y, X455L, X456L, X456M, X456R, X456S, X456V, X456W, X456Y, X457A, X457C, X457E, X457F, X457G, X457K, X457L, X457M, X457R, X457S, X457T, X457V, X457W, X458I, X458K, X458V, X458W, X459C, X459D, X459E, X459I, X459K, X459N, X459Q, X459R, X459V, X459Y, X45G, X45N, X460A, X460D, X460E, X460G, X460Q, X460R, X460S, X460T, X460V, X461A, X461E, X461F, X461K, X461L, X461M, X461N, X461Q, X461R, X461S, X461V, X462K, X463L, X465H, X465P, X465R, X465V, X467A, X467E, X467H, X468D, X468H, X470A, X470Q, X470T, X471E, X474F, X474H, X474P, X478A, X478E, X47S, X480D, X480E, X480M, X480R, X480Y, X482C, X482T, X482W, X48F, X48I, X48M, X48Y, X4A, X4C, X4K, X4M, X4Q, X4R, X4S, X51Q, X51T, X51V, X54D, X54G, X54Q, X59T, X5A, X5C, X5D, X5E, X5F, X5H, X5I, X5K, X5L, X5M, X5N, X5P, X5Q, X5R, X5V, X5Y, X60T, X63C, X63V, X6A, X6C, X6E, X6F, X6G, X6H, X6K, X6L, X6M, X6P, X6Q, X6S, X6T, X6V, X6W, X6Y, X70F, X70H, X70L, X70M, X70N, X70Y, X71D, X72C, X72D, X72E, X72N, X72T, X73L, X73N, X73Q, X75A, X75G, X75I, X75L, X75P, X75T, X75W, X76C, X76E, X76G, X76L, X76T, X76V, X7C, X7E, X7F, X7H, X7K, X7N, X7P, X7Q, X7R, X7S, X7V, X7W, X7Y, X81H, X81L, X82K, X82M, X83A, X83D, X83E, X83G, X83R, X83S, X86K, X87D, X87R, X89A, X89C, X89F, X89G, X89L, X89M, X89R, X89S, X8A, X8C, X8F, X8I, X8M, X8P, X8S, X8V, X8W, X8Y, X90A, X90D, X90E, X90F, X90G, X90I, X90M, X90N, X90Q, X90R, X90S, X90V, X90Y, X91C, X91D, X91E, X91F, X91G, X91H, X91I, X91K, X91L, X91M, X91N, X91Q, X91S, X91T, X91V, X91W, X91Y, X92D, X92M, X92V, X93K, X93Q, X94A, X94D, X94E, X94K, X94M, X94V, X94Y, X95E, X95I, X95L, X95V, X96K, X96N, X96Q, X97V, X98E, X98G, X98N, X99H, X99K, X99N, X100W, and X1G according to the numbering of the amino acid sequence set forth in SEQ ID NO: 3 and wherein said variant has alpha-amylase activity. Preferably, the amino acid residue at the above cited positions (i.e., X) corresponds to the amino acid residue shown in SEQ ID NO: 1 or 3, preferably in SEQ ID NO: 1, at the respective position (according to the numbering of SEQ ID NO: 3).
Particularly preferred, the alpha-amylase variant having alpha-amylase activity comprises compared to the parent alpha-amylase one or more amino acid substitutions selected from the group of amino acid substitutions consisting of X4Q, X7H, X7W, X25H, X25Y, X37M, X70H, X100W, X118D, X135T, X160W, X176K, X193E, X210C, X251E, X281N, X258Q, X323G, X361R, X363E, X363H, X363N, X368F, X405M, X434R, X441Q, X459N, X460G, X451L, and X482W according to the numbering of the amino acid sequence set forth in SEQ ID NO: 3 and wherein said variant has alpha-amylase activity. Preferably, the amino acid residue at the above cited positions (i.e., X) corresponds to the amino acid residue shown in SEQ ID NO: 1 or 3, preferably in SEQ ID NO: 1, at the respective position (according to the numbering of SEQ ID NO: 3).
Particularly preferred, the alpha-amylase variant having alpha-amylase activity comprises compared to the parent alpha-amylase one or more amino acid substitutions selected from the group of amino acid substitutions consisting of X25H, X25Y, X100W, X135T, X176K, X193E, and X460G according to the numbering of the amino acid sequence set forth in SEQ ID NO: 3 and wherein said variant has alpha-amylase activity. Preferably, the amino acid residue at the above cited positions (i.e., X) corresponds to the amino acid residue shown in SEQ ID NO: 1 or 3, preferably in SEQ ID NO: 1, at the respective position (according to the numbering of SEQ ID NO: 3).
Particularly preferred, the alpha-amylase variant having alpha-amylase activity comprises compared to the parent alpha-amylase one or more amino acid substitutions selected from the group of amino acid substitutions consisting of X4Q, X25H, X100W, X135E, X135T, X135W, X135D, X160W, X176K, X193E, X251E, X258Q, X276R, X299S, X323G, X363E, X363H, X382Q, X405M, X460G, and X482W according to the numbering of the amino acid sequence set forth in SEQ ID NO: 3 and wherein said variant has alpha-amylase activity. Preferably, the amino acid residue at the above cited positions (i.e., X) corresponds to the amino acid residue shown in SEQ ID NO: 1 or 3, preferably in SEQ ID NO: 1, at the respective position (according to the numbering of SEQ ID NO: 3).
Preferably, the alpha-amylase variant having alpha-amylase activity comprises compared to the parent alpha-amylase one or more amino acid substitutions selected from the group of amino acid substitutions consisting of H1G, H1V, H1R, H2S, H2I, N3Q, N3K, N3F, N3G, N3L, G4Q, G4R, G4S, G4K, G4M, G4A, G4C, T5F, T5E, T5M, T5Y, T5R, T5Q, T5K, T5L, T5A, T5V, T5C, T5N, T5H, T5D, T5P, T5I, N6T, N6E, N6A, N6K, N6Q, N6V, N6M, N6G, N6L, N6H, N6W, N6F, N6P, N6S, N6C, N6Y, G7Y, G7S, G7H, G7C, G7Q, G7F, G7P, G7W, G7R, G7K, G7E, G7V, G7N, T8C, T8V, T8F, T8Y, T8P, T8W, T8A, T8S, T8M, T8I, M10W, M10Y, M10L, M10C, M10E, M10F, M10A, Y12N, Y12S, F13A, F13Y, E14N, W15R, Y16F, Y16R, L17K, L17V, P18R, P18M, P18F, P18K, P18T, P18I, N19P, N19L, N19F, N19Y, N19T, N19I, N19Q, N19C, N19H, N19S, N19M, N19K, N19G, N19D, N19A, D20C, D20S, D20L, D20M, D20T, D20D, D20Q, D20F, D20P, D20G, D20V, D20W, D20A, D20Y, D20H, D20K, D20E, G21S, G21E, N22E, N22F, N22A, N22D, N22M, N22T, N22K, N22Y, N22G, N22R, N22Q, N22W, N22L, H23N, H23Q, H23W, W24G, N25H, N25Y, N25K, N25F, N25S, N25C, N25D, N25A, N25G, N25W, N25Q, N25M, N25L, R26S, R26V, R26M, R26A, R26D, R26E, R26F, R26P, R26Y, R26L, L27D, L27I, L27F, L27R, L27G, L27Q, L27V, L27T, L27A, L27H, R28N, R28S, R28F, R28C, R28E, R28T, R28G, R28K, R28D, R28V, R28I, R28Q, S29Y, S29L, S29P, S29W, S29K, S29F, S29N, S29H, S29D, S29V, S29G, S29Q, S29I, D30F, D30I, D30A, D30K, D30W, D30Y, D30M, D30L, D30H, D30Q, D30G, D30E, D30T, A31S, A31V, A31Q, A31T, A31N, A31W, S32T, S32H, S32W, S32F, S32Q, S32I, S32A, S32P, S32E, S32M, S32N, S32D, S32L, N33Y, N33D, N33M, N33Q, N33K, N33R, L34H, L34V, L34I, K35C, K35Y, K35P, K35T, K35M, K35H, K35A, K35V, K35I, K35G, K35Q, K35D, K35N, K35R, K35S, K35L, D36V, D36I, D36T, D36K, D36A, D36S, D36R, D36M, D36E, D36P, D36G, D36N, D36Q, K37P, K37V, K37W, K37A, K37G, G38N, I39E, I39K, T40I, T40S, A41G, A41Q, A41C, A41D, A41E, A41S, A41T, V42Q, V42V, V42I, V42C, P45N, P45G, A47S, W48F, W48I, W48M, A51T, G59T, A60T, L63V, L63C, N70N, N70L, N70M, N70F, N70Y, Q71D, K72T, K72E, K72D, K72N, K72C, G73Q, G73L, G73N, V75A, V75I, V75W, V75P, V75G, V75T, R76G, R76T, R76C, R76V, R76E, R76L, T81H, R82M, R82K, N83G, N83R, N83S, Q86K, V89F, V89A, V89C, V89L, V89G, V89M, V89S, V89R, T90Q, T90F, T90G, T90E, T90A, T90N, T90I, T90R, T90Y, T90V, T90S, T90M, T90D, A91S, A91Y, A91K, A91Q, A91F, A91H, A91T, A91I, A91W, A91N, A91V, A91L, A91C, A91G, A91D, A91E, A91M, L92V, L92M, L92D, K93K, K93Q, S94M, S94K, S94Y, S94D, S94A, S94V, N95V, N95I, N95E, N95L, G96N, G96K, I97V, Q98G, Q98E, Q98N, V99K, V99H, V99N, Y100D, Y100K, Y100L, Y100F, V103A, N106D, K108A, G109A, A113Y, A113H, A113M, A113V, A113F, A113W, A113R, T114D, T114H, T114N, T114W, T114E, T114C, T114G, T114L, T114M, T114Q, T114F, T114A, T114V, T114K, T114S, T114Y, T114R, T114I, E115C, E115K, E115N, E115T, E115M, E115S, E115Q, E115R, E115D, E115V, W116L, V117D, V117Y, V117N, V117C, V117R, V117W, V117A, V117I, V117P, V117F, A119Y, A119R, A119P, A119H, S125A, S125K, S125L, S125T, S125R, S125F, S125W, S125Q, S125V, S125Y, S125H, S125N, S125E, S125G, S125M, N126D, N128C, N128L, N128Y, N128E, N128W, N128M, Q129E, V131I, V131C, S132T, G133E, G133P, G133C, G133D, G133Q, G133S, G133H, G133A, G133N, G133K, D134Y, D134W, D134F, D134P, D134E, D134V, D134L, D134M, D134T, D134C, D134I, Y135M, T136D, T136E, T136F, T136M, T136P, T136H, T136C, T136A, T136W, T136L, T136N, A139C, T141V, K142C, K142W, K142Y, K142L, K142M, K142Q, K142F, K142E, K142R, F143F, D144Q, D144C, D144V, D144K, D144T, D144Y, D144E, D144G, D144N, D144S, D144R, F145A, P146C, P146H, P146K, P146T, P146E, P146S, P146D, P146W, P146F, P146L, P146G, G147M, G149E, N150Q, N150E, N150C, T151C, T151E, N154Y, N154A, F155Y, K156V, Y160W, H161T, F162V, G164V, V165W, Q169E, Q169V, S170F, S170C, Q172C, Q172K, Q172A, L173I, L173Y, Q174P, Q174M, Q174E, Q174S, N175G, N175Q, N175H, R176K, G184G, D188V, D188H, V191K, V191H, V191F, V191W, V191Y, Y203E, Y203R, H210D, H210C, H210E, H210N, H210F, H210Y, H210M, H210Q, H210S, H210A, P211N, P211C, P211T, P211E, P211G, P211V, P211Q, P211A, P211H, P211L, P211S, E212W, E212L, E212I, E212D, E212C, V213A, V213S, V213M, V213R, V213C, V214P, V214E, N215E, N215D, N215H, N215W, N215A, E216H, E216G, R218Q, R218E, R218C, R218N, R218F, R218S, R218G, R218K, R218I, R218T, R218D, R218Y, R218H, R218L, R218V, R218A, R218W, N219E, N219Q, N219K, N219T, N219M, N219D, N219F, N219S, N219R, N219A, N219Y, N219H, N219W, N219G, N219C, G221N, G221F, V222S, V222T, V222D, W223V, W223Y, W223L, W223C, Y224V, Y224F, T225A, T225R, T225N, T225E, T225H, T225F, T225C, T225Y, T225I, N226C, N226W, N226I, N226R, N226Y, N226D, N226L, N226M, N226G, N226T, N226Q, N226S, N226A, N226V, N226E, N226H, T227W, T227C, T227T, T227R, T227M, T227K, T227Y, T227V, T227F, T227I, T227H, T227L, T227G, L230A, L230F, D231N, D231C, F233T, F233W, F233H, F233C, F233I, F233Y, F233M, R234C, I235M, I235V, I235L, D236Y, A237C, A237I, V238T, H240M, K242M, Y243F, F245E, F245M, F245H, W249D, W249I, L250V, T251E, T251A, T251L, T251S, T251M, T251F, H252C, H252I, H252S, V253Y, V253G, N255T, N255V, N255D, N255F, N255I, T256C, T257Y, T257L, T257V, K259L, M261R, F262D, F262Y, F262C, F262P, F262E, F262H, A263I, V264T, V264W, V264H, A265S, W268G, N270A, N270Y, I272L, I272G, G273H, G273V, G273L, G273M, G273C, G273D, G273W, G273F, G273A, G273E, G273P, G273Y, G273R, G273I, A274S, E276Y, E276K, E276L, E276D, L279P, S280I, S280V, S280N, S280H, S280Y, S280K, S280R, W284Y, W284A, W284L, W284F, W284H, W284N, W284M, N285N, N285L, N285G, N285P, H286Q, S287E, S287H, S287A, S287D, S287T, V288K, V288P, V288Y, V288A, F289T, F289R, F289G, F289F, D290Q, D290N, D290M, D290D, D290W, V291D, V291Y, V291K, V291T, P292W, P292F, P292L, P292I, P292T, P292C, L293D, L293R, L293K, L293E, L293F, H294T, H294G, Y295F, N296Y, N296L, N296C, N296A, L297M, L297K, L297S, L297H, L297V, L297E, L297F, N299L, N299G, N299I, N299K, N299S, N299Y, S301F, R302H, R302I, R302Q, R302Y, R302V, S303P, S303M, S303E, S303I, S303R, S303K, S303N, S303L, S303H, S303T, G304H, G304A, G304R, G304Y, G304D, G304N, G304M, G304E, G304P, G304W, G304K, G304T, N306M, N306E, N306A, N306G, N306W, N306V, N306S, N306I, N306H, N306T, N306D, N306Q, N306Y, N306R, Y307F, Y307M, D308S, M309H, M309L, M309Q, R310Q, R310A, Q311G, Q311E, Q311H, Q311A, Q311K, Q311T, Q311N, Q311R, Q311Y, I312M, I312L, F313V, N314C, N314Q, G315C, G315A, G315H, G315T, G315E, G315K, V318T, V318S, V318I, Q319K, Q319H, Q319P, Q319A, Q319I, Q319D, Q319M, Q319S, Q319N, Q319T, Q319W, R320G, R320Y, R320L, R320N, R320A, R320D, R320K, R320Q, R320E, R320H, R320C, R320S, H321E, H321W, H321T, H321A, H321N, H321V, H321K, T323K, T323G, H324W, H324K, H324Y, V326Y, V326N, V326G, V326S, T327M, T327C, T327L, D333I, P336K, E337N, E337K, E337M, E337T, E337V, E337Q, E337S, E337R, E337I, E337Y, E337G, E337F, E337A, E337L, E338G, E338T, E338S, E341V, S342P, F343W, F343Y, V344I, V344Q, V344V, E345N, E345T, E345M, E345G, E345S, E345D, E345Q, E346D, E346C, E346H, E346A, E346G, E346Q, E346N, F348T, P350P, P350H, P350K, L351M, L351A, A352S, Y353H, A354Y, A354N, A354T, A354I, L355I, L355M, L357A, T358P, R359E, D360N, D360R, D360S, D360Y, D360V, D360L, D360A, D360I, D360T, D360G, D360F, G362T, G362M, G362V, G362N, G362Y, G362F, G362K, Y363M, Y363G, Y363S, Y363V, Y363C, Y363T, Y363H, Y363P, Y363D, Y363L, Y363R, Y363Q, Y363E, Y363Y, Y363K, Y363W, Y363A, P364G, P364T, P364S, P364A, P364V, P364C, P364K, P364L, P364N, V366I, V366L, V366T, F367S, F367E, Y368N, Y368L, Y368A, D370E, D370I, Y372C, Y372H, Y372A, Y372F, Y372M, Y372Q, Y372N, Y372E, P375K, P375A, P375E, P375R, P375T, P375I, P375W, P375Q, T376S, T376R, T376M, T376I, T376V, T376Q, T376L, G378E, G378R, G378D, G378C, V379L, V379A, V379S, A381V, A381E, M382N, M382H, M382Q, M382K, M382A, M382S, K383R, K383I, K383D, K383V, K383M, K383E, K383N, K383S, K383Q, K383Y, K383C, K383H, S384W, S384Y, S384V, S384R, S384N, S384E, S384A, S384D, S384T, S384C, S384F, S384L, S384I, S384M, S384Q, K385G, K385V, K385T, K385Y, K385I, K385D, K385C, K385Q, K385A, K385W, K385L, K385H, K385M, K385N, K385S, K385F, K385E, K385R, K385P, D387N, D387C, D387E, P388R, P388F, P388H, P388V, P388E, P388I, I389G, I389K, I389H, L390P, L390N, L390R, L390F, L390M, L390D, E391T, E391F, E391S, E391A, E391G, E391M, E391N, E391Y, E391Q, E391K, A392C, A392V, R393V, R393E, R393H, R393S, R393P, Q394C, Q394L, Q394R, Q394S, Q394A, Q394E, Q394N, Q394M, Q394H, Q394I, K395V, K395M, K395A, K395H, Y396P, Y396H, A397P, A397D, A397S, A397H, Y398M, G399P, T400L, T400P, T400I, T400S, T400A, T400R, T400Q, T400D, T400K, T400N, T400V, T400W, T400H, T400E, T400G, T400M, Q401M, Q401K, Q401T, Q401I, D403N, L405H, L405V, L405T, L405C, D406P, H407S, H407R, H407D, P408Q, P408E, P408I, V410R, V410K, V410I, V410L, V410H, W413S, T414E, T414C, T414S, R415I, R415E, E416S, D418C, D418P, D418N, H421P, H421N, S424A, L426D, L426W, A427Q, A427C, A427F, A427T, A427V, A427G, A427S, A427R, A427K, L429M, L429D, L429E, L429N, L429T, L429A, L429P, L429W, L429Q, L429S, L429F, L429G, L429I, L429V, S431I, G436E, K438P, K438F, K438Y, W439C, W439P, M440V, V442Q, G443T, G443H, N445M, N445T, N445A, N445G, N445S, N445F, N445R, N445Q, N445C, N445D, N445K, N445H, N445V, N446P, N446I, N446V, N446F, N446L, N446R, N446Q, N446W, A447V, G448E, G448H, G448N, G448D, E449V, E449Y, E449F, E449L, E449P, E449G, E449W, E449M, E449R, E449N, E449K, E449Q, E449S, E449A, W451L, D453G, D453I, D453P, D453Y, D453N, T455L, G456M, G456S, G456Y, G456V, G456L, G456W, G456R, N457K, N457C, N457L, N457S, N457R, N457F, N457T, N457A, N457V, N457W, N457E, N457M, N457G, Q458V, Q458W, Q458I, Q458K, T459Q, T459D, T459I, T459K, T459C, T459V, T459E, T459Y, N460E, N460Q, N460S, N460R, N460A, N460T, N460D, N460V, T461A, T461R, T461F, T461E, T461S, T461V, T461M, T461K, T461N, T461Q, V462K, T463L, N465P, N465V, N465H, N465R, D467A, D467E, D467H, G468H, G468D, G470Q, G470T, G470A, V474P, V474H, V474F, S478A, S478E, S480Y, S480M, S480E, S480D, S480R, Y482T, Y482C, A119S, A139S, A274F, D163A, D163Q, D163T, D188T, D20N, D231D, D271S, D370S, D36H, E276N, E439K, F180M, F180N, F180T, F180W, G182D, G182E, G182N, G182Q, G182S, G258F, G258Q, G258R, G273Q, G362C, G73C, G96Q, I177A, I177G, I177K, I177N, I177P, I177R, I177S, I177W, I272F, I272S, I275V, K156E, K185E, K185M, K185N, K242N, K269M, K281A, K281D, K281E, K281H, K281N, K281S, K37F, K37L, K37M, K37Y, K395C, L279A, L357C, L357F, N224F, N260H, N270Q, N277D, N277E, N277T, N314S, N33L, N83E, N95A, P322K, P434E, P434H, P434S, Q169D, Q169S, Q174G, Q174H, Q174N, Q311W, Q98A, Q98V, R118A, R118D, R118E, R176S, R176T, R359W, R82L, S280D, S280F, S280G, S365Y, S94E, T193D, T193E, T193G, T193V, T251T, T282V, T356C, T356L, T356V, T358M, T461L, T463A, T463V, V120E, V120G, V120M, V120S, V120W, V120Y, V131Q, V165S, V165T, V191M, V238A, V238F, V317M, V75H, W116I, W116M, W187A, W187D, W187M, W187V, W189I, W268F, Y100W, Y135D, Y135E, Y135G, Y135N, Y135P, Y135S, Y135T, Y135W, Y160D, Y160E, Y178F, Y243D, Y298V, Y363N, Y368F, Y372S, Y372T, Y396C, R393Q, P434R, V318L, T459N, H321Y, N460G, D387S, S365L, V253C, P388K, A352C, S365Q, Y396S, V379R, L405M, T81L, and R82C according to the numbering of the amino acid sequence set forth in SEQ ID NO: 3 and wherein said variant has alpha-amylase activity.
Particularly preferred, the alpha-amylase variant having alpha-amylase activity comprises compared to the parent alpha-amylase one or more amino acid substitutions selected from the group of amino acid substitutions consisting of G4Q, G7H, G7W, N25H, N25Y, K37M, N70H, Y100W, R118D, Y135T, Y160W, R176K, T193E, H210C, T251E, K281N, G258Q, T323G, Q361R, Y363E,Y363H, Y363N, Y368F, L405M, P434R, E441Q, T459N, N460G, T451L, and Y482W according to the numbering of the amino acid sequence set forth in SEQ ID NO: 3 and wherein said variant has alpha-amylase activity.
Particularly preferred, the alpha-amylase variant having alpha-amylase activity comprises compared to the parent alpha-amylase one or more amino acid substitutions selected from the group of amino acid substitutions consisting of N25H, Y100W, Y135T, R176K, T193E, and N460G according to the numbering of the amino acid sequence set forth in SEQ ID NO: 3 and wherein said variant has alpha-amylase activity.
Particularly preferred, the alpha-amylase variant having alpha-amylase activity comprises compared to the parent alpha-amylase one or more amino acid substitutions selected from the group of amino acid substitutions consisting of G4Q, N25H, Y100W, Y135E, Y135T, Y135W, Y135D, Y160W, R176K, T193E, T251E, G258Q, E276R, N299S, T323G, Y363E, Y363H, M382Q, L405M, N460G, and Y482W according to the numbering of the amino acid sequence set forth in SEQ ID NO: 3 and wherein said variant has alpha-amylase activity.
Preferably, the one or more amino acid alteration, preferably substitution, is at amino acid positions located on the surface of the alpha-amylase. Preferably, the one or more amino acid alteration, preferably substitution, at an amino acid positions located on the surface of the alpha-amylase is selected from the group consisting of 5, 7, 16, 18, 20, 22, 26, 28, 29, 32, 35, 36, 54, 70, 73, 75, 83, 86, 87, 90, 93, 94, 95, 96, 98, 113, 116, 125, 126, 128, 129, 131, 133, 135, 136, 146, 147, 149, 150, 151, 154, 158, 160, 169, 170, 172, 174, 175, 184, 191, 192, 208, 210, 224, 242, 249, 253, 256, 257, 268, 279, 297, 302, 303, 304, 306, 308, 309, 311, 312, 313, 318, 320, 321, 336, 337, 338, 345, 358, 370, 375, 376, 378, 379, 382, 383, 397, 398, 401, 405, 406, 416, 418, 421, 434, 442, 443, 446, 448, 455, 457, 458, 459, 461, 463, 465, 467, 471, and 474 according to the numbering of SEQ ID NO: 3. Preferably, the alpha-amylase of the present invention comprises one or more amino acid substitution at an amino acid position located on the surface of the alpha-amylase, wherein the substitution at an amino acid position located on the surface of the alpha-amylase is selected from the group consisting of X5F, X5E, X5M, X5Y, X5R, X5Q, X5K, X5L, X5A, X5V, X5C, X5N, X5H, X5D, X5P, X7Y, X7S, X7H, X7C, X7Q, X7F, X7P, X7W, X7R, X7K, X7E, X7V, X7N, X16F, X16R, X18R, X18M, X18F, X18K, X18T, X18I, X20C, X20S, X20L, X20M, X20T, X20D, X20Q, X20F, X20P, X20G, X20V, X20W, X20A, X20Y, X20H, X20K, X20E, X22E, X22F, X22A, X22D, X22M, X22T, X22K, X22Y, X22G, X22R, X22Q, X22W, X22L, X26S, X26V, X26M, X26A, X26D, X26E, X26F, X26P, X26Y, X26L, X28N, X28S, X28F, X28C, X28E, X28T, X28G, X28K, X28D, X28V, X28I, X28Q, X29Y, X29L, X29P, X29W, X29K, X29F, X29N, X29H, X29E, X29D, X29V, X29G, X29Q, X29I, X32T, X32H, X32W, X32F, X32Q, X32I, X32A, X32P, X32E, X32M, X32N, X32D, X32L, X35C, X35Y, X35P, X35T, X35M, X35H, X35A, X35V, X35I, X35G, X35Q, X35D, X35N, X35R, X35S, X35L, X36V, X36I, X36T, X36K, X36A, X36S, X36R, X36M, X36E, X36P, X36G, X36N, X36Q, X54D, X54G, X54Q, X70N, X70L, X70H, X70M, X70F, X70Y, X73Q, X73L, X73N, X75A, X75I, X75W, X75P, X75G, X75L, X75T, X83G, X83R, X83E, X83D, X83A, X83S, X86K, X87D, X87R, X90Q, X90F, X90G, X90E, X90A, X90N, X90I, X90R, X90Y, X90V, X90S, X90M, X90D, X93K, X93Q, X94M, X94K, X94Y, X94D, X94A, X94E, X94V, X95V, X95I, X95E, X95L, X96N, X96Q, X96K, X98G, X98E, X98N, X113Y, X113H, X113M, X113V, X113F, X113W, X113R, X116R, X116T, X116K, X116L, X125A, X125K, X125L, X125T, X125R, X125F, X125W, X125D, X125Q, X125V, X125Y, X125H, X125N, X125E, X125G, X125M, X126D, X128C, X128L, X128Y, X128E, X128W, X128M, X129E, X131I, X131L, X131W, X131C, X133E, X133P, X133C, X133D, X133Q, X133S, X133H, X133A, X133N, X133K, X135M, X135D, X135E, X135T, X135W, X136D, X136E, X136F, X136M, X136P, X136H, X136C, X136A, X136W, X136L, X136N, X146C, X146H, X146K, X146T, X146E, X146S, X146D, X146W, X146F, X146L, X146G, X147M, X149E, X150Q, X150E, X150C, X151C, X151E, X154Y, X154A, X158Y, X158N, X158H, X160W, X160D, X169E, X169A, X169V, X170F, X170C, X172C, X172K, X172D, X172N, X172A, X174P, X174M, X174E, X174D, X174T, X174S, X174H, X175G, X175Q, X175H, X175A, X184G, X191K, X191H, X191F, X191W, X191Y, X192A, X192T, X208I, X208Y, X208F, X210D, X210C, X210E, X210N, X210F, X210Y, X210M, X210Q, X210S, X210A, X224V, X224F, X242M, X249D, X249I, X253Y, X253G, X256C, X257Y, X257L, X257V, X268G, X279P, X297M, X297K, X297S, X297H, X297V, X297E, X297F, X302H, X302I, X302Q, X302Y, X302V, X303P, X303M, X303E, X303I, X303R, X303K, X303N, X303L, X303H, X303T, X304H, X304A, X304R, X304Y, X304D, X304N, X304M, X304E, X304P, X304W, X304K, X304T, X306M, X306E, X306A, X306G, X306W, X306V, X306S, X306I, X306H, X306T, X306D, X306Q, X306Y, X306R, X308S, X309H, X309L, X309Q, X311G, X311E, X311H, X311A, X311K, X311T, X311N, X311R, X311Y, X312M, X312L, X313V, X318T, X318S, X318I, X320G, X320Y, X320L, X320N, X320A, X320D, X320K, X320Q, X320E, X320H, X320C, X320S, X321E, X321W, X321T, X321A, X321N, X321V, X321K, X336K, X337N, X337K, X337M, X337T, X337V, X337Q, X337S, X337C, X337R, X337I, X337Y, X337G, X337F, X337A, X337L, X338G, X338T, X338S, X345N, X345T, X345M, X345G, X345S, X345D, X345Q, X358I, X358L, X358N, X358V, X358P, X370E, X370I, X375K, X375A, X375D, X375E, X375R, X375T, X375I, X375W, X375Y, X375Q, X376S, X376G, X376R, X376M, X376K, X376I, X376V, X376Q, X376L, X378E, X378R, X378D, X378C, X379L, X379A, X379S, X382N, X382H, X382Q, X382K, X382A, X382L, X382S, X383R, X383I, X383D, X383V, X383M, X383E, X383N, X383S, X383Q, X383Y, X383C, X383H, X397P, X397D, X397S, X397H, X398M, X401M, X401K, X401T, X401I, X405H, X405V, X405T, X405M, X405C, X406P, X416S, X418C, X418P, X418N, X421P, X421N, X434S, X442Q, X443T, X443H, X446P, X446I, X446V, X446F, X446L, X446R, X446Q, X446W, X448E, X448H, X448N, X448D, X455L, X457K, X457C, X457L, X457S, X457R, X457F, X457T, X457A, X457V, X457W, X457E, X457M, X457G, X458V, X458W, X458I, X458K, X459Q, X459D, X459I, X459K, X459C, X459V, X459E, X459R, X459N, X459Y, X461A, X461R, X461F, X461E, X461S, X461L, X461V, X461M, X461K, X461N, X461Q, X463L, X465P, X465V, X465H, X465R, X467A, X467E, X467H, X471E, X474P, X474H, and X474F according to the numbering of SEQ ID NO: 3.
Preferably, the one or more amino acid alteration, preferably substitution, is at amino acid positions located near the catalytic center of the alpha-amylase, preferably in a distance below 20 Angstrom from the catalytic center of the alpha-amylase. Preferably, the one or more amino acid alteration, preferably substitution, at an amino acid position located near the catalytic center of the alpha-amylase, preferably in a distance below 20 Angstrom from the catalytic center of the alpha-amylase, is selected from the group consisting of 14, 15, 16, 17, 18, 19, 20, 51, 54, 59, 60, 71, 72, 73, 76, 109, 123, 125, 126, 172, 173, 174, 175, 176, 192, 193, 203, 234, 236, 237, 238, 240, 268, 270, 333, 334, 337, 338, 341, and 342 according to the numbering of SEQ ID NO: 3. Preferably, the alpha-amylase of the present invention comprises one or more amino acid substitution at an amino acid positions located near the catalytic center of the alpha-amylase, preferably in a distance below 20 Angstrom from the catalytic center of the alpha-amylase, wherein the substitution at an amino acid positions located near the catalytic center of the alpha-amylase, preferably in a distance below 20 Angstrom from the catalytic center of the alpha-amylase is selected from the group consisting of X14N, X15R, X16F, X16R, X17K, X17V, X18R, X18M, X18F, X18K, X18T, X18I, X19P, X19L, X19F, X19Y, X19T, X19I, X19Q, X19C, X19H, X19S, X19M, X19K, X19G, X19D, X19A, X20C, X20S, X20L, X20M, X20T, X20D, X20Q, X20F, X20P, X20G, X20V, X20W, X20A, X20Y, X20H, X20K, X20E, X51V, X51Q, X51T, X54D, X54G, X54Q, X59T, X60T, X71D, X72T, X72E, X72D, X72N, X72C, X73Q, X73L, X73N, X76G, X76T, X76C, X76V, X76E, X76L, X109A, X123D, X123S, X125A, X125K, X125L, X125T, X125R, X125F, X125W, X125D, X125Q, X125V, X125Y, X125H, X125N, X125E, X125G, X125M, X126D, X172C, X172K, X172D, X172N, X172A, X173I, X173Y, X174P, X174M, X174E, X174D, X174T, X174S, X174H, X175G, X175Q, X175H, X175A, X176K, X192A, X192T, X193E, X203E, X203R, X234C, X236Y, X237C, X237I, X238T, X240M, X268G, X270A, X270Y, X333I, X334T, X337N, X337K, X337M, X337T, X337V, X337Q, X337S, X337C, X337R, X337I, X337Y, X337G, X337F, X337A, X337L, X338G, X338T, X338S, X341V, and X342P according to the numbering of SEQ ID NO: 3.
Preferably, the one or more amino acid alteration, preferably substitution, is at amino acid positions located near one of the Ca2+-binding sites of the alpha-amylase, preferably in a distance below 10 Angstrom from one of the Ca2+-binding sites of the alpha-amylase. Preferably, the one or more amino acid alteration, preferably substitution, at an amino acid position located near one of the Ca2+-binding sites of the alpha-amylase, preferably in a distance below 10 Angström from one of the Ca2+-binding sites of the alpha-amylase, is selected from the group consisting of 106, 108, 160, 161, 162, 164, 184, 188, 203, 208, 211, 212, 215, 234, 235, 236, 237, 238, 240, 297, 299, 301, 302, 303, 304, 306, 307, 308, 309, 310, 345, 346, 405, 406, 407, 408, 429, 431, and 474according to the numbering of SEQ ID NO: 3. Preferably, the alpha-amylase of the present invention comprises one or more amino acid substitution at an amino acid positions located near one of the Ca2+-binding sites of the alpha-amylase, preferably in a distance below 10 Angstrom from one of the Ca2+-binding sites of the alpha-amylase, wherein the substitution at an amino acid positions located near one of the Ca2+-binding sites of the alpha-amylase, preferably in a distance below 10 Angstrom from one of the Ca2+-binding sites of the alpha-amylase is selected from the group consisting of X106D, X108A, X160W, X160D, X161T, X162V, X164V, X184G, X188V, X188H, X203E, X203R, X208I, X208Y, X208F, X211D, X211N, X211C, X211T, X211E, X211G, X211V, X211Q, X211A, X211H, X211L, X211S, X212W, X212L, X212I, X212D, X212C, X215E, X215D, X215H, X215W, X215A, X234C, X235M, X235V, X235L, X236Y, X237C, X237I, X238T, X240M, X297M, X297K, X297S, X297H, X297V, X297E, X297F, X299L, X299G, X299I, X299K, X299S, X299Y, X301F, X302H, X302I, X302Q, X302Y, X302V, X303P, X303M, X303E, X303I, X303R, X303K, X303N, X303L, X303H, X303T, X304H, X304A, X304R, X304Y, X304D, X304N, X304M, X304E, X304P, X304W, X304K, X304T, X306M, X306E, X306A, X306G, X306W, X306V, X306S, X306I, X306H, X306T, X306D, X306Q, X306Y, X306R, X307F, X307M, X308S, X309H, X309L, X309Q, X310Q, X310A, X345N, X345T, X345M, X345G, X345S, X345D, X345Q, X346D, X346C, X346H, X346A, X346G, X346Q, X346N, X405H, X405V, X405T, X405M, X405C, X406P, X407S, X407R, X407D, X408Q, X408E, X408I, X429M, X429D, X429E, X429N, X429T, X429A, X429P, X429W, X429Q, X429S, X429F, X429G, X429I, X429V, X431I, X474P, X474H, and X474F according to the numbering of SEQ ID NO: 3.
Preferably, the alpha-amylase variant comprises 1 to 50, preferably 1 to 30 or 1 to 25 of any of the above cited amino acid alterations, preferably amino acid substitutions, compared to the parent alpha-amylase. Preferably, the alpha-amylase variant comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, or at least 25, but below 50, preferably below 30, preferably below 20 of the above cited substitutions compared to the parent alpha-amylase.
More preferably, the alpha-amylase variant wherein the number of the above cited substitutions is 1 to 30, preferably 1 to 25, 1 to 20, 1 to 15, 1 to 10, or 1 to 5 such as 1, 2, 3, 4, 5, 6, 7, 8, 9, 10, 11, 12, 13, 14, 15, 15, 16, 17, 18, 19, 20, 21, 22, 23, 24, or 25 substitutions. Further preferred, the alpha-amylase variant wherein the number of the above cited substitutions is 2 to 30, preferably 2 to 25, 2 to 20, 2 to 15 2 to 10, 2 to 8, or 2 to 5. Further preferred, the alpha-amylase variant wherein the number of the above cited substitutions is 3 to 30, preferably 3 to 25, 3 to 20, 3 to 15 3 to 10, 3 to 8, or 3 to 5. Further preferred, the alpha-amylase variant wherein the number of the above cited substitutions is 4 to 30, preferably 4 to 25, 4 to 20, 4 to 15, 4 to 10, or 4 to 8. Preferably, besides the above listed number of amino acid alterations, preferably substitutions, the alpha-amylase variant consists of the amino acid sequence shown in SEQ ID NO: 1. Preferably, the parent alpha-amylase for the alpha-amylase variant is an amylase according to SEQ ID NO: 1, SEQ ID NO: 3, or SEQ ID NO: 4, or any alpha-amylase having at least 60% sequence identity to SEQ IDNO: 1, SEQ ID NO: 3, or SEQ ID NO: 4, preferably the parent alpha-amylase for the alpha-amylase variant is an amylase according to according to SEQ ID NO: 1. In a preferred embodiment, the alpha-amylase variant according to the present invention comprises a combination of substitutions selected from the group consisting of: X25H+X176K+X186E+X206Y+X251 E+X482W, X4Q+X25H+X176K+X186E+X206Y+X251 E+X405M+X482W, X25H+X176K+X186E+X206Y+X482W, X25H+X186E+X206Y+X405M+X482W, X25H+X186E+X206Y+X482W, X25H+X176K+X186E+X193E+X206Y+X251E+X482W, X4Q+X176K+X186E+X193E+X206Y+X251 E+X405M+X482W, X25H+X176K+X186E+X482W, X25H+X176K+X186E+X193E+X206Y+X482W, X4Q+X25H+X176K+X186E+X251E+X405M+X482W, X25H+X176K+X186E+X206Y, X4Q+X176K+X186E+X193E+X206Y+X251E+X405M, X4Q+X206Y+X460G+X482W, X4Q+X25H+X176K+X206Y+X251E+X460G+X482W, X4Q+X25H+X176K+X193E+X251E+X186E+X482W, X4Q+X193E+X206Y+X276R+X186E+X482W, X186E+X25H+X482W, X25H+X193E+X206Y+X251E+X276R+X405M+X186E+X482W, X25H+X251 E+X186E+X482W, X25H+X160W+X176K+X186E+X251E+X405M+X482W, X193E+X206Y+X405M+X186E+X482W, X3I+X356V, X3I+X356I, X83D+X94E, X94D+X125E, X131I+X377Q+X410H, X48F+X94D, X48Y+X116K+X218K, X5L+X218K+X225S, X83E+X116R+X158Y+X181E, X51V+X218K, X83E+X181E, X4Q+X7W+X176K+X186E+X193E+X206Y+X251E+X405M, X4Q+X37M+X176K+X186E+X193E+X206Y+X251E+X405M, X4Q+X25D+X176K+X186E+X193E+X206Y+X251E+X405M, X4Q+X25H+X176K+X186E+X206Y+X251E+X405M+X460G, X4Q+X25H+X176K+X186E+X206Y+X251E+X460G, X4Q+X25Y+X176K+X186E+X193E+X206Y+X251E+X405M, X4Q+X118D+X176K+X186E+X193E+X206Y+X251E+X405M, X4Q+X176K+X182D+X186E+X193E+X206Y+X251E+X405M, X4Q+X176K+X186E+X193E+X206Y+X251E+X258Q+X405M, X4Q+X176K+X186E+X193E+X206Y+X251E+X405M, X4Q+X176K+X186E+X193E+X206Y+X251E+X405M+X460G, X4Q+X176K+X186E+X193E+X206Y+X251E+X363E+X405M, X4Q+X176K+X186E+X193E+X206Y+X251E+X363H+X405M, X4Q+X176K+X186E+X193E+X206Y+X251E+X363N+X405M, X4Q+X176K+X181D+X186E+X193E+X206Y+X251E+X405M, X4Q+X176K+X181F+X186E+X193E+X206Y+X251E+X405M, X4Q+X176K+X181N+X186E+X193E+X206Y+X251E+X405M, X4Q+X176K+X181Q+X186E+X193E+X206Y+X251E+X405M, X4Q+X136E+X176K+X186E+X193E+X206Y+X251E+X405M, X4Q+X100W+X176K+X186E+X193E+X206Y+X251E+X405M, X4Q+X135D+X176K+X186E+X193E+X206Y+X251E+X405M, X4Q+X135E+X176K+X186E+X193E+X206Y+X251E+X405M, X4Q+X135T+X176K+X186E+X193E+X206Y+X251E+X405M, X4Q+X135W+X176K+X186E+X193E+X206Y+X251E+X405M, X4Q+X160D+X176K+X186E+X193E+X206Y+X251E+X405M, X4Q+X160E+X176K+X186E+X193E+X206Y+X251E+X405M, X7H+X25H+X176K+X186E+X206Y,X7W+X25H+X176K+X186E+X206Y, X25D+X176K+X186E+X206Y, X25H+X186E+X206Y+X405M+X460G, X25H+X186E+X206Y+X460G,X25H+X37M+X176K+X186E+X206Y, X25H+X118D+X176K+X186E+X206Y,X25H+X176K+X182D+X186E+X206Y, X25H+X176K+X186E+X206Y,X25H+X176K+X186E+X206Y+X258Q, X25H+X176K+X186E+X206Y+X281N,X25H+X176K+X186E+X206Y+X460G, X25H+X176K+X186E+X206Y+X251E+X460G,X25H+X176K+X186E+X206Y+X363E, X25H+X176K+X186E+X206Y+X363H,X25H+X176K+X186E+X206Y+X363N, X25H+X176K+X186E+X460G, X25H+X176K+X186E+X193E+X206Y, X25H+X176K+X186E+X193E+X206Y+X460G, X25H+X176K+X186E+X193E+X206Y+X251E+X460G,X25H+X176K+X181D+X186E+X206Y, X25H+X176K+X181N+X186E+X206Y,X25H+X176K+X181Q+X186E+X206Y, X25H+X136E+X176K+X186E+X206Y,X25H+X100W+X176K+X186E+X206Y, X25H+X135E+X176K+X186E+X206Y,X25H+X135T+X176K+X186E+X206Y, X25H+X135W+X176K+X186E+X206Y, X25H+X160D+X176K+X186E+X206Y, X25H+X160E+X176K+X186E+X206Y,X25Y+X176K+X186E+X206Y, X87D+X186E+X315K+X420K,X87D+X186E+X226D+X314E+X420E+X461E, X87D+X186E+X226D+X420E,X87D+X186E+X226D+X420E+X461E, X87D+X186E+X314E+X471E,X87D+X186E+X311K+X314E+X420K, X87D+X186E+X160D+X461E,X87R+X186E+X315K+X461R,X87R+X186E+X226D+X471E, X87R+X186E+X226R+X314K+X420K+X461R,X87R+X186E+X226R+X311K+X420K, X87R+X186E+X314K+X315K,X87R+X186E+X311K+X314E,X87R+X186E+X420E+X450R, X87R+X186E+X450R+X452R,X186E+X315K+X420E+X452R, X186E+X226D+X314E+X461E+X471E,X186E+X226D+X314E+X452R, X186E+X226D+X314K+X460D+X471E+X485R,X186E+X226D+X311K+X460D, X186E+X226D+X461E+X471E,X186E+X314E+X460D+X461E, X186E+X314E+X420E+X452R+X461E,X186E+X314K+X450R+X460D, X186E+X460D+X471E+X485R,X186E+X311K+X314K+X452R, X186E+X311K+X461E+X485R, X186E+X311K+X420E+X471E, X186E+X420K+X450R+X471E+X485R,X186E+X420K+X450R+X485R, X186E+X452R+X461E+X471E,X4Q+X176K+X186E+X193E+X206Y+X251E+X405M, X25H+X176K+X186E+X206Y,X83E+X186E+X219D+X226R, X83E+X186E+X219R+X226D+X314E+X452R,X83E+X186E+X219R+X226R, X83E+X186E+X160D+X219D+X226R+X452R,X83R+X186E+X219R+X226D, X160D+X186E+X315K+X450R,X160D+X186E+X226D+X314K+X460D+X461E, X160D+X186E+X226D+X314K+X420E,X160D+X186E+X314K+X485R, X160D+X186E+X420E+X452R,X160D+X186E+X420K+X460D,X186E+X276R, X186E+X206Y,X186E+X206Y+X276R,X186E+X206Y+X276R+X405M, X186E+X206Y+X405M,X186E+X206Y+X251E,X186E+X206Y+X251E+X276R, X186E+X206Y+X251E+X276R+X405M,X186E+X206Y+X251E+X405M, X186E+X206Y+X251E+X323G+X405M, X186E+X206Y+X323G+X405M, X186E+X405M, X186E+X193E+X206Y+X405M,X186E+X193E+X206Y+X251E,X186E+X251E, X186E+X251E+X405M, X4Q+X186E, X4Q+X186E+X276R, X4Q+X186E+X206Y, X4Q+X186E+X206Y+X276R+X405M, X4Q+X186E+X206Y+X405M, X4Q+X186E+X206Y+X251E+X276R, X4Q+X186E+X206Y+X251E+X276R+X405M, X4Q+X186E+X206Y+X251E+X405M, X4Q+X186E+X206Y+X251E+X323G+X405M, X4Q+X186E+X405M, X4Q+X186E+X193E+X206Y+X276R, X4Q+X186E+X193E+X206Y+X405M,X4Q+X186E+X193E+X206Y+X251E+X405M, X4Q+X186E+X251E+X276R+X405M, X4Q+X25H+X186E+X206Y, X4Q+X25H+X186E+X206Y+X276R, X4Q+X25H+X186E+X206Y+X276R+X405M, X4Q+X25H+X186E+X206Y+X405M, X4Q+X25H+X186E+X206Y+X251E+X323A+X405M, X4Q+X25H+X186E+X206Y+X323G+X405M, X4Q+X25H+X186E+X405M, X4Q+X25H+X176K+X186E+X206Y+X276R, X4Q+X25H+X176K+X186E+X206Y+X276R+X405M, X4Q+X25H+X176K+X186E+X206Y+X251E+X276R+X405M, X4Q+X25H+X176K+X186E+X206Y+X251E+X405M, X4Q+X25H+X176K+X186E+X251E, X4Q+X25H+X176K+X186E+X251E+X405M, X4Q+X25H+X176K+X206Y, X4Q+X25H+X176K+X206Y+X405M N460G, X4Q+X25H+X176K+X206Y+X460G, X4Q+X25H+X176K+X206Y+X251E, X4Q+X25H+X176K+X206Y+X251E+X276R+X405M, X4Q+X25H+X176K+X206Y+X251E+X460G, X4Q+X25H+X176K+X206Y+X323G, X4Q+X25H+X160W+X186E+X206Y+X405M, X4Q+X25H+X160W+X186E+X206Y+X251E, X4Q+X25H+X160W+X186E+X206Y+X251E+X276R+X405M, X4Q+X25H+X160W+X186E+X206Y+X323G+X405M, X4Q+X25H+X160W+X186E+X405M, X4Q+X25H+X160W+X186E+X193E+X206Y+X276R, X4Q+X25H+X160W+X186E+X323G, X4Q+X25H+X160W+X169R+X186E+X206Y+X276R, X4Q+X25H+X160W+X176K+X186E+X276R+X405M, X4Q+X25H+X160W+X176K+X186E+X206Y+X276R, X4Q+X25H+X160W+X176K+X186E+X206Y+X251E, X4Q+X25H+X160W+X176K+X186E+X206Y+X251E+X405M, X4Q+X25H+X160W+X176K+X186E+X405M, X4Q+X25H+X160W+X176K+X186E+X251E+X276R+X405M, X4Q+X25H+X160W+X176K+X186E+X251E+X405M, X4Q+X25H+X160W+X176K+X206Y, X4Q+X25H+X160W+X176K+X206Y+X405M, X4Q+X25H+X160W+X176K+X206Y+X460G, X4Q+X25H+X160W+X176K+X206Y+X251E+X460G, X4Q+X176K+X186E+X276R+X405M, X4Q+X176K+X186E+X206Y+X276R, X4Q+X176K+X186E+X206Y+X276R+X405M, X4Q+X176K+X186E+X206Y+X405M, X4Q+X176K+X186E+X206Y+X251E+X276R+X405M, X4Q+X176K+X186E+X206Y+X251E+X405M, X4Q+X176K+X186E+X405M, X4Q+X176K+X186E+X193E+X206Y+X276R+X405M, X4Q+X176K+X186E+X193E+X206Y+X251E+X405M, X4Q+X176K+X186E+X251E+X276R+X405M, X4Q+X176K+X186E+X251E+X405M, X4Q+X176K+X186E+X323G+X405M,X4Q+X176K+X206Y, X4Q+X176K+X206Y+X276R, X4Q+X176K+X206Y+X405M, X4Q+X176K+X206Y+X405M N460G, X4Q+X176K+X206Y+X460G, X4Q+X176K+X206Y+X251E+X276R+X405M, X4Q+X176K+X206Y+X251E+X276R+X405M N460G, X4Q+X176K+X206Y+X251E+X405M, X4Q+X176K+X206Y+X251E+X405M N460G, X4Q+X176K+X206Y+X251E+X323A+X460G, X4Q+X160W+X186E+X276R+X405M, X4Q+X160W+X186E+X206Y, X4Q+X160W+X186E+X206Y+X276R+X323G, X4Q+X160W+X186E+X206Y+X405M, X4Q+X160W+X186E+X206Y+X251E,X4Q+X160W+X186E+X206Y+X251E+X276R+X405M, X4Q+X160W+X186E+X405M, X4Q+X160W+X186E+X251E+X405M, X4Q+X160W+X176K+X186E, X4Q+X160W+X176K+X186E+X276R+X405M, X4Q+X160W+X176K+X186E+X206Y+X251E, X4Q+X160W+X176K+X186E+X251E+X276R+X405M, X4Q+X160W+X176K+X186E+X251E+X405M, X4Q+X160W+X176K+X206Y, X4Q+X160W+X176K+X206Y+X251E+X276R+X460G, X4Q+X160W+X176K+X206Y+X251E+X323G+X405M+X460G, X25H+X186E, X25H+X186E+X206Y,X25H+X186E+X206Y+X276R+X405M, X25H+X186E+X206Y+X276R+X323G,X25H+X186E+X206Y+X405M, X25H+X186E+X206Y+X251E+X276R+X323G+X405M, X25H+X186E+X206Y+X251E+X405M, X25H+X186E+X405M, X25H+X186E+X2021+X206Y+X405M, X25H+X186E+X193E+X206Y+X276R+X405M, X25H+X186E+X193E+X206Y+X405M, X25H+X186E+X193E+X206Y+X251E+X276R+X405M, X25H+X186E+X251E, X25H+X186E+X251E+X276R,X25H+X176K+X186E, X25H+X176K+X186E+X276R+X323G+X405M, X25H+X176K+X186E+X206Y, X25H+X176K+X186E+X206Y+X276R, X25H+X176K+X186E+X206Y+X251E, X25H+X176K+X186E+X206Y+X251E+X276R+X405M, X25H+X176K+X186E+X206Y+X251E+X405M, X25H+X176K+X186E+X405M, X25H+X176K+X186E+X193E+X206Y, X25H+X176K+X186E+X193E+X206Y+X251E, X25H+X176K+X186E+X193E+X206Y+X251E+X276R+X405M, X25H+X176K+X186E+X251E+X276R, X25H+X176K+X206Y, X25H+X176K+X206Y+X276R, X25H+X176K+X206Y+X276R+X405M, X25H+X176K+X206Y+X276R+X460G, X25H+X176K+X206Y+X405M, X25H+X176K+X206Y+X460G, X25H+X176K+X206Y+X251E, X25H+X176K+X206Y+X251E+X276R+X405M+X460G, X25H+X176K+X206Y+X251E+X276R+X460G, X25H+X176K+X206Y+X251E+X405M, X25H+X160W+X186E+X206Y, X25H+X160W+X186E+X206Y+X251E, X25H+X160W+X186E+X206Y+X251E+X276R, X25H+X160W+X186E+X206Y+X251E+X276R+X323G+X405M, X25H+X160W+X186E+X206Y+X251E+X405M, X25H+X160W+X186E+X206Y+X323G+X405M, X25H+X160W+X186E+X251E+X276R+X405M, X25H+X160W+X176K+X186E+X206Y+X276R, X25H+X160W+X176K+X186E+X206Y+X276R+X405M, X25H+X160W+X176K+X186E+X206Y+X251E+X276R+X323G, X25H+X160W+X176K+X186E+X206Y+X251E+X405M, X25H+X160W+X176K+X186E+X405M, X25H+X160W+X176K+X186E+X193E+X206Y, X25H+X160W+X176K+X186E+X193E+X206Y+X405M, X25H+X160W+X176K+X186E+X193E+X206Y+X251E+X405M, X25H+X160W+X176K+X186E+X251E, X25H+X160W+X176K+X186E+X251E+X276R, X25H+X160W+X176K+X186E+X251E+X405M, X25H+X160W+X176K+X186E+X323G+X405M, X25H+X160W+X176K+X206Y+X276R+X323G+X405M, X25H+X160W+X176K+X206Y+X460G, X25H+X160W+X176K+X206Y+X251E, X25H+X160W+X176K+X206Y+X251E+X323G+X405M,X176K+X186E+X206Y, X176K+X186E+X206Y+X276R, X176K+X186E+X206Y+X276R+X405M, X176K+X186E+X206Y+X405M, X176K+X186E+X206Y+X251E+X405M, X176K+X186E+X405M,X176K+X186E+X193E+X206Y+X405M, X176K+X186E+X193E+X206Y+X251E+X405M, X176K+X186E+X193E+X405M, X176K+X186E+X251E+X276R+X405M, X176K+X186E+X251E+X405M, X176K+X206Y, X176K+X206Y+X276R, X176K+X206Y+X276R+X460G, X176K+X206Y+X405M, X176K+X206Y+X405M N460G, X176K+X206Y+X460G, X176K+X206Y+X251E, X176K+X206Y+X251E+X405M, X160W+X186E, X160W+X186E+X276R+X405M, X160W+X186E+X206Y+X276R, X160W+X186E+X206Y+X276R+X405M, X160W+X186E+X206Y+X405M, X160W+X186E+X206Y+X251E, X160W+X186E+X206Y+X251E+X405M, X160W+X186E+X405M, X160W+X186E+X193E+X206Y, X160W+X186E+X193E+X206Y+X405M, X160W+X186E+X251E+X276R, X160W+X186E+X251 E+X276R+X405M, X160W+X186E+X323G+X405M, X160W+X176K+X186E+X276R+X405M, X160W+X176K+X186E+X206Y, X160W+X176K+X186E+X206Y+X251E+X405M, X160W+X176K+X186E+X405M, X160W+X176K+X186E+X193E+X206Y, X160W+X176K+X186E+X193E+X206Y+X405M, X160W+X176K+X186E+X193E+X206Y+X251E, X160W+X176K+X206Y+X276R+X405M N460G, X160W+X176K+X206Y+X276R+X323G+X460G, X160W+X176K+X206Y+X405M, X160W+X176K+X206Y+X405M N460G, X160W+X176K+X206Y+X460G, X160W+X176K+X206Y+X251E+X276R, X160W+X176K+X206Y+X251E+X405M+X460G, X160W+X176K+X206Y+X323G+X405M+X460G, X4Q+X176K+X186E+X193E+X206Y+X251E+X405M, X4Q+X176K+X186E+X193E+X206Y+X251E+X405M+X258Q, X4Q+X176K+X186E+X193E+X206Y+X251 E+X405M+X258Q+X281 N, X4Q+X176K+X186E+X193E+X206Y+X251 E+X405M+X258Q+X281 N+X363H, X4Q+X176K+X186E+X193E+X206Y+X251E+X405M+X258Q+X363E, X4Q+X176K+X186E+X193E+X206Y+X251E+X405M+X281N, X4Q+X176K+X186E+X193E+X206Y+X251E+X405M+X281N+X363E, X4Q+X176K+X186E+X193E+X206Y+X251 E+X405M+X281 N+X363H, X4Q+X176K+X186E+X193E+X206Y+X251E+X405M+X181Q, X4Q+X176K+X186E+X193E+X206Y+X251E+X405M+X181Q+X258Q, X4Q+X176K+X186E+X193E+X206Y+X251E+X405M+X181Q+X258Q+X281N, X4Q+X176K+X186E+X193E+X206Y+X251E+X405M+X181Q+X258Q+X281N+X363H, X4Q+X176K+X186E+X193E+X206Y+X251E+X405M+X181Q+X281N, X4Q+X176K+X186E+X193E+X206Y+X251E+X405M+X181Q+X281N+X363E, X4Q+X176K+X186E+X193E+X206Y+X251E+X405M+X181Q+X363E, X4Q+X176K+X186E+X193E+X206Y+X251E+X405M+X181Q+X363H, X4Q+X176K+X186E+X193E+X206Y+X251E+X405M+X100W, X4Q+X176K+X186E+X193E+X206Y+X251E+X405M+X100W+X258Q, X4Q+X176K+X186E+X193E+X206Y+X251E+X405M+X100W+X258Q+X281N, X4Q+X176K+X186E+X193E+X206Y+X251E+X405M+X100W+X258Q+X281N+X363E, X4Q+X176K+X186E+X193E+X206Y+X251E+X405M+X100W+X258Q+X281N+X363H, X4Q+X176K+X186E+X193E+X206Y+X251E+X405M+X100W+X258Q+X281N+X363H+X272V, X4Q+X176K+X186E+X193E+X206Y+X251E+X405M+X100W+X258Q+X363E, X4Q+X176K+X186E+X193E+X206Y+X251E+X405M+X100W+X258Q+X363H, X4Q+X176K+X186E+X193E+X206Y+X251E+X405M+X100W+X281N, X4Q+X176K+X186E+X193E+X206Y+X251E+X405M+X100W+X281N+X363E, X4Q+X176K+X186E+X193E+X206Y+X251E+X405M+X100W+X281N+X363H, X4Q+X176K+X186E+X193E+X206Y+X251E+X405M+X100W+X181Q, X4Q+X176K+X186E+X193E+X206Y+X251E+X405M+X100W+X181Q+X258Q, X4Q+X176K+X186E+X193E+X206Y+X251E+X405M+X100W+X181Q+X258Q+X281N, X4Q+X176K+X186E+X193E+X206Y+X251E+X405M+X100W+X181Q+X258Q+X281N+X363E, X4Q+X176K+X186E+X193E+X206Y+X251E+X405M+X100W+X181Q+X258Q+X363E, X4Q+X176K+X186E+X193E+X206Y+X251E+X405M+X100W+X181Q+X258Q+X363H, X4Q+X176K+X186E+X193E+X206Y+X251E+X405M+X100W+X181Q+X281N, X4Q+X176K+X186E+X193E+X206Y+X251E+X405M+X100W+X181Q+X281N+X363H, X4Q+X176K+X186E+X193E+X206Y+X251E+X405M+X100W+X181Q+X363H, X4Q+X176K+X186E+X193E+X206Y+X251E+X405M+X100W+X181Q+X363H+X254Q, X4Q+X176K+X186E+X193E+X206Y+X251E+X405M+X100W+X135T, X4Q+X176K+X186E+X193E+X206Y+X251E+X405M+X100W+X135T+X258Q, X4Q+X176K+X186E+X193E+X206Y+X251E+X405M+X100W+X135T+X258Q+X281N+X363H, X4Q+X176K+X186E+X193E+X206Y+X251E+X405M+X100W+X135T+X258Q+X363E, X4Q+X176K+X186E+X193E+X206Y+X251E+X405M+X100W+X135T+X258Q+X363H, X4Q+X176K+X186E+X193E+X206Y+X251E+X405M+X100W+X135T+X181Q, X4Q+X176K+X186E+X193E+X206Y+X251E+X405M+X100W+X135T+X181Q+X258Q, X4Q+X176K+X186E+X193E+X206Y+X251E+X405M+X100W+X135T+X181Q+X258Q+X281N, X4Q+X176K+X186E+X193E+X206Y+X251E+X405M+X100W+X135T+X181Q+X258Q+X363E, X4Q+X176K+X186E+X193E+X206Y+X251E+X405M+X100W+X135T+X181Q+X258Q+X363H, X4Q+X176K+X186E+X193E+X206Y+X251E+X405M+X100W+X135T+X181Q+X281N+X363H, X4Q+X176K+X186E+X193E+X206Y+X251E+X405M+X100W+X135T+X181Q+X363E, X4Q+X176K+X186E+X193E+X206Y+X251E+X405M+X100W+X135T+X181Q+X363H, X4Q+X176K+X186E+X193E+X206Y+X251E+X405M+X100W+X135T+X363E, X4Q+X176K+X186E+X193E+X206Y+X251E+X405M+X100W+X135T+X363H, X4Q+X176K+X186E+X193E+X206Y+X251E+X405M+X100W+X363E, X4Q+X176K+X186E+X193E+X206Y+X251E+X405M+X100W+X363H, X4Q+X176K+X186E+X193E+X206Y+X251E+X405M+X135T, X4Q+X176K+X186E+X193E+X206Y+X251E+X405M+X135T+X258Q, X4Q+X176K+X186E+X193E+X206Y+X251E+X405M+X135T+X258Q+X8A, X4Q+X176K+X186E+X193E+X206Y+X251E+X405M+X135T+X258Q+X281N+X363E, X4Q+X176K+X186E+X193E+X206Y+X251E+X405M+X135T+X258Q+X363H, X4Q+X176K+X186E+X193E+X206Y+X251E+X405M+X135T+X281N, X4Q+X176K+X186E+X193E+X206Y+X251E+X405M+X135T+X181Q+X258Q, X4Q+X176K+X186E+X193E+X206Y+X251E+X405M+X135T+X181Q+X258Q+X281N, X4Q+X176K+X186E+X193E+X206Y+X251E+X405M+X135T+X181Q+X258Q+X363H, X4Q+X176K+X186E+X193E+X206Y+X251E+X405M+X135T+X363E, X4Q+X176K+X186E+X193E+X206Y+X251E+X405M+X135T+X363H, X4Q+X176K+X186E+X193E+X206Y+X251E+X405M+X363E, X4Q+X176K+X186E+X193E+X206Y+X251E+X405M+X363H,X25H+X176K+X186E+X206Y, X25H+X176K+X186E+X206Y+X258Q,X25H+X176K+X186E+X206Y+X258Q, X25H+X176K+X186E+X206Y+X258Q+X281N, X25H+X176K+X186E+X206Y+X258Q+X281N+X363E, X25H+X176K+X186E+X206Y+X258Q+X363E, X25H+X176K+X186E+X206Y+X258Q+X363E+X123D,X25H+X176K+X186E+X206Y+X281N, X25H+X176K+X186E+X206Y+X181Q,X25H+X176K+X186E+X206Y+X181Q+X193E, X25H+X176K+X186E+X206Y+X181Q+X363E,X25H+X176K+X186E+X206Y+X193E, X25H+X176K+X186E+X206Y+X193E+X258Q, X25H+X176K+X186E+X206Y+X193E+X281N+X363E, X25H+X176K+X186E+X206Y+X193E+X363E,X25H+X176K+X186E+X206Y+X100W, X25H+X176K+X186E+X206Y+X100W+X258Q, X25H+X176K+X186E+X206Y+X100W+X258Q+X281N, X25H+X176K+X186E+X206Y+X100W+X258Q+X281N+X363E, X25H+X176K+X186E+X206Y+X100W+X258Q+X363E, X25H+X176K+X186E+X206Y+X100W+X258Q+X363E+X135C, X25H+X176K+X186E+X206Y+X100W+X281N, X25H+X176K+X186E+X206Y+X100W+X281N+X363E, X25H+X176K+X186E+X206Y+X100W+X361R, X25H+X176K+X186E+X206Y+X100W+X181Q+X281N+X363E, X25H+X176K+X186E+X206Y+X100W+X181Q+X281N+X363E+X175D, X25H+X176K+X186E+X206Y+X100W+X181Q+X193E, X25H+X176K+X186E+X206Y+X100W+X181Q+X193E+X258Q+X281N, X25H+X176K+X186E+X206Y+X100W+X181Q+X193E+X258Q+X363E, X25H+X176K+X186E+X206Y+X100W+X181Q+X193E+X281N+X363E, X25H+X176K+X186E+X206Y+X100W+X181Q+X193E+X363E, X25H+X176K+X186E+X206Y+X100W+X181Q+X363E, X25H+X176K+X186E+X206Y+X100W+X193E, X25H+X176K+X186E+X206Y+X100W+X193E+X258Q, X25H+X176K+X186E+X206Y+X100W+X193E+X258Q+X473R, X25H+X176K+X186E+X206Y+X100W+X193E+X258Q+X281N, X25H+X176K+X186E+X206Y+X100W+X193E+X258Q+X281N+X363E, X25H+X176K+X186E+X206Y+X100W+X193E+X258Q+X363E, X25H+X176K+X186E+X206Y+X100W+X193E+X363E, X25H+X176K+X186E+X206Y+X100W+X135T, X25H+X176K+X186E+X206Y+X100W+X135T+X258Q, X25H+X176K+X186E+X206Y+X100W+X135T+X258Q+X281N, X25H+X176K+X186E+X206Y+X100W+X135T+X258Q+X281N+X363E, X25H+X176K+X186E+X206Y+X100W+X135T+X258Q+X363E, X25H+X176K+X186E+X206Y+X100W+X135T+X281N, X25H+X176K+X186E+X206Y+X100W+X135T+X281N+X363E, X25H+X176K+X186E+X206Y+X100W+X135T+X181Q, X25H+X176K+X186E+X206Y+X100W+X135T+X181Q+X258Q+X281N+X363E, X25H+X176K+X186E+X206Y+X100W+X135T+X181Q+X281N+X363E, X25H+X176K+X186E+X206Y+X100W+X135T+X181Q+X193E, X25H+X176K+X186E+X206Y+X100W+X135T+X181Q+X193E+X258Q+X363E, X25H+X176K+X186E+X206Y+X100W+X135T+X181Q+X193E+X363E, X25H+X176K+X186E+X206Y+X100W+X135T+X193E, X25H+X176K+X186E+X206Y+X100W+X135T+X193E+X258Q+X363E, X25H+X176K+X186E+X206Y+X100W+X135T+X193E+X363E, X25H+X176K+X186E+X206Y+X100W+X135T+X363E, X25H+X176K+X186E+X206Y+X100W+X363E,X25H+X176K+X186E+X206Y+X135T, X25H+X176K+X186E+X206Y+X135T+X258Q, X25H+X176K+X186E+X206Y+X135T+X258Q+X281N, X25H+X176K+X186E+X206Y+X135T+X258Q+X281N+X363E,and X25H+X176K+X186E+X206Y+X135T+X258Q+X363E
wherein the numbering is according to the amino acid sequence set forth in SEQ ID NO: 3 and wherein said variant has alpha-amylase activity. Preferably, the amino acid residue at the above cited positions (i.e., X) corresponds to the amino acid residue shown in SEQ ID NO: 1 or 3, preferably in SEQ ID NO: 1, at the respective position (according to the numbering of SEQ ID NO: 3).
In a particularly preferred embodiment, the alpha-amylase variant according to the present invention comprises a combination of substitutions selected from the group consisting of: N25H+R176K+G186E+I206Y+R181Q, N25H+R176K+G186E+I206Y+Y135T, N25H+R176K+G186E+I206Y+Y100W+Y363E, N25H+R176K+G186E+I206Y+Y100W+T193E, N25H+R176K+G186E+I206Y+T251E, G4Q+N25H+R176K+G186E+I206Y+T251E+L405M, N25H+R176K+G186E+1206Y, N25H+G186E+1206Y+L405M, N25H+G186E+1206Y, N25H+R176K+G186E+T193E+I206Y+T251E, G4Q+R176K+G186E+T193E+I206Y+T251E+L405M, N25H+R176K+G186E, N25H+R176K+G186E+T193E+I206Y, G4Q+N25H+R176K+G186E+T251E+L405M, N25H+R176K+G186E+1206Y, G4Q+R176K+G186E+T193E+I206Y+T251E+L405M, G4Q+l206Y+N460G, G4Q+N25H+R176K+I206Y+T251E+N460G, G4Q+N25H+R176K+T193E+T251E+G186E, G4Q+T193E+I206Y+E276R+G186E, G186E+N25H, N25H+T193E+I206Y+T251E+E276R+L405M+G186E, N25H+T251E+G186E, N25H+Y160W+R176K+G186E+T251E+L405M, T193E+1206Y+L405M+G186E, N25H+R176K+G186E+I206Y+G258Q+Y363E, N25H+R176K+G186E+I206Y+R181Q, N25H+R176K+G186E+I206Y+Y100W, N25H+R176K+G186E+I206Y+Y100W+Q359R, N25H+R176K+G186E+I206Y+Y100W+G258Q, N25H+R176K+G186E+I206Y+Y100W+G258Q+Y363E, N25H+R176K+G186E+I206Y+Y100W+Y135T, N25H+R176K+G186E+I206Y+Y100W+Y135T+Y363E, N25H+R176K+G186E+I206Y+Y135T, N25H+R176K+G186E+I206Y+Y135T+G258Q, N25H+R176K+G186E+I206Y+Y135T+G258Q+Y363E, N25H+R176K+G186E+I206Y+Y135T+Y363E, N25H+R176K+G186E+I206Y+Y363E, N25H+R176K+G186E+I206Y+T251E+Y482W, N25H+R176K+G186E+I206Y+Y482W, N25H+R176K+G186E+T193E+I206Y+T251E+Y482W, G4Q+R176K+G186E+T193E+I206Y+T251E+L405M+Y482W, G4Q+N25H+R176K+G186E+T251E+L405M+Y482W, N25H+R176K+G186E+T193E+I206Y+Y482W, G4Q+N25H+R176K+G186E+I206Y+T251E+L405M+Y482W, N25H+G186E+I206Y+L405M+Y482W, N25H+R176K+G186E+Y482W, N25H+R176K+G186E+T193E+I206Y+T251E+N460G, G4Q+N25H+R176K+G186E+I206Y+T251E+N460G, N25H+R176K+G186E+T193E+I206Y+T251E+N460G+Y482W, G4Q+N25H+R176K+G186E+I206Y+T251E+N460G+Y482W, N25H+R176K+G186E+N195F+T251E+Y482W, N25H+R176K+G186E+N195F+Y482W, N25H+R176K+G186E+T193E+N195F+T251E+Y482W, G4Q+R176K+G186E+T193E+N195F+T251E+L405M+Y482W, N25H+R176K+G186E+T193E+N195F+Y482W, G4Q+N25H+R176K+G186E+N195F+T251E+L405M+Y482W, N25H+G186E+N195F+L405M+Y482W, N25H+R176K+G186E+T193E+N195F+T251E+N460G, G4Q+N25H+R176K+G186E+N195F+T251E+N460G, N25H+R176K+G186E+T193E+N195F+T251E+N460G+Y482W, and G4Q+N25H+R176K+G186E+N195F+T251E+N460G+Y482W
wherein the numbering is according to the amino acid sequence set forth in SEQ ID NO: 3 and wherein said variant has alpha-amylase activity. Preferably, the amino acid residue at the above cited positions (i.e., X) corresponds to the amino acid residue shown in SEQ ID NO: 1 or 3, preferably in SEQ ID NO: 1, at the respective position (according to the numbering of SEQ ID NO: 3).
In another embodiment, the alpha-amylase variant according to the present invention having alpha-amylase activity comprises or consists of the amino acid sequence set forth in SEQ ID NO: 1 with 1 to 50 of the herein cited amino acid alterations, preferably 1 to 30, 1 to 25, 1 to 20, 1 to 15, 1 to 10, or 1 to 5, 2 to 30, 2 to 25, 2 to 20, 2 to 15 2 to 10, 2 to 8, or 2 to 5, preferably 3 to 30, 3 to 25, 3 to 20, 3 to 15 3 to 10, 3 to 8, or 3 to 5, preferably, 4 to 30, 4 to 25, 4 to 20, 4 to 15, 4 to 10, or 4 to 8 amino acid alterations, preferably substitutions. Preferably, wherein the alpha-amylase variant according to the present invention having alpha-amylase activity comprises or consists of the amino acid sequence set forth in SEQ ID NO: 1 with 1 to 50 of the above cited amino acid substitutions, preferably 1 to 30, 1 to 25, 1 to 20, 1 to 15, 1 to 10, or 1 to 5, 2 to 30, 2 to 25, 2 to 20, 2 to 15 2 to 10, 2 to 8, or 2 to 5, preferably 3 to 30, 3 to 25, 3 to 20, 3 to 15 3 to 10, 3 to 8, or 3 to 5, preferably, 4 to 30, 4 to 25, 4 to 20, 4 to 15, 4 to 10, or 4 to 8 amino acid substitutions and further comprises 1 to 50, preferably 1 to 30, 1 to 25, 1 to 20, 1 to 15, 1 to 10, or 1 to 5, 2 to 30, 2 to 25, 2 to 20, 2 to 15 2 to 10, 2 to 8, or 2 to 5, preferably 3 to 30, 3 to 25, 3 to 20, 3 to 15 3 to 10, 3 to 8, or 3 to 5, preferably, 4 to 30, 4 to 25, 4 to 20, 4 to 15, 4 to 10, or 4 to 8 conservative amino acid exchanges.
In another embodiment, the alpha-amylase variant according to the present invention having alpha-amylase activity comprises or consists of the amino acid sequence set forth in SEQ ID NO: 1 with 1 to 50 amino acid alterations, preferably 1 to 30, 1 to 25, 1 to 20, 1 to 15, 1 to 10, or 1 to 5, 2 to 30, 2 to 25, 2 to 20, 2 to 15 2 to 10, 2 to 8, or 2 to 5, preferably 3 to 30, 3 to 25, 3 to 20, 3 to 15 3 to 10, 3 to 8, or 3 to 5, preferably, 4 to 30, 4 to 25, 4 to 20, 4 to 15, 4 to 10, or 4 to 8 amino acid substitutions, wherein said amino acid substitutions are selected from the group consisting of X100D, X100F, X100K, X100L, X103A, X106D, X108A, X109A, X10A, X10C, X10E, X10F, X10L, X10W, X10Y, X113F, X113H, X113M, X113R, X113V, X113W, X113Y, X114A, X114C, X114D, X114E, X114F, X114G, X114H, X114I, X114K, X114L, X114M, X114N, X114Q, X114R, X114S, X114V, X114W, X114Y, X115C, X115D, X115K, X115M, X115N, X115Q, X115R, X115S, X115T, X115V, X116I, X116K, X116L, X116M, X116R, X116T, X117A, X117C, X117D, X117F, X117I, X117N, X117P, X117R, X117W, X117Y, X118A, X118D, X118E, X119H, X119P, X119R, X119S, X119Y, X120E, X120G, X120M, X120S, X120W, X120Y, X123D, X123S, X125A, X125D, X125E, X125F, X125G, X125H, X125K, X125L, X125M, X125N, X125Q, X125R, X125T, X125V, X125W, X125Y, X126D, X128C, X128E, X128L, X128M, X128W, X128Y, X129E, X12N, X12S, X131C, X131I, X131L, X131Q, X131W, X132T, X133A, X133C, X133D, X133E, X133H, X133K, X133N, X133P, X133Q, X133S, X134C, X134E, X134F, X134I, X134L, X134M, X134P, X134T, X134V, X134W, X134Y, X135D, X135E, X135G, X135M, X135N, X135P, X135S, X135T, X135W, X136A, X136C, X136D, X136E, X136F, X136H, X136L, X136M, X136N, X136P, X136W, X139C, X139S, X13A, X13Y, X141V, X142C, X142E, X142F, X142L, X142M, X142Q, X142R, X142W, X142Y, X143F, X144C, X144E, X144G, X144K, X144N, X144Q, X144R, X144S, X144T, X144V, X144Y, X145A, X146C, X146D, X146E, X146F, X146G, X146H, X146K, X146L, X146S, X146T, X146W, X147M, X149E, X14N, X150C, X150E, X150Q, X151C, X151E, X154A, X154Y, X155Y, X156E, X156V, X158H, X158N, X158Y, X15R, X160D, X160E, X160W, X161T, X162V, X163A, X163Q, X163T, X164V, X165S, X165T, X165W, X169A, X169D, X169E, X169S, X169V, X16F, X16R, X170C, X170F, X172A, X172C, X172D, X172K, X172N, X173I, X173Y, X174D, X174E, X174G, X174H, X174M, X174N, X174P, X174S, X174T, X175A, X175G, X175H, X175Q, X176K, X176S, X176T, X177A, X177G, X177K, X177N, X177P, X177R, X177S, X177W, X178F, X17K, X17V, X180M, X180N, X180T, X180W, X182D, X182E, X182N, X182Q, X182S, X184G, X185E, X185M, X185N, X187A, X187D, X187M, X187V, X188H, X188T, X188V, X189I, X18F, X18I, X18K, X18M, X18R, X18T, X191F, X191H, X191K, X191M, X191W, X191Y, X192A, X192T, X193D, X193E, X193G, X193V, X19A, X19C, X19D, X19F, X19G, X19H, X19I, X19K, X19L, X19M, X19P, X19Q, X19S, X19T, X19Y, X1R, X1V, X203E, X203R, X208F, X208I, X208Y, X20A, X20C, X20D, X20E, X20F, X20G, X20H, X20K, X20L, X20M, X20N, X20P, X20Q, X20S, X20T, X20V, X20W, X20Y, X210A, X210C, X210D, X210E, X210F, X210M, X210N, X210Q, X210S, X210Y, X211A, X211C, X211D, X211E, X211G, X211H, X211L, X211N, X211Q, X211S, X211T, X211V, X212C, X212D, X212I, X212L, X212W, X213A, X213C, X213M, X213R, X213S, X214E, X214P, X215A, X215D, X215E, X215H, X215W, X216G, X216H, X218A, X218C, X218D, X218E, X218F, X218G, X218H, X218I, X218K, X218L, X218N, X218Q, X218S, X218T, X218V, X218W, X218Y, X219A, X219C, X219D, X219E, X219F, X219G, X219H, X219K, X219M, X219Q, X219R, X219S, X219T, X219W, X219Y, X21E, X21S, X221F, X221N, X222D, X222K, X222S, X222T, X223C, X223L, X223V, X223Y, X224F, X224V, X225A, X225C, X225E, X225F, X225H, X225I, X225N, X225R, X225S, X225Y, X226A, X226C, X226D, X226E, X226G, X226H, X226I, X226L, X226M, X226Q, X226R, X226S, X226T, X226V, X226W, X226Y, X227C, X227F, X227G, X227H, X227I, X227K, X227L, X227M, X227R, X227T, X227V, X227W, X227Y, X22A, X22D, X22E, X22F, X22G, X22K, X22L, X22M, X22Q, X22R, X22T, X22W, X22Y, X230A, X230F, X231C, X231D, X231N, X233C, X233H, X233I, X233M, X233T, X233W, X233Y, X234C, X235L, X235M, X235V, X236Y, X237C, X237I, X238A, X238F, X238T, X23N, X23Q, X23W, X240M, X242M, X242N, X243D, X243F, X245E, X245H, X245M, X249D, X249I, X24G, X250V, X251A, X251E, X251F, X251L, X251M, X251S, X251T, X252C, X252I, X252S, X253C, X253G, X253Y, X255D, X255F, X255I, X255T, X255V, X256C, X257L, X257V, X257Y, X258F, X258Q, X258R, X259L, X25A, X25C, X25D, X25F, X25G, X25H, X25K, X25L, X25M, X25Q, X25S, X25W, X25Y, X260H, X261R, X262C, X262D, X262E, X262H, X262P, X262Y, X263I, X264H, X264T, X264W, X265S, X268F, X268G, X269M, X26A, X26D, X26E, X26F, X26L, X26M, X26P, X26S, X26V, X26Y, X270A, X270Q, X270Y, X271S, X272F, X272G, X272L, X272S, X273A, X273C, X273D, X273E, X273F, X273H, X273I, X273L, X273M, X273P, X273Q, X273R, X273V, X273W, X273Y, X274F, X274S, X275V, X276D, X276K, X276L, X276N, X276R, X276Y, X277D, X277E, X277T, X279A, X279P, X27A, X27D, X27F, X27G, X27H, X27I, X27Q, X27R, X27T, X27V, X280D, X280F, X280G, X280H, X280I, X280K, X280N, X280R, X280V, X280Y, X281A, X281D, X281E, X281H, X284A, X284F, X284H, X284L, X284M, X284N, X284Y, X285G, X285L, X285N, X285P, X286Q, X287A, X287D, X287E, X287H, X287T, X288A, X288K, X288P, X288Y, X289F, X289G, X289R, X289T, X28C, X28D, X28E, X28F, X28G, X28I, X28K, X28N, X28Q, X28S, X28T, X28V, X290D, X290M, X290N, X290Q, X290W, X291D, X291K, X291T, X291Y, X292C, X292D, X292F, X2921, X292L, X292T, X292W, X292Y, X293D, X293E, X293F, X293K, X293R, X294G, X294T, X295F, X296A, X296C, X296L, X296Y, X297E, X297F, X297H, X297K, X297M, X297S, X297V, X299G, X299I, X299K, X299L, X299S, X299Y, X29D, X29E, X29F, X29G, X29H, X29I, X29K, X29L, X29N, X29P, X29Q, X29V, X29W, X29Y, X2I, X2S, X301F, X302H, X302I, X302Q, X302V, X302Y, X303E, X303H, X303I, X303K, X303L, X303M, X303N, X303P, X303R, X303T, X304A, X304D, X304E, X304H, X304K, X304M, X304N, X304P, X304R, X304T, X304W, X304Y, X306A, X306D, X306E, X306G, X306H, X306I, X306M, X306Q, X306R, X306S, X306T, X306V, X306W, X306Y, X307F, X307M, X308S, X309H, X309L, X309Q, X30A, X30E, X30F, X30G, X30H, X30I, X30K, X30L, X30M, X30Q, X30T, X30W, X30Y, X310A, X310Q, X311A, X311E, X311G, X311H, X311K, X311N, X311R, X311T, X311Y, X312L, X312M, X313V, X314C, X314E, X314K, X314Q, X315A, X315C, X315E, X315H, X315K, X315T, X318I, X318S, X318T, X319A, X319D, X319H, X319I, X319K, X319M, X319N, X319P, X319S, X319T, X319W, X31N, X31Q, X31S, X31T, X31V, X31W, X320A, X320C, X320D, X320E, X320G, X320H, X320K, X320L, X320N, X320Q, X320S, X320Y, X321A, X321E, X321K, X321N, X321T, X321V, X321W, X323A, X323G, X323K, X323L, X323V, X324K, X324L, X324M, X324W, X324Y, X326G, X326N, X326S, X326Y, X327C, X327L, X327M, X32A, X32D, X32E, X32F, X32H, X32I, X32L, X32M, X32N, X32P, X32Q, X32T, X32W, X333I, X334T, X336K, X337A, X337C, X337F, X337G, X337I, X337K, X337L, X337M, X337N, X337Q, X337R, X337S, X337T, X337V, X337Y, X338G, X338S, X338T, X33D, X33E, X33H, X33K, X33M, X33Q, X33R, X33Y, X341V, X342P, X343L, X343T, X343W, X343Y, X344I, X344Q, X344V, X345D, X345G, X345M, X345N, X345Q, X345S, X345T, X346A, X346C, X346D, X346G, X346H, X346N, X346Q, X348T, X34H, X34I, X34V, X350H, X350K, X350P, X351A, X351M, X352S, X353H, X354I, X354N, X354T, X354Y, X355I, X355M, X356I, X356V, X357A, X358I, X358L, X358N, X358P, X358V, X359E, X35A, X35C, X35D, X35G, X35H, X35I, X35L, X35M, X35N, X35P, X35Q, X35R, X35S, X35T, X35V, X35Y, X360A, X360F, X360G, X360I, X360L, X360N, X360Q, X360R, X360S, X360T, X360V, X360Y, X362F, X362K, X362M, X362N, X362T, X362V, X362Y, X363A, X363C, X363D, X363E, X363G, X363H, X363K, X363L, X363M, X363P, X363Q, X363R, X363S, X363T, X363V, X363W, X363Y, X364A, X364C, X364G, X364K, X364L, X364N, X364S, X364T, X364V, X366I, X366L, X366T, X367E, X367S, X368A, X368F, X368L, X368N, X36A, X36E, X36G, X36I, X36K, X36M, X36N, X36P, X36Q, X36R, X36S, X36T, X36V, X370E, X370I, X372A, X372C, X372E, X372F, X372H, X372M, X372N, X372Q, X375A, X375D, X375E, X375I, X375K, X375Q, X375R, X375T, X375W, X375Y, X376G, X376I, X376K, X376L, X376M, X376Q, X376R, X376S, X376V, X377Q, X378C, X378D, X378E, X378R, X379A, X379L, X379S, X37A, X37G, X37M, X37P, X37T, X37V, X37W, X381E, X381V, X382A, X382H, X382K, X382L, X382N, X382Q, X382S, X383C, X383D, X383E, X383H, X383I, X383M, X383N, X383Q, X383R, X383S, X383V, X383Y, X384A, X384C, X384D, X384E, X384F, X384I, X384L, X384M, X384N, X384Q, X384R, X384T, X384V, X384W, X384Y, X385A, X385C, X385D, X385E, X385F, X385G, X385H, X385I, X385L, X385M, X385N, X385P, X385Q, X385R, X385S, X385T, X385V, X385W, X385Y, X387C, X387E, X387N, X388E, X388F, X388H, X388I, X388M, X388R, X388V, X389G, X389H, X389K, X38N, X390D, X390F, X390M, X390N, X390P, X390R, X391A, X391F, X391G, X391K, X391M, X391N, X391Q, X391S, X391T, X391Y, X392C, X392V, X393E, X393H, X393P, X393S, X393V, X394A, X394C, X394E, X394H, X394I, X394L, X394M, X394N, X394R, X394S, X395A, X395H, X395M, X395V, X396H, X396P, X397D, X397H, X397P, X397S, X398M, X399P, X39E, X39K, X3A, X3F, X3G, X3I, X3K, X3L, X3Q, X3V, X400A, X400D, X400E, X400G, X400H, X400I, X400K, X400L, X400M, X400N, X400P, X400Q, X400R, X400S, X400V, X400W, X401I, X401K, X401M, X401T, X403N, X405C, X405H, X405M, X405T, X405V, X406P, X407D, X407R, X407S, X408E, X408I, X408Q, X40I, X40S, X410H, X410I, X410K, X410L, X410P, X410R, X410Y, X413S, X414C, X414E, X414S, X415E, X415I, X416S, X418C, X418N, X418P, X41C, X41D, X41E, X41G, X41Q, X41S, X41T, X421N, X421P, X424A, X426D, X426W, X427C, X427F, X427G, X427K, X427Q, X427R, X427S, X427T, X427V, X429A, X429D, X429E, X429F, X429G, X429I, X429M, X429N, X429P, X429Q, X429S, X429T, X429V, X429W, X42C, X42I, X42Q, X42V, X431I, X434S, X436E, X438F, X438P, X438Y, X439C, X439P, X440V, X442Q, X443H, X443T, X445A, X445C, X445D, X445F, X445G, X445H, X445K, X445M, X445Q, X445R, X445S, X445T, X445V, X446F, X446I, X446L, X446P, X446Q, X446R, X446V, X446W, X447V, X448D, X448E, X448H, X448N, X449A, X449F, X449G, X449K, X449L, X449M, X449N, X449P, X449Q, X449R, X449S, X449V, X449W, X449Y, X451L, X453G, X453I, X453N, X453P, X453Y, X455L, X456L, X456M, X456R, X456S, X456V, X456W, X456Y, X457A, X457C, X457E, X457F, X457G, X457K, X457L, X457M, X457R, X457S, X457T, X457V, X457W, X458I, X458K, X458V, X458W, X459C, X459D, X459E, X459I, X459K, X459N, X459Q, X459R, X459V, X459Y, X45G, X45N, X460A, X460D, X460E, X460G, X460Q, X460R, X460S, X460T, X460V, X461A, X461E, X461F, X461K, X461L, X461M, X461N, X461Q, X461R, X461S, X461V, X462K, X463L, X465H, X465P, X465R, X465V, X467A, X467E, X467H, X468D, X468H, X470A, X470Q, X470T, X471E, X474F, X474H, X474P, X478A, X478E, X47S, X480D, X480E, X480M, X480R, X480Y, X482C, X482T, X482W, X48F, X48I, X48M, X48Y, X4A, X4C, X4K, X4M, X4Q, X4R, X4S, X51Q, X51T, X51V, X54D, X54G, X54Q, X59T, X5A, X5C, X5D, X5E, X5F, X5H, X5I, X5K, X5L, X5M, X5N, X5P, X5Q, X5R, X5V, X5Y, X60T, X63C, X63V, X6A, X6C, X6E, X6F, X6G, X6H, X6K, X6L, X6M, X6P, X6Q, X6S, X6T, X6V, X6W, X6Y, X70F, X70H, X70L, X70M, X70N, X70Y, X71D, X72C, X72D, X72E, X72N, X72T, X73L, X73N, X73Q, X75A, X75G, X75I, X75L, X75P, X75T, X75W, X76C, X76E, X76G, X76L, X76T, X76V, X7C, X7E, X7F, X7H, X7K, X7N, X7P, X7Q, X7R, X7S, X7V, X7W, X7Y, X81H, X81L, X82K, X82M, X83A, X83D, X83E, X83G, X83R, X83S, X86K, X87D, X87R, X89A, X89C, X89F, X89G, X89L, X89M, X89R, X89S, X8A, X8C, X8F, X8I, X8M, X8P, X8S, X8V, X8W, X8Y, X90A, X90D, X90E, X90F, X90G, X90I, X90M, X90N, X90Q, X90R, X90S, X90V, X90Y, X91C, X91D, X91E, X91F, X91G, X91H, X91I, X91K, X91L, X91M, X91N, X91Q, X91S, X91T, X91V, X91W, X91Y, X92D, X92M, X92V, X93K, X93Q, X94A, X94D, X94E, X94K, X94M, X94V, X94Y, X95E, X95I, X95L, X95V, X96K, X96N, X96Q, X97V, X98E, X98G, X98N, X99H, X99K, X99N, X100W, and X1G, preferably selected from the group consisting of X4Q, X25H, X100W, X135E, X135T, X135W, X135D, X160W, X176K, X193E, X251E, X258Q, X276R, X299S, X323G, X363E, X363H, X382Q, X405M, X460G, and X482W, wherein optionally, the alpha-amylase variant further comprises 1 to 50, preferably 1 to 30, 1 to 25, 1 to 20, 1 to 15, 1 to 10, or 1 to 5, 2 to 30, 2 to 25, 2 to 20, 2 to 15 2 to 10, 2 to 8, or 2 to 5, preferably 3 to 30, 3 to 25, 3 to 20, 3 to 15 3 to 10, 3 to 8, or 3 to 5, preferably, 4 to 30, 4 to 25, 4 to 20, 4 to 15, 4 to 10, or 4 to 8 conservative amino acid exchanges. Preferably, the amino acid residue at the above cited positions (i.e., X) corresponds to the amino acid residue shown in SEQ ID NO: 1 or 3, preferably in SEQ ID NO: 1, at the respective position (according to the numbering of SEQ ID NO: 3). In another embodiment, the alpha-amylase variant according to the present invention having alpha-amylase activity comprises or consists of the amino acid sequence set forth in SEQ ID NO: 1 with one of the combinations of amino acid substitutions selected from the group consisting of X25H+X176K+X186E+X206Y+X251E+X482W, X4Q+X25H+X176K+X186E+X206Y+X251E+X405M+X482W, X25H+X176K+X186E+X206Y+X482W, X25H+X186E+X206Y+X405M+X482W, X25H+X186E+X206Y+X482W, X25H+X176K+X186E+X193E+X206Y+X251E+X482W, X4Q+X176K+X186E+X193E+X206Y+X251E+X405M+X482W, X25H+X176K+X186E+X482W, X25H+X176K+X186E+X193E+X206Y+X482W, X4Q+X25H+X176K+X186E+X251E+X405M+X482W, X25H+X176K+X186E+X206Y, X4Q+X176K+X186E+X193E+X206Y+X251E+X405M, X4Q+X206Y+X460G+X482W, X4Q+X25H+X176K+X206Y+X251E+X460G+X482W, X4Q+X25H+X176K+X193E+X251E+X186E+X482W, X4Q+X193E+X206Y+X276R+X186E+X482W, X186E+X25H+X482W, X25H+X193E+X206Y+X251E+X276R+X405M+X186E+X482W, X25H+X251 E+X186E+X482W, X25H+X160W+X176K+X186E+X251E+X405M+X482W, X193E+X206Y+X405M+X186E+X482W, X3I+X356V, X3I+X356I, X83D+X94E, X94D+X125E, X131I+X377Q+X410H, X48F+X94D, X48Y+X116K+X218K, X5L+X218K+X225S, X83E+X116R+X158Y+X181E, X51V+X218K, X83E+X181E, X4Q+X7W+X176K+X186E+X193E+X206Y+X251E+X405M, X4Q+X37M+X176K+X186E+X193E+X206Y+X251E+X405M, X4Q+X25D+X176K+X186E+X193E+X206Y+X251E+X405M, X4Q+X25H+X176K+X186E+X206Y+X251E+X405M+X460G, X4Q+X25H+X176K+X186E+X206Y+X251E+X460G, X4Q+X25Y+X176K+X186E+X193E+X206Y+X251E+X405M, X4Q+X118D+X176K+X186E+X193E+X206Y+X251E+X405M, X4Q+X176K+X182D+X186E+X193E+X206Y+X251E+X405M, X4Q+X176K+X186E+X193E+X206Y+X251E+X258Q+X405M, X4Q+X176K+X186E+X193E+X206Y+X251E+X405M, X4Q+X176K+X186E+X193E+X206Y+X251E+X405M+X460G, X4Q+X176K+X186E+X193E+X206Y+X251E+X363E+X405M, X4Q+X176K+X186E+X193E+X206Y+X251E+X363H+X405M, X4Q+X176K+X186E+X193E+X206Y+X251E+X363N+X405M, X4Q+X176K+X181D+X186E+X193E+X206Y+X251E+X405M, X4Q+X176K+X181F+X186E+X193E+X206Y+X251E+X405M, X4Q+X176K+X181N+X186E+X193E+X206Y+X251E+X405M, X4Q+X176K+X181Q+X186E+X193E+X206Y+X251E+X405M, X4Q+X136E+X176K+X186E+X193E+X206Y+X251E+X405M, X4Q+X100W+X176K+X186E+X193E+X206Y+X251E+X405M, X4Q+X135D+X176K+X186E+X193E+X206Y+X251E+X405M, X4Q+X135E+X176K+X186E+X193E+X206Y+X251E+X405M, X4Q+X135T+X176K+X186E+X193E+X206Y+X251E+X405M, X4Q+X135W+X176K+X186E+X193E+X206Y+X251E+X405M, X4Q+X160D+X176K+X186E+X193E+X206Y+X251E+X405M, X4Q+X160E+X176K+X186E+X193E+X206Y+X251E+X405M, X7H+X25H+X176K+X186E+X206Y,X7W+X25H+X176K+X186E+X206Y, X25D+X176K+X186E+X206Y, X25H+X186E+X206Y+X405M+X460G, X25H+X186E+X206Y+X460G, X25H+X37M+X176K+X186E+X206Y, X25H+X118D+X176K+X186E+X206Y, X25H+X176K+X182D+X186E+X206Y, X25H+X176K+X186E+X206Y, X25H+X176K+X186E+X206Y+X258Q, X25H+X176K+X186E+X206Y+X281N, X25H+X176K+X186E+X206Y+X460G, X25H+X176K+X186E+X206Y+X251E+X460G,X25H+X176K+X186E+X206Y+X363E, X25H+X176K+X186E+X206Y+X363H,X25H+X176K+X186E+X206Y+X363N, X25H+X176K+X186E+X460G, X25H+X176K+X186E+X193E+X206Y, X25H+X176K+X186E+X193E+X206Y+X460G, X25H+X176K+X186E+X193E+X206Y+X251E+X460G, X25H+X176K+X181D+X186E+X206Y, X25H+X176K+X181N+X186E+X206Y, X25H+X176K+X181Q+X186E+X206Y, X25H+X136E+X176K+X186E+X206Y, X25H+X100W+X176K+X186E+X206Y, X25H+X135E+X176K+X186E+X206Y, X25H+X135T+X176K+X186E+X206Y, X25H+X135W+X176K+X186E+X206Y, X25H+X160D+X176K+X186E+X206Y, X25H+X160E+X176K+X186E+X206Y, X25Y+X176K+X186E+X206Y, X87D+X186E+X315K+X420K, X87D+X186E+X226D+X314E+X420E+X461E, X87D+X186E+X226D+X420E, X87D+X186E+X226D+X420E+X461E, X87D+X186E+X314E+X471E, X87D+X186E+X311K+X314E+X420K, X87D+X186E+X160D+X461E,X87R+X186E+X315K+X461R,X87R+X186E+X226D+X471E, X87R+X186E+X226R+X314K+X420K+X461R,X87R+X186E+X226R+X311K+X420K, X87R+X186E+X314K+X315K, X87R+X186E+X311K+X314E, X87R+X186E+X420E+X450R, X87R+X186E+X450R+X452R,X186E+X315K+X420E+X452R, X186E+X226D+X314E+X461E+X471E,X186E+X226D+X314E+X452R, X186E+X226D+X314K+X460D+X471E+X485R,X186E+X226D+X311K+X460D, X186E+X226D+X461E+X471E,X186E+X314E+X460D+X461E, X186E+X314E+X420E+X452R+X461E, X186E+X314K+X450R+X460D, X186E+X460D+X471E+X485R,X186E+X311K+X314K+X452R, X186E+X311K+X461E+X485R, X186E+X311K+X420E+X471E, X186E+X420K+X450R+X471E+X485R, X186E+X420K+X450R+X485R, X186E+X452R+X461E+X471E, X4Q+X176K+X186E+X193E+X206Y+X251E+X405M, X25H+X176K+X186E+X206Y, X83E+X186E+X219D+X226R, X83E+X186E+X219R+X226D+X314E+X452R,X83E+X186E+X219R+X226R, X83E+X186E+X160D+X219D+X226R+X452R, X83R+X186E+X219R+X226D, X160D+X186E+X315K+X450R, X160D+X186E+X226D+X314K+X460D+X461E, X160D+X186E+X226D+X314K+X420E,X160D+X186E+X314K+X485R, X160D+X186E+X420E+X452R, X160D+X186E+X420K+X460D, X186E+X276R, X186E+X206Y, X186E+X206Y+X276R, X186E+X206Y+X276R+X405M, X186E+X206Y+X405M, X186E+X206Y+X251E, X186E+X206Y+X251E+X276R, X186E+X206Y+X251E+X276R+X405M,X186E+X206Y+X251E+X405M, X186E+X206Y+X251E+X323G+X405M, X186E+X206Y+X323G+X405M, X186E+X405M, X186E+X193E+X206Y+X405M, X186E+X193E+X206Y+X251E, X186E+X251E, X186E+X251E+X405M, X4Q+X186E, X4Q+X186E+X276R, X4Q+X186E+X206Y, X4Q+X186E+X206Y+X276R+X405M, X4Q+X186E+X206Y+X405M, X4Q+X186E+X206Y+X251E+X276R, X4Q+X186E+X206Y+X251E+X276R+X405M, X4Q+X186E+X206Y+X251E+X405M, X4Q+X186E+X206Y+X251E+X323G+X405M, X4Q+X186E+X405M, X4Q+X186E+X193E+X206Y+X276R, X4Q+X186E+X193E+X206Y+X405M, X4Q+X186E+X193E+X206Y+X251E+X405M, X4Q+X186E+X251E+X276R+X405M, X4Q+X25H+X186E+X206Y, X4Q+X25H+X186E+X206Y+X276R, X4Q+X25H+X186E+X206Y+X276R+X405M, X4Q+X25H+X186E+X206Y+X405M, X4Q+X25H+X186E+X206Y+X251E+X323A+X405M, X4Q+X25H+X186E+X206Y+X323G+X405M, X4Q+X25H+X186E+X405M, X4Q+X25H+X176K+X186E+X206Y+X276R, X4Q+X25H+X176K+X186E+X206Y+X276R+X405M, X4Q+X25H+X176K+X186E+X206Y+X251E+X276R+X405M, X4Q+X25H+X176K+X186E+X206Y+X251E+X405M, X4Q+X25H+X176K+X186E+X251E, X4Q+X25H+X176K+X186E+X251E+X405M, X4Q+X25H+X176K+X206Y, X4Q+X25H+X176K+X206Y+X405M N460G, X4Q+X25H+X176K+X206Y+X460G, X4Q+X25H+X176K+X206Y+X251E, X4Q+X25H+X176K+X206Y+X251E+X276R+X405M, X4Q+X25H+X176K+X206Y+X251E+X460G, X4Q+X25H+X176K+X206Y+X323G, X4Q+X25H+X160W+X186E+X206Y+X405M, X4Q+X25H+X160W+X186E+X206Y+X251E, X4Q+X25H+X160W+X186E+X206Y+X251E+X276R+X405M, X4Q+X25H+X160W+X186E+X206Y+X323G+X405M, X4Q+X25H+X160W+X186E+X405M, X4Q+X25H+X160W+X186E+X193E+X206Y+X276R, X4Q+X25H+X160W+X186E+X323G, X4Q+X25H+X160W+X169R+X186E+X206Y+X276R, X4Q+X25H+X160W+X176K+X186E+X276R+X405M, X4Q+X25H+X160W+X176K+X186E+X206Y+X276R, X4Q+X25H+X160W+X176K+X186E+X206Y+X251E, X4Q+X25H+X160W+X176K+X186E+X206Y+X251E+X405M, X4Q+X25H+X160W+X176K+X186E+X405M, X4Q+X25H+X160W+X176K+X186E+X251E+X276R+X405M, X4Q+X25H+X160W+X176K+X186E+X251E+X405M, X4Q+X25H+X160W+X176K+X206Y, X4Q+X25H+X160W+X176K+X206Y+X405M, X4Q+X25H+X160W+X176K+X206Y+X460G, X4Q+X25H+X160W+X176K+X206Y+X251E+X460G, X4Q+X176K+X186E+X276R+X405M, X4Q+X176K+X186E+X206Y+X276R, X4Q+X176K+X186E+X206Y+X276R+X405M, X4Q+X176K+X186E+X206Y+X405M, X4Q+X176K+X186E+X206Y+X251E+X276R+X405M, X4Q+X176K+X186E+X206Y+X251E+X405M, X4Q+X176K+X186E+X405M, X4Q+X176K+X186E+X193E+X206Y+X276R+X405M, X4Q+X176K+X186E+X193E+X206Y+X251E+X405M, X4Q+X176K+X186E+X251E+X276R+X405M, X4Q+X176K+X186E+X251E+X405M, X4Q+X176K+X186E+X323G+X405M, X4Q+X176K+X206Y, X4Q+X176K+X206Y+X276R, X4Q+X176K+X206Y+X405M, X4Q+X176K+X206Y+X405M N460G, X4Q+X176K+X206Y+X460G, X4Q+X176K+X206Y+X251E+X276R+X405M, X4Q+X176K+X206Y+X251E+X276R+X405M N460G, X4Q+X176K+X206Y+X251E+X405M, X4Q+X176K+X206Y+X251E+X405M N460G, X4Q+X176K+X206Y+X251E+X323A+X460G, X4Q+X160W+X186E+X276R+X405M, X4Q+X160W+X186E+X206Y, X4Q+X160W+X186E+X206Y+X276R+X323G, X4Q+X160W+X186E+X206Y+X405M, X4Q+X160W+X186E+X206Y+X251E, X4Q+X160W+X186E+X206Y+X251E+X276R+X405M, X4Q+X160W+X186E+X405M, X4Q+X160W+X186E+X251E+X405M, X4Q+X160W+X176K+X186E, X4Q+X160W+X176K+X186E+X276R+X405M, X4Q+X160W+X176K+X186E+X206Y+X251E, X4Q+X160W+X176K+X186E+X251E+X276R+X405M, X4Q+X160W+X176K+X186E+X251E+X405M, X4Q+X160W+X176K+X206Y, X4Q+X160W+X176K+X206Y+X251E+X276R+X460G, X4Q+X160W+X176K+X206Y+X251E+X323G+X405M+X460G, X25H+X186E, X25H+X186E+X206Y,X25H+X186E+X206Y+X276R+X405M, X25H+X186E+X206Y+X276R+X323G,X25H+X186E+X206Y+X405M, X25H+X186E+X206Y+X251E+X276R+X323G+X405M, X25H+X186E+X206Y+X251E+X405M, X25H+X186E+X405M, X25H+X186E+X202I+X206Y+X405M, X25H+X186E+X193E+X206Y+X276R+X405M, X25H+X186E+X193E+X206Y+X405M, X25H+X186E+X193E+X206Y+X251E+X276R+X405M, X25H+X186E+X251E, X25H+X186E+X251E+X276R,X25H+X176K+X186E, X25H+X176K+X186E+X276R+X323G+X405M, X25H+X176K+X186E+X206Y, X25H+X176K+X186E+X206Y+X276R, X25H+X176K+X186E+X206Y+X251E, X25H+X176K+X186E+X206Y+X251E+X276R+X405M, X25H+X176K+X186E+X206Y+X251E+X405M, X25H+X176K+X186E+X405M, X25H+X176K+X186E+X193E+X206Y, X25H+X176K+X186E+X193E+X206Y+X251E, X25H+X176K+X186E+X193E+X206Y+X251E+X276R+X405M, X25H+X176K+X186E+X251E+X276R, X25H+X176K+X206Y, X25H+X176K+X206Y+X276R, X25H+X176K+X206Y+X276R+X405M, X25H+X176K+X206Y+X276R+X460G, X25H+X176K+X206Y+X405M, X25H+X176K+X206Y+X460G, X25H+X176K+X206Y+X251E, X25H+X176K+X206Y+X251E+X276R+X405M+X460G, X25H+X176K+X206Y+X251E+X276R+X460G, X25H+X176K+X206Y+X251E+X405M, X25H+X160W+X186E+X206Y, X25H+X160W+X186E+X206Y+X251E, X25H+X160W+X186E+X206Y+X251E+X276R, X25H+X160W+X186E+X206Y+X251E+X276R+X323G+X405M, X25H+X160W+X186E+X206Y+X251E+X405M, X25H+X160W+X186E+X206Y+X323G+X405M, X25H+X160W+X186E+X251E+X276R+X405M, X25H+X160W+X176K+X186E+X206Y+X276R, X25H+X160W+X176K+X186E+X206Y+X276R+X405M, X25H+X160W+X176K+X186E+X206Y+X251E+X276R+X323G, X25H+X160W+X176K+X186E+X206Y+X251E+X405M, X25H+X160W+X176K+X186E+X405M, X25H+X160W+X176K+X186E+X193E+X206Y, X25H+X160W+X176K+X186E+X193E+X206Y+X405M, X25H+X160W+X176K+X186E+X193E+X206Y+X251E+X405M, X25H+X160W+X176K+X186E+X251E, X25H+X160W+X176K+X186E+X251E+X276R, X25H+X160W+X176K+X186E+X251E+X405M, X25H+X160W+X176K+X186E+X323G+X405M, X25H+X160W+X176K+X206Y+X276R+X323G+X405M, X25H+X160W+X176K+X206Y+X460G, X25H+X160W+X176K+X206Y+X251E, X25H+X160W+X176K+X206Y+X251E+X323G+X405M, X176K+X186E+X206Y, X176K+X186E+X206Y+X276R, X176K+X186E+X206Y+X276R+X405M, X176K+X186E+X206Y+X405M, X176K+X186E+X206Y+X251E+X405M, X176K+X186E+X405M,X176K+X186E+X193E+X206Y+X405M, X176K+X186E+X193E+X206Y+X251E+X405M, X176K+X186E+X193E+X405M, X176K+X186E+X251E+X276R+X405M, X176K+X186E+X251E+X405M, X176K+X206Y, X176K+X206Y+X276R, X176K+X206Y+X276R+X460G, X176K+X206Y+X405M, X176K+X206Y+X405M N460G, X176K+X206Y+X460G, X176K+X206Y+X251E, X176K+X206Y+X251E+X405M, X160W+X186E, X160W+X186E+X276R+X405M, X160W+X186E+X206Y+X276R, X160W+X186E+X206Y+X276R+X405M, X160W+X186E+X206Y+X405M, X160W+X186E+X206Y+X251E, X160W+X186E+X206Y+X251E+X405M, X160W+X186E+X405M, X160W+X186E+X193E+X206Y, X160W+X186E+X193E+X206Y+X405M, X160W+X186E+X251E+X276R, X160W+X186E+X251 E+X276R+X405M, X160W+X186E+X323G+X405M, X160W+X176K+X186E+X276R+X405M, X160W+X176K+X186E+X206Y, X160W+X176K+X186E+X206Y+X251E+X405M, X160W+X176K+X186E+X405M, X160W+X176K+X186E+X193E+X206Y, X160W+X176K+X186E+X193E+X206Y+X405M, X160W+X176K+X186E+X193E+X206Y+X251E, X160W+X176K+X206Y+X276R+X405M N460G, X160W+X176K+X206Y+X276R+X323G+X460G, X160W+X176K+X206Y+X405M, X160W+X176K+X206Y+X405M N460G, X160W+X176K+X206Y+X460G, X160W+X176K+X206Y+X251E+X276R, X160W+X176K+X206Y+X251E+X405M+X460G, X160W+X176K+X206Y+X323G+X405M+X460G, X4Q+X176K+X186E+X193E+X206Y+X251E+X405M, X4Q+X176K+X186E+X193E+X206Y+X251E+X405M+X258Q, X4Q+X176K+X186E+X193E+X206Y+X251E+X405M+X258Q+X281N, X4Q+X176K+X186E+X193E+X206Y+X251E+X405M+X258Q+X281N+X363H, X4Q+X176K+X186E+X193E+X206Y+X251E+X405M+X258Q+X363E, X4Q+X176K+X186E+X193E+X206Y+X251E+X405M+X281N, X4Q+X176K+X186E+X193E+X206Y+X251E+X405M+X281N+X363E, X4Q+X176K+X186E+X193E+X206Y+X251 E+X405M+X281 N+X363H, X4Q+X176K+X186E+X193E+X206Y+X251E+X405M+X181Q, X4Q+X176K+X186E+X193E+X206Y+X251E+X405M+X181Q+X258Q, X4Q+X176K+X186E+X193E+X206Y+X251E+X405M+X181Q+X258Q+X281N, X4Q+X176K+X186E+X193E+X206Y+X251E+X405M+X181Q+X258Q+X281N+X363H, X4Q+X176K+X186E+X193E+X206Y+X251E+X405M+X181Q+X281N, X4Q+X176K+X186E+X193E+X206Y+X251E+X405M+X181Q+X281N+X363E, X4Q+X176K+X186E+X193E+X206Y+X251E+X405M+X181Q+X363E, X4Q+X176K+X186E+X193E+X206Y+X251E+X405M+X181Q+X363H, X4Q+X176K+X186E+X193E+X206Y+X251E+X405M+X100W, X4Q+X176K+X186E+X193E+X206Y+X251E+X405M+X100W+X258Q, X4Q+X176K+X186E+X193E+X206Y+X251E+X405M+X100W+X258Q+X281N, X4Q+X176K+X186E+X193E+X206Y+X251E+X405M+X100W+X258Q+X281N+X363E, X4Q+X176K+X186E+X193E+X206Y+X251E+X405M+X100W+X258Q+X281N+X363H, X4Q+X176K+X186E+X193E+X206Y+X251E+X405M+X100W+X258Q+X281N+X363H+X272V, X4Q+X176K+X186E+X193E+X206Y+X251E+X405M+X100W+X258Q+X363E, X4Q+X176K+X186E+X193E+X206Y+X251E+X405M+X100W+X258Q+X363H, X4Q+X176K+X186E+X193E+X206Y+X251E+X405M+X100W+X281N, X4Q+X176K+X186E+X193E+X206Y+X251E+X405M+X100W+X281N+X363E, X4Q+X176K+X186E+X193E+X206Y+X251E+X405M+X100W+X281N+X363H, X4Q+X176K+X186E+X193E+X206Y+X251E+X405M+X100W+X181Q, X4Q+X176K+X186E+X193E+X206Y+X251E+X405M+X100W+X181Q+X258Q, X4Q+X176K+X186E+X193E+X206Y+X251E+X405M+X100W+X181Q+X258Q+X281N, X4Q+X176K+X186E+X193E+X206Y+X251E+X405M+X100W+X181Q+X258Q+X281N+X363E, X4Q+X176K+X186E+X193E+X206Y+X251E+X405M+X100W+X181Q+X258Q+X363E, X4Q+X176K+X186E+X193E+X206Y+X251E+X405M+X100W+X181Q+X258Q+X363H, X4Q+X176K+X186E+X193E+X206Y+X251E+X405M+X100W+X181Q+X281N, X4Q+X176K+X186E+X193E+X206Y+X251E+X405M+X100W+X181Q+X281N+X363H, X4Q+X176K+X186E+X193E+X206Y+X251E+X405M+X100W+X181Q+X363H, X4Q+X176K+X186E+X193E+X206Y+X251E+X405M+X100W+X181Q+X363H+X254Q, X4Q+X176K+X186E+X193E+X206Y+X251E+X405M+X100W+X135T, X4Q+X176K+X186E+X193E+X206Y+X251E+X405M+X100W+X135T+X258Q, X4Q+X176K+X186E+X193E+X206Y+X251E+X405M+X100W+X135T+X258Q+X281N+X363H, X4Q+X176K+X186E+X193E+X206Y+X251E+X405M+X100W+X135T+X258Q+X363E, X4Q+X176K+X186E+X193E+X206Y+X251E+X405M+X100W+X135T+X258Q+X363H, X4Q+X176K+X186E+X193E+X206Y+X251E+X405M+X100W+X135T+X181Q, X4Q+X176K+X186E+X193E+X206Y+X251E+X405M+X100W+X135T+X181Q+X258Q, X4Q+X176K+X186E+X193E+X206Y+X251E+X405M+X100W+X135T+X181Q+X258Q+X281N, X4Q+X176K+X186E+X193E+X206Y+X251E+X405M+X100W+X135T+X181Q+X258Q+X363E, X4Q+X176K+X186E+X193E+X206Y+X251E+X405M+X100W+X135T+X181Q+X258Q+X363H, X4Q+X176K+X186E+X193E+X206Y+X251E+X405M+X100W+X135T+X181Q+X281N+X363H, X4Q+X176K+X186E+X193E+X206Y+X251E+X405M+X100W+X135T+X181Q+X363E, X4Q+X176K+X186E+X193E+X206Y+X251E+X405M+X100W+X135T+X181Q+X363H, X4Q+X176K+X186E+X193E+X206Y+X251E+X405M+X100W+X135T+X363E, X4Q+X176K+X186E+X193E+X206Y+X251E+X405M+X100W+X135T+X363H, X4Q+X176K+X186E+X193E+X206Y+X251E+X405M+X100W+X363E, X4Q+X176K+X186E+X193E+X206Y+X251E+X405M+X100W+X363H, X4Q+X176K+X186E+X193E+X206Y+X251E+X405M+X135T, X4Q+X176K+X186E+X193E+X206Y+X251E+X405M+X135T+X258Q, X4Q+X176K+X186E+X193E+X206Y+X251E+X405M+X135T+X258Q+X8A, X4Q+X176K+X186E+X193E+X206Y+X251E+X405M+X135T+X258Q+X281N+X363E, X4Q+X176K+X186E+X193E+X206Y+X251E+X405M+X135T+X258Q+X363H, X4Q+X176K+X186E+X193E+X206Y+X251E+X405M+X135T+X281N, X4Q+X176K+X186E+X193E+X206Y+X251E+X405M+X135T+X181Q+X258Q, X4Q+X176K+X186E+X193E+X206Y+X251E+X405M+X135T+X181Q+X258Q+X281N, X4Q+X176K+X186E+X193E+X206Y+X251E+X405M+X135T+X181Q+X258Q+X363H, X4Q+X176K+X186E+X193E+X206Y+X251E+X405M+X135T+X363E, X4Q+X176K+X186E+X193E+X206Y+X251E+X405M+X135T+X363H, X4Q+X176K+X186E+X193E+X206Y+X251E+X405M+X363E, X4Q+X176K+X186E+X193E+X206Y+X251E+X405M+X363H,X25H+X176K+X186E+X206Y, X25H+X176K+X186E+X206Y+X258Q, X25H+X176K+X186E+X206Y+X258Q, X25H+X176K+X186E+X206Y+X258Q+X281N, X25H+X176K+X186E+X206Y+X258Q+X281N+X363E, X25H+X176K+X186E+X206Y+X258Q+X363E, X25H+X176K+X186E+X206Y+X258Q+X363E+X123D, X25H+X176K+X186E+X206Y+X281N, X25H+X176K+X186E+X206Y+X181Q, X25H+X176K+X186E+X206Y+X181Q+X193E, X25H+X176K+X186E+X206Y+X181Q+X363E, X25H+X176K+X186E+X206Y+X193E, X25H+X176K+X186E+X206Y+X193E+X258Q, X25H+X176K+X186E+X206Y+X193E+X281N+X363E, X25H+X176K+X186E+X206Y+X193E+X363E, X25H+X176K+X186E+X206Y+X100W, X25H+X176K+X186E+X206Y+X100W+X258Q, X25H+X176K+X186E+X206Y+X100W+X258Q+X281N, X25H+X176K+X186E+X206Y+X100W+X258Q+X281N+X363E, X25H+X176K+X186E+X206Y+X100W+X258Q+X363E, X25H+X176K+X186E+X206Y+X100W+X258Q+X363E+X135C, X25H+X176K+X186E+X206Y+X100W+X281N, X25H+X176K+X186E+X206Y+X100W+X281N+X363E, X25H+X176K+X186E+X206Y+X100W+X361R, X25H+X176K+X186E+X206Y+X100W+X181Q+X281N+X363E, X25H+X176K+X186E+X206Y+X100W+X181Q+X281N+X363E+X175D, X25H+X176K+X186E+X206Y+X100W+X181Q+X193E, X25H+X176K+X186E+X206Y+X100W+X181Q+X193E+X258Q+X281N, X25H+X176K+X186E+X206Y+X100W+X181Q+X193E+X258Q+X363E, X25H+X176K+X186E+X206Y+X100W+X181Q+X193E+X281N+X363E, X25H+X176K+X186E+X206Y+X100W+X181Q+X193E+X363E, X25H+X176K+X186E+X206Y+X100W+X181Q+X363E, X25H+X176K+X186E+X206Y+X100W+X193E, X25H+X176K+X186E+X206Y+X100W+X193E+X258Q, X25H+X176K+X186E+X206Y+X100W+X193E+X258Q+X473R, X25H+X176K+X186E+X206Y+X100W+X193E+X258Q+X281N, X25H+X176K+X186E+X206Y+X100W+X193E+X258Q+X281N+X363E, X25H+X176K+X186E+X206Y+X100W+X193E+X258Q+X363E, X25H+X176K+X186E+X206Y+X100W+X193E+X363E, X25H+X176K+X186E+X206Y+X100W+X135T, X25H+X176K+X186E+X206Y+X100W+X135T+X258Q, X25H+X176K+X186E+X206Y+X100W+X135T+X258Q+X281N, X25H+X176K+X186E+X206Y+X100W+X135T+X258Q+X281N+X363E, X25H+X176K+X186E+X206Y+X100W+X135T+X258Q+X363E, X25H+X176K+X186E+X206Y+X100W+X135T+X281N, X25H+X176K+X186E+X206Y+X100W+X135T+X281N+X363E, X25H+X176K+X186E+X206Y+X100W+X135T+X181Q, X25H+X176K+X186E+X206Y+X100W+X135T+X181Q+X258Q+X281N+X363E, X25H+X176K+X186E+X206Y+X100W+X135T+X181Q+X281N+X363E, X25H+X176K+X186E+X206Y+X100W+X135T+X181Q+X193E, X25H+X176K+X186E+X206Y+X100W+X135T+X181Q+X193E+X258Q+X363E, X25H+X176K+X186E+X206Y+X100W+X135T+X181Q+X193E+X363E, X25H+X176K+X186E+X206Y+X100W+X135T+X193E, X25H+X176K+X186E+X206Y+X100W+X135T+X193E+X258Q+X363E, X25H+X176K+X186E+X206Y+X100W+X135T+X193E+X363E, X25H+X176K+X186E+X206Y+X100W+X135T+X363E, X25H+X176K+X186E+X206Y+X100W+X363E, X25H+X176K+X186E+X206Y+X135T, X25H+X176K+X186E+X206Y+X135T+X258Q, X25H+X176K+X186E+X206Y+X135T+X258Q+X281N, X25H+X176K+X186E+X206Y+X135T+X258Q+X281N+X363E, and X25H+X176K+X186E+X206Y+X135T+X258Q+X363E, preferably selected from the group consisting of N25H+R176K+G186E+I206Y+R181Q, N25H+R176K+G186E+I206Y+Y135T, N25H+R176K+G186E+I206Y+Y100W+Y363E, N25H+R176K+G186E+I206Y+Y100W+T193E, N25H+R176K+G186E+I206Y+T251E, G4Q+N25H+R176K+G186E+I206Y+T251E+L405M, N25H+R176K+G186E+1206Y, N25H+G186E+1206Y+L405M, N25H+G186E+1206Y, N25H+R176K+G186E+T193E+I206Y+T251E, G4Q+R176K+G186E+T193E+I206Y+T251E+L405M, N25H+R176K+G186E, N25H+R176K+G186E+T193E+I206Y, G4Q+N25H+R176K+G186E+T251E+L405M, N25H+R176K+G186E+1206Y, G4Q+R176K+G186E+T193E+I206Y+T251E+L405M, G4Q+I206Y+N460G, G4Q+N25H+R176K+I206Y+T251E+N460G, G4Q+N25H+R176K+T193E+T251E+G186E, G4Q+T193E+I206Y+E276R+G186E, G186E+N25H, N25H+T193E+I206Y+T251E+E276R+L405M+G186E, N25H+T251E+G186E, N25H+Y160W+R176K+G186E+T251E+L405M, T193E+1206Y+L405M+G186E, N25H+R176K+G186E+I206Y+G258Q+Y363E, N25H+R176K+G186E+I206Y+R181Q, N25H+R176K+G186E+I206Y+Y100W, N25H+R176K+G186E+I206Y+Y100W+Q359R, N25H+R176K+G186E+1206Y+Y100W+G258Q, N25H+R176K+G186E+1206Y+Y100W+G258Q+Y363E, N25H+R176K+G186E+1206Y+Y100W+Y135T, N25H+R176K+G186E+I206Y+Y100W+Y135T+Y363E, N25H+R176K+G186E+I206Y+Y135T, N25H+R176K+G186E+1206Y+Y135T+G258Q, N25H+R176K+G186E+1206Y+Y135T+G258Q+Y363E, N25H+R176K+G186E+I206Y+Y135T+Y363E, N25H+R176K+G186E+I206Y+Y363E, N25H+R176K+G186E+I206Y+T251E+Y482W, N25H+R176K+G186E+1206Y+Y482W, N25H+R176K+G186E+T193E+I206Y+T251E+Y482W, G4Q+R176K+G186E+T193E+I206Y+T251E+L405M+Y482W, G4Q+N25H+R176K+G186E+T251E+L405M+Y482W, N25H+R176K+G186E+T193E+1206Y+Y482W, G4Q+N25H+R176K+G186E+I206Y+T251E+L405M+Y482W, N25H+G186E+I206Y+L405M+Y482W, N25H+R176K+G186E+Y482W, N25H+R176K+G186E+T193E+I206Y+T251E+N460G, G4Q+N25H+R176K+G186E+1206Y+T251E+N460G, N25H+R176K+G186E+T193E+I206Y+T251E+N460G+Y482W, G4Q+N25H+R176K+G186E+1206Y+T251E+N460G+Y482W, N25H+R176K+G186E+N195F+T251E+Y482W, N25H+R176K+G186E+N195F+Y482W, N25H+R176K+G186E+T193E+N195F+T251E+Y482W, G4Q+R176K+G186E+T193E+N195F+T251E+L405M+Y482W, N25H+R176K+G186E+T193E+N195F+Y482W, G4Q+N25H+R176K+G186E+N195F+T251E+L405M+Y482W, N25H+G186E+N195F+L405M+Y482W, N25H+R176K+G186E+T193E+N195F+T251E+N460G, G4Q+N25H+R176K+G186E+N195F+T251E+N460G, N25H+R176K+G186E+T193E+N195F+T251E+N460G+Y482W, and G4Q+N25H+R176K+G186E+N195F+T251E+N460G+Y482W.
Preferably, the amino acid residue at the above cited positions (i.e., X) corresponds to the amino acid residue shown in SEQ ID NO: 1 or 3, preferably in SEQ ID NO: 1, at the respective position (according to the numbering of SEQ ID NO: 3).

The alpha-amylase variant according to the present invention having one or more amino acid alterations, preferably substitutions, as described herein and having alpha-amylase activity preferably comprises at least 65%, at least 70%, at least 75%, at least 80%, at least 85%, at least 90%, at least 95% identity, at least 96%, at least 97%, at least 98%, or at least 99%, but less than 100% sequence identity to the amino acid sequence of the parent amylase.
The alpha-amylase variant according to the present invention having one or more amino acid substitutions as described herein and having alpha-amylase activity preferably comprises at least 65%, at least 70%, at least 75%, at least 80%, at least 85%, at least 90%, at least 95% identity, at least 96%, at least 97%, at least 98%, or at least 99%, but less than 100% sequence identity to the amino acid sequence of the parent amylase.
Preferably, the alpha-amylase variant according to the present invention having alpha-amylase activity and having one or more of the amino acid substitutions described herein preferably comprises at least 65%, at least 70%, at least 75%, at least 80%, at least 85%, at least 90%, at least 95% identity, at least 96%, at least 97%, at least 98%, or at least 99%, but less than 100% sequence identity to the amino acid sequence set forth in SEQ ID NO: 1, SEQ ID NO: 3, or SEQ ID NO: 4, preferably SEQ ID NO: 1 or 3.

Preferably, the alpha-amylase variant according to the present invention having alpha-amylase activity and having one or more of the amino acid substitutions described herein preferably comprises at least 65%, at least 70%, at least 75%, at least 80%, at least 85%, at least 90%, at least 95% identity, at least 96%, at least 97%, at least 98%, or at least 99%, but less than 100% sequence identity to the amino acid sequence set forth in SEQ ID NO: 3.
More preferably, the alpha-amylase variant according to the present invention having alpha-amylase activity and having one or more of the amino acid substitutions described herein preferably comprises at least 65%, at least 70%, at least 75%, at least 80%, at least 85%, at least 90%, at least 95% identity, at least 96%, at least 97%, at least 98%, or at least 99%, but less than 100% sequence identity to the amino acid sequence set forth in SEQ ID NO: 1.
The alpha-amylase variant according to the present invention having alpha-amylase activity preferably comprises at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95% identity, at least 96%, at least 97%, at least 98%, or at least 99%, but less than 100% sequence identity or sequence similarity to the amino acid sequence set forth in SEQ ID NO: 1.
The alpha-amylase variant according to the present invention having alpha-amylase activity preferably comprises at least 65%, at least 70%, at least 75%, at least 80%, at least 85%, at least 90%, at least 95% identity, at least 96%, at least 97%, at least 98%, or at least 99%, but less than 100% sequence identity to the amino acid sequence set forth in SEQ ID NO: 1.
The alpha-amylase variant according to the present invention having alpha-amylase activity preferably comprises at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95% identity, at least 96%, at least 97%, at least 98%, or at least 99%, but less than 100% sequence identity to the amino acid sequence set forth in SEQ ID NO: 1.
Preferably, the alpha-amylase variant of the present invention having alpha-amylase activity comprises:
(a) an amino acid sequence having at least 80%, at least 85%, at least 90%, at least 91 %, at least 92%, at least 93%, at least 94%, at least 95%, at least, 96%, at least 97%, at least 98%, at least 99%, but less than 100% sequence identity to SEQ ID NO: 1;
(b) an amino acid sequence encoded by a polynucleotide having at least 80%, at least 85%, at least 90%, at least 91 %, at least 92%, at least 93%, at least 94%, at least 95%, at least, 96%, at least 97%, at least 98%, or at least 99%, but less than 100% sequence identity with SEQ ID NO: 2,
(c) an amino acid sequence encoded by a polynucleotide that hybridizes under high stringency conditions with the complement of
   (i) a coding sequence of SEQ ID NO: 1; or
   (ii) a polynucleotide shown in SEQ ID NO: 2;
(d) an amino acid sequence encoded by a polynucleotide that having at least 95%, but less than 100% sequence identity to SEQ ID NO: 2, wherein the polynucleotide further differs to SEQ ID NO: 2 merely by the degeneration of the genetic code, or
(e) a fragment of (a), (b), or (c) having amylase activity.
Particularly preferred, the alpha-amylase variant according to the present invention having alpha-amylase activity comprises at least at least 91.0%, at least 91.5%, at least 92.0%, at least 92.5%, at least 93.0%, at least 93.5%, 94.0%, at least 94.5%, at least 95.0%, at least 95.5%, at least 96.0%, at least 96.5%, at least 97.0%, at least 97.5%, at least 98.0%, at least 98.5%, at least 99.0%, at least 99.5%, at least 99.6%, at least 99.7%, at least 99.8%, or at least 99.9%, but less than 100% sequence identity or sequence similarity to the amino acid sequence set forth in SEQ ID NO: 1. The alpha-amylase variant according to the present invention having alpha-amylase activity preferably comprises at least at least 91.0%, at least 91.5%, at least 92.0%, at least 92.5%, at least 93.0%, at least 93.5%, 94.0%, at least 94.5%, at least 95.0%, at least 95.5%, at least 96.0%, at least 96.5%, at least 97.0%, at least 97.5%, at least 98.0%, at least 98.5%, at least 99.0%, at least 99.5%, at least 99.6%, at least 99.7%, at least 99.8%, or at least 99.9%, but less than 100% sequence identity to the amino acid sequence set forth in SEQ ID NO: 1.
The alpha-amylase variant according to the present invention having alpha-amylase activity preferably comprises at least 95.0%, at least 95.5%, at least 96.0%, at least 96.5%, at least 97.0%, at least 97.5%, at least 98.0%, at least 98.5%, at least 99.0%, at least 99.5%, at least 99.6%, at least 99.7%, at least 99.8%, or at least 99.9%, but less than 100% sequence identity or sequence similarity to the amino acid sequence set forth in SEQ ID NO: 1.
The alpha-amylase variant according to the present invention having alpha-amylase activity preferably comprises at least 95.0%, at least 95.5%, at least 96.0%, at least 96.5%, at least 97.0%, at least 97.5%, at least 98.0%, at least 98.5%, at least 99.0%, at least 99.5%, at least 99.6%, at least 99.7%, at least 99.8%, or at least 99.9%, but less than 100% sequence identity to the amino acid sequence set forth in SEQ ID NO: 1.
The alpha-amylase variant according to the present invention having alpha-amylase activity preferably comprises at least 95.0% but less than 100% sequence identity to the amino acid sequence set forth in SEQ ID NO: 1.
The alpha-amylase variant according to the present invention having alpha-amylase activity preferably comprises at least 65%, at least 70%, at least 75%, at least 80%, at least 85%, at least 90%, at least 95% identity, at least 96%, at least 97%, at least 98%, or at least 99%, but less than 100% sequence identity, to the amino acid sequence set forth in SEQ ID NO: 1 and comprises an A and B domain and a C domain wherein the amino acid sequence of the A and B domain is at least 75%, at least 80%, at least 85%, at least 90%, at least 91 %, at least 92%, at least 93%, at least 94%, at least 95%, at least, 96%, at least 97%, at least 98%, or at least 99%, but less than 100%, identical to the amino acid sequence of SEQ ID NO: 6 and the amino acid sequence of the C domain is at least 75%, at least 80%, at least 85%, at least 90%, at least 91 %, at least 92%, at least 93%, at least 94%, at least 95%, at least, 96%, at least 97%, at least 98%, or at least 99%, but less than 100%, identical to the amino acid sequence of SEQ ID NO: 8.
In another embodiment, the alpha-amylase variant according to the present invention having alpha-amylase activity comprises or consists of the amino acid sequence set forth in SEQ ID NO: 1 with one or more, preferably 1 to 50, preferably 1 to 30, preferably 1 to 25, preferably 1 to 20, preferably 1 to 15, preferably 1 to 10, or preferably 1 to 5, of the amino acid substitutions described above. In another embodiment, the alpha-amylase variant according to the present invention having alpha-amylase activity comprises or consists of the amino acid sequence set forth in SEQ ID NO: 1 with one or more, preferably 1 to 50, preferably 1 to 30, preferably 1 to 25, preferably 1 to 20, preferably 1 to 15, preferably 1 to 10, or preferably 1 to 5, of the amino acid substitutions described above and with 1-50, preferably 1-30, 1-20, 1-10, or 1-5 additional amino acid alterations, preferably amino acid substitutions. In one embodiment, these 1-50 additional amino acid substitutions are conservative amino acid substitutions, preferably outside any functional domain, preferably outside the catalytically active domain, of the parent alpha-amylase. Preferably, the alpha-amylase variant according to the present invention having alpha-amylase activity comprises or consists of the amino acid sequence set forth in SEQ ID NO: 1 with one or more of the amino acid substitutions described above and with 1-30, 1-20, 1-10, or 1-5 additional amino acid alterations, preferably amino acid substitutions. In one embodiment, these 1-30, 1-20, 1-10, or 1-5 additional amino acid substitutions are conservative amino acid substitutions, preferably outside any functional domain, preferably outside the catalytically active domain, of the parent alpha-amylase.
Preferably, the alpha-amylase variant according to the present invention having alpha-amylase activity comprises or consists of the amino acid sequence set forth in SEQ ID NO: 1 with one or more of the amino acid substitutions described above and with 1-30, 1-20, 1-10, or 1-5 additional amino acid substitutions outside the catalytically active domain. Preferably, the alpha-amylase variant according to the present invention having alpha-amylase activity comprises or consists of the amino acid sequence set forth in SEQ ID NO: 1 with one or more of the amino acid substitutions described above and with 1-30, 1-20, 1-10, or 1-5 additional conservative amino acid substitutions, preferably outside the catalytically active domain.

The structure of alpha-amylases comprises three distinct domains A, B and C, *see, e.g.,* Machius et al., 1995, J. Mol. Biol. 246: 545-559. The alpha-amylase variant described herein may further comprise one or more non-catalytic CBMs (carbohydrate-binding modules, also called carbohydrate binding domain or specifically for amylases starch binding domains). CBMs can improve the association of the enzyme with the substrate. CBMs are attached to the C-domain. Preferably, the amylase of the present invention does not comprise a carbohydrate binding domain. Preferably, the alpha-amylase variant of the present invention consists only of the three domains being A, B, and C domain.
As used herein, the "A and B domain" or "AB domain" of an alpha-amylase corresponds to the amino acids aligning with the amino acids 1-399 of SEQ ID NO: 3. As used herein, the "C domain" of an alpha-amylase corresponds to amino acids aligning with the amino acids 400-485 of SEQ ID NO: 3.
Preferably, the alpha-amylase variant comprising one or more of the amino acid alteration, preferably insertion, deletion, substitution, or combinations thereof, preferably substitution, as described above is a hybrid amylase comprising its domains, in particular its AB domain and its C domain from different parent amylases. The alpha-amylase variant may be produced by substituting the C domain or a portion thereof of an alpha-amylase with the C domain or a portion thereof of another alpha-amylase. Preferably, the A and B domain of the alpha-amylase variant described herein has at least 75% identity, such as at least 78%, at least 80%, at least 85%, at least 90%, at least 91%, at least 92%, at least 93%, at least 94%, at least 95%, at least 96%, at least 97%, at least 98%, or at least 99%, but less than 100%, identity to the A and B domain of the alpha-amylase of SEQ ID NO: 3.
Preferably, the A and B domain of the alpha-amylase variant described herein has at least 75% identity, preferably at least 78%, at least 80%, at least 85%, at least 90%, at least 91%, at least 92%, at least 93%, at least 94%, at least 95%, at least 96%, at least 97%, at least 98%, or at least 99%, but less than 100%, identity to SEQ ID NO: 6, meaning that the amino acid sequence that form the A and B domain has at least 75% identity, preferably at least 78%, at least 80%, at least 85%, at least 90%, at least 91%, at least 92%, at least 93%, at least 94%, at least 95%, at least 96%, at least 97%, at least 98%, or at least 99%, but less than 100%, sequence identity to SEQ ID NO: 6.
Preferably, the C domain of the alpha-amylase variant described herein comprises a C domain having at least 75% identity, preferably at least 78%, at least 80%, at least 85%, at least 90%, at least 91%, at least 92%, at least 93%, at least 94%, at least 95%, at least 96%, at least 97%, at least 98%, or at least 99%, but less than 100%, identity to the C domain of the alpha-amylase of SEQ ID NO: 5.
Preferably, the C domain of the alpha-amylase variant described herein has at least 75% identity, preferably at least 78%, at least 80%, at least 85%, at least 90%, at least 91%, at least 92%, at least 93%, at least 94%, at least 95%, at least 96%, at least 97%, at least 98, or at least 99%, but less than 100%, identity to SEQ ID NO: 8.
In one embodiment of the present invention, the amino acid sequence forming the A and B domain has at least 75% identity, preferably at least 78%, at least 80%, at least 85%, at least 90%, at least 91 %, at least 92%, at least 93%, at least 94%, at least 95%, at least 96%, at least 97%, at least 98%, or at least 99%, but less than 100%, identity to the amino acid sequence of SEQ ID NO: 6, and the amino acid sequence forming the C domain has at least 75% identity, preferably at least 78%, at least 80%, at least 85%, at least 90%, at least 91 %, at least 92%, at least 93%, at least 94%, at least 95%, at least 96%, at least 97%, at least 98%, or at least 99%, but less than 100%, identity, to SEQ ID NO: 8.
In one embodiment of the present invention, the amino acid sequence forming the A and B domain has 100% identity to the amino acid sequence of SEQ ID NO: 6, and the amino acid sequence forming the C domain has at least 75% identity, preferably at least 78%, at least 80%, at least 85%, at least 90%, at least 91 %, at least 92%, at least 93%, at least 94%, at least 95%, at least 96%, at least 97%, at least 98%, or at least 99%, but less than 100%, identity, to SEQ ID NO: 8.
In one embodiment of the present invention, the amino acid sequence forming the A and B domain has at least 75% identity, preferably at least 78%, at least 80%, at least 85%, at least 90%, at least 91 %, at least 92%, at least 93%, at least 94%, at least 95%, at least 96%, at least 97%, at least 98%, or at least 99%, but less than 100%, identity to the amino acid sequence of SEQ ID NO: 6, and the amino acid sequence forming the C domain has 100% identity to SEQ ID NO: 8.
In one embodiment, the present invention is directed to a method of making alpha-amylase variant comprising the step of making a hybrid from at least two different amylases, wherein the hybrid comprises an A and B domain and a C domain and wherein the amino acid sequence of the A and B domain is at least 75%, preferably at least 78%, at least 80%, at least 85%, at least 90%, at least 91 %, at least 92%, at least 93%, at least 94%, at least 95%, at least 96%, at least 97%, at least 98%, or at least 99%, or 100% identical to the amino acid sequence of SEQ ID NO: 6 and the amino acid sequence of the C domain is at least 75%, preferably at least 78%, at least 80%, at least 85%, at least 90%, at least 91 %, at least 92%, at least 93%, at least 94%, at least 95%, at least 96%, at least 97%, at least 98%, or at least 99%, or 100% identical to the amino acid sequence of SEQ ID NO: 8 and introducing into the hybrid one or more amino acid alterations as described herein.

In one embodiment, the alpha-amylase variant of the present invention exhibits one or more improved property compared to the parent alpha-amylase, preferably compared to an alpha-amylase as shown in SEQ ID NO: 1 and/or SEQ ID NO: 3, preferably compared to an alpha-amylase as shown in SEQ ID NO: 1.
Preferably, the improved property is expressed as an Improvement Factor (IF) of >1.0. Preferably, for improved thermostability and improved wash performance the improvement is expressed as an Improvement Factor. Preferably, the Improvement Factor is equal or greater 1.1, equal or greater 1.2, equal or greater 1.3, equal or greater 1.4, equal or greater 1.5, equal or greater 1.6, equal or greater 1.7, equal or greater 1.8, equal or greater 1.9, or equal or greater 2.0. Preferably, the IF for wash performance is equal or greater 1.1, equal or greater 1.2, or equal or greater 1.3. Preferably, the IF for thermostability is equal or greater 1.1, equal or greater 1.2, equal or greater 1.3, equal or greater 1.5, or equal or greater 2.0.
Alternatively, the improvement of the amylase property is indicated as percentage of improvement compared to the parent alpha amylase. Preferably, the alpha-amylase variants of the present invention exhibit at least 0.5%, at least 1%, at least 2%, at least 3%, at least 5%, at least 6%, at least 7%, at least 8%, at least 9%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, at least 160%, at least 170%, at least 180%, at least 190%, or at least 200% improved property compared to the parent alpha-amylase, preferably compared to an alpha-amylase as shown in SEQ ID NO: 1 or SEQ ID NO: 3, preferably compared to SEQ ID NO: 1.
Alternatively, in particular for stability improvement, the improvement of the amylase property is indicated as residual activity after stability challenge. Preferably, storage stability is indicated as residual activity after storage under the respective storage conditions, preferably, after storage in a detergent composition (preferably in a laundry or dishwash detergent, preferably laundry detergent). Preferably, the residual activity of the alpha-amylase variant is increased compared to the parent amylase. Preferably, the residual activity of the alpha-amylase variant is at least 0.5%, at least 1%, at least 2%, at least 3%, at least 5%, at least 6%, at least 7%, at least 8%, at least 9%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, at least 160%, at least 170%, at least 180%, at least 190%, or at least 200% improved compared to the parent amylase, preferably compared to an amylase as shown in SEQ ID NO: 1 and/or 3, preferably compared to SEQ ID NO: 1. The residual activity compared to the parent amylase can also be converted into an improvement factor by forming the ratio of the residual activity of the variant vs. the residual activity of the parent amylase.
Preferably, the improved property is one or more property selected from the group consisting of an increase in expression, activity, stability, thermostability, specific activity, substrate specificity, pH-dependent activity, pH stability, oxidative stability, catalytic efficiency, catalytic rate, chemical stability, pH activity, stability under storage conditions, substrate binding, substrate cleavage, substrate stability, surface properties, thermal activity, Ca2+ dependency, performance in a detergent, performance in a laundry detergent, performance in an ADW detergent. Preferably, the improved activity is improved specific activity, substrate specificity, pH-dependent activity, catalytic efficiency, catalytic rate, pH activity, substrate binding, substrate cleavage, thermal activity, Ca2+ dependency, wash performance, wash performance of a laundry detergent, and/or wash performance of an ADW detergent. Preferably, the improved stability is improved thermostability, thermostability in a detergent composition (preferably in laundry or dishwash detergent composition, preferably laundry detergent composition), pH stability, oxidative stability, chemical stability, stability under storage conditions, substrate stability, and/or thermal activity. Preferably, the improved property is improved expression, improved solubility, improved thermostability, improved thermostability in a detergent composition, improved stability under storage in a detergent composition, and/or an improved performance wash performance. Preferably, the improved property is improved thermostability, preferably, improved thermostability in a detergent composition, improved stability under storage in a detergent composition, and/or an improved wash performance. Preferably, the improved property is improved thermostability, improved thermostability in a detergent composition, improved stability under storage conditions, preferably improved stability under storage in a detergent composition, preferably improved stability under storage in a laundry detergent and/or an improved stability under storage in an ADW detergent, improved wash performance, preferably improved wash performance of a laundry detergent, and/or improved wash performance of an ADW detergent.

The alpha-amylase variant described herein may be active over a broad temperature range, wherein the temperature is any point in the range from about 10 °C to about 95°C. The alpha-amylase variant may be active at a temperature range from 10 ° C to 55 ° C, 10 ° C to 50 ° C, 10 ° C to 45 ° C, 10 ° C to 40 ° C, 10 ° C to 35 ° C, 10 ° C to 30 ° C, or 10 ° C to 25° C The variant polypeptides may be active at a temperature range from 20 ° C to 55 ° C, 20 ° C to 50 ° C, 20 ° C to 45 ° C, 20 ° C to 40 ° C, 20 ° C to 35 ° C, 20 ° C to 30 ° C, or 20 ° C to 25° C.

In one embodiment, the alpha-amylase variant described herein improve the thermostability compared to the parent alpha-amylase, preferably, improved thermostability in a detergent composition (preferably in laundry or dishwash detergent composition, preferably laundry detergent composition). In another embodiment the variant polypeptides improve the thermostability by 1 ° C, 2 ° C, 3 ° C, 4 ° C, 5 ° C, 6 ° C, 7 ° C, 8 ° C, 9 ° C, 10 ° C, 11 ° C, 12 ° C, 13 ° C, 14 ° C, 15 ° C, 16 ° C, 17 ° C, 18 ° C, 19 ° C, 20 ° C, or more degrees C when compared to the parent alpha-amylase. In another embodiment, the thermostability increase is measured at a temperature between 40 ° C and 100° C. In one embodiment, the thermostability is improved at 40°C, at 50°C, at 60°C, at 70°C, at 80°C, or at 90°C, preferably at 60 °C or 70°C. In another embodiment, the thermostability is improved in a temperature range between 40 °C and 90°C, preferably between 60 °C and 85 °C, preferably between 60 °C and 80 °C.
Thus, preferably, the improved property is improved stability, preferably thermostability. Preferably, the alpha-amylase variant of the present invention that exhibits improved stability, preferably thermostability, compared to a parent alpha-amylase, preferably compared to SEQ ID NO: 1, is an amylase comprising amino acid alteration, preferably insertion, deletion, substitution, or combinations thereof, preferably substitution, at one or more positions (according to the numbering of SEQ ID NO: 3) corresponding to positions 1, 2, 3, 4, 5, 6, 7, 8, 10, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 45, 47, 48, 59, 60, 63, 70, 71, 72, 73, 75, 76, 81, 82, 83, 86, 89, 90, 91, 92, 93, 94, 95, 96, 97, 98, 99, 100, 103, 106, 108, 109, 113, 114, 115, 117, 119, 125, 126, 128, 129, 131, 132, 133, 134, 136, 139, 141, 142, 143, 144, 145, 146, 147, 149, 150, 151, 154, 155, 156, 160, 161, 162, 164, 165, 170, 172, 173, 174, 175, 176, 184, 188, 191, 203, 210, 211, 212, 213, 214, 215, 216, 218, 219, 221, 222, 223, 224, 225, 226, 227, 230, 231, 233, 234, 235, 236, 237, 238, 240, 242, 243, 245, 249, 250, 251, 252, 253, 255, 256, 257, 259, 261, 262, 263, 264, 265, 268, 270, 272, 273, 274, 276, 279, 280, 284, 285, 286, 287, 288, 289, 290, 291, 292, 293, 294, 295, 296, 297, 299, 299, 301, 302, 303, 304, 306, 307, 308, 309, 310, 311, 312, 313, 314, 315, 318, 318, 319, 320, 321, 323, 324, 326, 327, 336, 337, 338, 341, 342, 343, 344, 345, 346, 348, 350, 351, 352, 353, 354, 355, 357, 359, 360, 362, 363, 364, 366, 367, 368, 370, 372, 375, 376, 378, 379, 381, 382, 383, 384, 385, 387, 388, 389, 390, 391, 392, 393, 394, 396, 397, 398, 399, 400, 401, 403, 405, 406, 407, 408, 410, 413, 414, 415, 416, 418, 421, 424, 426, 427, 429, 431, 436, 438, 439, 440, 442, 443, 445, 446, 447, 448, 449, 451, 453, 455, 456, 457, 458, 459, 460, 461, 462, 463, 465, 467, 468, 470, 474, 478, 480, and 482.
Preferably, the alpha-amylase variant of the present invention that exhibits improved stability, preferably thermostability, compared to a parent alpha-amylase, preferably compared to SEQ ID NO: 1, is an amylase comprising one or more amino acid substitutions selected from the group of amino acid substitutions (according to the numbering of SEQ ID NO: 3) consisting of X1G, X1V, X1R, X2S, X2I, X3Q, X3K, X3F, X3G, X3L, X4Q, X4R, X4S, X4K, X4M, X4A, X4C, X5F, X5E, X5M, X5Y, X5R, X5Q, X5K, X5L, X5A, X5V, X5C, X5N, X5H, X5D, X5P, X5I, X6T, X6E, X6A, X6K, X6Q, X6V, X6M, X6G, X6L, X6H, X6W, X6F, X6P, X6S, X6C, X6Y, X7Y, X7S, X7H, X7C, X7Q, X7F, X7P, X7W, X7R, X7K, X7E, X7V, X7N, X8C, X8V, X8F, X8Y, X8P, X8W, X8A, X8S, X8M, X8I, X10W, X10Y, X10L, X10C, X10E, X10F, X10A, X12N, X12S, X13A, X13Y, X14N, X15R, X16F, X16R, X17K, X17V, X18R, X18M, X18F, X18K, X18T, X18I, X19P, X19L, X19F, X19Y, X19T, X19I, X19Q, X19C, X19H, X19S, X19M, X19K, X19G, X19D, X19A, X20C, X20S, X20L, X20M, X20T, X20D, X20Q, X20F, X20P, X20G, X20V, X20W, X20A, X20Y, X20H, X20K, X20E, X21S, X21E, X22E, X22F, X22A, X22D, X22M, X22T, X22K, X22Y, X22G, X22R, X22Q, X22W, X22L, X23N, X23Q, X23W, X24G, X25H, X25Y, X25K, X25F, X25S, X25C, X25D, X25A, X25G, X25W, X25Q, X25M, X25L, X26S, X26V, X26M, X26A, X26D, X26E, X26F, X26P, X26Y, X26L, X27D, X27I, X27F, X27R, X27G, X27Q, X27V, X27T, X27A, X27H, X28N, X28S, X28F, X28C, X28E, X28T, X28G, X28K, X28D, X28V, X28I, X28Q, X29Y, X29L, X29P, X29W, X29K, X29F, X29N, X29H, X29D, X29V, X29G, X29Q, X29I, X30F, X30I, X30A, X30K, X30W, X30Y, X30M, X30L, X30H, X30Q, X30G, X30E, X30T, X31S, X31V, X31Q, X31T, X31N, X31W, X32T, X32H, X32W, X32F, X32Q, X32I, X32A, X32P, X32E, X32M, X32N, X32D, X32L, X33Y, X33D, X33M, X33Q, X33K, X33R, X34H, X34V, X34I, X35C, X35Y, X35P, X35T, X35M, X35H, X35A, X35V, X35I, X35G, X35Q, X35D, X35N, X35R, X35S, X35L, X36V, X36I, X36T, X36K, X36A, X36S, X36R, X36M, X36E, X36P, X36G, X36N, X36Q, X37P, X37V, X37W, X37A, X37G, X38N, X39E, X39K, X40I, X40S, X41G, X41Q, X41C, X41D, X41E, X41S, X41T, X42Q, X42V, X42I, X42C, X45N, X45G, X47S, X48F, X48I, X48M, X51T, X59T, X60T, X63V, X63C, X70N, X70L, X70M, X70F, X70Y, X71D, X72T, X72E, X72D, X72N, X72C, X73Q, X73L, X73N, X75A, X75I, X75W, X75P, X75G, X75T, X76G, X76T, X76C, X76V, X76E, X76L, X81H, X82M, X82K, X83G, X83R, X83S, X86K, X89F, X89A, X89C, X89L, X89G, X89M, X89S, X89R, X90Q, X90F, X90G, X90E, X90A, X90N, X90I, X90R, X90Y, X90V, X90S, X90M, X90D, X91S, X91Y, X91K, X91Q, X91F, X91H, X91T, X91I, X91W, X91N, X91V, X91L, X91C, X91G, X91D, X91E, X91M, X92V, X92M, X92D, X93K, X93Q, X94M, X94K, X94Y, X94D, X94A, X94V, X95V, X95I, X95E, X95L, X96N, X96K, X97V, X98G, X98E, X98N, X99K, X99H, X99N, X100D, X100K, X100L, X100F, X103A, X106D, X108A, X109A, X113Y, X113H, X113M, X113V, X113F, X113W, X113R, X114D, X114H, X114N, X114W, X114E, X114C, X114G, X114L, X114M, X114Q, X114F, X114A, X114V, X114K, X114S, X114Y, X114R, X114I, X115C, X115K, X115N, X115T, X115M, X115S, X115Q, X115R, X115D, X115V, X116L, X117D, X117Y, X117N, X117C, X117R, X117W, X117A, X117I, X117P, X117F, X119Y, X119R, X119P, X119H, X125A, X125K, X125L, X125T, X125R, X125F, X125W, X125Q, X125V, X125Y, X125H, X125N, X125E, X125G, X125M, X126D, X128C, X128L, X128Y, X128E, X128W, X128M, X129E, X131I, X131C, X132T, X133E, X133P, X133C, X133D, X133Q, X133S, X133H, X133A, X133N, X133K, X134Y, X134W, X134F, X134P, X134E, X134V, X134L, X134M, X134T, X134C, X134I, X135M, X136D, X136E, X136F, X136M, X136P, X136H, X136C, X136A, X136W, X136L, X136N, X139C, X141V, X142C, X142W, X142Y, X142L, X142M, X142Q, X142F, X142E, X142R, X143F, X144Q, X144C, X144V, X144K, X144T, X144Y, X144E, X144G, X144N, X144S, X144R, X145A, X146C, X146H, X146K, X146T, X146E, X146S, X146D, X146W, X146F, X146L, X146G, X147M, X149E, X150Q, X150E, X150C, X151C, X151E, X154Y, X154A, X155Y, X156V, X160W, X161T, X162V, X164V, X165W, X169E, X169V, X170F, X170C, X172C, X172K, X172A, X173I, X173Y, X174P, X174M, X174E, X174S, X174H, X175G, X175Q, X175H, X176K, X184G, X188V, X188H, X191K, X191H, X191F, X191W, X191Y, X203E, X203R, X210D, X210C, X210E, X210N, X210F, X210Y, X210M, X210Q, X210S, X210A, X211D, X211N, X211C, X211T, X211E, X211G, X211V, X211Q, X211A, X211H, X211L, X211S, X212W, X212L, X212I, X212D, X212C, X213A, X213S, X213M, X213R, X213C, X214P, X214E, X215E, X215D, X215H, X215W, X215A, X216H, X216G, X218Q, X218E, X218C, X218N, X218F, X218S, X218G, X218K, X218I, X218T, X218D, X218Y, X218H, X218L, X218V, X218A, X218W, X219E, X219Q, X219K, X219T, X219M, X219D, X219F, X219S, X219R, X219A, X219Y, X219H, X219W, X219G, X219C, X221N, X221F, X222S, X222T, X222D, X223V, X223Y, X223L, X223C, X224V, X224F, X225A, X225R, X225N, X225E, X225H, X225F, X225C, X225Y, X225I, X226C, X226W, X226I, X226R, X226Y, X226D, X226L, X226M, X226G, X226T, X226Q, X226S, X226A, X226V, X226E, X226H, X227W, X227C, X227T, X227R, X227M, X227K, X227Y, X227V, X227F, X227I, X227H, X227L, X227G, X230A, X230F, X231N, X231C, X233T, X233W, X233H, X233C, X233I, X233Y, X233M, X234C, X235M, X235V, X235L, X236Y, X237C, X237I, X238T, X240M, X242M, X243F, X245E, X245M, X245H, X249D, X249I, X250V, X251E, X251A, X251L, X251S, X251M, X251F, X252C, X252I, X252S, X253Y, X253G, X255T, X255V, X255D, X255F, X255I, X256C, X257Y, X257L, X257V, X259L, X261R, X262D, X262Y, X262C, X262P, X262E, X262H, X263I, X264T, X264W, X264H, X265S, X268G, X270A, X270Y, X272L, X272G, X273H, X273V, X273L, X273M, X273C, X273D, X273W, X273F, X273A, X273E, X273P, X273Y, X273R, X273I, X274S, X276Y, X276K, X276L, X276D, X279P, X280I, X280V, X280N, X280H, X280Y, X280K, X280R, X284Y, X284A, X284L, X284F, X284H, X284N, X284M, X285N, X285L, X285G, X285P, X286Q, X287E, X287H, X287A, X287D, X287T, X288K, X288P, X288Y, X288A, X289T, X289R, X289G, X289F, X290Q, X290N, X290M, X290D, X290W, X291D, X291Y, X291K, X291T, X292W, X292Y, X292F, X292D, X292L, X292I, X292T, X292C, X293D, X293R, X293K, X293E, X293F, X294T, X294G, X295F, X296Y, X296L, X296C, X296A, X297M, X297K, X297S, X297H, X297V, X297E, X297F, X299L, X299G, X299I, X299K, X299S, X299Y, X301F, X302H, X302I, X302Q, X302Y, X302V, X303P, X303M, X303E, X303I, X303R, X303K, X303N, X303L, X303H, X303T, X304H, X304A, X304R, X304Y, X304D, X304N, X304M, X304E, X304P, X304W, X304K, X304T, X306M, X306E, X306A, X306G, X306W, X306V, X306S, X306I, X306H, X306T, X306D, X306Q, X306Y, X306R, X307F, X307M, X308S, X309H, X309L, X309Q, X310Q, X310A, X311G, X311E, X311H, X311A, X311K, X311T, X311N, X311R, X311Y, X312M, X312L, X313V, X314C, X314Q, X315C, X315A, X315H, X315T, X315E, X315K, X318T, X318S, X318I, X319K, X319H, X319P, X319A, X319I, X319D, X319M, X319S, X319N, X319T, X319W, X320G, X320Y, X320L, X320N, X320A, X320D, X320K, X320Q, X320E, X320H, X320C, X320S, X321E, X321W, X321T, X321A, X321N, X321V, X321K, X323K, X323G, X324W, X324K, X324Y, X326Y, X326N, X326G, X326S, X327M, X327C, X327L, X333I, X336K, X337N, X337K, X337M, X337T, X337V, X337Q, X337S, X337R, X337I, X337Y, X337G, X337F, X337A, X337L, X338G, X338T, X338S, X341V, X342P, X343W, X343Y, X344I, X344Q, X344V, X345N, X345T, X345M, X345G, X345S, X345D, X345Q, X346D, X346C, X346H, X346A, X346G, X346Q, X346N, X348T, X350P, X350H, X350K, X351M, X351A, X352S, X353H, X354Y, X354N, X354T, X354I, X355I, X355M, X357A, X358P, X359E, X360N, X360R, X360S, X360Y, X360V, X360L, X360A, X360I, X360T, X360G, X360F, X362T, X362M, X362V, X362N, X362Y, X362F, X362K, X363M, X363G, X363S, X363V, X363C, X363T, X363H, X363P, X363D, X363L, X363R, X363Q, X363E, X363Y, X363K, X363W, X363A, X364G, X364T, X364S, X364A, X364V, X364C, X364K, X364L, X364N, X366I, X366L, X366T, X367S, X367E, X368N, X368L, X368A, X370E, X370I, X372C, X372H, X372A, X372F, X372M, X372Q, X372N, X372E, X375K, X375A, X375D, X375E, X375R, X375T, X375I, X375W, X375Y, X375Q, X376S, X376R, X376M, X376I, X376V, X376Q, X376L, X378E, X378R, X378D, X378C, X379L, X379A, X379S, X381V, X381E, X382N, X382H, X382Q, X382K, X382A, X382S, X383R, X383I, X383D, X383V, X383M, X383E, X383N, X383S, X383Q, X383Y, X383C, X383H, X384W, X384Y, X384V, X384R, X384N, X384E, X384A, X384D, X384T, X384C, X384F, X384L, X384I, X384M, X384Q, X385G, X385V, X385T, X385Y, X385I, X385D, X385C, X385Q, X385A, X385W, X385L, X385H, X385M, X385N, X385S, X385F, X385E, X385R, X385P, X387N, X387C, X387E, X388R, X388F, X388H, X388V, X388E, X388M, X388I, X389G, X389K, X389H, X390P, X390N, X390R, X390F, X390M, X390D, X391T, X391F, X391S, X391A, X391G, X391M, X391N, X391Y, X391Q, X391K, X392C, X392V, X393V, X393E, X393H, X393S, X393P, X394C, X394L, X394R, X394S, X394A, X394E, X394N, X394M, X394H, X394I, X395V, X395M, X395A, X395H, X396P, X396H, X397P, X397D, X397S, X397H, X398M, X399P, X400L, X400P, X400I, X400S, X400A, X400R, X400Q, X400D, X400K, X400N, X400V, X400W, X400H, X400E, X400G, X400M, X401M, X401K, X401T, X401I, X403N, X405H, X405V, X405T, X405C, X406P, X407S, X407R, X407D, X408Q, X408E, X408I, X410R, X410K, X410I, X410L, X410H, X413S, X414E, X414C, X414S, X415I, X415E, X416S, X418C, X418P, X418N, X421P, X421N, X424A, X426D, X426W, X427Q, X427C, X427F, X427T, X427V, X427G, X427S, X427R, X427K, X429M, X429D, X429E, X429N, X429T, X429A, X429P, X429W, X429Q, X429S, X429F, X429G, X429I, X429V, X431I, X436E, X438P, X438F, X438Y, X439C, X439P, X440V, X442Q, X443T, X443H, X445M, X445T, X445A, X445G, X445S, X445F, X445R, X445Q, X445C, X445D, X445K, X445H, X445V, X446P, X446I, X446V, X446F, X446L, X446R, X446Q, X446W, X447V, X448E, X448H, X448N, X448D, X449V, X449Y, X449F, X449L, X449P, X449G, X449W, X449M, X449R, X449N, X449K, X449Q, X449S, X449A, X451L, X453G, X453I, X453P, X453Y, X453N, X455L, X456M, X456S, X456Y, X456V, X456L, X456W, X456R, X457K, X457C, X457L, X457S, X457R, X457F, X457T, X457A, X457V, X457W, X457E, X457M, X457G, X458V, X458W, X458I, X458K, X459Q, X459D, X459I, X459K, X459C, X459V, X459E, X459Y, X460E, X460Q, X460S, X460R, X460A, X460T, X460D, X460V, X461A, X461R, X461F, X461E, X461S, X461V, X461M, X461K, X461N, X461Q, X462K, X463L, X465P, X465V, X465H, X465R, X467A, X467E, X467H, X468H, X468D, X470Q, X470T, X470A, X474P, X474H, X474F, X478A, X478E, X480Y, X480M, X480E, X480D, X480R, X482T, and X482C.

Preferably, the improved property is improved stability in a detergent composition, preferably a laundry detergent composition, preferably improved thermostability in a detergent composition. Preferably, the alpha-amylase variant of the present invention that exhibits improved stability, preferably thermostability, in a detergent composition compared to a parent alpha-amylase, preferably compared to SEQ ID NO: 1, is an amylase comprising amino acid substitution at one or more positions (according to the numbering of SEQ ID NO: 3) corresponding to positions selected from the group consisting of 4, 6, 20, 25, 27, 28, 33, 38, 41, 70, 72, 73, 75, 81, 93, 94, 96, 115, 125, 126, 128, 134, 139, 160, 174, 175, 176, 210, 213, 250, 251, 253, 276, 291, 292, 299, 314, 318, 319, 323, 326, 338, 343, 352, 356, 359, 363, 364, 368, 372, 378, 379, 382, 385, 387, 388, 390, 393, 396, 398, 400, 405, 418, 434, 447, 449, 459, 460, 461, and 465. Preferably, the alpha-amylase variant of the present invention that exhibits improved stability, preferably thermostability, in a detergent composition compared to a parent alpha-amylase, preferably compared to SEQ ID NO: 1, is an amylase comprising one or more amino acid substitutions selected from the group of amino acid substitutions (according to the numbering of SEQ ID NO: 3) consisting of X4Q, X6E, X6H, X20K, X25H, X25Y, X27I, X28V, X33M, X38V, X41C, X70H, X72C, X73C, X75L, X81L, X93Q, X94M, X96Q, X115T, X125K, X125R, X126D, X128C, X134F, X134Y, X139C, X160W, X174H, X175A, X176K, X210C, X210D, X213C, X250I, X251E, X253C, X276R, X291T, X292C, X299S, X314Q, X318L, X319K, X323G, X326Y, X338S, X338T, X343W, X352C, X356V, X359W, X363M, X363S, X364T, X368F, X372C, X378E, X379A, X379R, X382Q, X385W, X387E, X387N, X387S, X388E, X388K, X388Y, X390F, X393Q, X396S, X398G, X400A, X400S, X405M, X418F, X434S, X434R, X447L, X449G, X459N, X460G, X461L, X461A, and X465H.

Preferably, the alpha-amylase variant of the present invention that exhibits improved stability, preferably thermostability, in a detergent composition compared to a parent alpha-amylase, preferably compared to SEQ ID NO: 1, is an amylase comprising a combination of substitutions (according to the numbering of SEQ ID NO: 3) selected from the group consisting of X25H+X176K+X186E+X206Y+X251E+X482W, X4Q+X25H+X176K+X186E+X206Y+X251E+X405M+X482W, X25H+X176K+X186E+X206Y+X482W, X25H+X186E+X206Y+X405M+X482W, X25H+X186E+X206Y+X482W, X25H+X176K+X186E+X193E+X206Y+X251E+X482W, X4Q+X176K+X186E+X193E+X206Y+X251E+X405M+X482W, X25H+X176K+X186E+X482W, X25H+X176K+X186E+X193E+X206Y+X482W, X4Q+X25H+X176K+X186E+X251E+X405M+X482W, X25H+X176K+X186E+X206Y, X4Q+X176K+X186E+X193E+X206Y+X251E+X405M, X4Q+X206Y+X460G+X482W, X4Q+X25H+X176K+X206Y+X251E+X460G+X482W, X4Q+X25H+X176K+X193E+X251E+X186E+X482W, X4Q+X193E+X206Y+X276R+X186E+X482W, X186E+X25H+X482W, X25H+X193E+X206Y+X251E+X276R+X405M+X186E+X482W, X25H+X251 E+X186E+X482W, X25H+X160W+X176K+X186E+X251E+X405M+X482W, X193E+X206Y+X405M+X186E+X482W, X3I+X356V, X3I+X356I, X83D+X94E, X94D+X125E, X131I+X377Q+X410H, X48F+X94D, X48Y+X116K+X218K, X5L+X218K+X225S, X83E+X116R+X158Y+X181E, X51V+X218K, X83E+X181E, X4Q+X7W+X176K+X186E+X193E+X206Y+X251E+X405M, X4Q+X37M+X176K+X186E+X193E+X206Y+X251E+X405M, X4Q+X25D+X176K+X186E+X193E+X206Y+X251E+X405M, X4Q+X25H+X176K+X186E+X206Y+X251E+X405M+X460G, X4Q+X25H+X176K+X186E+X206Y+X251E+X460G, X4Q+X25Y+X176K+X186E+X193E+X206Y+X251E+X405M, X4Q+X118D+X176K+X186E+X193E+X206Y+X251E+X405M, X4Q+X176K+X182D+X186E+X193E+X206Y+X251E+X405M, X4Q+X176K+X186E+X193E+X206Y+X251E+X258Q+X405M, X4Q+X176K+X186E+X193E+X206Y+X251E+X405M, X4Q+X176K+X186E+X193E+X206Y+X251E+X405M+X460G, X4Q+X176K+X186E+X193E+X206Y+X251E+X363E+X405M, X4Q+X176K+X186E+X193E+X206Y+X251E+X363H+X405M, X4Q+X176K+X186E+X193E+X206Y+X251E+X363N+X405M, X4Q+X176K+X181D+X186E+X193E+X206Y+X251E+X405M, X4Q+X176K+X181F+X186E+X193E+X206Y+X251E+X405M, X4Q+X176K+X181N+X186E+X193E+X206Y+X251E+X405M, X4Q+X176K+X181Q+X186E+X193E+X206Y+X251E+X405M, X4Q+X136E+X176K+X186E+X193E+X206Y+X251E+X405M, X4Q+X100W+X176K+X186E+X193E+X206Y+X251E+X405M, X4Q+X135D+X176K+X186E+X193E+X206Y+X251E+X405M, X4Q+X135E+X176K+X186E+X193E+X206Y+X251E+X405M, X4Q+X135T+X176K+X186E+X193E+X206Y+X251E+X405M, X4Q+X135W+X176K+X186E+X193E+X206Y+X251E+X405M, X4Q+X160D+X176K+X186E+X193E+X206Y+X251E+X405M, X4Q+X160E+X176K+X186E+X193E+X206Y+X251E+X405M, X7H+X25H+X176K+X186E+X206Y, X7W+X25H+X176K+X186E+X206Y, X25D+X176K+X186E+X206Y, X25H+X186E+X206Y+X405M+X460G, X25H+X186E+X206Y+X460G, X25H+X37M+X176K+X186E+X206Y, X25H+X118D+X176K+X186E+X206Y, X25H+X176K+X182D+X186E+X206Y, X25H+X176K+X186E+X206Y, X25H+X176K+X186E+X206Y+X258Q, X25H+X176K+X186E+X206Y+X281N, X25H+X176K+X186E+X206Y+X460G, X25H+X176K+X186E+X206Y+X251E+X460G,X25H+X176K+X186E+X206Y+X363E, X25H+X176K+X186E+X206Y+X363H,X25H+X176K+X186E+X206Y+X363N, X25H+X176K+X186E+X460G, X25H+X176K+X186E+X193E+X206Y, X25H+X176K+X186E+X193E+X206Y+X460G, X25H+X176K+X186E+X193E+X206Y+X251E+X460G, X25H+X176K+X181D+X186E+X206Y, X25H+X176K+X181N+X186E+X206Y, X25H+X176K+X181Q+X186E+X206Y, X25H+X136E+X176K+X186E+X206Y, X25H+X100W+X176K+X186E+X206Y, X25H+X135E+X176K+X186E+X206Y, X25H+X135T+X176K+X186E+X206Y, X25H+X135W+X176K+X186E+X206Y, X25H+X160D+X176K+X186E+X206Y, X25H+X160E+X176K+X186E+X206Y, X25Y+X176K+X186E+X206Y, X87D+X186E+X315K+X420K, X87D+X186E+X226D+X314E+X420E+X461E, X87D+X186E+X226D+X420E, X87D+X186E+X226D+X420E+X461E, X87D+X186E+X314E+X471E, X87D+X186E+X311K+X314E+X420K, X87D+X186E+X160D+X461E,X87R+X186E+X315K+X461R,X87R+X186E+X226D+X471E, X87R+X186E+X226R+X314K+X420K+X461R,X87R+X186E+X226R+X311K+X420K, X87R+X186E+X314K+X315K, X87R+X186E+X311K+X314E, X87R+X186E+X420E+X450R, X87R+X186E+X450R+X452R,X186E+X315K+X420E+X452R, X186E+X226D+X314E+X461E+X471E,X186E+X226D+X314E+X452R, X186E+X226D+X314K+X460D+X471E+X485R, X186E+X226D+X311K+X460D, X186E+X226D+X461E+X471E,X186E+X314E+X460D+X461E, X186E+X314E+X420E+X452R+X461E, X186E+X314K+X450R+X460D, X186E+X460D+X471E+X485R, X186E+X311K+X314K+X452R, X186E+X311K+X461 E+X485R, X186E+X311 K+X420E+X471 E, X186E+X420K+X450R+X471E+X485R, X186E+X420K+X450R+X485R, X186E+X452R+X461E+X471E, X4Q+X176K+X186E+X193E+X206Y+X251E+X405M, X25H+X176K+X186E+X206Y, X83E+X186E+X219D+X226R, X83E+X186E+X219R+X226D+X314E+X452R, X83E+X186E+X219R+X226R, X83E+X186E+X160D+X219D+X226R+X452R, X83R+X186E+X219R+X226D, X160D+X186E+X315K+X450R, X160D+X186E+X226D+X314K+X460D+X461E, X160D+X186E+X226D+X314K+X420E, X160D+X186E+X314K+X485R, X160D+X186E+X420E+X452R, X160D+X186E+X420K+X460D,X186E+X276R, X186E+X206Y, X186E+X206Y+X276R, X186E+X206Y+X276R+X405M, X186E+X206Y+X405M, X186E+X206Y+X251E, X186E+X206Y+X251E+X276R, X186E+X206Y+X251E+X276R+X405M, X186E+X206Y+X251E+X405M, X186E+X206Y+X251E+X323G+X405M, X186E+X206Y+X323G+X405M, X186E+X405M, X186E+X193E+X206Y+X405M, X186E+X193E+X206Y+X251E, X186E+X251E, X186E+X251E+X405M, X4Q+X186E, X4Q+X186E+X276R, X4Q+X186E+X206Y, X4Q+X186E+X206Y+X276R+X405M, X4Q+X186E+X206Y+X405M, X4Q+X186E+X206Y+X251E+X276R, X4Q+X186E+X206Y+X251E+X276R+X405M, X4Q+X186E+X206Y+X251E+X405M, X4Q+X186E+X206Y+X251E+X323G+X405M, X4Q+X186E+X405M, X4Q+X186E+X193E+X206Y+X276R, X4Q+X186E+X193E+X206Y+X405M, X4Q+X186E+X193E+X206Y+X251E+X405M, X4Q+X186E+X251E+X276R+X405M, X4Q+X25H+X186E+X206Y, X4Q+X25H+X186E+X206Y+X276R, X4Q+X25H+X186E+X206Y+X276R+X405M, X4Q+X25H+X186E+X206Y+X405M, X4Q+X25H+X186E+X206Y+X251E+X323A+X405M, X4Q+X25H+X186E+X206Y+X323G+X405M, X4Q+X25H+X186E+X405M, X4Q+X25H+X176K+X186E+X206Y+X276R, X4Q+X25H+X176K+X186E+X206Y+X276R+X405M, X4Q+X25H+X176K+X186E+X206Y+X251E+X276R+X405M, X4Q+X25H+X176K+X186E+X206Y+X251E+X405M, X4Q+X25H+X176K+X186E+X251E, X4Q+X25H+X176K+X186E+X251E+X405M, X4Q+X25H+X176K+X206Y, X4Q+X25H+X176K+X206Y+X405M N460G, X4Q+X25H+X176K+X206Y+X460G, X4Q+X25H+X176K+X206Y+X251E, X4Q+X25H+X176K+X206Y+X251E+X276R+X405M, X4Q+X25H+X176K+X206Y+X251E+X460G, X4Q+X25H+X176K+X206Y+X323G, X4Q+X25H+X160W+X186E+X206Y+X405M, X4Q+X25H+X160W+X186E+X206Y+X251E, X4Q+X25H+X160W+X186E+X206Y+X251E+X276R+X405M, X4Q+X25H+X160W+X186E+X206Y+X323G+X405M, X4Q+X25H+X160W+X186E+X405M, X4Q+X25H+X160W+X186E+X193E+X206Y+X276R, X4Q+X25H+X160W+X186E+X323G, X4Q+X25H+X160W+X169R+X186E+X206Y+X276R, X4Q+X25H+X160W+X176K+X186E+X276R+X405M, X4Q+X25H+X160W+X176K+X186E+X206Y+X276R, X4Q+X25H+X160W+X176K+X186E+X206Y+X251E, X4Q+X25H+X160W+X176K+X186E+X206Y+X251E+X405M, X4Q+X25H+X160W+X176K+X186E+X405M, X4Q+X25H+X160W+X176K+X186E+X251E+X276R+X405M, X4Q+X25H+X160W+X176K+X186E+X251E+X405M, X4Q+X25H+X160W+X176K+X206Y, X4Q+X25H+X160W+X176K+X206Y+X405M, X4Q+X25H+X160W+X176K+X206Y+X460G, X4Q+X25H+X160W+X176K+X206Y+X251E+X460G, X4Q+X176K+X186E+X276R+X405M, X4Q+X176K+X186E+X206Y+X276R, X4Q+X176K+X186E+X206Y+X276R+X405M, X4Q+X176K+X186E+X206Y+X405M, X4Q+X176K+X186E+X206Y+X251E+X276R+X405M, X4Q+X176K+X186E+X206Y+X251E+X405M, X4Q+X176K+X186E+X405M, X4Q+X176K+X186E+X193E+X206Y+X276R+X405M, X4Q+X176K+X186E+X193E+X206Y+X251E+X405M, X4Q+X176K+X186E+X251E+X276R+X405M, X4Q+X176K+X186E+X251E+X405M, X4Q+X176K+X186E+X323G+X405M, X4Q+X176K+X206Y, X4Q+X176K+X206Y+X276R, X4Q+X176K+X206Y+X405M, X4Q+X176K+X206Y+X405M N460G, X4Q+X176K+X206Y+X460G, X4Q+X176K+X206Y+X251E+X276R+X405M, X4Q+X176K+X206Y+X251E+X276R+X405M N460G, X4Q+X176K+X206Y+X251E+X405M, X4Q+X176K+X206Y+X251E+X405M N460G, X4Q+X176K+X206Y+X251E+X323A+X460G, X4Q+X160W+X186E+X276R+X405M, X4Q+X160W+X186E+X206Y, X4Q+X160W+X186E+X206Y+X276R+X323G, X4Q+X160W+X186E+X206Y+X405M, X4Q+X160W+X186E+X206Y+X251E, X4Q+X160W+X186E+X206Y+X251E+X276R+X405M, X4Q+X160W+X186E+X405M, X4Q+X160W+X186E+X251E+X405M, X4Q+X160W+X176K+X186E, X4Q+X160W+X176K+X186E+X276R+X405M, X4Q+X160W+X176K+X186E+X206Y+X251E, X4Q+X160W+X176K+X186E+X251E+X276R+X405M, X4Q+X160W+X176K+X186E+X251E+X405M, X4Q+X160W+X176K+X206Y, X4Q+X160W+X176K+X206Y+X251E+X276R+X460G, X4Q+X160W+X176K+X206Y+X251E+X323G+X405M+X460G, X25H+X186E, X25H+X186E+X206Y, X25H+X186E+X206Y+X276R+X405M, X25H+X186E+X206Y+X276R+X323G,X25H+X186E+X206Y+X405M, X25H+X186E+X206Y+X251E+X276R+X323G+X405M, X25H+X186E+X206Y+X251E+X405M, X25H+X186E+X405M, X25H+X186E+X2021+X206Y+X405M, X25H+X186E+X193E+X206Y+X276R+X405M, X25H+X186E+X193E+X206Y+X405M, X25H+X186E+X193E+X206Y+X251E+X276R+X405M, X25H+X186E+X251E, X25H+X186E+X251E+X276R,X25H+X176K+X186E, X25H+X176K+X186E+X276R+X323G+X405M, X25H+X176K+X186E+X206Y, X25H+X176K+X186E+X206Y+X276R, X25H+X176K+X186E+X206Y+X251E, X25H+X176K+X186E+X206Y+X251E+X276R+X405M, X25H+X176K+X186E+X206Y+X251E+X405M, X25H+X176K+X186E+X405M, X25H+X176K+X186E+X193E+X206Y, X25H+X176K+X186E+X193E+X206Y+X251E, X25H+X176K+X186E+X193E+X206Y+X251E+X276R+X405M, X25H+X176K+X186E+X251 E+X276R, X25H+X176K+X206Y, X25H+X176K+X206Y+X276R, X25H+X176K+X206Y+X276R+X405M, X25H+X176K+X206Y+X276R+X460G, X25H+X176K+X206Y+X405M, X25H+X176K+X206Y+X460G, X25H+X176K+X206Y+X251E, X25H+X176K+X206Y+X251E+X276R+X405M+X460G, X25H+X176K+X206Y+X251E+X276R+X460G, X25H+X176K+X206Y+X251E+X405M, X25H+X160W+X186E+X206Y, X25H+X160W+X186E+X206Y+X251E, X25H+X160W+X186E+X206Y+X251E+X276R, X25H+X160W+X186E+X206Y+X251E+X276R+X323G+X405M, X25H+X160W+X186E+X206Y+X251E+X405M, X25H+X160W+X186E+X206Y+X323G+X405M, X25H+X160W+X186E+X251E+X276R+X405M, X25H+X160W+X176K+X186E+X206Y+X276R, X25H+X160W+X176K+X186E+X206Y+X276R+X405M, X25H+X160W+X176K+X186E+X206Y+X251E+X276R+X323G, X25H+X160W+X176K+X186E+X206Y+X251E+X405M, X25H+X160W+X176K+X186E+X405M, X25H+X160W+X176K+X186E+X193E+X206Y, X25H+X160W+X176K+X186E+X193E+X206Y+X405M, X25H+X160W+X176K+X186E+X193E+X206Y+X251E+X405M, X25H+X160W+X176K+X186E+X251E, X25H+X160W+X176K+X186E+X251E+X276R, X25H+X160W+X176K+X186E+X251E+X405M, X25H+X160W+X176K+X186E+X323G+X405M, X25H+X160W+X176K+X206Y+X276R+X323G+X405M, X25H+X160W+X176K+X206Y+X460G, X25H+X160W+X176K+X206Y+X251E, X25H+X160W+X176K+X206Y+X251E+X323G+X405M, X176K+X186E+X206Y, X176K+X186E+X206Y+X276R, X176K+X186E+X206Y+X276R+X405M, X176K+X186E+X206Y+X405M, X176K+X186E+X206Y+X251E+X405M, X176K+X186E+X405M,X176K+X186E+X193E+X206Y+X405M, X176K+X186E+X193E+X206Y+X251E+X405M, X176K+X186E+X193E+X405M, X176K+X186E+X251E+X276R+X405M, X176K+X186E+X251E+X405M, X176K+X206Y, X176K+X206Y+X276R, X176K+X206Y+X276R+X460G, X176K+X206Y+X405M, X176K+X206Y+X405M N460G, X176K+X206Y+X460G, X176K+X206Y+X251E, X176K+X206Y+X251E+X405M, X160W+X186E, X160W+X186E+X276R+X405M, X160W+X186E+X206Y+X276R, X160W+X186E+X206Y+X276R+X405M, X160W+X186E+X206Y+X405M, X160W+X186E+X206Y+X251E, X160W+X186E+X206Y+X251E+X405M, X160W+X186E+X405M, X160W+X186E+X193E+X206Y, X160W+X186E+X193E+X206Y+X405M, X160W+X186E+X251E+X276R, X160W+X186E+X251E+X276R+X405M, X160W+X186E+X323G+X405M, X160W+X176K+X186E+X276R+X405M, X160W+X176K+X186E+X206Y, X160W+X176K+X186E+X206Y+X251E+X405M, X160W+X176K+X186E+X405M, X160W+X176K+X186E+X193E+X206Y, X160W+X176K+X186E+X193E+X206Y+X405M, X160W+X176K+X186E+X193E+X206Y+X251E, X160W+X176K+X206Y+X276R+X405M N460G, X160W+X176K+X206Y+X276R+X323G+X460G, X160W+X176K+X206Y+X405M, X160W+X176K+X206Y+X405M N460G, X160W+X176K+X206Y+X460G, X160W+X176K+X206Y+X251E+X276R, X160W+X176K+X206Y+X251E+X405M+X460G, X160W+X176K+X206Y+X323G+X405M+X460G, X4Q+X176K+X186E+X193E+X206Y+X251E+X405M, X4Q+X176K+X186E+X193E+X206Y+X251E+X405M+X258Q, X4Q+X176K+X186E+X193E+X206Y+X251E+X405M+X258Q+X281 N, X4Q+X176K+X186E+X193E+X206Y+X251E+X405M+X258Q+X281 N+X363H, X4Q+X176K+X186E+X193E+X206Y+X251E+X405M+X258Q+X363E, X4Q+X176K+X186E+X193E+X206Y+X251E+X405M+X281N, X4Q+X176K+X186E+X193E+X206Y+X251E+X405M+X281N+X363E, X4Q+X176K+X186E+X193E+X206Y+X251E+X405M+X281N+X363H, X4Q+X176K+X186E+X193E+X206Y+X251E+X405M+X181Q, X4Q+X176K+X186E+X193E+X206Y+X251E+X405M+X181Q+X258Q, X4Q+X176K+X186E+X193E+X206Y+X251E+X405M+X181Q+X258Q+X281N, X4Q+X176K+X186E+X193E+X206Y+X251E+X405M+X181Q+X258Q+X281N+X363H, X4Q+X176K+X186E+X193E+X206Y+X251E+X405M+X181Q+X281N, X4Q+X176K+X186E+X193E+X206Y+X251E+X405M+X181Q+X281N+X363E, X4Q+X176K+X186E+X193E+X206Y+X251E+X405M+X181Q+X363E, X4Q+X176K+X186E+X193E+X206Y+X251E+X405M+X181Q+X363H, X4Q+X176K+X186E+X193E+X206Y+X251E+X405M+X100W, X4Q+X176K+X186E+X193E+X206Y+X251E+X405M+X100W+X258Q, X4Q+X176K+X186E+X193E+X206Y+X251E+X405M+X100W+X258Q+X281N, X4Q+X176K+X186E+X193E+X206Y+X251E+X405M+X100W+X258Q+X281N+X363E, X4Q+X176K+X186E+X193E+X206Y+X251E+X405M+X100W+X258Q+X281N+X363H, X4Q+X176K+X186E+X193E+X206Y+X251E+X405M+X100W+X258Q+X281N+X363H+X272V, X4Q+X176K+X186E+X193E+X206Y+X251E+X405M+X100W+X258Q+X363E, X4Q+X176K+X186E+X193E+X206Y+X251E+X405M+X100W+X258Q+X363H, X4Q+X176K+X186E+X193E+X206Y+X251E+X405M+X100W+X281N, X4Q+X176K+X186E+X193E+X206Y+X251E+X405M+X100W+X281N+X363E, X4Q+X176K+X186E+X193E+X206Y+X251E+X405M+X100W+X281N+X363H, X4Q+X176K+X186E+X193E+X206Y+X251E+X405M+X100W+X181Q, X4Q+X176K+X186E+X193E+X206Y+X251E+X405M+X100W+X181Q+X258Q, X4Q+X176K+X186E+X193E+X206Y+X251E+X405M+X100W+X181Q+X258Q+X281N, X4Q+X176K+X186E+X193E+X206Y+X251E+X405M+X100W+X181Q+X258Q+X281N+X363E, X4Q+X176K+X186E+X193E+X206Y+X251E+X405M+X100W+X181Q+X258Q+X363E, X4Q+X176K+X186E+X193E+X206Y+X251E+X405M+X100W+X181Q+X258Q+X363H, X4Q+X176K+X186E+X193E+X206Y+X251E+X405M+X100W+X181Q+X281N, X4Q+X176K+X186E+X193E+X206Y+X251E+X405M+X100W+X181Q+X281N+X363H, X4Q+X176K+X186E+X193E+X206Y+X251E+X405M+X100W+X181Q+X363H, X4Q+X176K+X186E+X193E+X206Y+X251E+X405M+X100W+X181Q+X363H+X254Q, X4Q+X176K+X186E+X193E+X206Y+X251E+X405M+X100W+X135T, X4Q+X176K+X186E+X193E+X206Y+X251E+X405M+X100W+X135T+X258Q, X4Q+X176K+X186E+X193E+X206Y+X251E+X405M+X100W+X135T+X258Q+X281N+X363H, X4Q+X176K+X186E+X193E+X206Y+X251E+X405M+X100W+X135T+X258Q+X363E, X4Q+X176K+X186E+X193E+X206Y+X251E+X405M+X100W+X135T+X258Q+X363H, X4Q+X176K+X186E+X193E+X206Y+X251E+X405M+X100W+X135T+X181Q, X4Q+X176K+X186E+X193E+X206Y+X251E+X405M+X100W+X135T+X181Q+X258Q, X4Q+X176K+X186E+X193E+X206Y+X251E+X405M+X100W+X135T+X181Q+X258Q+X281N, X4Q+X176K+X186E+X193E+X206Y+X251E+X405M+X100W+X135T+X181Q+X258Q+X363E, X4Q+X176K+X186E+X193E+X206Y+X251E+X405M+X100W+X135T+X181Q+X258Q+X363H, X4Q+X176K+X186E+X193E+X206Y+X251E+X405M+X100W+X135T+X181Q+X281N+X363H, X4Q+X176K+X186E+X193E+X206Y+X251E+X405M+X100W+X135T+X181Q+X363E, X4Q+X176K+X186E+X193E+X206Y+X251E+X405M+X100W+X135T+X181Q+X363H, X4Q+X176K+X186E+X193E+X206Y+X251E+X405M+X100W+X135T+X363E, X4Q+X176K+X186E+X193E+X206Y+X251E+X405M+X100W+X135T+X363H, X4Q+X176K+X186E+X193E+X206Y+X251E+X405M+X100W+X363E, X4Q+X176K+X186E+X193E+X206Y+X251E+X405M+X100W+X363H, X4Q+X176K+X186E+X193E+X206Y+X251E+X405M+X135T, X4Q+X176K+X186E+X193E+X206Y+X251E+X405M+X135T+X258Q, X4Q+X176K+X186E+X193E+X206Y+X251E+X405M+X135T+X258Q+X8A, X4Q+X176K+X186E+X193E+X206Y+X251E+X405M+X135T+X258Q+X281N+X363E, X4Q+X176K+X186E+X193E+X206Y+X251E+X405M+X135T+X258Q+X363H, X4Q+X176K+X186E+X193E+X206Y+X251E+X405M+X135T+X281N, X4Q+X176K+X186E+X193E+X206Y+X251E+X405M+X135T+X181Q+X258Q, X4Q+X176K+X186E+X193E+X206Y+X251E+X405M+X135T+X181Q+X258Q+X281N, X4Q+X176K+X186E+X193E+X206Y+X251E+X405M+X135T+X181Q+X258Q+X363H, X4Q+X176K+X186E+X193E+X206Y+X251E+X405M+X135T+X363E, X4Q+X176K+X186E+X193E+X206Y+X251E+X405M+X135T+X363H, X4Q+X176K+X186E+X193E+X206Y+X251E+X405M+X363E, X4Q+X176K+X186E+X193E+X206Y+X251E+X405M+X363H, X25H+X176K+X186E+X206Y, X25H+X176K+X186E+X206Y+X258Q, X25H+X176K+X186E+X206Y+X258Q, X25H+X176K+X186E+X206Y+X258Q+X281N, X25H+X176K+X186E+X206Y+X258Q+X281N+X363E, X25H+X176K+X186E+X206Y+X258Q+X363E, X25H+X176K+X186E+X206Y+X258Q+X363E+X123D, X25H+X176K+X186E+X206Y+X281N, X25H+X176K+X186E+X206Y+X181Q, X25H+X176K+X186E+X206Y+X181Q+X193E, X25H+X176K+X186E+X206Y+X181Q+X363E, X25H+X176K+X186E+X206Y+X193E, X25H+X176K+X186E+X206Y+X193E+X258Q, X25H+X176K+X186E+X206Y+X193E+X281N+X363E, X25H+X176K+X186E+X206Y+X193E+X363E, X25H+X176K+X186E+X206Y+X100W, X25H+X176K+X186E+X206Y+X100W+X258Q, X25H+X176K+X186E+X206Y+X100W+X258Q+X281N, X25H+X176K+X186E+X206Y+X100W+X258Q+X281N+X363E, X25H+X176K+X186E+X206Y+X100W+X258Q+X363E, X25H+X176K+X186E+X206Y+X100W+X258Q+X363E+X135C, X25H+X176K+X186E+X206Y+X100W+X281N, X25H+X176K+X186E+X206Y+X100W+X281N+X363E, X25H+X176K+X186E+X206Y+X100W+X361R, X25H+X176K+X186E+X206Y+X100W+X181Q+X281N+X363E, X25H+X176K+X186E+X206Y+X100W+X181Q+X281N+X363E+X175D, X25H+X176K+X186E+X206Y+X100W+X181Q+X193E, X25H+X176K+X186E+X206Y+X100W+X181Q+X193E+X258Q+X281N, X25H+X176K+X186E+X206Y+X100W+X181Q+X193E+X258Q+X363E, X25H+X176K+X186E+X206Y+X100W+X181Q+X193E+X281N+X363E, X25H+X176K+X186E+X206Y+X100W+X181Q+X193E+X363E, X25H+X176K+X186E+X206Y+X100W+X181Q+X363E, X25H+X176K+X186E+X206Y+X100W+X193E, X25H+X176K+X186E+X206Y+X100W+X193E+X258Q, X25H+X176K+X186E+X206Y+X100W+X193E+X258Q+X473R, X25H+X176K+X186E+X206Y+X100W+X193E+X258Q+X281N, X25H+X176K+X186E+X206Y+X100W+X193E+X258Q+X281N+X363E, X25H+X176K+X186E+X206Y+X100W+X193E+X258Q+X363E, X25H+X176K+X186E+X206Y+X100W+X193E+X363E, X25H+X176K+X186E+X206Y+X100W+X135T, X25H+X176K+X186E+X206Y+X100W+X135T+X258Q, X25H+X176K+X186E+X206Y+X100W+X135T+X258Q+X281N, X25H+X176K+X186E+X206Y+X100W+X135T+X258Q+X281N+X363E, X25H+X176K+X186E+X206Y+X100W+X135T+X258Q+X363E, X25H+X176K+X186E+X206Y+X100W+X135T+X281N, X25H+X176K+X186E+X206Y+X100W+X135T+X281N+X363E, X25H+X176K+X186E+X206Y+X100W+X135T+X181Q, X25H+X176K+X186E+X206Y+X100W+X135T+X181Q+X258Q+X281N+X363E, X25H+X176K+X186E+X206Y+X100W+X135T+X181Q+X281N+X363E, X25H+X176K+X186E+X206Y+X100W+X135T+X181Q+X193E, X25H+X176K+X186E+X206Y+X100W+X135T+X181Q+X193E+X258Q+X363E, X25H+X176K+X186E+X206Y+X100W+X135T+X181Q+X193E+X363E, X25H+X176K+X186E+X206Y+X100W+X135T+X193E, X25H+X176K+X186E+X206Y+X100W+X135T+X193E+X258Q+X363E, X25H+X176K+X186E+X206Y+X100W+X135T+X193E+X363E, X25H+X176K+X186E+X206Y+X100W+X135T+X363E, X25H+X176K+X186E+X206Y+X100W+X363E, X25H+X176K+X186E+X206Y+X135T, X25H+X176K+X186E+X206Y+X135T+X258Q, X25H+X176K+X186E+X206Y+X135T+X258Q+X281N, X25H+X176K+X186E+X206Y+X135T+X258Q+X281N+X363E, and X25H+X176K+X186E+X206Y+X135T+X258Q+X363E.

Preferably, the amino acid residue at the above cited positions (i.e., X) corresponds to the amino acid residue shown in SEQ ID NO: 1 or 3, preferably in SEQ ID NO: 1, at the respective position (according to the numbering of SEQ ID NO: 3).

Preferably, the improved property is improved wash performance. Preferably, the alpha-amylase variant of the present invention that exhibits improved wash performance compared to a parent alpha-amylase, preferably compared to SEQ ID NO: 1, is an amylase comprising amino acid substitution (according to the numbering of SEQ ID NO: 3) at one or more positions corresponding to positions selected from the group consisting of 3, 4, 5, 6, 7, 20, 25, 33, 36, 37, 41, 51, 54, 72, 73, 75, 81, 82, 83, 94, 95, 96, 98, 100, 116, 118, 119, 120, 123, 126, 131, 135, 136, 139, 156, 158, 160, 163, 165, 169, 172, 174, 176, 177, 178, 180, 182, 185, 187, 188, 189, 191, 193, 208, 210, 218, 219, 222, 224, 225, 231, 238, 242, 243, 251, 252, 253, 258, 260, 268, 269, 270, 271, 272, 273, 274, 275, 276, 277, 279, 280, 281, 282, 286, 297, 298, 299, 311, 314, 317, 318, 320, 321, 322, 323, 324, 326, 337, 343, 344, 350, 352, 356, 357, 358, 359, 360, 362, 363, 364, 365, 368, 370, 372, 378, 379, 382, 385, 387, 388, 390, 391, 393, 395, 396, 397, 400, 405, 410, 429, 434, 439, 456, 459, 460, 461, 463, and 465.
Preferably, the alpha-amylase variant of the present invention that exhibits improved wash performance compared to a parent alpha-amylase, preferably compared to SEQ ID NO: 1, is an amylase comprising one or more amino acid substitutions (according to the numbering of SEQ ID NO: 3) selected from the group of amino acid substitutions consisting of X100W, X116I, X116M, X118A, X118D, X118E, X119S, X120E, X120G, X120M, X120S, X120W, X120Y, X126D, X131Q, X135D, X135E, X135G, X135N, X135N, X135P, X135S, X135T, X135T, X135W, X136E, X139S, X156E, X160D, X160E, X163A, X163Q, X163T, X165S, X165T, X169D, X169S, X174E, X174G, X174H, X174H, X174M, X174N, X176S, X176T, X177A, X177G, X177K, X177N, X177P, X177R, X177S, X177W, X178F, X180M, X180N, X180T, X180W, X182D, X182E, X182N, X182Q, X182S, X185E, X185M, X185N, X187A, X187D, X187M, X187V, X188T, X189I, X191M, X193D, X193E, X193E, X193G, X193V, X193V, X20N, X210D, X219Q, X224F, X231D, X238A, X238F, X242N, X243D, X251T, X252S, X253C, X258F, X258Q, X258Q, X258R, X25D, X25D, X25G, X25Y, X25Y, X260H, X260H, X268F, X269M, X270A, X270Q, X271S, X272F, X272S, X273Q, X274F, X274F, X274F, X275V, X276N, X277D, X277D, X277D, X277E, X277T, X279A, X280D, X280F, X280G, X280K, X280R, X281A, X281D, X281E, X281H, X281H, X281N, X281S, X282V, X282V, X286Q, X297M, X298V, X299S, X299S, X311G, X311W, X314Q, X314Q, X314S, X317M, X318L, X318L, X320A, X321Y, X322K, X323G, X324K, X326Y, X33L, X343W, X344V, X350P, X352C, X356C, X356L, X356V, X356V, X357C, X357F, X358M, X359W, X359W, X359W, X359W, X359W, X360T, X362C, X362M, X363E, X363H, X363M, X363N, X363S, X363Y, X363Y, X364T, X365L, X365L, X365Q, X365Q, X365Y, X368F, X368F, X36H, X36K, X370S, X372C, X372M, X372S, X372T, X372T, X378E, X379R, X37F, X37L, X37M, X37W, X37Y, X382Q, X382Q, X385W, X387E, X387N, X387S, X387S, X388E, X388H, X388K, X390F, X391K, X393Q, X395C, X395M, X396C, X396S, X397S, X400A, X400L, X400S, X405H, X405M, X41C, X41G, X429P, X429P, X434E, X434H, X434R, X434S, X439K, X456S, X459N, X459N, X460G, X460G, X461A, X461L, X461R, X463A, X463V, X465H, X4Q, X5D, X6E, X6H, X6P, X72C, X73C, X73N, X75H, X75I, X75L, X75L, X75T, X7F, X7H, X7W, X81L, X82C, X82L, X83E, X94E, X95A, X96Q, X98A, X98N, and X98V.
Preferably, the alpha-amylase variant of the present invention that exhibits improved wash performance compared to a parent alpha-amylase, preferably compared to SEQ ID NO: 1, is an amylase comprising a combination of substitutions (according to the numbering of SEQ ID NO: 3) selected from the group consisting of X4Q+X7W+X176K+X186E+X193E+X206Y+X251E+X405M, X4Q+X37M+X176K+X186E+X193E+X206Y+X251E+X405M, X4Q+X25H+X176K+X186E+X206Y+X251E+X405M+X460G, X4Q+X25H+X176K+X186E+X206Y+X251E+X460G, X4Q+X25Y+X176K+X186E+X193E+X206Y+X251E+X405M, X4Q+X176K+X182D+X186E+X193E+X206Y+X251E+X405M, X4Q+X176K+X186E+X193E+X206Y+X251E+X258Q+X405M, X4Q+X176K+X186E+X193E+X206Y+X251E+X281N+X405M, X4Q+X176K+X186E+X193E+X206Y+X251E+X405M+X460G, X4Q+X176K+X186E+X193E+X206Y+X251E+X363E+X405M, X4Q+X176K+X186E+X193E+X206Y+X251E+X363H+X405M, X4Q+X176K+X186E+X193E+X206Y+X251E+X363N+X405M, X4Q+X176K+X181D+X186E+X193E+X206Y+X251E+X405M, X4Q+X176K+X181F+X186E+X193E+X206Y+X251E+X405M, X4Q+X176K+X181N+X186E+X193E+X206Y+X251E+X405M, X4Q+X176K+X181Q+X186E+X193E+X206Y+X251E+X405M, X4Q+X136E+X176K+X186E+X193E+X206Y+X251E+X405M, X4Q+X100W+X176K+X186E+X193E+X206Y+X251E+X405M, X4Q+X135E+X176K+X186E+X193E+X206Y+X251E+X405M, X4Q+X135T+X176K+X186E+X193E+X206Y+X251E+X405M, X4Q+X135W+X176K+X186E+X193E+X206Y+X251E+X405M, X4Q+X160D+X176K+X186E+X193E+X206Y+X251E+X405M, X4Q+X160E+X176K+X186E+X193E+X206Y+X251E+X405M, X7H+X25H+X176K+X186E+X206Y, X7W+X25H+X176K+X186E+X206Y, X25D+X176K+X186E+X206Y, X25H+X186E+X206Y+X405M+X460G, X25H+X186E+X206Y+X460G,X25H+X37M+X176K+X186E+X206Y, X25H+X118D+X176K+X186E+X206Y, X25H+X176K+X186E+X206Y+X258Q, X25H+X176K+X186E+X206Y+X281N,X25H+X176K+X186E+X206Y+X460G, X25H+X176K+X186E+X206Y+X251E+X460G,X25H+X176K+X186E+X206Y+X363E, X25H+X176K+X186E+X206Y+X363H,X25H+X176K+X186E+X206Y+X363N, X25H+X176K+X186E+X460G, X25H+X176K+X186E+X193E+X206Y, X25H+X176K+X186E+X193E+X206Y+X460G, X25H+X176K+X186E+X193E+X206Y+X251E+X460G, X25H+X176K+X181D+X186E+X206Y, X25H+X176K+X181N+X186E+X206Y, X25H+X176K+X181Q+X186E+X206Y, X25H+X136E+X176K+X186E+X206Y, X25H+X100W+X176K+X186E+X206Y, X25H+X135D+X176K+X186E+X206Y, X25H+X135E+X176K+X186E+X206Y, X25H+X135T+X176K+X186E+X206Y, X25H+X135W+X176K+X186E+X206Y, X25H+X160D+X176K+X186E+X206Y, X25H+X160E+X176K+X186E+X206Y, X25Y+X176K+X186E+X206Y, X51Q+X116K+X410I, X51V+X356I, X33E+X54G+X83D+X116T+X337L, X3A+X116K+X172N+X323A+X356V, X3I+X33H+X54D+X208Y+X218E+X320A, X3I+X54D+X116K+X125E+X225A, X3I+X125D+X174D+X222D+X376L+X420D, X3I+X356I, X3I+X356V, X3I+X48Y+X116T, X54D+X116K+X225S+X410Y, X83A+X94E+X158N+X172D+X323A, X83D+X125E, X83E+X181E, X83E+X116R+X158Y+X181E, X169E+X377Q, X158N+X172D, X125D+X225A+X337A+X410H, X29D+X33H+X83D+X125D+X337C+X343W, X29E+X33K+X123D+X208I+X222D+X356V, X94D+X125E, X225A+X376G+X410Y, X5L+X33K+X222D+X320K+X324M+X420E, X5L+X131L+X172K+X225S+X410l+X420E, X5L+X116T+X123D+X208F, X131I+X377Q+X410H, X131L+X320K, X116K+X181T+X225A+X320K, X116K+X125N+X158H+X225S, X116K+X131I+X158H+X174E, X116R+X169E+X420E, X116T+X181Q+X343T, X116T+X125D+X172K+X337A+X410I, X116T+X125E+X131I, X186E+X206Y, X186E+X206Y+X405M, X186E+X206Y+X251E, X186E+X206Y+X251E+X276R+X405M,X186E+X206Y+X251E+X405M, X186E+X206Y+X251E+X323G+X405M, X186E+X206Y+X323G+X405M, X186E+X405M, X186E+X193E+X206Y+X251E, X186E+X251E+X405M, X4Q+X186E+X206Y, X4Q+X186E+X206Y+X405M, X4Q+X186E+X206Y+X251E+X276R, X4Q+X186E+X206Y+X251E+X405M, X4Q+X186E+X206Y+X251E+X323G+X405M, X4Q+X186E+X193E+X206Y+X405M, X4Q+X186E+X193E+X206Y+X251E+X405M, X4Q+X25H+X186E+X206Y, X4Q+X25H+X186E+X206Y+X405M, X4Q+X25H+X186E+X206Y+X251E+X323A+X405M, X4Q+X25H+X186E+X206Y+X323G+X405M, X4Q+X25H+X176K+X186E+X206Y+X251E+X405M, X4Q+X25H+X176K+X186E+X251E, X4Q+X25H+X176K+X186E+X251E+X405M, X4Q+X25H+X176K+X206Y+X405M+X460G, X4Q+X25H+X176K+X206Y+X460G, X4Q+X25H+X176K+X206Y+X251E, X4Q+X25H+X176K+X206Y+X251E+X460G, X4Q+X25H+X176K+X206Y+X323G, X4Q+X25H+X160W+X186E+X206Y+X405M, X4Q+X25H+X160W+X186E+X206Y+X251E, X4Q+X25H+X160W+X186E+X206Y+X323G+X405M, X4Q+X25H+X160W+X186E+X405M, X4Q+X25H+X160W+X186E+X193E+X206Y+X276R, X4Q+X25H+X160W+X186E+X323G, X4Q+X25H+X160W+X176K+X186E+X206Y+X251E, X4Q+X25H+X160W+X176K+X186E+X206Y+X251E+X405M, X4Q+X25H+X160W+X176K+X186E+X405M, X4Q+X25H+X160W+X176K+X186E+X251E+X405M, X4Q+X25H+X160W+X176K+X206Y, X4Q+X25H+X160W+X176K+X206Y+X251E+X460G, X4Q+X176K+X186E+X206Y+X405M, X4Q+X176K+X186E+X206Y+X251E+X405M, X4Q+X176K+X186E+X405M, X4Q+X176K+X186E+X193E+X206Y+X276R+X405M, X4Q+X176K+X186E+X193E+X206Y+X251E+X405M, X4Q+X176K+X186E+X251E+X405M, X4Q+X176K+X186E+X323G+X405M, X4Q+X176K+X206Y, X4Q+X176K+X206Y+X405M, X4Q+X176K+X206Y+X405M+X460G, X4Q+X176K+X206Y+X460G, X4Q+X176K+X206Y+X251E+X405M, X4Q+X176K+X206Y+X251E+X405M+X460G, X4Q+X176K+X206Y+X251E+X323A+X460G, X4Q+X160W+X176K+X186E+X206Y+X251E, X4Q+X160W+X176K+X186E+X251E+X405M, X4Q+X160W+X176K+X206Y+X251E+X323G+X405M+X460G, X25H+X186E+X206Y, X25H+X186E+X206Y+X405M, X25H+X186E+X206Y+X251E+X405M, X25H+X186E+X405M, X25H+X186E+X193E+X206Y+X405M, X25H+X176K+X186E, X25H+X176K+X186E+X276R+X323G+X405M, X25H+X176K+X186E+X206Y, X25H+X176K+X186E+X206Y+X276R, X25H+X176K+X186E+X206Y+X251E, X25H+X176K+X186E+X206Y+X251E+X405M, X25H+X176K+X186E+X405M, X25H+X176K+X186E+X193E+X206Y, X25H+X176K+X186E+X193E+X206Y+X251E, X25H+X176K+X186E+X193E+X206Y+X251E+X276R+X405M, X25H+X176K+X186E+X251E+X276R, X25H+X176K+X206Y, X25H+X176K+X206Y+X405M, X25H+X176K+X206Y+X460G, X25H+X176K+X206Y+X251E, X25H+X176K+X206Y+X251E+X405M, X25H+X160W+X186E+X206Y, X25H+X160W+X186E+X206Y+X251E, X25H+X160W+X186E+X206Y+X251E+X405M, X25H+X160W+X186E+X206Y+X323G+X405M, X25H+X160W+X176K+X186E+X206Y+X251E+X405M, X25H+X160W+X176K+X186E+X405M, X25H+X160W+X176K+X186E+X193E+X206Y, X25H+X160W+X176K+X186E+X193E+X206Y+X405M, X25H+X160W+X176K+X186E+X193E+X206Y+X251E+X405M, X25H+X160W+X176K+X186E+X251E, X25H+X160W+X176K+X186E+X251E+X405M, X25H+X160W+X176K+X206Y+X251E+X323G+X405M, X176K+X186E+X206Y, X176K+X186E+X206Y+X405M, X176K+X186E+X206Y+X251E+X405M, X176K+X186E+X405M+X405M, X176K+X186E+X193E+X206Y+X405M, X176K+X186E+X206Y+X251E+X405M, X176K+X186E+X251E+X405M, X176K+X206Y, X176K+X206Y+X405M, X176K+X206Y+X405M+X460G, X176K+X206Y+X460G, X176K+X206Y+X251E, X160W+X186E+X193E+X206Y+X405M, X160W+X176K+X186E+X206Y, X160W+X176K+X186E+X405M, X160W+X176K+X186E+X193E+X206Y, X160W+X176K+X186E+X193E+X206Y+X405M, X160W+X176K+X186E+X193E+X206Y+X251E, X160W+X176K+X206Y+X405M, X160W+X176K+X206Y+X323G+X405M+X460G, X186E+X193E, X4Q+X460G, X208C+X323G, X25H+X186E, X176K+X400A, X457N+X460G+X461L, X160W+X186E, X160W+X186Q, X160W+X434S, X4Q+X176K+X186E+X193E+X206Y+X251E+X258Q+X281N+X405M, X4Q+X176K+X186E+X193E+X206Y+X251E+X258Q+X405M, X4Q+X176K+X186E+X193E+X206Y+X251E+X258Q+X363E+X405M, X4Q+X176K+X186E+X193E+X206Y+X251E+X281N+X363E+X405M, X4Q+X176K+X186E+X193E+X206Y+X251E+X405M, X4Q+X176K+X186E+X193E+X206Y+X251E+X363E+X405M, X4Q+X176K+X186E+X193E+X206Y+X251E+X363H+X405M, X4Q+X176K+X181Q+X186E+X193E+X206Y+X251E+X258Q+X405M, X4Q+X176K+X181Q+X186E+X193E+X206Y+X251E+X405M, X4Q+X176K+X181Q+X186E+X193E+X206Y+X251E+X363E+X405M, X4Q+X8A+X135T+X176K+X186E+X193E+X206Y+X251E+X258Q+X405M, X4Q+X100W+X176K+X186E+X193E+X206Y+X251E+X258Q+X281N+X405M, X4Q+X100W+X176K+X186E+X193E+X206Y+X251E+X258Q+X281N+X363E+X405M, X4Q+X100W+X176K+X186E+X193E+X206Y+X251E+X258Q+X281N+X363H+X405M, X4Q+X100W+X176K+X186E+X193E+X206Y+X251E+X258Q+X363E+X405M, X4Q+X100W+X176K+X186E+X193E+X206Y+X251E+X258Q+X363H+X405M, X4Q+X100W+X176K+X186E+X193E+X206Y+X251E+X281N+X405M, X4Q+X100W+X176K+X186E+X193E+X206Y+X251E+X281N+X363H+X405M, X4Q+X100W+X176K+X186E+X193E+X206Y+X251E+X405M, X4Q+X100W+X176K+X186E+X193E+X206Y+X251E+X363E+X405M, X4Q+X100W+X176K+X186E+X193E+X206Y+X251E+X363H+X405M, X4Q+X100W+X176K+X181Q+X186E+X193E+X206Y+X251E+X258Q+X281N+X363E+X405M, X4Q+X100W+X176K+X181Q+X186E+X193E+X206Y+X251E+X363H+X405M, X4Q+X100W+X135T+X176K+X186E+X193E+X206Y+X251E+X258Q+X281N+X405M, X4Q+X100W+X135T+X176K+X186E+X193E+X206Y+X251E+X258Q+X405M, X4Q+X100W+X135T+X176K+X186E+X193E+X206Y+X251E+X258Q+X363H+X405M, X4Q+X100W+X135T+X176K+X186E+X193E+X206Y+X251E+X281N+X405M, X4Q+X100W+X135T+X176K+X186E+X193E+X206Y+X251E+X405M, X4Q+X100W+X135T+X176K+X186E+X193E+X206Y+X251E+X363E+X405M, X4Q+X100W+X135T+X176K+X186E+X193E+X206Y+X251E+X363E+X405M, X4Q+X100W+X135T+X176K+X181Q+X186E+X193E+X206Y+X251E+X258Q+X405M, X4Q+X100W+X135T+X176K+X181Q+X186E+X193E+X206Y+X251E+X405M, X4Q+X135T+X176K+X186E+X193E+X206Y+X251E+X258Q+X281N+X405M, X4Q+X135T+X176K+X186E+X193E+X206Y+X251E+X258Q+X281N+X363E+X405M, X4Q+X135T+X176K+X186E+X193E+X206Y+X251E+X258Q+X405M, X4Q+X135T+X176K+X186E+X193E+X206Y+X251E+X258Q+X363H+X405M, X4Q+X135T+X176K+X186E+X193E+X206Y+X251E+X281N+X405M, X4Q+X135T+X176K+X186E+X193E+X206Y+X251E+X405M, X4Q+X135T+X176K+X186E+X193E+X206Y+X251E+X363E+X405M, X4Q+X135T+X176K+X186E+X193E+X206Y+X251E+X363H+X405M, X4Q+X135T+X176K+X181Q+X186E+X193E+X206Y+X251E+X258Q+X281N+X405M, X4Q+X135T+X176K+X181Q+X186E+X193E+X206Y+X251E+X281N+X363H+X405M, X25H+X176K+X186E+X206Y, X25H+X176K+X186E+X206Y+X258Q, X25H+X176K+X186E+X206Y+X258Q+X281N+X363E, X25H+X176K+X186E+X206Y+X258Q+X363E, X25H+X176K+X186E+X206Y+X258Q+X363E+XN123D, X25H+X176K+X186E+X206Y+X281N,X25H+X176K+X186E+X206Y+X363E, X25H+X176K+X186E+X193E+X206Y,X25H+X176K+X186E+X193E+X206Y+X258Q, X25H+X176K+X186E+X193E+X206Y+X281N+X363E, X25H+X176K+X186E+X193E+X206Y+X363E, X25H+X176K+X181Q+X186E+X206Y, X25H+X176K+X181Q+X186E+X206Y+X363E, X25H+X176K+X181Q+X186E+X193E+X206Y, X25H+X100W+X176K+X186E+X206Y, X25H+X100W+X176K+X186E+X206Y+X258Q, X25H+X100W+X176K+X186E+X206Y+X258Q+X281N, X25H+X100W+X176K+X186E+X206Y+X258Q+X281N+X363E, X25H+X100W+X176K+X186E+X206Y+X258Q+X363E, X25H+X100W+X176K+X186E+X206Y+X281N, X25H+X100W+X176K+X186E+X206Y+X281N+X363E, X25H+X100W+X176K+X186E+X206Y+X361R, X25H+X100W+X176K+X186E+X206Y+X363E, X25H+X100W+X176K+X186E+X193E+X206Y, X25H+X100W+X176K+X186E+X193E+X206Y+X258Q, X25H+X100W+X176K+X186E+X193E+X206Y+X258Q+X473R, X25H+X100W+X176K+X186E+X193E+X206Y+X258Q+X281N, X25H+X100W+X176K+X186E+X193E+X206Y+X258Q+X281N+X363E, X25H+X100W+X176K+X186E+X193E+X206Y+X258Q+X363E, X25H+X100W+X176K+X186E+X193E+X206Y+X363E, X25H+X100W+X176K+X181Q+X186E+X206Y+X363E, X25H+X100W+X176K+X181Q+X186E+X193E+X206Y, X25H+X100W+X176K+X181Q+X186E+X193E+X206Y+X258Q+X281N, X25H+X100W+X176K+X181Q+X186E+X193E+X206Y+X258Q+X363E, X25H+X100W+X135C+X176K+X186E+X206Y+X258Q+X363E, X25H+X100W+X135T+X176K+X186E+X206Y, X25H+X100W+X135T+X176K+X186E+X206Y+X258Q, X25H+X100W+X135T+X176K+X186E+X206Y+X258Q+X281N, X25H+X100W+X135T+X176K+X186E+X206Y+X258Q+X281N+X363E, X25H+X100W+X135T+X176K+X186E+X206Y+X258Q+X363E, X25H+X100W+X135T+X176K+X186E+X206Y+X281N, X25H+X100W+X135T+X176K+X186E+X206Y+X281N+X363E, X25H+X100W+X135T+X176K+X186E+X206Y+X363E, X25H+X100W+X135T+X176K+X186E+X193E+X206Y, X25H+X100W+X135T+X176K+X186E+X193E+X206Y+X258Q+X363E, X25H+X100W+X135T+X176K+X186E+X193E+X206Y+X363E, X25H+X100W+X135T+X176K+X181Q+X186E+X206Y, X25H+X100W+X135T+X176K+X181Q+X186E+X206Y+X258Q+X281N, X25H+X100W+X135T+X176K+X181Q+X186E+X193E+X206Y, X25H+X100W+X135T+X176K+X181Q+X186E+X193E+X206Y+X258Q, X25H+X100W+X135T+X176K+X181Q+X186E+X193E+X206Y+X363E, X25H+X135T+X176K+X186E+X206Y, X25H+X135T+X176K+X186E+X206Y+X258Q, X25H+X135T+X176K+X186E+X206Y+X258Q+X281N, X25H+X135T+X176K+X186E+X206Y+X258Q+X281N+X363E, X25H+X135T+X176K+X186E+X206Y+X258Q+X363E, X25H+X135T+X176K+X186E+X206Y+X281N, X25H+X135T+X176K+X186E+X206Y+X281N+X363E, X25H+X135T+X176K+X186E+X206Y+X281N+X363E+X415S, X25H+X135T+X176K+X186E+X206Y+X363E, X25H+X135T+X176K+X186E+X193E+X206Y+X258Q, X25H+X135T+X176K+X181Q+X186E+X206Y+X258Q, X25H+X135T+X176K+X181Q+X186E+X206Y+X258Q+X363E, and X25H+X135T+X176K+X181Q+X186E+X193E+X206Y+X258Q+X281N. Preferably, the amino acid residue at the above cited positions (i.e., X) corresponds to the amino acid residue shown in SEQ ID NO: 1 or 3, preferably in SEQ ID NO: 1, at the respective position (according to the numbering of SEQ ID NO: 3).

Preferably, the alpha-amylase variant of the present invention that exhibits improved wash performance and/or improved stability compared to a parent alpha-amylase, preferably compared to SEQ ID NO: 1, is an amylase comprising a combination of substitutions (according to the numbering of SEQ ID NO: 3) selected from the group consisting of: X4Q+X7W+X176K+X186E+X193E+X206Y+X251E+X405M, X4Q+X37M+X176K+X186E+X193E+X206Y+X251E+X405M, X4Q+X25H+X176K+X186E+X206Y+X251E+X405M+X460G, X4Q+X25H+X176K+X186E+X206Y+X251E+X460G, X4Q+X25Y+X176K+X186E+X193E+X206Y+X251E+X405M, X4Q+X176K+X182D+X186E+X193E+X206Y+X251E+X405M, X4Q+X176K+X186E+X193E+X206Y+X251E+X258Q+X405M, X4Q+X176K+X186E+X193E+X206Y+X251E+X281N+X405M, X4Q+X176K+X186E+X193E+X206Y+X251E+X405M+X460G, X4Q+X176K+X186E+X193E+X206Y+X251E+X363E+X405M, X4Q+X176K+X186E+X193E+X206Y+X251E+X363H+X405M, X4Q+X176K+X186E+X193E+X206Y+X251E+X363N+X405M, X4Q+X176K+X181D+X186E+X193E+X206Y+X251E+X405M, X4Q+X176K+X181F+X186E+X193E+X206Y+X251E+X405M, X4Q+X176K+X181N+X186E+X193E+X206Y+X251E+X405M, X4Q+X176K+X181Q+X186E+X193E+X206Y+X251E+X405M, X4Q+X136E+X176K+X186E+X193E+X206Y+X251E+X405M, X4Q+X100W+X176K+X186E+X193E+X206Y+X251E+X405M, X4Q+X135E+X176K+X186E+X193E+X206Y+X251E+X405M, X4Q+X135T+X176K+X186E+X193E+X206Y+X251E+X405M, X4Q+X135W+X176K+X186E+X193E+X206Y+X251E+X405M, X4Q+X160D+X176K+X186E+X193E+X206Y+X251E+X405M, X4Q+X160E+X176K+X186E+X193E+X206Y+X251E+X405M, X7H+X25H+X176K+X186E+X206Y, X7W+X25H+X176K+X186E+X206Y, X25D+X176K+X186E+X206Y, X25H+X186E+X206Y+X405M+X460G, X25H+X186E+X206Y+X460G,X25H+X37M+X176K+X186E+X206Y, X25H+X118D+X176K+X186E+X206Y, X25H+X176K+X186E+X206Y+X258Q, X25H+X176K+X186E+X206Y+X281N, X25H+X176K+X186E+X206Y+X460G, X25H+X176K+X186E+X206Y+X251E+X460G, X25H+X176K+X186E+X206Y+X363E, X25H+X176K+X186E+X206Y+X363H, X25H+X176K+X186E+X206Y+X363N, X25H+X176K+X186E+X460G, X25H+X176K+X186E+X193E+X206Y, X25H+X176K+X186E+X193E+X206Y+X460G, X25H+X176K+X186E+X193E+X206Y+X251E+X460G, X25H+X176K+X181D+X186E+X206Y, X25H+X176K+X181N+X186E+X206Y, X25H+X176K+X181Q+X186E+X206Y, X25H+X136E+X176K+X186E+X206Y, X25H+X100W+X176K+X186E+X206Y, X25H+X135D+X176K+X186E+X206Y, X25H+X135E+X176K+X186E+X206Y, X25H+X135T+X176K+X186E+X206Y, X25H+X135W+X176K+X186E+X206Y, X25H+X160D+X176K+X186E+X206Y, X25H+X160E+X176K+X186E+X206Y, X25Y+X176K+X186E+X206Y, X51Q+X116K+X410I, X51V+X356I, X33E+X54G+X83D+X116T+X337L, X3A+X116K+X172N+X323A+X356V, X3I+X33H+X54D+X208Y+X218E+X320A, X3I+X54D+X116K+X125E+X225A, X3I+X125D+X174D+X222D+X376L+X420D, X3I+X356I, X3I+X356V, X3I+X48Y+X116T, X54D+X116K+X225S+X410Y, X83A+X94E+X158N+X172D+X323A, X83D+X125E, X83E+X181E, X83E+X116R+X158Y+X181E, X169E+X377Q, X158N+X172D, X125D+X225A+X337A+X410H, X29D+X33H+X83D+X125D+X337C+X343W, X29E+X33K+X123D+X208I+X222D+X356V, X94D+X125E, X225A+X376G+X410Y, X5L+X33K+X222D+X320K+X324M+X420E, X5L+X131L+X172K+X225S+X410l+X420E, X5L+X116T+X123D+X208F, X1311+X377Q+X410H, X131L+X320K, X116K+X181T+X225A+X320K, X116K+X125N+X158H+X225S, X116K+X1311+X158H+X174E, X116R+X169E+X420E, X116T+X181Q+X343T, X116T+X125D+X172K+X337A+X410I, X116T+X125E+X131I, X186E, X186E+X206Y, X186E+X206Y+X405M, X186E+X206Y+X251E, X186E+X206Y+X251E+X276R+X405M,X186E+X206Y+X251E+X405M, X186E+X206Y+X251E+X323G+X405M, X186E+X206Y+X323G+X405M, X186E+X405M, X186E+X193E+X206Y+X251E, X186E+X251E+X405M, X4Q+X186E+X206Y, X4Q+X186E+X206Y+X405M, X4Q+X186E+X206Y+X251E+X276R, X4Q+X186E+X206Y+X251E+X405M, X4Q+X186E+X206Y+X251E+X323G+X405M, X4Q+X186E+X193E+X206Y+X405M, X4Q+X186E+X193E+X206Y+X251E+X405M, X4Q+X25H+X186E+X206Y,X4Q+X25H+X186E+X206Y+X405M, X4Q+X25H+X186E+X206Y+X251E+X323A+X405M, X4Q+X25H+X186E+X206Y+X323G+X405M, X4Q+X25H+X176K+X186E+X206Y+X251E+X405M, X4Q+X25H+X176K+X186E+X251E, X4Q+X25H+X176K+X186E+X251E+X405M, X4Q+X25H+X176K+X206Y+X405M+X460G, X4Q+X25H+X176K+X206Y+X460G, X4Q+X25H+X176K+X206Y+X251E, X4Q+X25H+X176K+X206Y+X251E+X460G, X4Q+X25H+X176K+X206Y+X323G, X4Q+X25H+X160W+X186E+X206Y+X405M, X4Q+X25H+X160W+X186E+X206Y+X251E, X4Q+X25H+X160W+X186E+X206Y+X323G+X405M, X4Q+X25H+X160W+X186E+X405M, X4Q+X25H+X160W+X186E+X193E+X206Y+X276R, X4Q+X25H+X160W+X186E+X323G, X4Q+X25H+X160W+X176K+X186E+X206Y+X251E, X4Q+X25H+X160W+X176K+X186E+X206Y+X251E+X405M, X4Q+X25H+X160W+X176K+X186E+X405M, X4Q+X25H+X160W+X176K+X186E+X251E+X405M, X4Q+X25H+X160W+X176K+X206Y, X4Q+X25H+X160W+X176K+X206Y+X251E+X460G, X4Q+X176K+X186E+X206Y+X405M, X4Q+X176K+X186E+X206Y+X251E+X405M, X4Q+X176K+X186E+X405M, X4Q+X176K+X186E+X193E+X206Y+X276R+X405M, X4Q+X176K+X186E+X193E+X206Y+X251E+X405M, X4Q+X176K+X186E+X251E+X405M, X4Q+X176K+X186E+X323G+X405M, X4Q+X176K+X206Y, X4Q+X176K+X206Y+X405M, X4Q+X176K+X206Y+X405M+X460G, X4Q+X176K+X206Y+X460G, X4Q+X176K+X206Y+X251E+X405M, X4Q+X176K+X206Y+X251E+X405M+X460G, X4Q+X176K+X206Y+X251E+X323A+X460G, X4Q+X160W+X176K+X186E+X206Y+X251E, X4Q+X160W+X176K+X186E+X251E+X405M, X4Q+X160W+X176K+X206Y+X251E+X323G+X405M+X460G, X25H+X186E+X206Y, X25H+X186E+X206Y+X405M, X25H+X186E+X206Y+X251E+X405M, X25H+X186E+X405M, X25H+X186E+X193E+X206Y+X405M, X25H+X176K+X186E, X25H+X176K+X186E+X276R+X323G+X405M, X25H+X176K+X186E+X206Y, X25H+X176K+X186E+X206Y+X276R, X25H+X176K+X186E+X206Y+X251E, X25H+X176K+X186E+X206Y+X251E+X405M, X25H+X176K+X186E+X405M, X25H+X176K+X186E+X193E+X206Y+X251E, X25H+X176K+X186E+X193E+X206Y+X251E+X276R+X405M, X25H+X176K+X186E+X251E+X276R, X25H+X176K+X206Y, X25H+X176K+X206Y+X405M, X25H+X176K+X206Y+X460G, X25H+X176K+X206Y+X251E, X25H+X176K+X206Y+X251E+X405M, X25H+X160W+X186E+X206Y, X25H+X160W+X186E+X206Y+X251E, X25H+X160W+X186E+X206Y+X251E+X405M, X25H+X160W+X186E+X206Y+X323G+X405M, X25H+X160W+X176K+X186E+X206Y+X251E+X405M, X25H+X160W+X176K+X186E+X405M, X25H+X160W+X176K+X186E+X193E+X206Y, X25H+X160W+X176K+X186E+X193E+X206Y+X405M, X25H+X160W+X176K+X186E+X193E+X206Y+X251E+X405M, X25H+X160W+X176K+X186E+X251E, X25H+X160W+X176K+X186E+X251E+X405M, X25H+X160W+X176K+X206Y+X251E+X323G+X405M, X176K+X186E+X206Y, X176K+X186E+X206Y+X405M, X176K+X186E+X206Y+X251E+X405M, X176K+X186E+X405M+X405M, X176K+X186E+X193E+X206Y+X405M, X176K+X186E+X251E+X405M, X176K+X206Y, X176K+X206Y+X405M, X176K+X206Y+X405M+X460G, X176K+X206Y+X460G, X176K+X206Y+X251E, X160W+X186E+X193E+X206Y+X405M, X160W+X176K+X186E+X206Y, X160W+X176K+X186E+X405M, X160W+X176K+X186E+X193E+X206Y, X160W+X176K+X186E+X193E+X206Y+X405M, X160W+X176K+X186E+X193E+X206Y+X251E, X160W+X176K+X206Y+X405M, X160W+X176K+X206Y+X323G+X405M+X460G, X186E+X193E, X4Q+X460G, X208C+X323G, X25H+X186E, X176K+X400A, X457N+X460G+X461L, X160W+X186E, X160W+X186Q, X160W+X434S, X4Q+X176K+X186E+X193E+X206Y+X251E+X258Q+X281N+X405M, X4Q+X176K+X186E+X193E+X206Y+X251E+X258Q+X363E+X405M, X4Q+X176K+X186E+X193E+X206Y+X251E+X281N+X363E+X405M, X4Q+X176K+X181Q+X186E+X193E+X206Y+X251E+X258Q+X405M, X4Q+X176K+X181Q+X186E+X193E+X206Y+X251E+X363E+X405M, X4Q+X8A+X135T+X176K+X186E+X193E+X206Y+X251E+X258Q+X405M, X4Q+X100W+X176K+X186E+X193E+X206Y+X251E+X258Q+X281N+X405M, X4Q+X100W+X176K+X186E+X193E+X206Y+X251E+X258Q+X281N+X363E+X405M, X4Q+X100W+X176K+X186E+X193E+X206Y+X251E+X258Q+X281N+X363H+X405M, X4Q+X100W+X176K+X186E+X193E+X206Y+X251E+X258Q+X363E+X405M, X4Q+X100W+X176K+X186E+X193E+X206Y+X251E+X258Q+X363H+X405M, X4Q+X100W+X176K+X186E+X193E+X206Y+X251E+X281N+X405M, X4Q+X100W+X176K+X186E+X193E+X206Y+X251E+X281N+X363H+X405M, X4Q+X100W+X176K+X186E+X193E+X206Y+X251E+X363E+X405M, X4Q+X100W+X176K+X186E+X193E+X206Y+X251E+X363H+X405M, X4Q+X100W+X176K+X181Q+X186E+X193E+X206Y+X251E+X258Q+X281N+X363E+X405M, X4Q+X100W+X176K+X181Q+X186E+X193E+X206Y+X251E+X363H+X405M, X4Q+X100W+X135T+X176K+X186E+X193E+X206Y+X251E+X258Q+X281N+X405M, X4Q+X100W+X135T+X176K+X186E+X193E+X206Y+X251E+X258Q+X405M, X4Q+X100W+X135T+X176K+X186E+X193E+X206Y+X251E+X258Q+X363H+X405M, X4Q+X100W+X135T+X176K+X186E+X193E+X206Y+X251E+X281N+X405M, X4Q+X100W+X135T+X176K+X186E+X193E+X206Y+X251E+X405M, X4Q+X100W+X135T+X176K+X186E+X193E+X206Y+X251E+X363E+X405M, X4Q+X100W+X135T+X176K+X181Q+X186E+X193E+X206Y+X251E+X258Q+X405M, X4Q+X100W+X135T+X176K+X181Q+X186E+X193E+X206Y+X251E+X405M, X4Q+X135T+X176K+X186E+X193E+X206Y+X251E+X258Q+X281N+X405M, X4Q+X135T+X176K+X186E+X193E+X206Y+X251E+X258Q+X281N+X363E+X405M, X4Q+X135T+X176K+X186E+X193E+X206Y+X251E+X258Q+X405M, X4Q+X135T+X176K+X186E+X193E+X206Y+X251E+X258Q+X363H+X405M, X4Q+X135T+X176K+X186E+X193E+X206Y+X251E+X281N+X405M, X4Q+X135T+X176K+X186E+X193E+X206Y+X251E+X363E+X405M, X4Q+X135T+X176K+X186E+X193E+X206Y+X251E+X363H+X405M, X4Q+X135T+X176K+X181Q+X186E+X193E+X206Y+X251E+X258Q+X281N+X405M, X4Q+X135T+X176K+X181Q+X186E+X193E+X206Y+X251E+X281N+X363H+X405M, X25H+X176K+X186E+X206Y+X258Q+X281N+X363E, X25H+X176K+X186E+X206Y+X258Q+X363E, X25H+X176K+X186E+X206Y+X258Q+X363E+XN123D, X25H+X176K+X186E+X193E+X206Y+X258Q, X25H+X176K+X186E+X193E+X206Y+X281N+X363E, X25H+X176K+X186E+X193E+X206Y+X363E, X25H+X176K+X181Q+X186E+X206Y+X363E, X25H+X176K+X181Q+X186E+X193E+X206Y, X25H+X100W+X176K+X186E+X206Y+X258Q, X25H+X100W+X176K+X186E+X206Y+X258Q+X281N, X25H+X100W+X176K+X186E+X206Y+X258Q+X281N+X363E, X25H+X100W+X176K+X186E+X206Y+X258Q+X363E, X25H+X100W+X176K+X186E+X206Y+X281N, X25H+X100W+X176K+X186E+X206Y+X281N+X363E, X25H+X100W+X176K+X186E+X206Y+X361R, X25H+X100W+X176K+X186E+X206Y+X363E, X25H+X100W+X176K+X186E+X193E+X206Y, X25H+X100W+X176K+X186E+X193E+X206Y+X258Q, X25H+X100W+X176K+X186E+X193E+X206Y+X258Q+X473R, X25H+X100W+X176K+X186E+X193E+X206Y+X258Q+X281N, X25H+X100W+X176K+X186E+X193E+X206Y+X258Q+X281N+X363E, X25H+X100W+X176K+X186E+X193E+X206Y+X258Q+X363E, X25H+X100W+X176K+X186E+X193E+X206Y+X363E, X25H+X100W+X176K+X181Q+X186E+X206Y+X363E, X25H+X100W+X176K+X181Q+X186E+X193E+X206Y, X25H+X100W+X176K+X181Q+X186E+X193E+X206Y+X258Q+X281N, X25H+X100W+X176K+X181Q+X186E+X193E+X206Y+X258Q+X363E, X25H+X100W+X135C+X176K+X186E+X206Y+X258Q+X363E, X25H+X100W+X135T+X176K+X186E+X206Y, X25H+X100W+X135T+X176K+X186E+X206Y+X258Q, X25H+X100W+X135T+X176K+X186E+X206Y+X258Q+X281N, X25H+X100W+X135T+X176K+X186E+X206Y+X258Q+X281N+X363E, X25H+X100W+X135T+X176K+X186E+X206Y+X258Q+X363E, X25H+X100W+X135T+X176K+X186E+X206Y+X281N, X25H+X100W+X135T+X176K+X186E+X206Y+X281N+X363E, X25H+X100W+X135T+X176K+X186E+X206Y+X363E, X25H+X100W+X135T+X176K+X186E+X193E+X206Y, X25H+X100W+X135T+X176K+X186E+X193E+X206Y+X258Q+X363E, X25H+X100W+X135T+X176K+X186E+X193E+X206Y+X363E, X25H+X100W+X135T+X176K+X181Q+X186E+X206Y, X25H+X100W+X135T+X176K+X181Q+X186E+X206Y+X258Q+X281N, X25H+X100W+X135T+X176K+X181Q+X186E+X193E+X206Y, X25H+X100W+X135T+X176K+X181Q+X186E+X193E+X206Y+X258Q, X25H+X100W+X135T+X176K+X181Q+X186E+X193E+X206Y+X363E, X25H+X135T+X176K+X186E+X206Y+X258Q, X25H+X135T+X176K+X186E+X206Y+X258Q+X281N, X25H+X135T+X176K+X186E+X206Y+X258Q+X281N+X363E, X25H+X135T+X176K+X186E+X206Y+X258Q+X363E, X25H+X135T+X176K+X186E+X206Y+X281N, X25H+X135T+X176K+X186E+X206Y+X281N+X363E, X25H+X135T+X176K+X186E+X206Y+X281N+X363E+X415S, X25H+X135T+X176K+X186E+X206Y+X363E, X25H+X135T+X176K+X186E+X193E+X206Y+X258Q, X25H+X135T+X176K+X181Q+X186E+X206Y+X258Q, X25H+X135T+X176K+X181Q+X186E+X206Y+X258Q+X363E, X25H+X135T+X176K+X181Q+X186E+X193E+X206Y+X258Q+X281N, X25H+X176K+X186E+X206Y+X251E+X482W, X4Q+X25H+X176K+X186E+X206Y+X251E+X405M+X482W, X25H+X176K+X186E+X206Y+X482W, X25H+X186E+X206Y+X405M+X482W, X25H+X186E+X206Y+X482W, X25H+X176K+X186E+X193E+X206Y+X251E+X482W, X4Q+X176K+X186E+X193E+X206Y+X251E+X405M+X482W, X25H+X176K+X186E+X482W, X25H+X176K+X186E+X193E+X206Y+X482W, X4Q+X25H+X176K+X186E+X251E+X405M+X482W, X4Q+X206Y+X460G+X482W, X4Q+X25H+X176K+X206Y+X251E+X460G+X482W, X4Q+X25H+X176K+X193E+X251E+X186E+X482W, X4Q+X193E+X206Y+X276R+X186E+X482W, X186E+X25H+X482W, X25H+X193E+X206Y+X251E+X276R+X405M+X186E+X482W, X25H+X251E+X186E+X482W, X25H+X160W+X176K+X186E+X251E+X405M+X482W, and X193E+X206Y+X405M+X186E+X482W.
Preferably, the amino acid residue at the above cited positions (i.e., X) corresponds to the amino acid residue shown in SEQ ID NO: 1 or 3, preferably in SEQ ID NO: 1, at the respective position (according to the numbering of SEQ ID NO: 3).

Preferably, the improved property is improved expression. Preferably, the alpha-amylase variant of the present invention that exhibits improved expression compared to a parent alpha-amylase, preferably compared to SEQ ID NO: 1, is an amylase comprising amino acid substitution at one or more positions (according to the numbering of SEQ ID NO: 3) corresponding to positions selected from the group consisting of 4, 6, 20, 25, 27, 28, 37, 70, 72, 75, 81, 94, 96, 160, 175, 176, 193, 276, 277, 299, 323, 368, 372, 382, 400, 405, 434, 459, and 461.
Preferably, the alpha-amylase variant of the present invention that exhibits improved expression compared to a parent alpha-amylase, preferably compared to SEQ ID NO: 1, is an amylase comprising one or more amino acid substitutions selected from the group of amino acid substitutions (according to the numbering of SEQ ID NO: 3) consisting of X4Q, X6E, X20K, X25H, X25Y, X27I, X28V, X37M, X70H, X72C, X75L, X81L, X94M, X96Q, X160W, X175A, X176K, X193E, X276R, X277D, X299S, X323G, X368F, X372C, X382Q, X400A, X405M, X434S, X459N, and X461L. Preferably, the amino acid residue at the above cited positions (i.e., X) corresponds to the amino acid residue shown in SEQ ID NO: 1 or 3, preferably in SEQ ID NO: 1, at the respective position (according to the numbering of SEQ ID NO: 3).

Preferably, the improved property is improved solubility. Preferably, the alpha-amylase variant of the present invention that exhibits improved solubility compared to a parent alpha-amylase, preferably compared to SEQ ID NO: 1, is an amylase comprising amino acid substitution at one or more positions (according to the numbering of SEQ ID NO: 3) corresponding to positions selected from the group consisting of 4, 25, 83, 87, 160, 176, 193, 219, 226, 251, 311, 314, 315, 378, 405, 439, 459, 460, 461, and 471.
Preferably, the alpha-amylase variant of the present invention that exhibits improved t solubility compared to a parent alpha-amylase, preferably compared to SEQ ID NO: 1, is an amylase comprising one or more amino acid substitutions (according to the numbering of SEQ ID NO: 3) selected from the group of amino acid substitutions consisting of X4K, X25H, X83E, X87D, X87R, X160D, X176K, X193E, X219D, X219R, X226R, X226D, X251E, X311K, X314E, X314K, X315K, X378K, X405M, X439K, X459R, X460G, X460D, X461E, and X471E. Preferably, the amino acid residue at the above cited positions (i.e., X) corresponds to the amino acid residue shown in SEQ ID NO: 1 or 3, preferably in SEQ ID NO: 1, at the respective position (according to the numbering of SEQ ID NO: 3).

In another embodiment, the alpha-amylase variant according to the present invention having alpha-amylase activity comprises the one or more of the amino acid substitutions described above and one or more additional amino acids of specific kind, preferably one or more additional amino acid alterations, preferably amino acid substitutions, compared to the parent alpha-amylase.
Preferably, the alpha-amylase variant according to the present invention having alpha-amylase activity comprises 1-50 additional amino acid alterations, preferably 1-35, 1-31, 1-30, 1-20, 1-15, 1-10, or 1-5 amino acid alterations, preferably substitutions compared to the parent alpha-amylase.
Preferably, the alpha-amylase variant according to the present invention having alpha-amylase activity further comprises 1-4, preferably 1-2, amino acid deletions compared to the parent alpha-amylase.
In one embodiment, these additional amino acid alterations are conservative substitutions and/or substitutions as described below.
Preferably, the alpha-amylase variant of the present invention having alpha-amylase activity comprises an additional alteration, preferably additional substitution, of one or more, preferably of up to 1, up to 2, up to 3, up to 4, up to 5, up to 6, up to 7, up to 8, up to 9, up to 10, up to 15, up to 20, up to 25, or all amino acids at positions selected from the group consisting of 9, 130, 179, 181, 186, 190, 195, 202, 206, 244, 402, 419, 420, 422, 423, 428, 430, 435, 441, 444, 450, 452, 454, 466, 469, 473, 475, 476, 479, 483, and 485 wherein the numbering is according to the amino acid sequence set forth in SEQ ID NO: 3 and wherein said alpha-amylase variant has alpha-amylase activity, preferably one or more amino acid substitutions selected from the group of amino acid substitutions consisting of X9D, X9F, X9K, X9L, X9N, X9P, X9Q, X9S, X9T, X9Y, X130V, X179G, X179L, X181D, X181E, X181F, X181H, X181I, X181N, X181Q, X181S, X181T, X181V, X181W, X181Y, X186A, X186C, X186D, X186E, X186F, X186H, X186I, X186K, X186L, X186M, X186N, X186Q, X186R, X186S, X186V, X186W, X186Y, X190H, X195F, X195H, X195K, X195L, X195W, X195Y, X202L X206C, X206H, X206L, X206M, X206Y, X244A, X244C, X244D, X244E, X244F, X244G, X244H, X244M, X244N, X244Q, X244V, X402T, X419C, X420D, X420E, X420G, X420H, X420K, X420L, X420Q, X422C, X422N, X422H, X423F, X423M, X423Q, X423S, X428A, X428C, X428D, X428E, X428G, X428I, X428K, X428L, X428M, X428N, X428R, X428S, X428V, X428W, X428Y, X430A, X430C, X430D, X430E, X430F, X430G, X430I, X430L, X430P, X430Q, X430S, X430T, X430V, X435E, X435K, X435P, X435R, X435S, X435A, X435D, X437A, X437L, X437T, X437W, X441C, X441D, X441K, X441L, X441M, X441N, X441S, X444E, X444H, X444K, X444M, X444N, X444R, X444T, X450C, X450D, X450E, X450H, X450I, X450L, X450M, X450P, X450Q, X450R, X450T, X452A, X452C, X452E, X452F, X452I, X452K, X452M, X452N, X452R, X452T, X454A, X454E, X454K, X454L, X454M, X454P, X454S, X454T, X466E, X466W, X469F, X469L, X469Y, X473H, X473Q, X473R, X475A, X475K, X475N, X475E, X475L, X476G, X476E, X479A, X479I, X479K, X479M, X483F, X483I, X483L, X483Q, X483R, X485K, and X485R wherein the numbering is according to the amino acid sequence set forth in SEQ ID NO: 3. Preferably, the amino acid residue at the above cited positions (i.e., X) corresponds to the amino acid residue shown in SEQ ID NO: 1 or 3, preferably in SEQ ID NO: 1, at the respective position (according to the numbering of SEQ ID NO: 3).
Preferably, the alpha-amylase variant of the present invention having alpha-amylase activity comprises an additional alteration, preferably additional substitution, of one or more, preferably of up to 1, up to 2, up to 3, up to 4, up to 5, or all amino acids at positions selected from the group consisting of 181, 186, 195, 206, 441, and 473, preferably at position 186, 195, or 206, more preferably at position 206 or 195, wherein the numbering is according to the amino acid sequence set forth in SEQ ID NO: 3, preferably, wherein alpha-amylase variant further comprises one or more additional substitutions selected from the group consisting of X181D, X181E, X181F, X181H, X181I, X181N, X181Q, X181S, X181T, X181V, X181W, X181Y, X186A, X186C, X186D, X186E, X186F, X186H, X186I, X186K, X186L, X186M, X186N, X186Q, X186R, X186S, X186V, X186W, X186Y, X190H, X195F, X195H, X195K, X195L, X195W, X195Y, X206C, X206H, X206L, X206M, X206Y, X441C, X441D, X441K, X441L, X441M, X441N, X441S, X473H, X473Q, and X473R, (according to the numbering of SEQ ID NO: 3).
Most preferably, as additional alteration, preferably in combination with one or more additional alterations described above, the alpha-amylase variant of the present invention having one or more amino acid alterations, preferably substitutions, as described herein and having alpha-amylase activity comprises a deletion of one or more amino acids corresponding to positions selected from the group consisting of 181, 182, 183 and 184 corresponding to the numbering of SEQ ID NO: 3, preferably a deletion of two or more amino acids corresponding to positions selected from the group consisting of 181, 182, 183 and 184, preferably a deletion of amino acids corresponding to positions 181 and 182, 182 and 183, or 183 and 184 (according to the numbering of SEQ ID NO: 3). Preferably, the alpha-amylase variant of the present invention having one or more amino acid alterations, preferably substitutions, as described herein and having alpha-amylase activity comprises a deletion of one or more amino acids corresponding to positions 182 and 183, preferably a deletion of both amino acids corresponding to positions 182 and 183 (according to the numbering of SEQ ID NO: 3). Preferably, the alpha-amylase variant of the present invention having one or more amino acid alterations, preferably substitutions, as described herein and having alpha-amylase activity comprises a deletion of one or more, preferably of two or more, most preferably of two, amino acids corresponding to positions selected from the group consisting of R181, G182, D183, and G184, preferably D183* and G184*, wherein the numbering is according to the amino acid sequence set forth in SEQ ID NO: 3.

Preferably, the present invention is directed to an alpha-amylase variant of a parent alpha-amylase, wherein said variant comprises
(i) amino acid substitution at one or more positions corresponding to positions selected from the group consisting of 1, 2, 3, 4, 5, 6, 7, 8, 10, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 45, 47, 48, 51, 54, 59, 60, 63, 70, 71, 72, 73, 75, 76, 81, 82, 83, 86, 87, 89, 90, 91, 92, 93, 94, 95, 96, 97, 98, 99, 100, 103, 106, 108, 109, 113, 114, 115, 116, 117, 119, 120, 123, 125, 126, 128, 129, 131, 132, 133, 134, 135, 136, 139, 141, 142, 143, 144, 145, 146, 147, 149, 150, 151, 154, 155, 156, 158, 160, 161, 162, 163, 164, 165, 169, 170, 172, 173, 174, 175, 176, 177, 178, 180, 182, 184, 185, 187, 188, 189, 191, 192, 193, 203, 208, 210, 211, 212, 213, 214, 215, 216, 218, 219, 221, 222, 223, 224, 225, 226, 227, 230, 231, 233, 234, 235, 236, 237, 238, 240, 242, 243, 245, 249, 250, 251, 252, 253, 255, 256, 257, 258, 259, 260, 261, 262, 263, 264, 265, 268, 269, 270, 271, 272, 273, 274, 275, 276, 277, 279, 280, 281, 282, 284, 285, 286, 287, 288, 289, 290, 291, 292, 293, 294, 295, 296, 297, 298, 299, 301, 302, 303, 304, 306, 307, 308, 309, 310, 311, 312, 313, 314, 315, 317, 318, 319, 320, 321, 322, 323, 324, 326, 327, 333, 334, 336, 337, 338, 341, 342, 343, 344, 345, 346, 348, 350, 351, 352, 353, 354, 355, 356, 357, 358, 359, 360, 362, 363, 364, 365, 366, 367, 368, 370, 372, 375, 376, 378, 379, 381, 382, 383, 384, 385, 387, 388, 389, 390, 391, 392, 393, 394, 395, 396, 397, 398, 399, 400, 401, 403, 405, 406, 407, 408, 410, 413, 414, 415, 416, 418, 421, 424, 426, 427, 429, 431, 432, 434, 436, 438, 439, 440, 442, 443, 445, 446, 447, 448, 449, 451, 453, 455, 456, 457, 458, 459, 460, 461, 462, 463, 465, 467, 468, 470, 471, 474, 478, 480, and 482 according to the numbering of the amino acid sequence set forth in SEQ ID NO: 3,
(ii) said variant has at least 60%, preferably at least 80% but less than 100% sequence identity with the amino acid sequence set forth in SEQ ID NO: 1,
(iii) said variant comprises a deletion of one or more, preferably of two or more, most preferably of two, amino acids corresponding to positions selected from the group consisting of R181, G182, D183, and G184, preferably D183* and G184*, wherein the numbering is according to the amino acid sequence set forth in SEQ ID NO: 3,
(iv) optionally said variant comprises one or more additional amino acid substitutions as described herein,
(v) said variant comprises an improved property as described herein, and
(vi) said variant has alpha-amylase activity, preferably wherein the parent amylase is SEQ ID NO: 1.
In an alternative embodiment, the present invention is directed to an alpha-amylase variant of a parent alpha-amylase, wherein said variant comprises
(i) compared to a parent sequence one or more amino acid substitutions selected from the group consisting of X9D, X9F, X9K, X9N, X9P, X9Q, X9S, X9T, X9Y, X179G, X181D, X181F, X181H, X181I, X181N, X181Q, X181S, X181T, X181V, X181W, X181Y X186A, X186C, X186D, X186F, X186H, X186I, X186K, X186L, X186M, X186R, X186V, X186W, X186Y, X190H, X195H, X195K, X195L, X195W, X195Y, X206C, X206H, X206M, X206Y, X244A, X244C, X244D, X244E, X244F, X244G, X244H, X244M, X244N, X244V, X402T X419C, X420D, X420E, X420G, X420H, X420K, X420L, X420Q, X422C, X422N, X422H, X423F, X423M, X423Q, X423S, X428C, X428D, X428E, X428G, X428I, X428K, X428L, X428M, X428N, X428R, X428S, X428V, X428W, X428Y, X430A, X430C, X430D, X430E, X430F, X430G, X430L, X430P, X430Q, X430S, X430T, X430V, X435K, X435P, X435S, X435A, X435D, X437L, X437T, X437W, X441C, X441K, X441L, X441M, X441S, X444H, X444M, X444N, X444R, X444T, X450C, X450D, X450E, X450H, X450L, X450M, X450P, X450Q, X450R, X450T, X452A, X452C, X452E, X452F, X452I, X452K, X452M, X452N, X452T, X454A, X454E, X454K, X454L, X454P, X454S, X454T, X466E, X466W, X469F, X469L, X469Y, X475A, X475K, X475E, X475L, X479I, X479K, X479M, X483F, X483L, X483Q, and X483R, preferably one or more amino acid substitutions selected from the group consisting of X181D, X181F, X181H, X181I, X181N, X181Q, X181S, X181T, X181V, X181W, X181Y X186A, X186C, X186D, X186F, X186H, X186I, X186K, X186L, X186M, X186R, X186V, X186W, X186Y, X195H, X195K, X195L, X195W, X195Y, X206C, X206H, X206M, X206Y, X441C, X441K, X441L, X441M, and X441S, according to the numbering of the amino acid sequence set forth in SEQ ID NO: 3 and wherein said variant has alpha-amylase activity, preferably wherein the parent alpha-amylase for the alpha-amylase variant of the present invention is an amylase according to SEQ ID NO: 1 or SEQ ID NO: 3 or any alpha-amylase having at least 60% sequence identity to SEQ IDNO: 1 or SEQ ID NO: 3, most preferably the parent alpha-amylase for the alpha-amylase variant is an amylase according to according to SEQ ID NO: 1,
(ii) said variant has at least 60%, preferably at least 80% but less than 100% sequence identity with the amino acid sequence set forth in SEQ ID NO: 1,
(iii) said variant comprises a deletion of one or more, preferably of two or more, most preferably of two, amino acids corresponding to positions selected from the group consisting of R181, G182, D183, and G184, preferably D183* and G184*, wherein the numbering is according to the amino acid sequence set forth in SEQ ID NO: 3, and
(iv) said variant comprises an improved property as described herein, and
(v) said variant has alpha-amylase activity.

### Nucleic acid construct

The present invention also refers to a polynucleotide encoding the alpha-amylase variant of the present invention. Preferably, the polynucleotide is a codon-optimized polynucleotide for improving expression in a specific host cell, preferably a Bacillus cell.

In a further embodiment, the invention also relates to polypeptides which are encoded by a polynucleotide that hybridizes high stringency conditions, preferably under very high stringency conditions, with (i) the mature polypeptide coding sequence as described herein or (ii) the full-length complement of (i).

The present invention thus also refers to a nucleic acid, preferably an isolated, a synthetic, and/or a recombinant nucleic acid comprising:
(a) a nucleic acid sequence having at least at least 60%, at least 65%, at least 70%, at least 75%, at least 80%, at least 85%, at least 90%, at least 91 %, at least 92%, at least 93%, at least 94%, at least 95%, at least, 96%, at least 97%, at least 98%, at least 99%, but less than 100% identity to SEQ ID NO: 2, wherein the nucleic acid encodes a polypeptide having amylase activity;
(b) a nucleic acid sequence encoding a polypeptide having at least at least 60%, at least 65%, at least 70%, at least 75%, at least 80%, at least 85%, at least 90%, at least 91 %, at least 92%, at least 93%, at least 94%, at least 95%, at least, 96%, at least 97%, at least 98%, at least 99%, but less than 100% identity to SEQ ID NO: 1, wherein the polypeptide has amylase activity or encoding any alpha-amylase variant having amylase activity described herein;
(c) a polynucleotide that hybridizes under high stringency conditions, preferably under very high stringency conditions, with the complement of
   (i) a coding sequence of SEQ ID NO: 1 or a coding sequence of any alpha-amylase variant having amylase activity described herein; or
   (ii) a polynucleotide shown in SEQ ID NO: 2;
(d) a fragment of (a), (b), or (c), wherein the fragment encodes a polypeptide having amylase activity; or
(e) a nucleic acid sequence fully complementary to any of (a) to (d).
(f) a polynucleotide that differs from any of the nucleic acid sequences described in (a) to (e) merely by the degeneracy of the genetic code.

The present invention also refers to a nucleic acid construct, preferably an expression cassette, comprising the polynucleotide as described herein.

Typically, the expression cassette comprises three elements: a promoter sequence, an open reading frame, and a 3' untranslated region that, in eukaryotes, usually contains a polyadenylation site. Additional regulatory elements may include transcriptional as well as translational enhancers. An intron sequence may also be added to the 5' untranslated region (UTR) or in the coding sequence to increase the amount of the mature message that accumulates in the cytosol. The expression cassette may be part of a vector or may be integrated into the genome of a host cell and replicated together with the genome of its host cell. The expression cassette usually is capable of increasing or decreasing expression.

The present invention also refers to an expression vector comprising the polynucleotide or the nucleic acid construct as described herein. The expression vector can be a low copy number vector or high copy number vector.

A vector as used herein may provide segments for transcription and translation of a foreign polynucleotide upon transformation into a host cell or host cell organelles. Such additional segments may include regulatory nucleotide sequences, one or more origins of replication that is required for its maintenance and/or replication in a specific cell type, one or more selectable markers, a polyadenylation signal, a suitable site for the insertion of foreign coding sequences such as a multiple cloning site etc. One example is when a vector is required to be maintained in a bacterial cell as an episomal genetic element (e.g. plasmid or cosmid molecule). Non-limiting examples of suitable origins of replication include the f1-ori and colE1.

A vector may replicate without integrating into the genome of a host cell, e.g. as a plasmid in a bacterial host cell, or it may integrate part or all of its DNA into the genome of the host cell and thus lead to replication and expression of its DNA.

The polynucleotide encoding the alpha-amylase variant may be introduced into a vector by means of standard recombinant DNA techniques. Once introduced into the vector, the polynucleotide comprising a coding sequence may be suitable to be introduced (transformed, transduced, transfected, etc.) into a host cell or host cell organelles. A cloning vector may be chosen suitable for expression of the polynucleotide sequence in the host cell or host cell organelles.

### Host cell

The present invention also refers to a host cell comprising the polynucleotide encoding the alpha-amylase variant as described herein, the nucleic acid construct as described herein, or the expression vector as described herein. In one embodiment of the invention, a vector is used for transformation of a host cell.

The polynucleotide encoding the alpha-amylase variant as described herein may be transiently or stably introduced into a host cell and may be maintained non-integrated, for example, as a plasmid. Usually stable transformation is due to integration of nucleic acid comprising a foreign coding sequence into the chromosomes or as an episome (separate piece of nuclear DNA). Usually transient transformation is due to nucleic acid comprising a foreign nucleic acid sequence is not integrated into the chromosomes or as an episome. Alternatively, the polynucleotide encoding the alpha-amylase variant as described herein may be integrated into the host genome.

The introduction of nucleic acid into a host cell may, for instance, but not limited thereto, be effected by protoplast transformation (see, e.g., Chang and Cohen, 1979, Molecular General Genetics 168: 111-115), by using competent cells (see, e.g., Young and Spizizen, 1961, Journal of Bacteriology 81: 823-829, or Dubnau and Davidoff-Abelson, 1971, Journal of Molecular Biology 56: 209-221), by electroporation (see, e.g., Shigekawa and Dower, 1988, Biotechniques 6: 742-751), or by conjugation (see, e.g., Koehler and Thorne, 1987, Journal of Bacteriology 169: 5271-5278). Specific transformation protocols are known in the art for various types of host cells (see, e.g., for E. coli protoplast transformation see Hanahan, 1983, J. Mol. Biol. 166: 557-580). Various host cells can be used for expressing the nucleic acid construct described herein. Host cells comprising the genetic constructs described herein can be obtained by one of the methods described herein for introducing the polynucelotides into such host cells. The host cell of the present invention does not naturally express the alpha-amylase variant. Thus, the host cell is a recombinant host cell; the nucleic acid construct described herein is heterologous for the host cell.

In one embodiment, the host cell is a prokaryote or a eukaryote. In another embodiment, the host cell is a bacteria, an archaea, a fungal cell, a yeast cell or a eukaryotic cell. In another embodiment, the host cell is a non-human host cell.

In one embodiment, the host cell is a bacterial cell. The bacterial host cell may be any gram-positive bacterium or a gram-negative bacterium. Gram-positive bacteria include, but are not limited to, Bacillus, Brevibacterium, Corynebacterium, Streptococcus, Streptomyces, Staphylococcus, Enterococcus, Lactobacillus, Lactococcus, Clostridium, Geobacillus, and Oceanobacillus. Gram-negative bacteria include, but are not limited to, Escherichia, Pseudomonas, Salmonella, Campylobacter, Helicobacter, Acetobacter, Flavobacterium, Fusobacterium, Gluconobacter. In a specific embodiment, the bacterial host cell is a Echerichia coli cell. In one embodiment, the host cell is a bacterial cell. In a specific embodiment the host cell is of the genus Escherichia or Bacillus.

In the methods of the present invention, the bacterial host cell may be any Bacillus cell. Bacillus cells useful in the practice of the present invention include, but are not limited to, Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus methylotrophicus, Bacillus cereus Bacillus paralicheniformis, Bacillus subtilis, and Bacillus thuringiensis cells. In one embodiment, the bacterial host cell is a Bacillus amyloliquefaciens, Bacillus pumilus, Bacillus lentus, Bacillus licheniformis, Bacillus stearothermophilus or Bacillus subtilis cell. In preferred embodiment, the bacterial host cell is a Bacillus licheniformis cell, a Bacillus pumilus, or a Bacillus subtilis cell. Preferably, the bacterial host cell is a Bacillus licheniformis cell.

In the methods of the present invention, the bacterial host cell may be Lactobacillus acidophilus, Lactobacillus plantarum, Lactobacillus gasseri, Lactobacillus bulgaricusk, Lactobacillus reuteri, Escherichia coli, Staphylococcus aureus, Corynebacterium glutamicum, Corynebacterium acetoglutamicum, Corynebacterium acetoacidophilum, Corynebacterium callunae, Corynebacterium ammoniagenes, Corynebacterium thermoaminogenes, Corynebacterium melassecola, Corynebacterium effiziens, Corynebacterium efficiens, Corynebacterium deserti, Brevibacterium flavum, Brevibacterium lactofermentum, Brevibacterium divarecatum, Pseudomonas putida, Pseudomonas syringae, Streptomyces coelicolor, Streptomyces lividans, Streptomyces albus, Streptomyces avermitilis, Gluconobacter oxydans, Gluconobacter morbifer, Gluconobacter thailandicus, Acetobacter aceti, Clostridium acetobutylicum, Clostridium saccharobutylicum, Clostridium beijerinckii, Streptococcus equisimilis, Streptococcus pyogenes, Streptococcus uberis, Streptococcus equi subsp., Zooepidemicus or Basfia succiniciproducens.

In another embodiment, the bacterial host cell may additionally contain modifications, e.g., deletions or disruptions, of other genes that may be detrimental to the production, recovery or application of a polypeptide of interest. In one embodiment, a bacterial host cell is a protease-deficient cell. In another embodiment, the bacterial host cell, e.g., Bacillus cell, comprises a disruption or deletion of extracellular protease genes including but not limited to aprE, mpr, vpr, bpr, and / or epr. In one embodiment, the bacterial host cell does not produce spores. In another embodiment, the bacterial host cell, e.g., Bacillus cell, comprises a disruption or deletion of spoIIAC, sigE, and / or sigG. In one embodiment, the bacterial host cell, e.g., Bacillus cell, comprises a disruption or deletion of one of the genes involved in the biosynthesis of surfactin, e.g., srfA, srfB, srfC, and / or srfD. See, for example, U.S. Patent No. 5,958,728. In another embodiment, the bacterial host cell comprises a disruption or deletion of one of the genes involved in the biosynthesis of polyglutamic acid. Other genes, including but not limited to the amyE gene, which are detrimental to the production, recovery or application of a polypeptide of interest may also be disrupted or deleted.

In another embodiment, the bacterial host cell is a standard E. coli cloning host cell, including but not limited to DH5alpha (Invitrogen), DH10B, (Invitrogen), Omnimax (Invitrogen), INV110 (Invitrogen), TOP10 (Invitrogen), HB101 (Promega), SURE (Stratagene), XL1-Blue (Stratagen), TG1 (Lucigen), and JM109 (NEB). In another embodiment, the bacterial host cell is a standard Bacillus subtilis cloning host cell, including but not limited to B. subtilis carrying a defective hsd(RI)R-M- locus such as B. subtilis IG-20 (BGSC 1A436) or a defective hsdRM1 mutation such as B. subtilis1012 WT (Mobitec).

Alternative further host cells include but are not limited to: Aspergillus niger, Aspergillus oryzae, Hansenula polymorpha, Thermomyces lanuginosus, fusarium oxysporum, Fusarium heterosporum, Pichia pastoris (also known as Komagataella phaffii), Myceliopthora thermophile (C1), Themothelomyces thermophila, Schizosaccharomyces pombe, Trichoderma, preferably Trichoderma reesei and Saccharomyces, preferably Saccharomyces cerevisiae, or Rhizomucor.

### Methods of making

Another embodiment of the present invention is a method of obtaining an alpha-amylase variant of a parent alpha-amylase comprising the steps of:
a) introducing into a parent alpha-amylase, preferably SEQ ID NO: 1, an amino acid alteration, preferably insertion, deletion, substitution, or combinations thereof, preferably substitution, at one or more positions corresponding to positions selected from the group consisting of 1, 2, 3, 4, 5, 6, 7, 8, 10, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 45, 47, 48, 51, 54, 59, 60, 63, 70, 71, 72, 73, 75, 76, 81, 82, 83, 86, 87, 89, 90, 91, 92, 93, 94, 95, 96, 97, 98, 99, 100, 103, 106, 108, 109, 113, 114, 115, 116, 117, 119, 120, 123, 125, 126, 128, 129, 131, 132, 133, 134, 135, 136, 139, 141, 142, 143, 144, 145, 146, 147, 149, 150, 151, 154, 155, 156, 158, 160, 161, 162, 163, 164, 165, 169, 170, 172, 173, 174, 175, 176, 177, 178, 180, 182, 184, 185, 187, 188, 189, 191, 192, 193, 203, 208, 210, 211, 212, 213, 214, 215, 216, 218, 219, 221, 222, 223, 224, 225, 226, 227, 230, 231, 233, 234, 235, 236, 237, 238, 240, 242, 243, 245, 249, 250, 251, 252, 253, 255, 256, 257, 258, 259, 260, 261, 262, 263, 264, 265, 268, 269, 270, 271, 272, 273, 274, 275, 276, 277, 279, 280, 281, 282, 284, 285, 286, 287, 288, 289, 290, 291, 292, 293, 294, 295, 296, 297, 298, 299, 301, 302, 303, 304, 306, 307, 308, 309, 310, 311, 312, 313, 314, 315, 317, 318, 319, 320, 321, 322, 323, 324, 326, 327, 333, 334, 336, 337, 338, 341, 342, 343, 344, 345, 346, 348, 350, 351, 352, 353, 354, 355, 356, 357, 358, 359, 360, 362, 363, 364, 365, 366, 367, 368, 370, 372, 375, 376, 378, 379, 381, 382, 383, 384, 385, 387, 388, 389, 390, 391, 392, 393, 394, 395, 396, 397, 398, 399, 400, 401, 403, 405, 406, 407, 408, 410, 413, 414, 415, 416, 418, 421, 424, 426, 427, 429, 431, 432, 434, 436, 438, 439, 440, 442, 443, 445, 446, 447, 448, 449, 451, 453, 455, 456, 457, 458, 459, 460, 461, 462, 463, 465, 467, 468, 470, 471, 474, 478, 480, and 482 according to the numbering of the amino acid sequence set forth in SEQ ID NO: 3,
b) optionally introducing a deletion at two or more positions in the parent alpha-amylase said positions R181, G182, D183, and G184 according to the numbering of the amino acid sequence set forth in SEQ ID NO: 3;
and said method thereby providing an alpha-amylase variant of said parent alpha-amylase, wherein said variant has at least 60% sequence identity to the amino acid sequence to the polypeptide of SEQ ID NO: 1, and wherein said alpha-amylase variant has alpha-amylase activity and preferably wherein the alpha-amylase variant has an improved property relative to said parent.

Ways of introducing amino acid alterations, e.g., a substitution or a deletion, into protein sequence are well known in the art. The variants may be prepared using any mutagenesis procedure known in the art, such as site-directed mutagenesis, synthetic gene construction, semi-synthetic gene construction, random mutagenesis, shuffling, etc. Site-directed mutagenesis is a technique in which one or more (several) mutations are created at one or more defined sites in a polynucleotide encoding the parent. Site-directed mutagenesis can be accomplished *in vitro* by PCR involving the use of oligonucleotide primers containing the desired mutation. Site-directed mutagenesis can also be performed *in vitro* by cassette mutagenesis involving the cleavage by a restriction enzyme at a site in the plasmid comprising a polynucleotide encoding the parent and subsequent ligation of an oligonucleotide containing the mutation in the polynucleotide. Usually the restriction enzyme that digests at the plasmid and the oligonucleotide is the same, permitting sticky ends of the plasmid and insert to ligate to one another. See, *e.g*., Scherer and Davis, 1979, Proc. Natl. Acad. Sci. USA 76: 4949-4955; and Barton et al., 1990, Nucleic Acids Res. 18: 7349-4966. Site-directed mutagenesis can also be accomplished *in vivo* by methods known in the art. See, *e.g*., U.S. Patent Application Publication No. 2004/0171154; Storici et al., 2001, Nature Biotechnol. 19: 773-776; Kren et al., 1998, Nat. Med. 4: 285-290; and Calissano and Macino, 1996, Fungal Genet. Newslett. 43: 15-16. Any site-directed mutagenesis procedure can be used in the present invention. There are many commercial kits available that can be used to prepare variants.

Synthetic gene construction entails *in vitro* synthesis of a designed polynucleotide molecule to encode a polypeptide of interest. Gene synthesis can be performed utilizing a number of techniques, such as the multiplex microchip-based technology described by Tian et al. (2004, Nature 432: 1050-1054) and similar technologies wherein oligonucleotides are synthesized and assembled upon photo-programable microfluidic chips.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (*e.g.,* Lowman et al., 1991, Biochemistry 30: 10832-10837; U.S. Patent No. 5,223,409; WO 92/06204) and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

Semi-synthetic gene construction is accomplished by combining aspects of synthetic gene construction, and/or site-directed mutagenesis, and/or random mutagenesis, and/or shuffling. Semi-synthetic construction is typified by a process utilizing polynucleotide fragments that are synthesized, in combination with PCR techniques. Defined regions of genes may thus be synthesized *de novo,* while other regions may be amplified using site-specific mutagenic primers, while yet other regions may be subjected to error-prone PCR or non-error prone PCR amplification. Polynucleotide subsequences may then be shuffled.

The obtained alpha-amylase variant can be produced in an industrial scale and subsequently purified. Industrial production of enzymes usually is done by cultivating a host cell (also called fermentation) which expresses the enzyme. Suitable host cells are described herein. A nucleic acid sequence encoding the alpha-amylase variant described herein can be transformed into the host cell, which is subsequently cultivated under conditions suitable for the host cell to produce the alpha-amylase variant. In a preferred embodiment, the alpha-amylase variant is purified from the host cell.

Hence, in yet another embodiment, the present invention is directed to a method of producing an alpha-amylase variant, comprising the steps of
(a) providing a host cell comprising a heterologous nucleic acid construct comprising a polynucleotide encoding the alpha-amylase variant described herein by introducing the nucleic acid construct comprising the polynucleotide encoding the alpha-amylase variant as described herein into the host cell;
(b) cultivating the recombinant host cell of step (a) under conditions conductive for the expression of the polynucleotide; and
(c) optionally, recovering the alpha-amylase variant encoded by the polynucleotide.

Cultivation of the host cell normally takes place in a suitable nutrient medium allowing the recombinant cells to grow and express the desired protein. At the end of fermentation, the fermentation broth is collected and may be further processed, wherein the fermentation broth comprises a liquid fraction and a solid fraction. The enzyme of interest may be further purified from the fermentation broth.

The alpha-amylase variant described herein may be secreted (into the liquid fraction of the fermentation broth) or may not be secreted from the microbial cells (and therefore is comprised in the cells of the fermentation broth). Depending on this, the alpha-amylase variant may be recovered from the liquid fraction of the fermentation broth or from cell lysates. Preferably, the alpha-amylase variant is secreted from the cell into the fermentation broth, preferably by means of a secretion signal peptide added to the terminus of the amino acid sequence of the alpha-amlyase variant. Recovery of the alpha-amylase variant can be achieved by methods known to those skilled in the art. Suitable methods for recovery of proteins from fermentation broth include but are not limited to collection, centrifugation, filtration, extraction, and precipitation. If the product of interest precipitates or crystallizes in the fermentation broth or binds at least in part to the particulate matter of the fermentation broth additional treatment steps might be needed to release the protein of interest from the biomass or to solubilize protein of interest crystals and precipitates. US 6,316,240 B1 and WO 2008/110498 A1 describe a method for recovering a protein of interest, which precipitates and/or crystallizes during fermentation, from the fermentation broth. In case the desired protein is comprised in the cells of the fermentation broth release of the product of interest from the cells might be needed. Release from the cells can be achieved for instance, but not being limited thereto, by cell lysis with techniques well known to the skilled person, e.g., lysozyme treatment, ultrasonic treatment, French press or combinations thereof.

The alpha-amylase variant may be purified from the fermentation broth by methods known in the art. For example, the alpha-amylase variant may be isolated from the fermentation broth by conventional procedures including, but not limited to, centrifugation, filtration, extraction, spray-drying, evaporation, or precipitation. The isolated polypeptide may then be further purified by a variety of procedures known in the art including, but not limited to, chromatography (e.g., ion exchange, affinity, hydrophobic, chromatofocusing, and size exclusion), electrophoretic procedures (e.g., preparative isoelectric focusing (IEF), differential solubility (e.g., ammonium sulfate precipitation), or extraction (see, e.g., Protein Purification, J.-C. Janson and Lars Ryden, editors, VCH Publishers, New York, 1989). The purified polypeptide may then be concentrated by procedures known in the art including, but not limited to, ultrafiltration and evaporation, in particular, thin film evaporation.

### Compositions

The purified solution of the alpha-amylase variant described herein may be further processed to form an alpha-amylase containing composition, i.e., an "alpha-amylase variant formulation". Hence, also claimed herein is a composition comprising the alpha-amylase variant described herein and at least one additional component.

The alpha-amylase variant formulation can be either solid or liquid. Protein formulations can be obtained by using techniques known in the art. For instance, without being limited thereto, solid enzyme formulations can be obtained by extrusion or granulation. Suitable extrusion and granulation techniques are known in the art and are described for instance in WO 94/19444 A1 and WO 97/43482 A1. Liquid enzyme formulations may comprise amounts of enzyme in the range of 0.1% to 40%, 0.5% to 30%, 1% to 25%, 1% to 10%, or preferably 1-6% by weight, all relative to the total weight of the enzyme formulation.

Preferably, the enzyme formulation comprises the alpha-amylase variant of the present invention in an amount of 1-100 g active alpha-amylase variant per kg of enzyme formulation, preferably 5-80 g/kg, 5-60 g/kg, or 10-40 g/kg.

In one embodiment, the enzyme formulation, in particular the liquid enzyme formulation, comprises in addition one or more additional compounds selected from the group consisting of solvent, salt, pH regulator, preservative, enzyme stabilizer, and thickening agent. The solvent may be water and/or an organic solvent. Aqueous enzyme formulations of the invention may comprise water in amounts of more than about 50% by weight, more than about 60% by weight, more than about 70% by weight, or more than about 80% by weight, all relative to the total weight of the enzyme formulation. The organic solvent may be a water-miscible solvent. The organic solvent may be one or more selected from the group consisting of glycerol, propanediol, polypropylene glycol, and polyethylene glycol.

In one embodiment, the amylase formulation comprises at least one preservative. Preferably, preservative means substances that are added to a liquid composition for the purpose of preservation, meaning more preferably that compounds known to have preserving features comprised in a liquid composition formed in the production process are excluded from the term preservatives. In one embodiment, the preservative is selected from the group consisting of 2-phenoxyethanol, glutaraldehyde, 2-bromo-2-nitropropane-1,3-diol, and formic acid in acid form or as its salt, and 4,4'-dichloro 2-hydroxydiphenylether. Usually, the liquid compositions of the invention comprise at least one preservative in amounts below 10ppm, such as in amounts ranging from 2 ppm to 5% by weight relative to the total weight of the liquid composition. Preferably, the amylase formulation is free from preservatives, meaning that preservatives are comprised in amounts less than 1 ppm.

In one embodiment, the enzyme formulation described herein may comprise at least one enzyme stabilizer. Preferred enzyme stabilizers are selected from the group consisting of polyols, (e.g., 1,3-propanediol, ethylene glycol, glycerol, and 1,2-propanediol), salts (e.g., CaCl2, MgCl2, NaCl), formic acid, formiate (e.g., sodium formiate), acetate and any combination thereof. If proteases are present, protease inhibitors may be added, preferably selected from borate, boric acid, boronic acids (e.g., phenylboronic acids like 4-FPBA), peptide aldehydes, peptide acetals, and peptide aldehyde hydrosulfite adducts, preferably peptide aldehydes like Z-VAL-H or Z-GAY-H. Liquid enzyme formulations of the invention may comprise residual components such as salts originating from the fermentation medium, cell debris originating from the production host cells, metabolites produced by the production host cells during fermentation. In one embodiment, residual components may be comprised in liquid enzyme formulations in amounts less than 30% by weight, less than 20% by weight less, than 10% by weight, or less than 5% by weight, all relative to the total weight of the aqueous enzyme formulation.

### Secondary enzymes

In another embodiment, the composition comprising an alpha-amylase variant as described herein further comprises one or more second enzyme different from the alpha-amylase variant. Preferably, the second enzyme is selected from the group consisting of oxidoreductase, transferase, hydrolase, lyase, isomerase, ligase, aminopeptidase, amylase, asparaginase, carbohydrase, carboxypeptidase, catalase, cellulase, chitinase, cutinase, cyclodextrin glycosyltransferase, deoxyribonuclease, esterase, alpha-galactosidase, betagalactosidase, glucoamylase, alpha-glucosidase, beta-glucosidase, hyaluronic acid synthase, invertase, laccase, lipase, mannosidase, mutanase, oxidase, a pectinolytic enzyme, peroxidase, phytase, polyphenoloxidase, protease, ribonuclease, transglutaminase, and xylanase. In particular, the second enzyme is selected from the group consisting of a protease, a second amylase, a lipase, a cellulase, a laccase, a mannanase, a pectinase, xylanase, a nuclease, and any combination thereof. Preferably, the second enzyme is selected from the group consisting of amylase different to the protease, amylase of the present invention, cellulase, mannanase, and lipase, Preferably, the second enzyme is selected from the group consisting of protease, mannanase, and lipase, particularly preferred a protease or a mannanase, most preferably, a protease, preferably a subtilisin protease.

### Proteases

Proteases are active proteins exerting "protease activity" or "proteolytic activity". Proteolytic activity is related to the rate of degradation of protein by a protease or proteolytic enzyme in a defined course of time.

Preferably, the second enzyme different from the alpha-amylase variant is a protease with at least 40 to 100% identity to the full-length polypeptide sequence of any of SEQ ID NO: 10-14. In one embodiment, the protease comprises an amino acid sequence having at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the full length polypeptide sequence shown in SEQ ID NO: 10, 11, 12,13, or 14, preferably SEQ ID NO: 10.

Preferably, the protease used in combination with the alpha-amylase variant described herein comprises an amino acid sequence having at least 80%, at least 85%, at least 90%, at least 91%, at least 92%, at least 93%, at least 94%, at least 95%, at least 96%, at least 97%, at least 98%, or at least 99%, but below 100% sequence identity with SEQ ID NO: 10 and further comprises amino acid substitutions in one or more of the following positions 3, 4, 9, 15, 24, 27, 33, 36, 57, 68, 76, 77, 87, 95, 96, 97, 98, 99, 100, 101, 102, 103, 104, 106, 118, 120, 123, 128, 129, 130, 131, 154, 160, 167, 170, 194, 199, 205, 206, 217, 218, 222, 224, 232, 235, 236, 245, 248, 252 and 274 (according to the BPN' numbering), which has proteolytic activity. In one embodiment, such a protease is not mutated at positions Asp32, His64 and Ser221 (according to BPN' numbering). Preferably, the protease used in combination with the alpha-amylase variant described herein comprises an amino acid sequence having at least 80%, at least 85%, at least 90%, at least 91%, at least 92%, at least 93%, at least 94%, at least 95%, at least 96%, at least 97%, at least 98%, or at least 99%, but below 100% sequence identity with SEQ ID NO: 10 and is further characterized by having amino acid glutamic acid (E), or aspartic acid (D), or asparagine (N), or glutamine (Q), or alanine (A), or glycine (G), or serine (S), preferably glutamic acid (E), at position 101 (according to BPN' numbering) and has proteolytic activity. Mostly preferred is a protease has at least 80%, but below 100% sequence identity with SEQ ID NO: 10 and is characterized by having amino acid glutamic acid (E) at position 101 (according to BPN' numbering) and has proteolytic activity. The protease may comprise an amino acid substitution at position 101, such as R101E alone or in combination with one or more substitutions at positions 3, 4, 9, 15, 24, 27, 33, 36, 57, 68, 76, 77, 87, 95, 96, 97, 98, 99, 100, 102, 103, 104, 106, 118, 120, 123, 128, 129, 130, 131, 154, 160, 167, 170, 194, 199, 205, 206, 217, 218, 222, 224, 232, 235, 236, 245, 248, 252 and/or 274 (according to BPN' numbering) and has proteolytic activity. In one embodiment, said protease comprises one or more further substitutions: (a) threonine at position 3 (3T), (b) isoleucine at position 4 (41), (c) alanine, threonine or arginine at position 63 (63A, 63T, or 63R), (d) aspartic acid or glutamic acid at position 156 (156D or 156E), (e) proline at position 194 (194P), (f) methionine at position 199 (199M), (g) isoleucine at position 205 (205I), (h) aspartic acid, glutamic acid or glycine at position 217 (217D, 217E or 217G), (i) combinations of two or more amino acids according to (a) to (h). A suitable protease may be at least 80% identical to SEQ ID NO: 10 and is characterized by comprising one amino acid (according to (a)-(h)) or combinations according to (i) together with the amino acid 101E, 101D, 101N, 101Q, 101A, 101G, or 101S (according to BPN' numbering) and has proteolytic activity. In one embodiment, the protease is at least 80% identical to SEQ ID NO:10 and is characterized by comprising the mutation (according to BPN' numbering) R101E, or S3T + V4I + V205I, or S3T + V4I + R101E + V205I or S3T + V4I + V199M + V205I + L217D, and has proteolytic activity. In another embodiment, the protease comprises an amino acid sequence having at least 80% identity to SEQ ID NO: 10 and being further characterized by comprising S3T + V4I + S9R + A15T + V68A + D99S + R101S + A103S + I104V + N218D (according to the BPN' numbering) and has proteolytic activity. In another embodiment, the protease may have an amino acid sequence being at least 80% identical to SEQ ID NO:10 and being further characterized by comprising R101E, and one or more substitutions selected from the group consisting of S156D, L262E, Q137H, S3T, R45E,D,Q, P55N, T58W,Y,L, Q59D,M,N,T, G61 D,R, S87E, G97S, A98D,E,R, S106A,W, N117E, H120V,D,K,N, S125M, P129D, E136Q, S144W, S161T, S163A,G, Y171 L, A172S, N185Q, V199M, Y209W, M222Q, N238H, V244T, N261T,D and L262N,Q,D (according to the BPN' numbering), and has proteolytic activity.

### Lipases

"Lipases", "lipolytic enzyme", "lipid esterase", all refer to an enzyme of EC class 3.1.1 ("carboxylic ester hydrolase"). Lipase means active protein having lipase activity (or lipolytic activity; triacylglycerol lipase, EC 3.1.1.3), cutinase activity (EC 3.1.1.74; enzymes having cutinase activity may be called cutinase herein), sterol esterase activity (EC 3.1.1.13) and/or wax-ester hydrolase activity (EC 3.1.1.50). Lipases include those of bacterial or fungal origin.

In one aspect of the invention, a suitable lipase (component (b)) is selected from the following: lipases from *Humicola* (synonym *Thermomyces*)*,* e.g. from *H. lanuginosa* (*T. lanuginosus*) as described in EP 258068, EP 305216, WO 92/05249 and WO 2009/109500 or from *H. insolens* as described in WO 96/13580; lipases derived from *Rhizomucor miehei* as described in WO 92/05249; lipase from strains of *Pseudomonas* (some of these now renamed to *Burkholderia*), e.g. from *P*. *alcaligenes* or *P*. *pseudoalcaligenes* (EP 218272, WO 94/25578, WO 95/30744, WO 95/35381, WO 96/00292), P. *cepacia* (EP 331376), *P. stutzeri* (GB 1372034), *P*. *fluorescens, Pseudomonas sp.* strain SD705 (WO 95/06720 and WO 96/27002), *P*. *wisconsinensis* (WO 96/12012), *Pseudomonas mendocina* (WO 95/14783), *P. glumae* (WO 95/35381, WO 96/00292); lipase from *Streptomyces griseus* (WO 2011/150157) and *S*. *pristinaespiralis* (WO 2012/137147), GDSL-type *Streptomyces* lipases (WO 2010/065455); lipase from *Thermobifida fusca* as disclosed in WO 2011/084412; lipase from *Geobacillus stearothermophilus* as disclosed in WO 2011/084417; *Bacillus* lipases, e.g. as disclosed in WO 00/60063, lipases from B. *subtilis* as disclosed in Dartois et al. (1992), Biochemica et Biophysica Acta, 1131, 253-360 or WO 2011/084599, *B. stearothermophilus* (JP S64-074992) or *B*. *pumilus* (WO 91/16422); lipase from *Candida antarctica* as disclosed in WO 94/01541; cutinase from *Pseudomonas mendocina* (US 5389536, WO 88/09367); cutinase from *Magnaporthe grisea* (WO 2010/107560); cutinase from *Fusarum solani pisi* as disclosed in WO 90/09446, WO 00/34450 and WO 01/92502; and cutinase from *Humicola lanuginosa* as disclosed in WO 00/34450 and WO 01/92502.

Such suitable lipase variants are e.g. those which are developed by methods as disclosed in WO 95/22615, WO 97/04079, WO 97/07202, WO 00/60063, WO 2007/087508, EP 407225 and EP 260105.

Commercially available lipase enzymes include but are not limited to those sold under the trade names Lipolase^{™}, Lipex^{™}, Lipolex^{™} and Lipoclean^{™} (Novozymes A/S), Lumafast (originally from Genencor), Preferenz L (DuPont), and Lipomax (Gist-Brocades/ now DSM).

In one embodiment, lipase is selected from fungal triacylglycerol lipase (EC class 3.1.1.3). Fungal triacylglycerol lipase may be selected from lipases of *Thermomyces lanuginosus.* In one embodiment, the *Thermomyces lanuginosa* lipase is selected from triacylglycerol lipase according to amino acids 1-269 of SEQ ID NO: 2 of US5869438 and variants thereof having lipolytic activity.

*Thermomyces lanuginosa* lipase may be selected from variants having lipolytic activity which are at least 90%, at least 91%, at least 92%, at least 93%, at least 94%, at least 95%, at least 96%, at least 97%, at least 98% or at least 99% identical when compared to the full length polypeptide sequence of amino acids 1-269 of SEQ ID NO: 2 of US5869438.

*Thermomyces lanuginosa* lipase may be selected from variants having lipolytic activity comprising conservative mutations only, which do not pertain the functional domain of amino acids 1-269 of SEQ ID NO: 2 of US5869438. Lipase variants of this embodiment having lipolytic activity may be at least 95%, at least 96%, at least 97%, at least 98% or at least 99% similar when compared to the full-length polypeptide sequence of amino acids 1-269 of SEQ ID NO: 2 of US5869438.

*Thermomyces lanuginosa* lipase may be selected from variants having lipolytic activity comprising the following amino acid substitutions when compared to amino acids 1-269 of SEQ ID NO: 2 of US5869438: T231R and N233R. Said lipase variants may further comprise one or more of the following amino acid exchanges when compared to amino acids 1-269 of SEQ ID NO: 2 of US5869438: Q4V, V60S, A150G, L227G, P256K.

*Thermomyces lanuginosa* lipase may be selected from variants having lipolytic activity comprising the amino acid substitutions T231R, N233R, Q4V, V60S, A150G, L227G, P256K within the polypeptide sequence of amino acids 1-269 of SEQ ID NO: 2 of US5869438 and are at least 95%, at least 96%, or at least 97% similar when compared to the full length polypeptide sequence of amino acids 1-269 of SEQ ID NO: 2 of US5869438.

*Thermomyces lanuginosa* lipase may be selected from variants having lipolytic activity comprising the amino acid substitutions T231R and N233R within amino acids 1-269 of SEQ ID NO: 2 of US5869438 and are at least 95%, at least 96%, at least 97%, at least 98%, or at least 99% similar when compared to the full length polypeptide sequence of amino acids 1-269 of SEQ ID NO: 2 of US5869438.

*Thermomyces lanuginosa* lipase may be a variant of amino acids 1-269 of SEQ ID NO: 2 of US5869438 having lipolytic activity, wherein the variant of amino acids 1-269 of SEQ ID NO: 2 of US5869438 is characterized in containing the amino acid substitutions T231R and N233R. *Thermomyces lanuginosa* lipase may be selected from variants having lipolytic activity comprising the amino acid substitutions N11K/A18K/G23K/K24A/V77I/D130A/V154I/V187T/T189Q or N11K/A18K/G23K/K24A/L75R/V77I/D130A/V154I/V187T/T189Q within the polypeptide sequence of amino acids 1-269 of SEQ ID NO: 1 of WO2015/01009 and are at least 95%, at least 96%, or at least 97% similar when compared to the full length polypeptide sequence of amino acids 1-269 of SEQ ID NO: 1 of WO2015/01009.

### Amylases

Amylases different from the alpha-amylase variant described herein can be selected from alpha- or beta-amylases (EC 3.2.1.1 and 3.2.1.2, respectively). Preferably, the amylase different from the alpha-amylase variant is also an alpha-amylases (EC 3.2.1.1).

Amylases according to the invention have "amylolytic activity" or "amylase activity" involving (endo)hydrolysis of glucosidic linkages in polysaccharides. Alpha-amylase activity may be determined by assays for measurement of alpha-amylase activity which are known to those skilled in the art.

In one aspect of the present invention, the amylase different from the alpha-amylase variant may be selected from:
- amylases from *Bacillus licheniformis* having SEQ ID NO:2 as described in WO 95/10603. Suitable variants are described in WO 95/10603 comprising one or more substitutions in the following positions: 15, 23, 105, 106, 124, 128, 133, 154, 156, 178, 179, 181, 188, 190, 197, 201, 202, 207, 208, 209, 211, 243, 264, 304, 305, 391, 408, and 444 which have amylolytic activity. Variants are described in WO 94/02597, WO 94/018314, WO 97/043424 and SEQ ID NO:4 of WO 99/019467.
- amylases from *B*. *stearothermophilus* having SEQ ID NO:6 as disclosed in WO 02/10355 or an amylase with optionally having a C-terminal truncation over the wildtype sequence. Suitable variants of SEQ ID NO:6 include those comprising a deletion in positions 181 and/or 182 and/or a substitution in position 193.
- amylases from *Bacillus sp.707* having SEQ ID NO:6 as disclosed in WO 99/19467. Preferred variants of SEQ NO: 6 are those having a substitution, a deletion or an insertion in one or more of the following positions: R181, G182, H183, G184, N195, I206, E212, E216 and K269.
- amylases from *Bacillus halmapalus* having SEQ ID NO:2 or SEQ ID NO:7 as described in WO 96/23872, also described herein as SP-722. Preferred variants are described in WO 97/3296, WO 99/194671 and WO 2013/001078.
- amylases from *Bacillus sp.* DSM 12649 having SEQ ID NO:4 as disclosed in WO 00/22103.
- amylases from *Bacillus* strain TS-23 having SEQ ID NO:2 as disclosed in WO 2009/061380. Further amylases which can be used are amylases having SEQ ID NO:2 of WO 2009/061380 or variants thereof having 90% sequence identity to SEQ ID NO:2. Preferred variants of SEQ ID NO:2 are those having a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO:2 are those having the substitution in one of more of the following positions: Q87E,R, Q98R, S125A, N128C, T131I, T165I, K178L, T182G, M201L, F202Y, N225E,R, N272E,R, 243Q,A,E,D, Y305R, R309A, Q320R, Q359E, K444E and G475K and/or deletion in position R180 and/or S181. Most preferred amylase variants of SEQ ID NO:2 are those having the substitutions: N128C + K178L + T182G + Y305R + G475K, N128C + K178L + T182G + F202Y + Y305R + D319T + G475K, S125A + N128C + K178L + T182G + Y305R + G475K or S125A + N128C + T131I + T165I + K178L + T182G + Y305R + G475K, wherein the variant optionally further comprises a substitution at position 243 and/or a deletion at position 180 and/or 181.
- amylases from *Cytophaga sp.* having SEQ ID NO:1 as disclosed in WO 2013/184577.
- amylases from *Bacillus megaterium* DSM 90 having SEQ ID NO:1 as disclosed in WO 2010/104675.
- amylases from *Bacillus sp.* comprising amino acids 1 to 485 of SEQ ID NO:2 as described in WO 00/60060.
- amylases from *Bacillus amyloliquefaciens* or variants thereof, preferably selected from amylases according to SEQ ID NO: 3 as described in WO 2016/092009.
- amylases having SEQ ID NO:12 as described in WO 2006/002643 or amylase variants comprising the substitutions Y295F and M202LITV within said SEQ ID NO:12.
- amylases having SEQ ID NO:6 as described in WO 2011/098531 or amylase variants comprising a substitution at one or more positions selected from the group consisting of 193 [G,A,S,T or M], 195 [F,W,Y,L,I or V], 197 [F,W,Y,L,I or V], 198 [Q or N], 200 [F,W,Y,L,I or V], 203 [F,W,Y,L,I or V], 206 [F,W,Y,N,L,I,V,H,Q,D or E], 210 [F,W,Y,L,I or V], 212 [F,W,Y,L,I or V], 213 [G,A,S,T or M] and 243 [F,W,Y,L,I or V] within said SEQ ID NO:6.
- amylases having SEQ ID NO:1 as described in WO 2013/001078 or amylase variants comprising an alteration at two or more (several) positions corresponding to positions G304, W140, W189, D134, E260, F262, W284, W347, W439, W469, G476, and G477 within said SEQ ID NO:1.
- amylases having SEQ ID NO:2 as described in WO 2013/001087 or amylase variants comprising a deletion of positions 181+182, or 182+183, or 183+184, within said SEQ ID NO:2, optionally comprising one or two or more modifications in any of positions corresponding to W140, W159, W167, Q169, W189, E194, N260, F262, W284, F289, G304, G305, R320, W347, W439, W469, G476 and G477 within said SEQ ID NO:2.
- amylases which are hybrid alpha-amylases from above mentioned amylases as for example as described in WO 2006/066594; Other amylase examples are hybrid α-amylases comprising residues 1 to 33 of the α-amylase derived from B. amyloliquefaciens shown in SEQ ID NO:6 of WO 2006/066594 and residues 36 to 483 of the B. licheniformis α-amylase shown in SEQ ID NO:4 of WO 2006/066594 or variants having 90% sequence identity thereof. Preferred variants of this hybrid α-amylase are those having a substitution, a deletion or an insertion in one of more of the following positions: G48, T49, G107, H156, A181, N190, M197, 1201, A209 and Q264. Most preferred variants of the hybrid α-amylase comprising residues 1 to 33 of SEQ ID NO:6 of WO 2006/066594 and residues 36 to 483 of SEQ ID NO:4 of WO 2006/066594 are those having the substitutions: M197T, H156Y + A181T + N190F + A209V + Q264S or G48 + T49 + G107 + H156 + A181 + N190 + 1201 + A209 + Q264.
- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;
- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 3 as disclosed in WO 2014/183921 and a C domain having at least 90% identity to SEQ ID NO: 6 of WO 2014/183921, wherein the hybrid amylase has amylolytic activity; preferably, the hybrid alpha-amylase is at least 95% identical to SEQ ID NO: 30 as disclosed in WO 2014/183921 and having amylolytic activity.
- Other amylases are variants of SEQ ID NO:1 of WO 2016/203064 having at least 75% sequence identity to SEQ ID NO:1 thereof. Preferred variants are variants comprising a modification in one or more positions corresponding to positions 1, 54, 56, 72, 109, 113, 116, 134, 140, 159, 167, 169, 172, 173, 174, 181, 182, 183, 184, 189, 194, 195, 206, 255, 260, 262, 265, 284, 289, 304, 305, 347, 391, 395, 439, 469, 444, 473, 476, and 477 of SEQ ID NO:1, wherein said α-amylase variant has a sequence identity of at least 75% but less than 100% to SEQ ID NO:1.
- Other examples of amylases are the α-amylase having SEQ ID NO:12 in WO 2001/066712 or a variant having at least 90% sequence identity to SEQ ID NO:12. Preferred amylase variants are those having a substitution, a deletion or an insertion in one of more of the following positions of SEQ ID NO:12 in WO 2001/066712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

In one embodiment, at least one amylase is selected from commercially available amylases which include but are not limited to products sold under the trade names Duramyl^{™}, Termamyl^{™}, Fungamyl^{™}, Stainzyme^{™}, Stainzyme Plus^{™}, Natalase^{™}, Liquozyme X and BAN^{™}, Amplify^{™}, Amplify Prime^{™} (from Novozymes A/S), and Rapidase^{™}, Pura-star^{™}, Powerase^{™}, Effectenz^{™} (M100 from DuPont), Preferenz^{™} (S1000, S110, S210 and F1000; from DuPont), Excellenz^{™} (S3300), PrimaGreen^{™} (ALL; DuPont), Optisize^{™} (DuPont).

### Mannanases

"Mannanase" as described herein are enzymes selected from the group of mannan degrading enzyme. The mannan degrading enzyme may be selected from β-mannosidase (EC 3.2.1.25), endo-1,4-β-mannosidase (EC 3.2.1.78), and 1,4-β-mannobiosidase (EC 3.2.1.100). Preferably, the mannan degrading enzyme is selected from the group of endo-1,4-β-mannosidase (EC 3.2.1.78), a group of enzymes which may be called endo-β-1,4-D-mannanase, β-mannanase, or mannanase herein.

The mannanase may be selected from mannanases originating from Bacillus organisms, such as described in JP-0304706 [beta-mannanase from *Bacillus* sp.], JP-63056289 [alkaline, thermostable beta-mannanase], JP-63036774 [*Bacillus* microorganism FERM P-8856 producing beta-mannanase and beta-mannosidase at an alkaline pH], JP-08051975 [alkaline beta-mannanases from alkalophilic *Bacillus* sp. AM-001], WO 97/11164 [mannanase from *Bacillus amyloliquefaciens*], WO 91/18974 [mannanase active at an extreme pH and temperature], WO 97/11164 [mannanase from *Bacillus amyloliquefaciens*], WO 2014/100018 [endo-(3-mannanase1 cloned from a *Bacillus circulans* or *Bacillus lentus* strain CMG1240 (Bleman1; see US 5,476,775)]. Suitable mannanases are described in WO 99/064619].

The mannanase may be selected from mannanases originating from *Trichoderma* organisms, such as disclosed in WO 93/24622.

The mannanase may be selected from a commercially available mannanase such as Mannaway^{®} (Novozymes A/S) or Preferenz^{®} (M100) (DuPont).

### Detergent compositions

In one embodiment, the present invention is directed to the use of the alpha-amylase variant in a detergent composition. Thus, the present invention is also directed to a detergent composition comprising the alpha-amylase variant described herein and one or more detergent components. The one or more detergent component may be selected from the group consisting of additional enzyme different from the alpha-amylase of the invention, surfactant, defoamer, building agent (builder), polymer, bleaching system (bleach), rheology modifier, hydrotrope, softening agent, desiccant, whitening agent, buffer, preservative, anti-corrosion additive, dyestuff and fragrance. Preferably, at least one component of the detergent is a surfactant. Preferably, at least one further component of the detergent is a second enzyme as described herein different from the alpha-amylase of the present invention.

Detergent components may have more than one function in the final application of a detergent formulation, therefore any detergent component mentioned in the context of a specific function herein, may also have another function in the final application of a detergent formulation. The function of a specific detergent component in the final application of a detergent formulation usually depends on its amount within the detergent formulation, i.e. the effective amount of a detergent component. Detergent components vary in type and/or amount in a detergent formulation depending on the desired application such as laundering white textiles, colored textiles, and wool. The component(s) chosen further depend on physical form of a detergent formulation (liquid, solid, gel, provided in pouches or as a tablet, etc.). The component(s) chosen e.g. for laundering formulations further depend on regional conventions which themselves are related to aspects like washing temperatures used, mechanics of laundry machine (vertical vs. horizontal axis machines), water consumption per wash cycle etc. and geographical characteristics like average hardness of water.

In one embodiment, a detergent formulation is a formulation of more than two detergent components, wherein at least one component is effective in stain-removal, at least one component is effective in providing the optimal cleaning conditions, and at least one component is effective in maintaining the physical characteristics of the detergent.

The detergent composition can be a liquid or solid detergent composition or a combination of liquid and solid detergent composition. The liquid detergent composition is preferably a gel detergent composition. The solid detergent composition can be a soap bar or a powder detergent composition, preferably a powder detergent composition, wherein the powder detergent composition can be pressed to a tablet.

The detergent composition can be a unit dose or multi dose composition. The detergent composition can be in the form of a pouch, including multi-compartment pouches. The detergent composition can be a laundry or dish washing detergent composition, suitable for home care and/or industrial and institutional (I&I) cleaning. Both laundry and dish wash composition can be in the form of a hand wash or automated wash composition. Preferably the dish wash composition is an Automatic Dish Wash (ADW).

Detergent pouches can be of any form, shape and material which is suitable for holding the composition, e.g. without allowing the release of the composition from the pouch prior to water contact. The pouch is made from water-soluble film, which encloses an inner volume. Said inner volume can be divided into compartments of the pouch. Preferred films are polymeric materials preferably polymers which are formed into a film or sheet. Preferred polymers, copolymers or derivates thereof are selected polyacrylates, and water-soluble acrylate copolymers, methyl cellulose, carboxy methyl cellulose, sodium dextrin, ethyl cellulose, hydroxyethyl cellulose, hydroxypropyl methyl cellulose, malto dextrin, poly methacrylates, most preferably polyvinyl alcohol copolymers and, hydroxypropyl methyl cellulose (HPMC). Preferably, the level of polymer in the film for example PVA is at least about 60%. Preferred average molecular weight will typically be about 20,000 to about 150,000. Films can also be blend compositions comprising hydrolytically degradable and water-soluble polymer blends such as polyactide and polyvinyl alcohol (known under the Trade reference M8630 as sold by Chris Craft In. Prod. Of Gary, Ind., US) plus plasticizers like glycerol, ethylene glycerol, Propylene glycol, sorbitol and mixtures thereof. The pouches can comprise a solid laundry cleaning composition or part components and/or a liquid cleaning composition or part components separated by the water-soluble film. The compartment for liquid components can be different in composition from compartments containing solids (see e.g. US 2009/0011970).

In one embodiment, the alpha-amylase variant according to the present invention may be added to a detergent composition in an amount corresponding to 1-1000 mg of active alpha-amylase variant per kg of detergent composition, preferably, 5-500 mg/kg, 5-300 mg/kg, 10-200 mg/kg, or 50-200 mg/kg.

In one embodiment, the detergent formulation has a pH in the range of 5-12, preferably in the range of 6-11, more preferably in a range selected from 6-10, 7-9, and 7.5-8.5. In one embodiment, the formulation is a detergent formulation, preferably a liquid detergent formulation.

In one embodiment, the detergent formulations according to the invention comprise one or more surfactant(s). "Surfactant" (synonymously used herein with "surface active agent") means an organic chemical that, when added to a liquid, changes the properties of that liquid at an interface. According to its ionic charge, a surfactant is called non-ionic, anionic, cationic, or amphoteric.

The detergent composition of the present invention may comprise one or more surfactants, which may be anionic and/or cationic and/or non-ionic and/or semi-polar and/or zwitterionic, or a mixture thereof. In preferred embodiments, the detergent compositions of the invention comprise at least one surfactant. In a particular embodiment, the detergent composition of the present invention includes a mixture of one or more nonionic surfactants and one or more anionic surfactants. The surfactant(s) is/are typically present at a level of from about 0.1 to 60 wt.-%, such as 1 to 40 wt.-%, 3 to 20 wt.-% or 3 to 10 wt.-%. The surfactant(s) is/are chosen based on the desired cleaning application, and includes any conventional surfactant(s) known in the art. Any surfactant known in the art for use in detergents may be utilized. Non-limiting examples of surfactants are disclosed McCutcheon's 2016 Detergents and Emulsifiers, and McCutcheon's 2016 Functional Materials, both North American and International Edition, MC Publishing Co, 2016 edition. Further useful examples are disclosed in earlier editions of the same publications which are known to those skilled in the art.

When included therein, the detergent will usually comprise from about 1 to 40 wt.-%, such as 5 to 30 wt.-%, 5 to 15 wt.-% or 20 to 25 wt.-%, of an anionic surfactant. Non-limiting examples of anionic surfactants include sulfates and sulfonates, in particular linear alkyl benzene sulfonates (LAS), isomers of LAS, branched alkyl benzene sulfonates (BABS), phenyl alkane sulfonates, alpha-olefin sulfonates (AOS), olefin sulfonates, alkene sulfonates, alkane-2,3-diylbis(sulfates), hydroxy alkane sulfonates and disulfonates, alkyl sulfates (AS) such as sodium dodecyl sulfate (SDS), fatty alcohol sulfates (FAS), primary alcohol sulfates (PAS), alcohol ether sulfates (AES or AEOS or FES, also known as alcohol ethoxy sulfates or fatty alcohol ether sulfates), secondary alkane sulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenyl succinic acid, dodecenyl/tetradecenyl succinic acid (DTSA), fatty acid derivatives of amino acids, diesters and monoesters of sulfo succinic acid or soap, and combinations thereof.

When included therein, the detergent will usually comprise from about 0 to 10 wt.-% of a cationic surfactant. Non-limiting examples of cationic surfactants include alkyl dimethyl ethanolamine quat (ADMEAQ), cetyl trimethyl ammonium bromide (CTAB), dimethyl distearyl ammonium chloride (DSDMAC), and alkyl benzyl dimethyl ammonium, alkyl quaternary ammonium compounds, alkoxylated quaternary ammonium (AQA) compounds, and combinations thereof. When included therein, the detergent will usually comprise from about 0.2 to 40 wt.-% of a nonionic surfactant, e.g. 0.5 to 30 wt.-%, in particular 1 to 20 wt.-%, 3 to 10 wt.-%, 3 to 5 wt.-% or 8 to 12 wt.-%. Non-limiting examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkyl phenol ethoxylates (APE), nonyl phenol ethoxylates (NPE), alkyl polyglycosides (APG), alkoxylated amines, fatty acid monoethanolamides (FAM), fatty acid diethanol amides (FADA), ethoxylated fatty acid monoethanolamides (EFAM), propoxylated fatty acid monoethanolamides (PFAM), polyhydroxy alkyl fatty acid amides, or N-acyl N-alkyl derivatives of glucosamine (glucamides, GA, or fatty acid glucamide, FAGA), as well as products available under the trade names SPAN and TWEEN, and combinations thereof.

When included therein, the detergent will usually comprise from about 0 to 10 wt.-% of a semi-polar surfactant. Non-limiting examples of semipolar surfactants include amine oxides (AO) such as alkyl dimethyl amine oxide, N-(coco alkyl)-N,N-dimethyl amine oxide and N-(tallow-alkyl)-N,N-bis-(2-hydroxy ethyl) amine oxide, fatty acid alkanol amides and ethoxylated fatty acid alkanol amides, and combinations thereof.

When included therein, the detergent will usually comprise from about 0 to 10 wt.-% of a zwitterionic surfactant. Non-limiting examples of zwitterionic surfactants include betaine, alkyl dimethyl betaine, sulfo betaine, and combinations thereof.

The detergent formulations according to the invention may comprise one or more compounds selected from complexing agents (chelating agents (chelants), sequestrating agents), precipitating agents, and ion exchange compounds which may form water-soluble complexes with calcium and magnesium. Such compounds may be called "builders" or "building agents" herein, without meaning to limit such compounds to this function in the final application of a detergent formulation.

In one embodiment, the detergent formulation of the invention comprises at least one builder selected from non-phosphate based builders such as sodium gluconate, citrate(s), silicate(s), carbonate(s), phosphonate(s), amino carboxylate(s), polycarboxylate(s), polysulfonate(s), and polyphosphonate(s). In one embodiment, the detergent formulation of the invention comprises a strong sequestering builder. Preferably, detergent compositions of the current invention are free from phosphate, meaning essentially free from phosphate-based builders. Herein, "essentially free from phosphate" is to be understood as meaning that the content of phosphate and polyphosphate is in sum in the range of 10 ppm to 1% by weight, determined by gravimetry and referring to the respective inventive detergent composition. In another preferred embodiment, the detergent formulation comprises phosphonate, wherein the phosphonate is preferably DTPMP and/or HEDP.

In one embodiment, the detergent formulations of the invention comprise at least one "citrate" selected from the mono- and the dialkali metal salts and in particular the mono- and preferably the trisodium salt of citric acid, ammonium or substituted ammonium salts of citric acid as well as citric acid as such. Citrate can be used as the anhydrous compound or as the hydrate, for example as sodium citrate dihydrate. The citrate may be comprised in a total amount in the range of 0% to about 20% by weight, in the range of about 0.5% to about 10% by weight, or in the range of 1-5% by weight, all relative to the total weight of the detergent formulation. In one embodiment, the detergent formulation of the invention comprises a total amount of citrate in the range of about 1-3% relative to the total weight of the detergent formulation.

Detergent compositions of the invention may comprise one or more silicates. "Silicate(s)" in the context of the present invention include in particular sodium disilicate and sodium metasilicate, aluminosilicates such as sodium aluminosilicates like zeolith A (i.e. Na₁₂(AlO₂)₁₂(SiO₂)₁₂*27H₂O), and sheet silicates, in particular those of the formula alpha-Na₂Si₂O₅, beta-Na₂Si₂O₅, and delta-Na₂Si₂O₅.

Detergent compositions of the invention may comprise one or more carbonates. The term "carbonate(s)" includes alkali metal carbonates and alkali metal hydrogen carbonates, preferred are the sodium salts. Particularly suitable is sodium carbonate (Na₂CO₃).

Detergent compositions of the invention may comprise one or more phosphonates. "Phosphonates" include, but are not limited to 2-phosphinobutane-1,2,4-tricarboxylic acid (PBTC); ethylenediaminetetra(methylenephosphonic acid) (EDTMPA); 1-hydroxyethane-1,1-diphosphonic acid (HEDP), CH₂C(OH)[PO(OH)₂]₂; aminotris(methylenephosphonic acid) (ATMP), N[CH₂PO(OH)₂]₃; aminotris(methylenephosphonate), sodium salt (ATMP), N[CH₂PO(ONa)₂]₃; 2-hydroxyethyliminobis(methylenephosphonic acid), HOCH₂CH₂N[CH₂PO(OH)₂]₂; diethylenetriaminepenta(methylenephosphonic acid) (DTPMP), (HO)₂POCH₂N[CH₂CH₂N[CH₂PO(OH)₂]₂]₂; diethylenetriaminepenta(methylenephosphonate), sodium salt, C₉H₍₂₈₋ₓ₎N₃NaₓO₁₅P₅ (x=7); hexamethylenediamine(tetramethylenephosphonate), potassium salt, C₁₀H₍₂₈₋ₓ₎N₂KₓO₁₂P₄ (x=6); and bis(hexamethylene)triamine(pentamethylenephosphonic acid), (HO₂)POCH₂N[(CH₂)₂N[CH₂PO(OH)₂]₂]₂. Salts thereof may be suitable, too.

Detergent compositions of the invention may comprise one or more aminocarboxylates. Non-limiting examples of suitable "amino carboxylates" include, but are not limited to: diethanol glycine (DEG), dimethylglycine (DMG), nitrilitriacetic acid (NTA), N-hydroxyethylaminodiacetic acid, ethylenediaminetetraacetic acid (EDTA), N-(2hydroxyethyl)iminodiacetic acid (HEIDA), hydroxy-ethylenediaminetriacetic acid, N-hydroxyethyl-ethylenediaminetriacetic acid (HEDTA), hydroxy-ethylenediaminetetraacetic acid, diethylenetriaminepentaacetic acid (DTPA), and methylgly-cinediacetic acid (MGDA), glutamic acid-diacetic acid (GLDA), iminodisuccinic acid (IDS), hydroxyiminodisuccinic acid, ethylenediaminedisuccinic acid (EDDS), aspartic acid-diacetic acid, and alkali metal salts or ammonium salts thereof. Further suitable are aspartic acid-N-monoacetic acid (ASMA), aspartic acid-N,N-diacetic acid (ASDA), aspartic acid-N-monopropionic acid (ASMP), N-(2-sulfomethyl) aspartic acid (SMAS), N-(2-sulfoethyl) aspartic acid (SEAS), N-(2-sulfomethyl) glutamic acid (SMGL), N-(2-sulfoethyl) glutamic acid (SEGL), N-methyliminodiacetic acid (MIDA), alpha-alanine-N,N-diacetic acid (alpha-ALDA), serine-N,N-diacetic acid (SEDA), isoserine-N,N-diacetic acid (ISDA), phenylalanine-N,N-diacetic acid (PHDA), anthranilic acid-N,N-diacetic acid (ANDA), sulfanilic acid-N,N-diacetic acid (SLDA), taurine-N,N-diacetic acid (TUDA) and sulfomethyl-N,N-diacetic acid (SMDA) and alkali metal salts or ammonium salts thereof. The term "ammonium salts" as used in in this context refers to salts with at least one cation that bears a nitrogen atom that is permanently or temporarily quaternized. Examples of cations that bear at least one nitrogen atom that is permanently quaternized include tetramethylammonium, tetraethylammonium, dimethyldiethyl ammonium, and n-C₁₀-C₂₀-alkyl trimethyl ammonium. Examples of cations that bear at least one nitrogen atom that is temporarily quaternized include protonated amines and ammonia, such as monomethyl ammonium, dimethyl ammonium, trimethyl ammonium, monoethyl ammonium, diethyl ammonium, triethyl ammonium, n-C₁₀-C₂₀-alkyl dimethyl ammonium 2-hydroxyethylammonium, bis(2-hydroxyethyl) ammonium, tris(2-hydroxyethyl)ammonium, N-methyl 2-hydroxyethyl ammonium, N,N-dimethyl-2-hydroxyethylammonium, and especially NH₄⁺.

In one embodiment, detergent compositions of the invention comprise more than one builder. Preferably, inventive detergent compositions contain less than 0.2% by weight of nitrilotriacetic acid (NTA), or 0.01 to 0.1% NTA by weight relative to the total weight of the detergent composition.

In one embodiment, the detergent composition of the invention comprises of at least one aminocarboxylate selected from methylglycine diacetate (MGDA), glutamic acid diacetate (GLDA), and the respective salts thereof, e.g., alkali (such as sodium) salts thereof in amounts in the range of 0.1% to 25.0% by weight, in the range of 1.0% to 18.0% by weight, in the range of 3.0% to 15.0% by weight, in the range of 3.0% to 10.0% by weight, or in the range of 5.0% to 8.0% by weight relative to the total weight of the detergent composition.

The detergent formulations of the invention may comprise one or more hydrotropes. One or more hydrotropes may be selected from organic solvents such as ethanol, isopropanol, ethylene glycol, 1,2-propylene glycol, and further organic solvents known in the art that are water-miscible under normal conditions without limitation. In one embodiment, the detergent formulation of the invention comprises 1,2-propylene glycol in a total amount in the range of 5-10% by weight, preferably of about 6% by weight, all relative to the total weight of the detergent formulation. Further non-limiting examples of hydrotropes include sodium benzene sulfonate, sodium p-toluene sulfonate (STS), sodium xylene sulfonate (SXS), sodium cumene sulfonate (SCS), sodium cymene sulfonate, amine oxides, alcohols and polyglycol ethers, sodium hydroxy naphthoate, sodium hydroxy naphthalene sulfonate, sodium ethyl hexyl sulfate, and combinations thereof.

In one embodiment, the detergent composition comprises at least one preservative. Preferably, preservative means substances that are added to a liquid composition for the purpose of preservation, meaning more preferably that compounds known to have preserving features comprised in a liquid composition formed in the production process are excluded from the term preservatives. In one embodiment, the preservative is selected from the group consisting of 2-phenoxyethanol, glutaraldehyde, 2-bromo-2-nitropropane-1,3-diol, and formic acid in acid form or as its salt, and 4,4'-dichloro 2-hydroxydiphenylether. Usually, the liquid compositions of the invention comprise at least one preservative in amounts below 10ppm, such as in amounts ranging from 2 ppm to 5% by weight relative to the total weight of the liquid composition. Preferably, the liquid composition is free from preservatives, meaning that preservatives are comprised in amounts less than 1 ppm.

The detergent formulations may comprise one or more other enzyme(s) than a alpha-amylase variant according to the invention, which are selected from the group consisting of proteases, amylases different from the alpha-amylase variant of the invention, cellulases, lipases, xylanases, mannanases, cutinases, esterases, phytases, DNAses, pectinases, pectate lyases, pectinolytic enzymes, carbohydrases, arabinases, galactanases, xanthanases, xyloglucanases, laccases, peroxidases and oxidases. Preferably, the detergent formulations comprise at least a protease as disclosed herein, preferably a protease comprising an amino acid sequence having at least 80% sequence identity to any of SEQ ID NO: 10-14, preferably SEQ ID NO: 10. Particular preferred additional enzymes are disclosed elsewhere herein and that description is incorporated by reference also to this part of the description.

The detergent formulations may comprise water-soluble sources of calcium and/or magnesium ions. In one embodiment, at the detergent formulation comprises at least one enzyme stabilizer selected from polyols and water-soluble salts as disclosed above.

The detergent formulation may comprise at least one enzyme stabilizer selected from boron containing compounds and peptide stabilizers as disclosed above.

In one embodiment, the invention relates to a method to provide a liquid detergent formulation, more preferably a liquid laundering detergent formulation, comprising the steps of mixing in one or more steps
(a) at least one alpha-amylase variant according to the invention, preferably wherein the amylase is provided within an enzyme preparation of the invention; and
(b) at least one detergent component selected from surfactants, builders, and hydrotropes present in amounts effective in cleaning performance or effective in maintaining the physical characteristics of the detergent.

Preferably, the detergent composition is according to the Table 1a below.

| | Wt. % of active substance in raw material | Wt.% of active substance in formulation | | |
|---|---|---|---|---|
| | | Formulation A | Formulation B | Formulation C |
| Water demin. | 100 | ad 95 | ad 98 | ad 98 |
| Boric acid | 100 | 0.3-1 | 0.3-1 | |
| Citric acid | 100 | 1-2.5 | 0.1-2 | 0.1-2 |
| Glycerin | 99,5 | 0.75-1.5 | 0.3-1 | 0.3-1 |
| Propylenglycol | 100 | 3-8 | | |
| Monoethanolamin | 99 | 2-5 | | |
| Soil Release Polymer | 70 | 0.1-1 | | |
| anionic surfactant | 70/96 | 8-20 | 3-10 | 3-10 |
| Nonionic surfactant | 100 | 4-10 | 2-6 | 2-6 |
| NaOH | 50 | 0.3-1 | 0.3-1 | 0.3-1 |
| soap | 100 | 1.25-2.5 | 0.5-1.5 | 0.5-1.5 |
| Preservating agent | 100 | | 0.05-0.1 | 0.05-0.1 |
| DTPMP (Phosphonate) | 40 | | 0.1-2 | |
| HEDP (Phosphonate) | 60 | 0.5-3 | | |
| NaCl | 100 | | 1-2 | 1-2 |
| Dye, Perfume, Antifoam, Optical Brightener | t.q. | Minors | Minors | Minors |
| pH | | 7.8-8.2 | 8.2-8.6 | 8.2-8.6 |

An alternative detergent formulation is a detergent composition according to the Table 1b below.

| ES1-C | | wt% |
|---|---|---|
| Maranil DBS/LC | linear alkylbenzene sulfonate, anionic surfactant | 5.5 |
| Edenor coco fatty acid | C₁₂-C₁₈ coco fatty acid | 2.4 |
| Lutensol AO7 | alkyl polyethyleneglycol ether, non-ionic surfactant | 5.5 |
| Texapon N70 | sodium laureth sulfate 2EO, anionic surfactant | 5.5 |
| 1,2 propylene glycol | | 6.0 |
| C₂H₅OH | | 2.0 |
| KOH | | 2.2 |
| Citrate | | 3.0 |
| pH | | 8-8.5 |
| H₂O | up to 100% | |

To the detergent formulations shown in Table 1a and Table 1b one or more enzymes can be added as described herein.

### Methods of use

The present invention is also directed to the use of an alpha-amylase variant as described herein in a cleaning process such as laundry or hard surface cleaning including home care and I&I cleaning. Thus, the present invention also refers to the use of a composition comprising an alpha-amylase variant described herein for providing an improved alpha-amylase stability in the detergent and/or wash performance of the detergent composition.

The present invention therefore also refers to a method for providing a detergent composition with improved alpha-amylase stability and/or wash performance comprising the use of a detergent composition comprising
a) an alpha-amylase variant as described herein; and
b) one or more detergent component.

### Preferred embodiments

Particularly, preferred herein is
Preferred embodiment 1.) An alpha-amylase variant of a parent alpha-amylase, wherein said variant comprises
   (i)
      a) amino acid substitution at one or more positions corresponding to positions selected from the group consisting of 1, 2, 3, 4, 5, 6, 7, 8, 10, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 45, 47, 48, 51, 54, 59, 60, 63, 70, 71, 72, 73, 75, 76, 81, 82, 83, 86, 87, 89, 90, 91, 92, 93, 94, 95, 96, 97, 98, 99, 100, 103, 106, 108, 109, 113, 114, 115, 116, 117, 119, 120, 123, 125, 126, 128, 129, 131, 132, 133, 134, 135, 136, 139, 141, 142, 143, 144, 145, 146, 147, 149, 150, 151, 154, 155, 156, 158, 160, 161, 162, 163, 164, 165, 169, 170, 172, 173, 174, 175, 176, 177, 178, 180, 182, 184, 185, 187, 188, 189, 191, 192, 193, 203, 208, 210, 211, 212, 213, 214, 215, 216, 218, 219, 221, 222, 223, 224, 225, 226, 227, 230, 231, 233, 234, 235, 236, 237, 238, 240, 242, 243, 245, 249, 250, 251, 252, 253, 255, 256, 257, 258, 259, 260, 261, 262, 263, 264, 265, 268, 269, 270, 271, 272, 273, 274, 275, 276, 277, 279, 280, 281, 282, 284, 285, 286, 287, 288, 289, 290, 291, 292, 293, 294, 295, 296, 297, 298, 299, 301, 302, 303, 304, 306, 307, 308, 309, 310, 311, 312, 313, 314, 315, 317, 318, 319, 320, 321, 322, 323, 324, 326, 327, 333, 334, 336, 337, 338, 341, 342, 343, 344, 345, 346, 348, 350, 351, 352, 353, 354, 355, 356, 357, 358, 359, 360, 362, 363, 364, 365, 366, 367, 368, 370, 372, 375, 376, 378, 379, 381, 382, 383, 384, 385, 387, 388, 389, 390, 391, 392, 393, 394, 395, 396, 397, 398, 399, 400, 401, 403, 405, 406, 407, 408, 410, 413, 414, 415, 416, 418, 421, 424, 426, 427, 429, 431, 432, 434, 436, 438, 439, 440, 442, 443, 445, 446, 447, 448, 449, 451, 453, 455, 456, 457, 458, 459, 460, 461, 462, 463, 465, 467, 468, 470, 471, 474, 478, 480, and 482 according to the numbering of the amino acid sequence set forth in SEQ ID NO: 3,
         or
      b) compared to a parent sequence one or more amino acid substitutions selected from the group consisting of X9D, X9F, X9K, X9N, X9P, X9Q, X9S, X9T, X9Y, X179G, X181D, X181F, X181H, X181I, X181N, X181Q, X181S, X181T, X181V, X181W, X181Y X186A, X186C, X186D, X186F, X186H, X186I, X186K, X186L, X186M, X186R, X186V, X186W, X186Y, X190H, X195H, X195K, X195L, X195W, X195Y, X206C, X206H, X206M, X206Y, X244A, X244C, X244D, X244E, X244F, X244G, X244H, X244M, X244N, X244V, X402T X419C, X420D, X420E, X420G, X420H, X420K, X420L, X420Q, X422C, X422N, X422H, X423F, X423M, X423Q, X423S, X428C, X428D, X428E, X428G, X428I, X428K, X428L, X428M, X428N, X428R, X428S, X428V, X428W, X428Y, X430A, X430C, X430D, X430E, X430F, X430G, X430L, X430P, X430Q, X430S, X430T, X430V, X435K, X435P, X435S, X435A, X435D, X437L, X437T, X437W, X441C, X441K, X441L, X441M, X441S, X444H, X444M, X444N, X444R, X444T, X450C, X450D, X450E, X450H, X450L, X450M, X450P, X450Q, X450R, X450T, X452A, X452C, X452E, X452F, X452I, X452K, X452M, X452N, X452T, X454A, X454E, X454K, X454L, X454P, X454S, X454T, X466E, X466W, X469F, X469L, X469Y, X475A, X475K, X475E, X475L, X479I, X479K, X479M, X483F, X483L, X483Q, and X483R, preferably one or more amino acid substitutions selected from the group consisting of X181D, X181F, X181H, X181I, X181N, X181Q, X181S, X181T, X181V, X181W, X181Y X186A, X186C, X186D, X186F, X186H, X186I, X186K, X186L, X186M, X186R, X186V, X186W, X186Y, X195H, X195K, X195L, X195W, X195Y, X206C, X206H, X206M, X206Y, X441C, X441K, X441L, X441M, and X441S, according to the numbering of the amino acid sequence set forth in SEQ ID NO: 3,
   (ii) said variant has at least 91%, at least 91.5%, at least 92.0%, at least 92.5%, at least 93.0%, at least 93.5%, 94.0%, at least 94.5%, more preferably at least 95%, preferably at least 95.5%, at least 96.0%, at least 96.5%, at least 97.0%, at least 97.5%, at least 98.0%, at least 98.5%, at least 99.0%, at least 99.5%, at least 99.6%, at least 99.7%, at least 99.8%, or at least 99.9%, but less than 100% sequence identity with the amino acid sequence set forth in SEQ ID NO: 1, and
   (iii) said variant has alpha-amylase activity.
Preferred embodiment 2.): An alpha-amylase variant according to the preceding preferred embodiment, wherein the parent alpha-amylase for the alpha-amylase variant is an amylase according to SEQ ID NO: 1 or SEQ ID NO: 3 or any alpha-amylase having at least 60% sequence identity to SEQ IDNO: 1 or SEQ ID NO: 3, most preferably the parent alpha-amylase for the alpha-amylase variant is an amylase according to according to SEQ ID NO: 1, preferably the resulting amino acid residue of the amino acid substitution does not equal to an amino acid residue in SEQ ID NO: 3 or 5 at the corresponding position.
Preferred embodiment 3.): An alpha-amylase according to any of the preceding preferred embodiments, wherein the alpha-amylase variant comprises 1-30, preferably 1 to 25, 1 to 20, 1 to 15, 1 to 10, or 1 to 5, 2 to 30, 2 to 25, 2 to 20, 2 to 15 2 to 10, 2 to 8, or 2 to 5, preferably 3 to 30, 3 to 25, 3 to 20, 3 to 15 3 to 10, 3 to 8, or 3 to 5, preferably, 4 to 30, 4 to 25, 4 to 20, 4 to 15, 4 to 10, or 4 to 8 of the amino acid substitutions at the above-cited positions.
Preferred embodiment 4.): An alpha-amylase according to any of the preceding preferred embodiments, wherein the alpha-amylase variant optionally further comprises 1-30, 1-20, 1-10, or 1-5 additional conservative amino acid substitutions, preferably outside any functional domain, preferably outside the catalytically active domain, of the parent alpha-amylase.
Preferred embodiment 5.): An alpha-amylase according to any of the preceding preferred embodiments, wherein the variant comprises an A and B domain and a C domain wherein the amino acid sequence of the A and B domain is at least 75%, but less than 100%, identical to the amino acid sequence of SEQ ID NO: 6 and the amino acid sequence of the C domain is at least 75%, but less than 100%, identical to the amino acid sequence of SEQ ID NO: 8.
Preferred embodiment 6.): An alpha-amylase variant according to any of the preceding preferred embodiments, wherein one or more amino acid substitution is selected from the group consisting of 4, 25, 100, 135, 135, 135, 135, 160, 176, 193, 251, 258, 276, 299, 323, 363, 363, 382, 405, 460, and 482 or selected from the group consisting of 4, 7, 25, 37, 70, 100, 118, 135, 160, 176, 193, 210, 251, 281, 258, 323, 361, 363, 368, 405, 434, 441, 459, 460, 451, and 482, preferably selected from the group consisting of 25, 100, 135, 176, 193, and 460, according to the numbering of the amino acid sequence set forth in SEQ ID NO: 3.
Preferred embodiment 7.): An alpha-amylase variant according to any of the preceding preferred embodiments, wherein one or more amino acid substitutions is selected from the group of amino acid substitutions consisting of X100D, X100F, X100K, X100L, X103A, X106D, X108A, X109A, X10A, X10C, X10E, X10F, X10L, X10W, X10Y, X113F, X113H, X113M, X113R, X113V, X113W, X113Y, X114A, X114C, X114D, X114E, X114F, X114G, X114H, X114I, X114K, X114L, X114M, X114N, X114Q, X114R, X114S, X114V, X114W, X114Y, X115C, X115D, X115K, X115M, X115N, X115Q, X115R, X115S, X115T, X115V, X116I, X116K, X116L, X116M, X116R, X116T, X117A, X117C, X117D, X117F, X117I, X117N, X117P, X117R, X117W, X117Y, X118A, X118D, X118E, X119H, X119P, X119R, X119S, X119Y, X120E, X120G, X120M, X120S, X120W, X120Y, X123D, X123S, X125A, X125D, X125E, X125F, X125G, X125H, X125K, X125L, X125M, X125N, X125Q, X125R, X125T, X125V, X125W, X125Y, X126D, X128C, X128E, X128L, X128M, X128W, X128Y, X129E, X12N, X12S, X131C, X131I, X131L, X131Q, X131W, X132T, X133A, X133C, X133D, X133E, X133H, X133K, X133N, X133P, X133Q, X133S, X134C, X134E, X134F, X134I, X134L, X134M, X134P, X134T, X134V, X134W, X134Y, X135D, X135E, X135G, X135M, X135N, X135P, X135S, X135T, X135W, X136A, X136C, X136D, X136E, X136F, X136H, X136L, X136M, X136N, X136P, X136W, X139C, X139S, X13A, X13Y, X141V, X142C, X142E, X142F, X142L, X142M, X142Q, X142R, X142W, X142Y, X143F, X144C, X144E, X144G, X144K, X144N, X144Q, X144R, X144S, X144T, X144V, X144Y, X145A, X146C, X146D, X146E, X146F, X146G, X146H, X146K, X146L, X146S, X146T, X146W, X147M, X149E, X14N, X150C, X150E, X150Q, X151C, X151E, X154A, X154Y, X155Y, X156E, X156V, X158H, X158N, X158Y, X15R, X160D, X160E, X160W, X161T, X162V, X163A, X163Q, X163T, X164V, X165S, X165T, X165W, X169A, X169D, X169E, X169S, X169V, X16F, X16R, X170C, X170F, X172A, X172C, X172D, X172K, X172N, X173I, X173Y, X174D, X174E, X174G, X174H, X174M, X174N, X174P, X174S, X174T, X175A, X175G, X175H, X175Q, X176K, X176S, X176T, X177A, X177G, X177K, X177N, X177P, X177R, X177S, X177W, X178F, X17K, X17V, X180M, X180N, X180T, X180W, X182D, X182E, X182N, X182Q, X182S, X184G, X185E, X185M, X185N, X187A, X187D, X187M, X187V, X188H, X188T, X188V, X189I, X18F, X18I, X18K, X18M, X18R, X18T, X191F, X191H, X191K, X191M, X191W, X191Y, X192A, X192T, X193D, X193E, X193G, X193V, X19A, X19C, X19D, X19F, X19G, X19H, X19I, X19K, X19L, X19M, X19P, X19Q, X19S, X19T, X19Y, X1R, X1V, X203E, X203R, X208F, X208I, X208Y, X20A, X20C, X20D, X20E, X20F, X20G, X20H, X20K, X20L, X20M, X20N, X20P, X20Q, X20S, X20T, X20V, X20W, X20Y, X210A, X210C, X210D, X210E, X210F, X210M, X210N, X210Q, X210S, X210Y, X211A, X211C, X211D, X211E, X211G, X211H, X211L, X211N, X211Q, X211S, X211T, X211V, X212C, X212D, X212I, X212L, X212W, X213A, X213C, X213M, X213R, X213S, X214E, X214P, X215A, X215D, X215E, X215H, X215W, X216G, X216H, X218A, X218C, X218D, X218E, X218F, X218G, X218H, X218I, X218K, X218L, X218N, X218Q, X218S, X218T, X218V, X218W, X218Y, X219A, X219C, X219D, X219E, X219F, X219G, X219H, X219K, X219M, X219Q, X219R, X219S, X219T, X219W, X219Y, X21E, X21S, X221F, X221N, X222D, X222K, X222S, X222T, X223C, X223L, X223V, X223Y, X224F, X224V, X225A, X225C, X225E, X225F, X225H, X225I, X225N, X225R, X225S, X225Y, X226A, X226C, X226D, X226E, X226G, X226H, X226I, X226L, X226M, X226Q, X226R, X226S, X226T, X226V, X226W, X226Y, X227C, X227F, X227G, X227H, X227I, X227K, X227L, X227M, X227R, X227T, X227V, X227W, X227Y, X22A, X22D, X22E, X22F, X22G, X22K, X22L, X22M, X22Q, X22R, X22T, X22W, X22Y, X230A, X230F, X231C, X231D, X231N, X233C, X233H, X233I, X233M, X233T, X233W, X233Y, X234C, X235L, X235M, X235V, X236Y, X237C, X237I, X238A, X238F, X238T, X23N, X23Q, X23W, X240M, X242M, X242N, X243D, X243F, X245E, X245H, X245M, X249D, X249I, X24G, X250V, X251A, X251E, X251F, X251L, X251M, X251S, X251T, X252C, X252I, X252S, X253C, X253G, X253Y, X255D, X255F, X255I, X255T, X255V, X256C, X257L, X257V, X257Y, X258F, X258Q, X258R, X259L, X25A, X25C, X25D, X25F, X25G, X25H, X25K, X25L, X25M, X25Q, X25S, X25W, X25Y, X260H, X261R, X262C, X262D, X262E, X262H, X262P, X262Y, X263I, X264H, X264T, X264W, X265S, X268F, X268G, X269M, X26A, X26D, X26E, X26F, X26L, X26M, X26P, X26S, X26V, X26Y, X270A, X270Q, X270Y, X271S, X272F, X272G, X272L, X272S, X273A, X273C, X273D, X273E, X273F, X273H, X273I, X273L, X273M, X273P, X273Q, X273R, X273V, X273W, X273Y, X274F, X274S, X275V, X276D, X276K, X276L, X276N, X276R, X276Y, X277D, X277E, X277T, X279A, X279P, X27A, X27D, X27F, X27G, X27H, X27I, X27Q, X27R, X27T, X27V, X280D, X280F, X280G, X280H, X280I, X280K, X280N, X280R, X280V, X280Y, X281A, X281D, X281E, X281H, X284A, X284F, X284H, X284L, X284M, X284N, X284Y, X285G, X285L, X285N, X285P, X286Q, X287A, X287D, X287E, X287H, X287T, X288A, X288K, X288P, X288Y, X289F, X289G, X289R, X289T, X28C, X28D, X28E, X28F, X28G, X28I, X28K, X28N, X28Q, X28S, X28T, X28V, X290D, X290M, X290N, X290Q, X290W, X291D, X291K, X291T, X291Y, X292C, X292D, X292F, X292I, X292L, X292T, X292W, X292Y, X293D, X293E, X293F, X293K, X293R, X294G, X294T, X295F, X296A, X296C, X296L, X296Y, X297E, X297F, X297H, X297K, X297M, X297S, X297V, X299G, X299I, X299K, X299L, X299S, X299Y, X29D, X29E, X29F, X29G, X29H, X29I, X29K, X29L, X29N, X29P, X29Q, X29V, X29W, X29Y, X2I, X2S, X301F, X302H, X302I, X302Q, X302V, X302Y, X303E, X303H, X303I, X303K, X303L, X303M, X303N, X303P, X303R, X303T, X304A, X304D, X304E, X304H, X304K, X304M, X304N, X304P, X304R, X304T, X304W, X304Y, X306A, X306D, X306E, X306G, X306H, X306I, X306M, X306Q, X306R, X306S, X306T, X306V, X306W, X306Y, X307F, X307M, X308S, X309H, X309L, X309Q, X30A, X30E, X30F, X30G, X30H, X30I, X30K, X30L, X30M, X30Q, X30T, X30W, X30Y, X310A, X310Q, X311A, X311E, X311G, X311H, X311K, X311N, X311R, X311T, X311Y, X312L, X312M, X313V, X314C, X314E, X314K, X314Q, X315A, X315C, X315E, X315H, X315K, X315T, X318I, X318S, X318T, X319A, X319D, X319H, X319I, X319K, X319M, X319N, X319P, X319S, X319T, X319W, X31N, X31Q, X31S, X31T, X31V, X31W, X320A, X320C, X320D, X320E, X320G, X320H, X320K, X320L, X320N, X320Q, X320S, X320Y, X321A, X321E, X321K, X321N, X321T, X321V, X321W, X323A, X323G, X323K, X323L, X323V, X324K, X324L, X324M, X324W, X324Y, X326G, X326N, X326S, X326Y, X327C, X327L, X327M, X32A, X32D, X32E, X32F, X32H, X32I, X32L, X32M, X32N, X32P, X32Q, X32T, X32W, X333I, X334T, X336K, X337A, X337C, X337F, X337G, X337I, X337K, X337L, X337M, X337N, X337Q, X337R, X337S, X337T, X337V, X337Y, X338G, X338S, X338T, X33D, X33E, X33H, X33K, X33M, X33Q, X33R, X33Y, X341V, X342P, X343L, X343T, X343W, X343Y, X344I, X344Q, X344V, X345D, X345G, X345M, X345N, X345Q, X345S, X345T, X346A, X346C, X346D, X346G, X346H, X346N, X346Q, X348T, X34H, X34I, X34V, X350H, X350K, X350P, X351A, X351M, X352S, X353H, X354I, X354N, X354T, X354Y, X355I, X355M, X356I, X356V, X357A, X358I, X358L, X358N, X358P, X358V, X359E, X35A, X35C, X35D, X35G, X35H, X35I, X35L, X35M, X35N, X35P, X35Q, X35R, X35S, X35T, X35V, X35Y, X360A, X360F, X360G, X360I, X360L, X360N, X360Q, X360R, X360S, X360T, X360V, X360Y, X362F, X362K, X362M, X362N, X362T, X362V, X362Y, X363A, X363C, X363D, X363E, X363G, X363H, X363K, X363L, X363M, X363P, X363Q, X363R, X363S, X363T, X363V, X363W, X363Y, X364A, X364C, X364G, X364K, X364L, X364N, X364S, X364T, X364V, X366I, X366L, X366T, X367E, X367S, X368A, X368F, X368L, X368N, X36A, X36E, X36G, X36I, X36K, X36M, X36N, X36P, X36Q, X36R, X36S, X36T, X36V, X370E, X370I, X372A, X372C, X372E, X372F, X372H, X372M, X372N, X372Q, X375A, X375D, X375E, X375I, X375K, X375Q, X375R, X375T, X375W, X375Y, X376G, X376I, X376K, X376L, X376M, X376Q, X376R, X376S, X376V, X377Q, X378C, X378D, X378E, X378R, X379A, X379L, X379S, X37A, X37G, X37M, X37P, X37T, X37V, X37W, X381E, X381V, X382A, X382H, X382K, X382L, X382N, X382Q, X382S, X383C, X383D, X383E, X383H, X383I, X383M, X383N, X383Q, X383R, X383S, X383V, X383Y, X384A, X384C, X384D, X384E, X384F, X384I, X384L, X384M, X384N, X384Q, X384R, X384T, X384V, X384W, X384Y, X385A, X385C, X385D, X385E, X385F, X385G, X385H, X385I, X385L, X385M, X385N, X385P, X385Q, X385R, X385S, X385T, X385V, X385W, X385Y, X387C, X387E, X387N, X388E, X388F, X388H, X388I, X388M, X388R, X388V, X389G, X389H, X389K, X38N, X390D, X390F, X390M, X390N, X390P, X390R, X391A, X391F, X391G, X391K, X391M, X391N, X391Q, X391S, X391T, X391Y, X392C, X392V, X393E, X393H, X393P, X393S, X393V, X394A, X394C, X394E, X394H, X394I, X394L, X394M, X394N, X394R, X394S, X395A, X395H, X395M, X395V, X396H, X396P, X397D, X397H, X397P, X397S, X398M, X399P, X39E, X39K, X3A, X3F, X3G, X3I, X3K, X3L, X3Q, X3V, X400A, X400D, X400E, X400G, X400H, X400I, X400K, X400L, X400M, X400N, X400P, X400Q, X400R, X400S, X400V, X400W, X401I, X401K, X401M, X401T, X403N, X405C, X405H, X405M, X405T, X405V, X406P, X407D, X407R, X407S, X408E, X408I, X408Q, X40I, X40S, X410H, X410I, X410K, X410L, X410P, X410R, X410Y, X413S, X414C, X414E, X414S, X415E, X415I, X416S, X418C, X418N, X418P, X41C, X41D, X41E, X41G, X41Q, X41S, X41T, X421N, X421P, X424A, X426D, X426W, X427C, X427F, X427G, X427K, X427Q, X427R, X427S, X427T, X427V, X429A, X429D, X429E, X429F, X429G, X429I, X429M, X429N, X429P, X429Q, X429S, X429T, X429V, X429W, X42C, X42I, X42Q, X42V, X431I, X434S, X436E, X438F, X438P, X438Y, X439C, X439P, X440V, X442Q, X443H, X443T, X445A, X445C, X445D, X445F, X445G, X445H, X445K, X445M, X445Q, X445R, X445S, X445T, X445V, X446F, X446I, X446L, X446P, X446Q, X446R, X446V, X446W, X447V, X448D, X448E, X448H, X448N, X449A, X449F, X449G, X449K, X449L, X449M, X449N, X449P, X449Q, X449R, X449S, X449V, X449W, X449Y, X451L, X453G, X453I, X453N, X453P, X453Y, X455L, X456L, X456M, X456R, X456S, X456V, X456W, X456Y, X457A, X457C, X457E, X457F, X457G, X457K, X457L, X457M, X457R, X457S, X457T, X457V, X457W, X458I, X458K, X458V, X458W, X459C, X459D, X459E, X459I, X459K, X459N, X459Q, X459R, X459V, X459Y, X45G, X45N, X460A, X460D, X460E, X460G, X460Q, X460R, X460S, X460T, X460V, X461A, X461E, X461F, X461K, X461L, X461M, X461N, X461Q, X461R, X461S, X461V, X462K, X463L, X465H, X465P, X465R, X465V, X467A, X467E, X467H, X468D, X468H, X470A, X470Q, X470T, X471E, X474F, X474H, X474P, X478A, X478E, X47S, X480D, X480E, X480M, X480R, X480Y, X482C, X482T, X482W, X48F, X48I, X48M, X48Y, X4A, X4C, X4K, X4M, X4Q, X4R, X4S, X51Q, X51T, X51V, X54D, X54G, X54Q, X59T, X5A, X5C, X5D, X5E, X5F, X5H, X5I, X5K, X5L, X5M, X5N, X5P, X5Q, X5R, X5V, X5Y, X60T, X63C, X63V, X6A, X6C, X6E, X6F, X6G, X6H, X6K, X6L, X6M, X6P, X6Q, X6S, X6T, X6V, X6W, X6Y, X70F, X70H, X70L, X70M, X70N, X70Y, X71D, X72C, X72D, X72E, X72N, X72T, X73L, X73N, X73Q, X75A, X75G, X75I, X75L, X75P, X75T, X75W, X76C, X76E, X76G, X76L, X76T, X76V, X7C, X7E, X7F, X7H, X7K, X7N, X7P, X7Q, X7R, X7S, X7V, X7W, X7Y, X81H, X81L, X82K, X82M, X83A, X83D, X83E, X83G, X83R, X83S, X86K, X87D, X87R, X89A, X89C, X89F, X89G, X89L, X89M, X89R, X89S, X8A, X8C, X8F, X8I, X8M, X8P, X8S, X8V, X8W, X8Y, X90A, X90D, X90E, X90F, X90G, X90I, X90M, X90N, X90Q, X90R, X90S, X90V, X90Y, X91C, X91D, X91E, X91F, X91G, X91H, X91I, X91K, X91L, X91M, X91N, X91Q, X91S, X91T, X91V, X91W, X91Y, X92D, X92M, X92V, X93K, X93Q, X94A, X94D, X94E, X94K, X94M, X94V, X94Y, X95E, X95I, X95L, X95V, X96K, X96N, X96Q, X97V, X98E, X98G, X98N, X99H, X99K, X99N, X100W, and X1G according to the numbering of the amino acid sequence set forth in SEQ ID NO: 3.
Preferred embodiment 8.): An alpha-amylase variant according to any of the preceding preferred embodiments, wherein the alpha-amylase variant further comprising a deletion at one or more amino acids corresponding to positions selected from the group consisting of 181, 182, 183 and 184, preferably a deletion of two or more amino acids corresponding to positions selected from the group consisting of 181, 182, 183 and 184, preferably a deletion of amino acids corresponding to positions 181 and 182, 182 and 183, or 183 and 184, the deletion D183* and G184*, wherein the numbering is according to the amino acid sequence set forth in SEQ ID NO: 3.
Preferred embodiment 9.): An alpha-amylase variant according to any of the preceding preferred embodiments, wherein the variant exhibits one or more improved property relative to said parent alpha-amylase, preferably wherein said improved property is measured as an Improvement Factor (IF) of >1.0 and wherein preferably the Improvement Factor is equal or greater than 1.2, preferably, equal or greater than 1.3.
Preferred embodiment 10.): An alpha-amylase variant according to any of the preceding preferred embodiments, wherein one or more amino acid substitutions is selected from the group of amino acid substitutions consisting of X4Q, X25H, X100W, X135E, X135T, X135W, X135D, X160W, X176K, X193E, X251E, X258Q, X276R, X299S, X323G, X363E, X363H, X382Q, X405M, X460G, and X482W or wherein one or more amino acid substitutions is selected from the group of amino acid substitutions consisting of X4Q, X7H, X7W, X25H, X25Y, X37M, X70H, X100W, X118D, X135T, X160W, X176K, X193E, X210C, X251E, X281N, X258Q, X323G, X361R, X363E, X363H, X363N, X368F, X405M, X434R, X441Q, X459N, X460G, X451L, and X482W preferably, wherein one or more amino acid substitutions is selected from the group of amino acid substitutions consisting of X25H, X25Y, X100W, X135T, X176K, X193E, and X460G according to the amino acid sequence set forth in SEQ ID NO: 3.
Preferred embodiment 11.): An alpha-amylase variant according to any of the preceding preferred embodiments, wherein said variant comprises a combination of substitutions selected from the group consisting of X25H+X176K+X186E+X206Y+X251E+X482W, X4Q+X25H+X176K+X186E+X206Y+X251E+X405M+X482W, X25H+X176K+X186E+X206Y+X482W, X25H+X186E+X206Y+X405M+X482W, X25H+X186E+X206Y+X482W, X25H+X176K+X186E+X193E+X206Y+X251E+X482W, X4Q+X176K+X186E+X193E+X206Y+X251E+X405M+X482W, X25H+X176K+X186E+X482W, X25H+X176K+X186E+X193E+X206Y+X482W, X4Q+X25H+X176K+X186E+X251E+X405M+X482W, X25H+X176K+X186E+X206Y, X4Q+X176K+X186E+X193E+X206Y+X251E+X405M, X4Q+X206Y+X460G+X482W, X4Q+X25H+X176K+X206Y+X251E+X460G+X482W, X4Q+X25H+X176K+X193E+X251E+X186E+X482W, X4Q+X193E+X206Y+X276R+X186E+X482W, X186E+X25H+X482W, X25H+X193E+X206Y+X251E+X276R+X405M+X186E+X482W, X25H+X251 E+X186E+X482W, X25H+X160W+X176K+X186E+X251E+X405M+X482W, X193E+X206Y+X405M+X186E+X482W, X3I+X356V, X3I+X356I, X83D+X94E, X94D+X125E, X131I+X377Q+X410H, X48F+X94D, X48Y+X116K+X218K, X5L+X218K+X225S, X83E+X116R+X158Y+X181E, X51V+X218K, X83E+X181E, X4Q+X7W+X176K+X186E+X193E+X206Y+X251E+X405M, X4Q+X37M+X176K+X186E+X193E+X206Y+X251E+X405M, X4Q+X25D+X176K+X186E+X193E+X206Y+X251E+X405M, X4Q+X25H+X176K+X186E+X206Y+X251E+X405M+X460G, X4Q+X25H+X176K+X186E+X206Y+X251E+X460G, X4Q+X25Y+X176K+X186E+X193E+X206Y+X251E+X405M, X4Q+X118D+X176K+X186E+X193E+X206Y+X251E+X405M, X4Q+X176K+X182D+X186E+X193E+X206Y+X251E+X405M, X4Q+X176K+X186E+X193E+X206Y+X251E+X258Q+X405M, X4Q+X176K+X186E+X193E+X206Y+X251E+X405M, X4Q+X176K+X186E+X193E+X206Y+X251E+X405M+X460G, X4Q+X176K+X186E+X193E+X206Y+X251E+X363E+X405M, X4Q+X176K+X186E+X193E+X206Y+X251E+X363H+X405M, X4Q+X176K+X186E+X193E+X206Y+X251E+X363N+X405M, X4Q+X176K+X181D+X186E+X193E+X206Y+X251E+X405M, X4Q+X176K+X181F+X186E+X193E+X206Y+X251E+X405M, X4Q+X176K+X181N+X186E+X193E+X206Y+X251E+X405M, X4Q+X176K+X181Q+X186E+X193E+X206Y+X251E+X405M, X4Q+X136E+X176K+X186E+X193E+X206Y+X251E+X405M, X4Q+X100W+X176K+X186E+X193E+X206Y+X251E+X405M, X4Q+X135D+X176K+X186E+X193E+X206Y+X251E+X405M, X4Q+X135E+X176K+X186E+X193E+X206Y+X251E+X405M, X4Q+X135T+X176K+X186E+X193E+X206Y+X251E+X405M, X4Q+X135W+X176K+X186E+X193E+X206Y+X251E+X405M, X4Q+X160D+X176K+X186E+X193E+X206Y+X251E+X405M, X4Q+X160E+X176K+X186E+X193E+X206Y+X251E+X405M, X7H+X25H+X176K+X186E+X206Y,X7W+X25H+X176K+X186E+X206Y, X25D+X176K+X186E+X206Y, X25H+X186E+X206Y+X405M+X460G, X25H+X186E+X206Y+X460G, X25H+X37M+X176K+X186E+X206Y, X25H+X118D+X176K+X186E+X206Y, X25H+X176K+X182D+X186E+X206Y, X25H+X176K+X186E+X206Y, X25H+X176K+X186E+X206Y+X258Q, X25H+X176K+X186E+X206Y+X281N, X25H+X176K+X186E+X206Y+X460G, X25H+X176K+X186E+X206Y+X251E+X460G,X25H+X176K+X186E+X206Y+X363E, X25H+X176K+X186E+X206Y+X363H,X25H+X176K+X186E+X206Y+X363N, X25H+X176K+X186E+X460G, X25H+X176K+X186E+X193E+X206Y, X25H+X176K+X186E+X193E+X206Y+X460G, X25H+X176K+X186E+X193E+X206Y+X251E+X460G, X25H+X176K+X181D+X186E+X206Y, X25H+X176K+X181N+X186E+X206Y, X25H+X176K+X181Q+X186E+X206Y, X25H+X136E+X176K+X186E+X206Y, X25H+X100W+X176K+X186E+X206Y, X25H+X135E+X176K+X186E+X206Y, X25H+X135T+X176K+X186E+X206Y, X25H+X135W+X176K+X186E+X206Y, X25H+X160D+X176K+X186E+X206Y, X25H+X160E+X176K+X186E+X206Y, X25Y+X176K+X186E+X206Y, X87D+X186E+X315K+X420K, X87D+X186E+X226D+X314E+X420E+X461E, X87D+X186E+X226D+X420E, X87D+X186E+X226D+X420E+X461E, X87D+X186E+X314E+X471E, X87D+X186E+X311K+X314E+X420K, X87D+X186E+X160D+X461E,X87R+X186E+X315K+X461R,X87R+X186E+X226D+X471E, X87R+X186E+X226R+X314K+X420K+X461R,X87R+X186E+X226R+X311K+X420K, X87R+X186E+X314K+X315K, X87R+X186E+X311K+X314E, X87R+X186E+X420E+X450R, X87R+X186E+X450R+X452R,X186E+X315K+X420E+X452R, X186E+X226D+X314E+X461E+X471E,X186E+X226D+X314E+X452R, X186E+X226D+X314K+X460D+X471E+X485R, X186E+X226D+X311K+X460D, X186E+X226D+X461E+X471E,X186E+X314E+X460D+X461E, X186E+X314E+X420E+X452R+X461E, X186E+X314K+X450R+X460D, X186E+X460D+X471E+X485R, X186E+X311K+X314K+X452R, X186E+X311K+X461E+X485R, X186E+X311K+X420E+X471E, X186E+X420K+X450R+X471E+X485R, X186E+X420K+X450R+X485R, X186E+X452R+X461E+X471E, X4Q+X176K+X186E+X193E+X206Y+X251E+X405M, X25H+X176K+X186E+X206Y, X83E+X186E+X219D+X226R, X83E+X186E+X219R+X226D+X314E+X452R,X83E+X186E+X219R+X226R, X83E+X186E+X160D+X219D+X226R+X452R, X83R+X186E+X219R+X226D, X160D+X186E+X315K+X450R, X160D+X186E+X226D+X314K+X460D+X461E, X160D+X186E+X226D+X314K+X420E,X160D+X186E+X314K+X485R, X160D+X186E+X420E+X452R, X160D+X186E+X420K+X460D, X186E+X276R, X186E+X206Y, X186E+X206Y+X276R, X186E+X206Y+X276R+X405M, X186E+X206Y+X405M, X186E+X206Y+X251E, X186E+X206Y+X251E+X276R, X186E+X206Y+X251E+X276R+X405M,X186E+X206Y+X251E+X405M, X186E+X206Y+X251E+X323G+X405M, X186E+X206Y+X323G+X405M, X186E+X405M, X186E+X193E+X206Y+X405M, X186E+X193E+X206Y+X251E, X186E+X251E, X186E+X251E+X405M, X4Q+X186E, X4Q+X186E+X276R,X4Q+X186E+X206Y, X4Q+X186E+X206Y+X276R+X405M, X4Q+X186E+X206Y+X405M, X4Q+X186E+X206Y+X251E+X276R, X4Q+X186E+X206Y+X251E+X276R+X405M, X4Q+X186E+X206Y+X251E+X405M, X4Q+X186E+X206Y+X251E+X323G+X405M, X4Q+X186E+X405M, X4Q+X186E+X193E+X206Y+X276R, X4Q+X186E+X193E+X206Y+X405M, X4Q+X186E+X193E+X206Y+X251E+X405M, X4Q+X186E+X251E+X276R+X405M, X4Q+X25H+X186E+X206Y, X4Q+X25H+X186E+X206Y+X276R, X4Q+X25H+X186E+X206Y+X276R+X405M, X4Q+X25H+X186E+X206Y+X405M, X4Q+X25H+X186E+X206Y+X251E+X323A+X405M, X4Q+X25H+X186E+X206Y+X323G+X405M, X4Q+X25H+X186E+X405M, X4Q+X25H+X176K+X186E+X206Y+X276R, X4Q+X25H+X176K+X186E+X206Y+X276R+X405M, X4Q+X25H+X176K+X186E+X206Y+X251E+X276R+X405M, X4Q+X25H+X176K+X186E+X206Y+X251E+X405M ,X4Q+X25H+X176K+X186E+X251E, X4Q+X25H+X176K+X186E+X251E+X405M, X4Q+X25H+X176K+X206Y, X4Q+X25H+X176K+X206Y+X405M N460G, X4Q+X25H+X176K+X206Y+X460G, X4Q+X25H+X176K+X206Y+X251E, X4Q+X25H+X176K+X206Y+X251E+X276R+X405M, X4Q+X25H+X176K+X206Y+X251E+X460G, X4Q+X25H+X176K+X206Y+X323G, X4Q+X25H+X160W+X186E+X206Y+X405M, X4Q+X25H+X160W+X186E+X206Y+X251E, X4Q+X25H+X160W+X186E+X206Y+X251E+X276R+X405M, X4Q+X25H+X160W+X186E+X206Y+X323G+X405M, X4Q+X25H+X160W+X186E+X405M, X4Q+X25H+X160W+X186E+X193E+X206Y+X276R, X4Q+X25H+X160W+X186E+X323G, X4Q+X25H+X160W+X169R+X186E+X206Y+X276R, X4Q+X25H+X160W+X176K+X186E+X276R+X405M, X4Q+X25H+X160W+X176K+X186E+X206Y+X276R, X4Q+X25H+X160W+X176K+X186E+X206Y+X251E, X4Q+X25H+X160W+X176K+X186E+X206Y+X251E+X405M, X4Q+X25H+X160W+X176K+X186E+X405M, X4Q+X25H+X160W+X176K+X186E+X251E+X276R+X405M, X4Q+X25H+X160W+X176K+X186E+X251E+X405M, X4Q+X25H+X160W+X176K+X206Y, X4Q+X25H+X160W+X176K+X206Y+X405M, X4Q+X25H+X160W+X176K+X206Y+X460G, X4Q+X25H+X160W+X176K+X206Y+X251E+X460G, X4Q+X176K+X186E+X276R+X405M, X4Q+X176K+X186E+X206Y+X276R, X4Q+X176K+X186E+X206Y+X276R+X405M, X4Q+X176K+X186E+X206Y+X405M, X4Q+X176K+X186E+X206Y+X251E+X276R+X405M, X4Q+X176K+X186E+X206Y+X251E+X405M ,X4Q+X176K+X186E+X405M, X4Q+X176K+X186E+X193E+X206Y+X276R+X405M, X4Q+X176K+X186E+X193E+X206Y+X251E+X405M, X4Q+X176K+X186E+X251E+X276R+X405M, X4Q+X176K+X186E+X251E+X405M, X4Q+X176K+X186E+X323G+X405M, X4Q+X176K+X206Y,X4Q+X176K+X206Y+X276R, X4Q+X176K+X206Y+X405M, X4Q+X176K+X206Y+X405M N460G, X4Q+X176K+X206Y+X460G, X4Q+X176K+X206Y+X251E+X276R+X405M, X4Q+X176K+X206Y+X251E+X276R+X405M N460G, X4Q+X176K+X206Y+X251E+X405M, X4Q+X176K+X206Y+X251E+X405M N460G, X4Q+X176K+X206Y+X251E+X323A+X460G, X4Q+X160W+X186E+X276R+X405M, X4Q+X160W+X186E+X206Y, X4Q+X160W+X186E+X206Y+X276R+X323G, X4Q+X160W+X186E+X206Y+X405M, X4Q+X160W+X186E+X206Y+X251E, X4Q+X160W+X186E+X206Y+X251E+X276R+X405M, X4Q+X160W+X186E+X405M, X4Q+X160W+X186E+X251E+X405M, X4Q+X160W+X176K+X186E, X4Q+X160W+X176K+X186E+X276R+X405M, X4Q+X160W+X176K+X186E+X206Y+X251E, X4Q+X160W+X176K+X186E+X251E+X276R+X405M, X4Q+X160W+X176K+X186E+X251E+X405M, X4Q+X160W+X176K+X206Y, X4Q+X160W+X176K+X206Y+X251E+X276R+X460G, X4Q+X160W+X176K+X206Y+X251E+X323G+X405M+X460G, X25H+X186E, X25H+X186E+X206Y,X25H+X186E+X206Y+X276R+X405M, X25H+X186E+X206Y+X276R+X323G,X25H+X186E+X206Y+X405M, X25H+X186E+X206Y+X251E+X276R+X323G+X405M, X25H+X186E+X206Y+X251E+X405M, X25H+X186E+X405M, X25H+X186E+X2021+X206Y+X405M, X25H+X186E+X193E+X206Y+X276R+X405M, X25H+X186E+X193E+X206Y+X405M, X25H+X186E+X193E+X206Y+X251E+X276R+X405M, X25H+X186E+X251E, X25H+X186E+X251E+X276R,X25H+X176K+X186E, X25H+X176K+X186E+X276R+X323G+X405M, X25H+X176K+X186E+X206Y, X25H+X176K+X186E+X206Y+X276R, X25H+X176K+X186E+X206Y+X251E, X25H+X176K+X186E+X206Y+X251E+X276R+X405M, X25H+X176K+X186E+X206Y+X251E+X405M, X25H+X176K+X186E+X405M, X25H+X176K+X186E+X193E+X206Y, X25H+X176K+X186E+X193E+X206Y+X251E, X25H+X176K+X186E+X193E+X206Y+X251E+X276R+X405M, X25H+X176K+X186E+X251E+X276R, X25H+X176K+X206Y, X25H+X176K+X206Y+X276R, X25H+X176K+X206Y+X276R+X405M, X25H+X176K+X206Y+X276R+X460G, X25H+X176K+X206Y+X405M, X25H+X176K+X206Y+X460G,X25H+X176K+X206Y+X251E, X25H+X176K+X206Y+X251E+X276R+X405M+X460G, X25H+X176K+X206Y+X251E+X276R+X460G, X25H+X176K+X206Y+X251E+X405M, X25H+X160W+X186E+X206Y, X25H+X160W+X186E+X206Y+X251E, X25H+X160W+X186E+X206Y+X251E+X276R, X25H+X160W+X186E+X206Y+X251E+X276R+X323G+X405M, X25H+X160W+X186E+X206Y+X251E+X405M, X25H+X160W+X186E+X206Y+X323G+X405M, X25H+X160W+X186E+X251E+X276R+X405M, X25H+X160W+X176K+X186E+X206Y+X276R, X25H+X160W+X176K+X186E+X206Y+X276R+X405M, X25H+X160W+X176K+X186E+X206Y+X251E+X276R+X323G, X25H+X160W+X176K+X186E+X206Y+X251E+X405M, X25H+X160W+X176K+X186E+X405M, X25H+X160W+X176K+X186E+X193E+X206Y, X25H+X160W+X176K+X186E+X193E+X206Y+X405M, X25H+X160W+X176K+X186E+X193E+X206Y+X251E+X405M, X25H+X160W+X176K+X186E+X251E, X25H+X160W+X176K+X186E+X251E+X276R, X25H+X160W+X176K+X186E+X251E+X405M, X25H+X160W+X176K+X186E+X323G+X405M, X25H+X160W+X176K+X206Y+X276R+X323G+X405M, X25H+X160W+X176K+X206Y+X460G, X25H+X160W+X176K+X206Y+X251E, X25H+X160W+X176K+X206Y+X251E+X323G+X405M, X176K+X186E+X206Y, X176K+X186E+X206Y+X276R, X176K+X186E+X206Y+X276R+X405M, X176K+X186E+X206Y+X405M, X176K+X186E+X206Y+X251E+X405M, X176K+X186E+X405M,X176K+X186E+X193E+X206Y+X405M, X176K+X186E+X193E+X206Y+X251E+X405M, X176K+X186E+X193E+X405M, X176K+X186E+X251E+X276R+X405M, X176K+X186E+X251E+X405M, X176K+X206Y, X176K+X206Y+X276R,X176K+X206Y+X276R+X460G, X176K+X206Y+X405M, X176K+X206Y+X405M N460G, X176K+X206Y+X460G, X176K+X206Y+X251E, X176K+X206Y+X251E+X405M, X160W+X186E, X160W+X186E+X276R+X405M, X160W+X186E+X206Y+X276R, X160W+X186E+X206Y+X276R+X405M, X160W+X186E+X206Y+X405M, X160W+X186E+X206Y+X251E, X160W+X186E+X206Y+X251E+X405M, X160W+X186E+X405M, X160W+X186E+X193E+X206Y, X160W+X186E+X193E+X206Y+X405M, X160W+X186E+X251E+X276R, X160W+X186E+X251E+X276R+X405M, X160W+X186E+X323G+X405M, X160W+X176K+X186E+X276R+X405M, X160W+X176K+X186E+X206Y, X160W+X176K+X186E+X206Y+X251E+X405M, X160W+X176K+X186E+X405M, X160W+X176K+X186E+X193E+X206Y, X160W+X176K+X186E+X193E+X206Y+X405M, X160W+X176K+X186E+X193E+X206Y+X251E, X160W+X176K+X206Y+X276R+X405M N460G, X160W+X176K+X206Y+X276R+X323G+X460G, X160W+X176K+X206Y+X405M, X160W+X176K+X206Y+X405M N460G, X160W+X176K+X206Y+X460G, X160W+X176K+X206Y+X251E+X276R, X160W+X176K+X206Y+X251E+X405M+X460G, X160W+X176K+X206Y+X323G+X405M+X460G, X4Q+X176K+X186E+X193E+X206Y+X251E+X405M, X4Q+X176K+X186E+X193E+X206Y+X251E+X405M+X258Q, X4Q+X176K+X186E+X193E+X206Y+X251E+X405M+X258Q+X281N, X4Q+X176K+X186E+X193E+X206Y+X251E+X405M+X258Q+X281 N+X363H, X4Q+X176K+X186E+X193E+X206Y+X251E+X405M+X258Q+X363E, X4Q+X176K+X186E+X193E+X206Y+X251E+X405M+X281N, X4Q+X176K+X186E+X193E+X206Y+X251E+X405M+X281N+X363E, X4Q+X176K+X186E+X193E+X206Y+X251E+X405M+X281N+X363H, X4Q+X176K+X186E+X193E+X206Y+X251E+X405M+X181Q, X4Q⁺X176K+X186E+X193E+X206Y+X251E+X405M+X181Q⁺X258Q, X4Q⁺X176K+X186E+X193E+X206Y+X251E+X405M+X181Q⁺X258Q⁺X281N, X4Q+X176K+X186E+X193E+X206Y+X251E+X405M+X181Q+X258Q+X281N+X363H, X4Q+X176K+X186E+X193E+X206Y+X251E+X405M+X181Q+X281N, X4Q+X176K+X186E+X193E+X206Y+X251E+X405M+X181Q+X281N+X363E, X4Q+X176K+X186E+X193E+X206Y+X251E+X405M+X181Q+X363E, X4Q+X176K+X186E+X193E+X206Y+X251E+X405M+X181Q+X363H, X4Q+X176K+X186E+X193E+X206Y+X251E+X405M+X100W, X4Q+X176K+X186E+X193E+X206Y+X251E+X405M+X100W+X258Q, X4Q+X176K+X186E+X193E+X206Y+X251E+X405M+X100W+X258Q+X281N, X4Q+X176K+X186E+X193E+X206Y+X251 E+X405M+X1 00W+X258Q+X281N+X363E, X4Q+X176K+X186E+X193E+X206Y+X251E+X405M+X100W+X258Q+X281N+X363H, X4Q+X176K+X186E+X193E+X206Y+X251E+X405M+X100W+X258Q+X281N+X363H+X272V, X4Q+X176K+X186E+X193E+X206Y+X251 E+X405M+X100W+X258Q+X363E, X4Q+X176K+X186E+X193E+X206Y+X251E+X405M+X100W+X258Q+X363H, X4Q+X176K+X186E+X193E+X206Y+X251E+X405M+X100W+X281N, X4Q+X176K+X186E+X193E+X206Y+X251E+X405M+X100W+X281 N+X363E, X4Q+X176K+X186E+X193E+X206Y+X251E+X405M+X100W+X281N+X363H, X4Q+X176K+X186E+X193E+X206Y+X251 E+X405M+X100W+X181Q, X4Q+X176K+X186E+X193E+X206Y+X251E+X405M+X100W+X181Q⁺X258Q, X4Q+X176K+X186E+X193E+X206Y+X251E+X405M+X100W+X181Q⁺X258Q⁺X281N, X4Q+X176K+X186E+X193E+X206Y+X251E+X405M+X100W+X181Q⁺X258Q⁺X281N+X363E, X4Q+X176K+X186E+X193E+X206Y+X251E+X405M+X100W+X181Q⁺X258Q⁺X363E, X4Q+X176K+X186E+X193E+X206Y+X251E+X405M+X100W+X181Q⁺X258Q⁺X363H, X4Q+X176K+X186E+X193E+X206Y+X251E+X405M+X100W+X181Q+X281 N, X4Q+X176K+X186E+X193E+X206Y+X251E+X405M+X100W+X181Q+X281N+X363H, X4Q+X176K+X186E+X193E+X206Y+X251E+X405M+X100W+X181Q+X363H, X4Q+X176K+X186E+X193E+X206Y+X251E+X405M+X100W+X181Q⁺X363H+X254Q, X4Q+X176K+X186E+X193E+X206Y+X251E+X405M+X100W+X135T, X4Q+X176K+X186E+X193E+X206Y+X251E+X405M+X100W+X135T+X258Q, X4Q+X176K+X186E+X193E+X206Y+X251E+X405M+X100W+X135T+X258Q+X281N+X363H, X4Q+X176K+X186E+X193E+X206Y+X251E+X405M+X1 00W+X135T+X258Q+X363E, X4Q+X176K+X186E+X193E+X206Y+X251E+X405M+X1 00W+X135T+X258Q+X363H, X4Q+X176K+X186E+X193E+X206Y+X251E+X405M+X100W+X135T+X181 Q, X4Q⁺X176K+X186E+X193E+X206Y+X251E+X405M+X100W+X135T+X181Q⁺X258Q, X4Q⁺X176K+X186E+X193E+X206Y+X251E+X405M+X100W+X135T+X181Q⁺X258Q⁺X281N, X4Q⁺X176K+X186E+X193E+X206Y+X251E+X405M+X100W+X135T+X181Q⁺X258Q⁺X363E, X4Q⁺X176K+X186E+X193E+X206Y+X251E+X405M+X100W+X135T+X181Q⁺X258Q⁺X363H, X4Q+X176K+X186E+X193E+X206Y+X251E+X405M+X100W+X135T+X181Q+X281N+X363H, X4Q+X176K+X186E+X193E+X206Y+X251E+X405M+X100W+X135T+X181Q+X363E, X4Q+X176K+X186E+X193E+X206Y+X251E+X405M+X100W+X135T+X181Q+X363H, X4Q+X176K+X186E+X193E+X206Y+X251E+X405M+X100W+X135T+X363E, X4Q+X176K+X186E+X193E+X206Y+X251E+X405M+X100W+X135T+X363H, X4Q+X176K+X186E+X193E+X206Y+X251E+X405M+X100W+X363E, X4Q+X176K+X186E+X193E+X206Y+X251E+X405M+X100W+X363H, X4Q+X176K+X186E+X193E+X206Y+X251E+X405M+X135T, X4Q+X176K+X186E+X193E+X206Y+X251E+X405M+X135T+X258Q, X4Q+X176K+X186E+X193E+X206Y+X251E+X405M+X135T+X258Q+X8A, X4Q+X176K+X186E+X193E+X206Y+X251E+X405M+X135T+X258Q+X281N+X363E, X4Q+X176K+X186E+X193E+X206Y+X251E+X405M+X135T+X258Q+X363H, X4Q+X176K+X186E+X193E+X206Y+X251E+X405M+X135T+X281N, X4Q⁺X176K+X186E+X193E+X206Y+X251E+X405M+X135T+X181Q⁺X258Q, X4Q⁺X176K+X186E+X193E+X206Y+X251E+X405M+X135T+X181Q⁺X258Q⁺X281N, X4Q⁺X176K+X186E+X193E+X206Y+X251E+X405M+X135T+X181Q⁺X258Q⁺X363H, X4Q+X176K+X186E+X193E+X206Y+X251E+X405M+X135T+X363E, X4Q+X176K+X186E+X193E+X206Y+X251E+X405M+X135T+X363H, X4Q+X176K+X186E+X193E+X206Y+X251E+X405M+X363E, X4Q+X176K+X186E+X193E+X206Y+X251E+X405M+X363H, X25H+X176K+X186E+X206Y, X25H+X176K+X186E+X206Y+X258Q, X25H+X176K+X186E+X206Y+X258Q, X25H+X176K+X186E+X206Y+X258Q⁺X281N, X25H+X176K+X186E+X206Y+X258Q⁺X281N+X363E, X25H+X176K+X186E+X206Y+X258Q⁺X363E, X25H+X176K+X186E+X206Y+X258Q+X363E+X123D,X25H+X176K+X186E+X206Y+X281N, X25H+X176K+X186E+X206Y+X181Q, X25H+X176K+X186E+X206Y+X181Q⁺X193E, X25H+X176K+X186E+X206Y+X181Q+X363E, X25H+X176K+X186E+X206Y+X193E, X25H+X176K+X186E+X206Y+X193E+X258Q, X25H+X176K+X186E+X206Y+X193E+X281N+X363E, X25H+X176K+X186E+X206Y+X193E+X363E, X25H+X176K+X186E+X206Y+X100W, X25H+X176K+X186E+X206Y+X100W+X258Q, X25H+X176K+X186E+X206Y+X100W+X258Q⁺X281N, X25H+X176K+X186E+X206Y+X100W+X258Q⁺X281N+X363E, X25H+X176K+X186E+X206Y+X100W+X258Q⁺X363E, X25H+X176K+X186E+X206Y+X100W+X258Q⁺X363E+X135C, X25H+X176K+X186E+X206Y+X100W+X281N, X25H+X176K+X186E+X206Y+X100W+X281N+X363E, X25H+X176K+X186E+X206Y+X100W+X361R, X25H+X176K+X186E+X206Y+X100W+X181Q⁺X281N+X363E, X25H+X176K+X186E+X206Y+X100W+X181Q⁺X281N+X363E+X175D, X25H+X176K+X186E+X206Y+X100W+X181Q⁺X193E, X25H+X176K+X186E+X206Y+X100W+X181Q⁺X193E+X258Q⁺X281N, X25H+X176K+X186E+X206Y+X100W+X181Q⁺X193E+X258Q⁺X363E, X25H+X176K+X186E+X206Y+X100W+X181Q⁺X193E+X281N+X363E, X25H+X176K+X186E+X206Y+X100W+X181Q⁺X193E+X363E, X25H+X176K+X186E+X206Y+X100W+X181Q⁺X363E, X25H+X176K+X186E+X206Y+X100W+X193E, X25H+X176K+X186E+X206Y+X100W+X193E+X258Q, X25H+X176K+X186E+X206Y+X100W+X193E+X258Q⁺X473R, X25H+X176K+X186E+X206Y+X100W+X193E+X258Q⁺X281N, X25H+X176K+X186E+X206Y+X100W+X193E+X258Q⁺X281N+X363E, X25H+X176K+X186E+X206Y+X100W+X193E+X258Q⁺X363E, X25H+X176K+X186E+X206Y+X100W+X193E+X363E, X25H+X176K+X186E+X206Y+X100W+X135T, X25H+X176K+X186E+X206Y+X100W+X135T+X258Q, X25H+X176K+X186E+X206Y+X100W+X135T+X258Q⁺X281N, X25H+X176K+X186E+X206Y+X100W+X135T+X258Q⁺X281N+X363E, X25H+X176K+X186E+X206Y+X100W+X135T+X258Q⁺X363E, X25H+X176K+X186E+X206Y+X100W+X135T+X281N, X25H+X176K+X186E+X206Y+X100W+X135T+X281N+X363E, X25H+X176K+X186E+X206Y+X100W+X135T+X181Q, X25H+X176K+X186E+X206Y+X100W+X135T+X181Q⁺X258Q⁺X281N+X363E, X25H+X176K+X186E+X206Y+X100W+X135T+X181Q⁺X281N+X363E, X25H+X176K+X186E+X206Y+X100W+X135T+X181Q⁺X193E, X25H+X176K+X186E+X206Y+X100W+X135T+X181Q⁺X193E+X258Q⁺X363E, X25H+X176K+X186E+X206Y+X100W+X135T+X181Q⁺X193E+X363E, X25H+X176K+X186E+X206Y+X100W+X135T+X193E, X25H+X176K+X186E+X206Y+X100W+X135T+X193E+X258Q⁺X363E, X25H+X176K+X186E+X206Y+X100W+X135T+X193E+X363E, X25H+X176K+X186E+X206Y+X100W+X135T+X363E, X25H+X176K+X186E+X206Y+X100W+X363E, X25H+X176K+X186E+X206Y+X135T, X25H+X176K+X186E+X206Y+X135T+X258Q, X25H+X176K+X186E+X206Y+X135T+X258Q⁺X281N, X25H+X176K+X186E+X206Y+X135T+X258Q+X281N+X363E, and X25H+X176K+X186E+X206Y+X135T+X258Q⁺X363E.
Preferred embodiment 12.): An alpha-amylase variant according to any of the preceding preferred embodiments, wherein the amino acid residue at the unspecified position (i.e., X) corresponds to the amino acid residue shown in SEQ ID NO: 1 or 3, preferably in SEQ ID NO: 1, at the respective position (according to the numbering of SEQ ID NO: 3).
Preferred embodiment 13.): An alpha-amylase variant according to any of the preceding preferred embodiments, wherein the variant comprises the deletion D183* and G184* according to the numbering of SEQ ID NO: 3.
Preferred embodiment 14.): An alpha-amylase variant according to any of the preceding preferred embodiments further comprising additional amino acid substitutions at one or more positions corresponding to positions selected from the group consisting of 9, 130, 179, 181, 186, 190, 195, 202, 206, 244, 402, 419, 420, 422, 423, 428, 430, 435, 441, 444, 450, 452, 454, 466, 469, 473, 475, 476, 479, 483, and 485, preferably one or more additional amino acid substitutions selected from the group of amino acid substitutions consisting of X9D, X9F, X9K, X9L, X9N, X9P, X9Q, X9S, X9T, X9Y, X130V, X179G, X179L, X181 D, X181E, X181F, X181H, X181I, X181N, X181Q, X181S, X181T, X181V, X181W, X181Y, X186A, X186C, X186D, X186E, X186F, X186H, X186I, X186K, X186L, X186M, X186N, X186Q, X186R, X186S, X186V, X186W, X186Y, X190H, X195F, X195H, X195K, X195L, X195W, X195Y, X202L X206C, X206H, X206L, X206M, X206Y, X244A, X244C, X244D, X244E, X244F, X244G, X244H, X244M, X244N, X244Q, X244V, X402T, X419C, X420D, X420E, X420G, X420H, X420K, X420L, X420Q, X422C, X422N, X422H, X423F, X423M, X423Q, X423S, X428A, X428C, X428D, X428E, X428G, X428I, X428K, X428L, X428M, X428N, X428R, X428S, X428V, X428W, X428Y, X430A, X430C, X430D, X430E, X430F, X430G, X430I, X430L, X430P, X430Q, X430S, X430T, X430V, X435E, X435K, X435P, X435R, X435S, X435A, X435D, X437A, X437L, X437T, X437W, X441C, X441D, X441K, X441L, X441M, X441N, X441S, X444E, X444H, X444K, X444M, X444N, X444R, X444T, X450C, X450D, X450E, X450H, X450I, X450L, X450M, X450P, X450Q, X450R, X450T, X452A, X452C, X452E, X452F, X452I, X452K, X452M, X452N, X452R, X452T, X454A, X454E, X454K, X454L, X454M, X454P, X454S, X454T, X466E, X466W, X469F, X469L, X469Y, X473H, X473Q, X473R, X475A, X475K, X475N, X475E, X475L, X476G, X476E, X479A, X479I, X479K, X479M, X483F, X483I, X483L, X483Q, X483R, X485K, and X485R wherein the numbering is according to the amino acid sequence set forth in SEQ ID NO: 3.
Preferred embodiment 15.): An alpha-amylase variant according to any of the preceding preferred embodiments, wherein the number of substitutions compared to SEQ ID NO: 1 is 1 to 30, preferably 1 to 25, 1 to 20, 1 to 15, 1 to 10, or 1 to 5, 2 to 30, 2 to 25, 2 to 20, 2 to 15 2 to 10, 2 to 8, or 2 to 5, preferably 3 to 30, 3 to 25, 3 to 20, 3 to 15 3 to 10, 3 to 8, or 3 to 5, preferably, 4 to 30, 4 to 25, 4 to 20, 4 to 15, 4 to 10, or 4 to 8.
Preferred embodiment 16.): An alpha-amylase variant according to any of the preceding preferred embodiments, wherein said variant comprises or consists of the amino acid sequence set forth in SEQ ID NO: 1 with one or more, preferably 1-30, of the amino acid substitutions selected from the group of amino acid substitutions consisting of X100D, X100F, X100K, X100L, X103A, X106D, X108A, X109A, X10A, X10C, X10E, X10F, X10L, X10W, X10Y, X113F, X113H, X113M, X113R, X113V, X113W, X113Y, X114A, X114C, X114D, X114E, X114F, X114G, X114H, X1141, X114K, X114L, X114M, X114N, X114Q, X114R, X114S, X114V, X114W, X114Y, X115C, X115D, X115K, X115M, X115N, X115Q, X115R, X115S, X115T, X115V, X116I, X116K, X116L, X116M, X116R, X116T, X117A, X117C, X117D, X117F, X117I, X117N, X117P, X117R, X117W, X117Y, X118A, X118D, X118E, X119H, X119P, X119R, X119S, X119Y, X120E, X120G, X120M, X120S, X120W, X120Y, X123D, X123S, X125A, X125D, X125E, X125F, X125G, X125H, X125K, X125L, X125M, X125N, X125Q, X125R, X125T, X125V, X125W, X125Y, X126D, X128C, X128E, X128L, X128M, X128W, X128Y, X129E, X12N, X12S, X131C, X1311, X131L, X131Q, X131W, X132T, X133A, X133C, X133D, X133E, X133H, X133K, X133N, X133P, X133Q, X133S, X134C, X134E, X134F, X134I, X134L, X134M, X134P, X134T, X134V, X134W, X134Y, X135D, X135E, X135G, X135M, X135N, X135P, X135S, X135T, X135W, X136A, X136C, X136D, X136E, X136F, X136H, X136L, X136M, X136N, X136P, X136W, X139C, X139S, X13A, X13Y, X141V, X142C, X142E, X142F, X142L, X142M, X142Q, X142R, X142W, X142Y, X143F, X144C, X144E, X144G, X144K, X144N, X144Q, X144R, X144S, X144T, X144V, X144Y, X145A, X146C, X146D, X146E, X146F, X146G, X146H, X146K, X146L, X146S, X146T, X146W, X147M, X149E, X14N, X150C, X150E, X150Q, X151C, X151E, X154A, X154Y, X155Y, X156E, X156V, X158H, X158N, X158Y, X15R, X160D, X160E, X160W, X161T, X162V, X163A, X163Q, X163T, X164V, X165S, X165T, X165W, X169A, X169D, X169E, X169S, X169V, X16F, X16R, X170C, X170F, X172A, X172C, X172D, X172K, X172N, X173I, X173Y, X174D, X174E, X174G, X174H, X174M, X174N, X174P, X174S, X174T, X175A, X175G, X175H, X175Q, X176K, X176S, X176T, X177A, X177G, X177K, X177N, X177P, X177R, X177S, X177W, X178F, X17K, X17V, X180M, X180N, X180T, X180W, X182D, X182E, X182N, X182Q, X182S, X184G, X185E, X185M, X185N, X187A, X187D, X187M, X187V, X188H, X188T, X188V, X189I, X18F, X18I, X18K, X18M, X18R, X18T, X191F, X191H, X191K, X191M, X191W, X191Y, X192A, X192T, X193D, X193E, X193G, X193V, X19A, X19C, X19D, X19F, X19G, X19H, X19I, X19K, X19L, X19M, X19P, X19Q, X19S, X19T, X19Y, X1R, X1V, X203E, X203R, X208F, X208I, X208Y, X20A, X20C, X20D, X20E, X20F, X20G, X20H, X20K, X20L, X20M, X20N, X20P, X20Q, X20S, X20T, X20V, X20W, X20Y, X210A, X210C, X210D, X210E, X210F, X210M, X210N, X210Q, X210S, X210Y, X211A, X211C, X211D, X211E, X211G, X211H, X211L, X211N, X211Q, X211S, X211T, X211V, X212C, X212D, X212I, X212L, X212W, X213A, X213C, X213M, X213R, X213S, X214E, X214P, X215A, X215D, X215E, X215H, X215W, X216G, X216H, X218A, X218C, X218D, X218E, X218F, X218G, X218H, X218I, X218K, X218L, X218N, X218Q, X218S, X218T, X218V, X218W, X218Y, X219A, X219C, X219D, X219E, X219F, X219G, X219H, X219K, X219M, X219Q, X219R, X219S, X219T, X219W, X219Y, X21E, X21S, X221F, X221N, X222D, X222K, X222S, X222T, X223C, X223L, X223V, X223Y, X224F, X224V, X225A, X225C, X225E, X225F, X225H, X225I, X225N, X225R, X225S, X225Y, X226A, X226C, X226D, X226E, X226G, X226H, X226I, X226L, X226M, X226Q, X226R, X226S, X226T, X226V, X226W, X226Y, X227C, X227F, X227G, X227H, X227I, X227K, X227L, X227M, X227R, X227T, X227V, X227W, X227Y, X22A, X22D, X22E, X22F, X22G, X22K, X22L, X22M, X22Q, X22R, X22T, X22W, X22Y, X230A, X230F, X231C, X231D, X231N, X233C, X233H, X233I, X233M, X233T, X233W, X233Y, X234C, X235L, X235M, X235V, X236Y, X237C, X237I, X238A, X238F, X238T, X23N, X23Q, X23W, X240M, X242M, X242N, X243D, X243F, X245E, X245H, X245M, X249D, X249I, X24G, X250V, X251A, X251E, X251F, X251L, X251M, X251S, X251T, X252C, X252I, X252S, X253C, X253G, X253Y, X255D, X255F, X255I, X255T, X255V, X256C, X257L, X257V, X257Y, X258F, X258Q, X258R, X259L, X25A, X25C, X25D, X25F, X25G, X25H, X25K, X25L, X25M, X25Q, X25S, X25W, X25Y, X260H, X261R, X262C, X262D, X262E, X262H, X262P, X262Y, X263I, X264H, X264T, X264W, X265S, X268F, X268G, X269M, X26A, X26D, X26E, X26F, X26L, X26M, X26P, X26S, X26V, X26Y, X270A, X270Q, X270Y, X271S, X272F, X272G, X272L, X272S, X273A, X273C, X273D, X273E, X273F, X273H, X273I, X273L, X273M, X273P, X273Q, X273R, X273V, X273W, X273Y, X274F, X274S, X275V, X276D, X276K, X276L, X276N, X276R, X276Y, X277D, X277E, X277T, X279A, X279P, X27A, X27D, X27F, X27G, X27H, X27I, X27Q, X27R, X27T, X27V, X280D, X280F, X280G, X280H, X280I, X280K, X280N, X280R, X280V, X280Y, X281A, X281D, X281E, X281H, X284A, X284F, X284H, X284L, X284M, X284N, X284Y, X285G, X285L, X285N, X285P, X286Q, X287A, X287D, X287E, X287H, X287T, X288A, X288K, X288P, X288Y, X289F, X289G, X289R, X289T, X28C, X28D, X28E, X28F, X28G, X28I, X28K, X28N, X28Q, X28S, X28T, X28V, X290D, X290M, X290N, X290Q, X290W, X291D, X291K, X291T, X291Y, X292C, X292D, X292F, X292I, X292L, X292T, X292W, X292Y, X293D, X293E, X293F, X293K, X293R, X294G, X294T, X295F, X296A, X296C, X296L, X296Y, X297E, X297F, X297H, X297K, X297M, X297S, X297V, X299G, X299I, X299K, X299L, X299S, X299Y, X29D, X29E, X29F, X29G, X29H, X29I, X29K, X29L, X29N, X29P, X29Q, X29V, X29W, X29Y, X2I, X2S, X301F, X302H, X302I, X302Q, X302V, X302Y, X303E, X303H, X303I, X303K, X303L, X303M, X303N, X303P, X303R, X303T, X304A, X304D, X304E, X304H, X304K, X304M, X304N, X304P, X304R, X304T, X304W, X304Y, X306A, X306D, X306E, X306G, X306H, X306I, X306M, X306Q, X306R, X306S, X306T, X306V, X306W, X306Y, X307F, X307M, X308S, X309H, X309L, X309Q, X30A, X30E, X30F, X30G, X30H, X30I, X30K, X30L, X30M, X30Q, X30T, X30W, X30Y, X310A, X310Q, X311A, X311E, X311G, X311H, X311K, X311N, X311R, X311T, X311Y, X312L, X312M, X313V, X314C, X314E, X314K, X314Q, X315A, X315C, X315E, X315H, X315K, X315T, X318I, X318S, X318T, X319A, X319D, X319H, X319I, X319K, X319M, X319N, X319P, X319S, X319T, X319W, X31N, X31Q, X31S, X31T, X31V, X31W, X320A, X320C, X320D, X320E, X320G, X320H, X320K, X320L, X320N, X320Q, X320S, X320Y, X321A, X321E, X321K, X321N, X321T, X321V, X321W, X323A, X323G, X323K, X323L, X323V, X324K, X324L, X324M, X324W, X324Y, X326G, X326N, X326S, X326Y, X327C, X327L, X327M, X32A, X32D, X32E, X32F, X32H, X32I, X32L, X32M, X32N, X32P, X32Q, X32T, X32W, X333I, X334T, X336K, X337A, X337C, X337F, X337G, X337I, X337K, X337L, X337M, X337N, X337Q, X337R, X337S, X337T, X337V, X337Y, X338G, X338S, X338T, X33D, X33E, X33H, X33K, X33M, X33Q, X33R, X33Y, X341V, X342P, X343L, X343T, X343W, X343Y, X344I, X344Q, X344V, X345D, X345G, X345M, X345N, X345Q, X345S, X345T, X346A, X346C, X346D, X346G, X346H, X346N, X346Q, X348T, X34H, X34I, X34V, X350H, X350K, X350P, X351A, X351M, X352S, X353H, X354I, X354N, X354T, X354Y, X355I, X355M, X356I, X356V, X357A, X358I, X358L, X358N, X358P, X358V, X359E, X35A, X35C, X35D, X35G, X35H, X35I, X35L, X35M, X35N, X35P, X35Q, X35R, X35S, X35T, X35V, X35Y, X360A, X360F, X360G, X360I, X360L, X360N, X360Q, X360R, X360S, X360T, X360V, X360Y, X362F, X362K, X362M, X362N, X362T, X362V, X362Y, X363A, X363C, X363D, X363E, X363G, X363H, X363K, X363L, X363M, X363P, X363Q, X363R, X363S, X363T, X363V, X363W, X363Y, X364A, X364C, X364G, X364K, X364L, X364N, X364S, X364T, X364V, X366I, X366L, X366T, X367E, X367S, X368A, X368F, X368L, X368N, X36A, X36E, X36G, X36I, X36K, X36M, X36N, X36P, X36Q, X36R, X36S, X36T, X36V, X370E, X370I, X372A, X372C, X372E, X372F, X372H, X372M, X372N, X372Q, X375A, X375D, X375E, X375I, X375K, X375Q, X375R, X375T, X375W, X375Y, X376G, X376I, X376K, X376L, X376M, X376Q, X376R, X376S, X376V, X377Q, X378C, X378D, X378E, X378R, X379A, X379L, X379S, X37A, X37G, X37M, X37P, X37T, X37V, X37W, X381E, X381V, X382A, X382H, X382K, X382L, X382N, X382Q, X382S, X383C, X383D, X383E, X383H, X383I, X383M, X383N, X383Q, X383R, X383S, X383V, X383Y, X384A, X384C, X384D, X384E, X384F, X384I, X384L, X384M, X384N, X384Q, X384R, X384T, X384V, X384W, X384Y, X385A, X385C, X385D, X385E, X385F, X385G, X385H, X385I, X385L, X385M, X385N, X385P, X385Q, X385R, X385S, X385T, X385V, X385W, X385Y, X387C, X387E, X387N, X388E, X388F, X388H, X388I, X388M, X388R, X388V, X389G, X389H, X389K, X38N, X390D, X390F, X390M, X390N, X390P, X390R, X391A, X391F, X391G, X391K, X391M, X391N, X391Q, X391S, X391T, X391Y, X392C, X392V, X393E, X393H, X393P, X393S, X393V, X394A, X394C, X394E, X394H, X394I, X394L, X394M, X394N, X394R, X394S, X395A, X395H, X395M, X395V, X396H, X396P, X397D, X397H, X397P, X397S, X398M, X399P, X39E, X39K, X3A, X3F, X3G, X3I, X3K, X3L, X3Q, X3V, X400A, X400D, X400E, X400G, X400H, X400I, X400K, X400L, X400M, X400N, X400P, X400Q, X400R, X400S, X400V, X400W, X401I, X401K, X401M, X401T, X403N, X405C, X405H, X405M, X405T, X405V, X406P, X407D, X407R, X407S, X408E, X408I, X408Q, X40I, X40S, X410H, X410I, X410K, X410L, X410P, X410R, X410Y, X413S, X414C, X414E, X414S, X415E, X415I, X416S, X418C, X418N, X418P, X41C, X41D, X41E, X41G, X41Q, X41S, X41T, X421N, X421P, X424A, X426D, X426W, X427C, X427F, X427G, X427K, X427Q, X427R, X427S, X427T, X427V, X429A, X429D, X429E, X429F, X429G, X429I, X429M, X429N, X429P, X429Q, X429S, X429T, X429V, X429W, X42C, X42I, X42Q, X42V, X431I, X434S, X436E, X438F, X438P, X438Y, X439C, X439P, X440V, X442Q, X443H, X443T, X445A, X445C, X445D, X445F, X445G, X445H, X445K, X445M, X445Q, X445R, X445S, X445T, X445V, X446F, X446I, X446L, X446P, X446Q, X446R, X446V, X446W, X447V, X448D, X448E, X448H, X448N, X449A, X449F, X449G, X449K, X449L, X449M, X449N, X449P, X449Q, X449R, X449S, X449V, X449W, X449Y, X451L, X453G, X453I, X453N, X453P, X453Y, X455L, X456L, X456M, X456R, X456S, X456V, X456W, X456Y, X457A, X457C, X457E, X457F, X457G, X457K, X457L, X457M, X457R, X457S, X457T, X457V, X457W, X458I, X458K, X458V, X458W, X459C, X459D, X459E, X459I, X459K, X459N, X459Q, X459R, X459V, X459Y, X45G, X45N, X460A, X460D, X460E, X460G, X460Q, X460R, X460S, X460T, X460V, X461A, X461E, X461F, X461K, X461L, X461M, X461N, X461Q, X461R, X461S, X461V, X462K, X463L, X465H, X465P, X465R, X465V, X467A, X467E, X467H, X468D, X468H, X470A, X470Q, X470T, X471E, X474F, X474H, X474P, X478A, X478E, X47S, X480D, X480E, X480M, X480R, X480Y, X482C, X482T, X482W, X48F, X48I, X48M, X48Y, X4A, X4C, X4K, X4M, X4Q, X4R, X4S, X51Q, X51T, X51V, X54D, X54G, X54Q, X59T, X5A, X5C, X5D, X5E, X5F, X5H, X5I, X5K, X5L, X5M, X5N, X5P, X5Q, X5R, X5V, X5Y, X60T, X63C, X63V, X6A, X6C, X6E, X6F, X6G, X6H, X6K, X6L, X6M, X6P, X6Q, X6S, X6T, X6V, X6W, X6Y, X70F, X70H, X70L, X70M, X70N, X70Y, X71D, X72C, X72D, X72E, X72N, X72T, X73L, X73N, X73Q, X75A, X75G, X75I, X75L, X75P, X75T, X75W, X76C, X76E, X76G, X76L, X76T, X76V, X7C, X7E, X7F, X7H, X7K, X7N, X7P, X7Q, X7R, X7S, X7V, X7W, X7Y, X81H, X81L, X82K, X82M, X83A, X83D, X83E, X83G, X83R, X83S, X86K, X87D, X87R, X89A, X89C, X89F, X89G, X89L, X89M, X89R, X89S, X8A, X8C, X8F, X8I, X8M, X8P, X8S, X8V, X8W, X8Y, X90A, X90D, X90E, X90F, X90G, X90I, X90M, X90N, X90Q, X90R, X90S, X90V, X90Y, X91C, X91D, X91E, X91F, X91G, X91H, X91I, X91K, X91L, X91M, X91N, X91Q, X91S, X91T, X91V, X91W, X91Y, X92D, X92M, X92V, X93K, X93Q, X94A, X94D, X94E, X94K, X94M, X94V, X94Y, X95E, X95I, X95L, X95V, X96K, X96N, X96Q, X97V, X98E, X98G, X98N, X99H, X99K, X99N, X100W, and X1G according to the numbering of SEQ ID NO: 3, preferably further comprising 1-10 conservation amino acid substitutions and / or further comprising additional amino acid substitutions at 1-10 positions corresponding to positions selected from the group consisting of 9, 130, 179, 181, 186, 190, 195, 202, 206, 244, 402, 419, 420, 422, 423, 428, 430, 435, 441, 444, 450, 452, 454, 466, 469, 473, 475, 476, 479, 483, and 485, preferably 1-10 additional amino acid substitutions selected from the group of amino acid substitutions consisting of X9D, X9F, X9K, X9L, X9N, X9P, X9Q, X9S, X9T, X9Y, X130V, X179G, X179L, X181D, X181E, X181F, X181H, X181I, X181N, X181Q, X181S, X181T, X181V, X181W, X181Y, X186A, X186C, X186D, X186E, X186F, X186H, X186I, X186K, X186L, X186M, X186N, X186Q, X186R, X186S, X186V, X186W, X186Y, X190H, X195F, X195H, X195K, X195L, X195W, X195Y, X202L X206C, X206H, X206L, X206M, X206Y, X244A, X244C, X244D, X244E, X244F, X244G, X244H, X244M, X244N, X244Q, X244V, X402T, X419C, X420D, X420E, X420G, X420H, X420K, X420L, X420Q, X422C, X422N, X422H, X423F, X423M, X423Q, X423S, X428A, X428C, X428D, X428E, X428G, X428I, X428K, X428L, X428M, X428N, X428R, X428S, X428V, X428W, X428Y, X430A, X430C, X430D, X430E, X430F, X430G, X430I, X430L, X430P, X430Q, X430S, X430T, X430V, X435E, X435K, X435P, X435R, X435S, X435A, X435D, X437A, X437L, X437T, X437W, X441C, X441D, X441K, X441L, X441M, X441N, X441S, X444E, X444H, X444K, X444M, X444N, X444R, X444T, X450C, X450D, X450E, X450H, X450I, X450L, X450M, X450P, X450Q, X450R, X450T, X452A, X452C, X452E, X452F, X452I, X452K, X452M, X452N, X452R, X452T, X454A, X454E, X454K, X454L, X454M, X454P, X454S, X454T, X466E, X466W, X469F, X469L, X469Y, X473H, X473Q, X473R, X475A, X475K, X475N, X475E, X475L, X476G, X476E, X479A, X479I, X479K, X479M, X483F, X483I, X483L, X483Q, X483R, X485K, and X485R wherein the numbering is according to the amino acid sequence set forth in SEQ ID NO: 3.
Preferred embodiment 17.): An alpha-amylase variant according to any of the preceding preferred embodiments, wherein said variant comprises a combination of substitutions selected from any of Tables 8-12, preferably selected from the group consisting of N25H+R176K+G186E+I206Y+R181Q, N25H+R176K+G186E+I206Y+Y135T, N25H+R176K+G186E+I206Y+Y100W+Y363E, N25H+R176K+G186E+I206Y+Y100W+T193E, N25H+R176K+G186E+I206Y+T251E, G4Q+N25H+R176K+G186E+I206Y+T251E+L405M, N25H+R176K+G186E+1206Y, N25H+G186E+1206Y+L405M, N25H+G186E+1206Y, N25H+R176K+G186E+T193E+I206Y+T251E, G4Q+R176K+G186E+T193E+I206Y+T251E+L405M, N25H+R176K+G186E, N25H+R176K+G186E+T193E+I206Y, G4Q+N25H+R176K+G186E+T251E+L405M, N25H+R176K+G186E+1206Y, G4Q+R176K+G186E+T193E+I206Y+T251E+L405M, G4Q+l206Y+N460G, G4Q+N25H+R176K+I206Y+T251E+N460G, G4Q+N25H+R176K+T193E+T251E+G186E, G4Q+T193E+I206Y+E276R+G186E, G186E+N25H, N25H+T193E+I206Y+T251E+E276R+L405M+G186E, N25H+T251E+G186E, N25H+Y160W+R176K+G186E+T251E+L405M, T193E+1206Y+L405M+G186E, N25H+R176K+G186E+I206Y+G258Q+Y363E, N25H+R176K+G186E+I206Y+R181Q, N25H+R176K+G186E+I206Y+Y100W, N25H+R176K+G186E+I206Y+Y100W+Q359R, N25H+R176K+G186E+1206Y+Y100W+G258Q, N25H+R176K+G186E+1206Y+Y100W+G258Q+Y363E, N25H+R176K+G186E+1206Y+Y100W+Y135T, N25H+R176K+G186E+I206Y+Y100W+Y135T+Y363E, N25H+R176K+G186E+I206Y+Y135T, N25H+R176K+G186E+1206Y+Y135T+G258Q, N25H+R176K+G186E+1206Y+Y135T+G258Q+Y363E, N25H+R176K+G186E+I206Y+Y135T+Y363E, N25H+R176K+G186E+I206Y+Y363E, N25H+R176K+G186E+I206Y+T251E+Y482W, N25H+R176K+G186E+1206Y+Y482W, N25H+R176K+G186E+T193E+I206Y+T251E+Y482W, G4Q+R176K+G186E+T193E+I206Y+T251E+L405M+Y482W, G4Q+N25H+R176K+G186E+T251E+L405M+Y482W, N25H+R176K+G186E+T193E+I206Y+Y482W, G4Q+N25H+R176K+G186E+I206Y+T251E+L405M+Y482W, N25H+G186E+I206Y+L405M+Y482W, N25H+R176K+G186E+Y482W, N25H+R176K+G186E+T193E+I206Y+T251E+N460G, G4Q+N25H+R176K+G186E+I206Y+T251E+N460G, N25H+R176K+G186E+T193E+I206Y+T251E+N460G+Y482W, G4Q+N25H+R176K+G186E+I206Y+T251E+N460G+Y482W, N25H+R176K+G186E+N195F+T251E+Y482W, N25H+R176K+G186E+N195F+Y482W, N25H+R176K+G186E+T193E+N195F+T251E+Y482W, G4Q+R176K+G186E+T193E+N195F+T251E+L405M+Y482W, N25H+R176K+G186E+T193E+N195F+Y482W, G4Q+N25H+R176K+G186E+N195F+T251E+L405M+Y482W, N25H+G186E+N195F+L405M+Y482W, N25H+R176K+G186E+T193E+N195F+T251E+N460G, G4Q+N25H+R176K+G186E+N195F+T251E+N460G, N25H+R176K+G186E+T193E+N195F+T251E+N460G+Y482W, and G4Q+N25H+R176K+G186E+N195F+T251 E+N460G+Y482W according to the numbering of SEQ ID NO: 3.
Preferred embodiment 18.): A polynucleotide encoding the alpha-amylase variant according to any one of the preceding embodiments.
Preferred embodiment 19.): A nucleic acid construct or an expression vector comprising the polynucleotide according to embodiment 18.).
Preferred embodiment 20.): A host cell comprising the polynucleotide according to embodiment 18.), the nucleic acid construct according to embodiment 19.), or the expression vector according to embodiment 19.).
Preferred embodiment 21.): A composition comprising the alpha-amylase variant described in the preceding embodiments, wherein the composition, wherein the composition comprises one or more second enzyme different from the alpha-amylase variant, preferably, wherein the second enzyme is a protease, lipase or mannanase, preferably a mannanase or a protease, particularly preferred a protease, preferably a subtilisin protease, preferably a subtilisin protease having at least 60% sequence identity to any of SEQ ID NO: 10-14, preferably, to SEQ ID NO: 1, preferably wherein the protease comprises a glutamic acid at position 101 according to BPN' numbering.
Preferred embodiment 22.): A composition comprising an alpha-amylase variant as described in the preceding preferred embodiments, wherein the composition is a detergent composition, preferably comprising the alpha-amylase variant in the preceding preferred embodiments in an amount 1-1000 mg of active alpha-amylase variant per kg of detergent composition, preferably, 5-500 mg/kg, 5-300 mg/kg, 10-200 mg/kg, or 50-200 mg/kg, preferably comprising at least one additional component selected from the group consisting of an additional enzyme different from the alpha-amylase of the invention, surfactant, defoamer, building agent (builder), polymer, bleaching system (bleach), rheology modifier, hydrotrope, softening agent, desiccant, whitening agent, buffer, preservative, anti-corrosion additive, dyestuff and fragrance, preferably, the at least one component of the detergent is a surfactant and/or a builder, preferably a strong-sequestering builder.
Preferred embodiment 23.): A composition according to embodiment 21.) and 22.), wherein the detergent composition is liquid or solid, preferably a laundry detergent composition or an Automatic Dish Wash (ADW) detergent composition, preferably in the form of a pouch.
Preferred embodiment 24.): A method of producing an alpha-amylase variant, comprising:
   a. cultivating said host cell according to embodiment 20.) under conditions suitable for expression of said alpha-amylase variant; and
   b. recovering said alpha-amylase variant.
Preferred embodiment 25.): Use of an alpha-amylase variant as described in the preceding embodiments in a cleaning process such as laundry or hard surface cleaning.

### Examples

### Example 1

### Improved parent alpha-amylase

The ALBA amylase (SEQ ID NO: 3) was modified by deleting the amino acids G182 and D183 causing an increased stability of the amylase. The resulting amylase variant is Amylase A as shown in SEQ ID NO: 4. The wash performance of Amylase A was compared to Amylase B (shown in SEQ ID NO: 1), which served as a parent amylase for the alpha-amylase variants described herein, using microscale wash trials and a Launderometer.
For the microscale cleaning performance trials the amylase A and B were used to remove soil from aged corn starch stains (CS-126) in 3.3 g/L ES1-C (as described in Table 1b above) diluted with water (15°dH) at room temperature at 0.02, 0.05 and 0.1 ppm of amylase added to the wash for 60 minutes before being rinsed for 5 min under running water. and dried.
The wash performance is measured as the brightness of the color of the textile washed. Brightness can also be expressed as the intensity of the light reflected from the sample when illuminated with white light. When the sample is stained the intensity of the reflected light is lower than that of a clean sample. Expressed another way, a cleaner sample will reflect more light and will have a higher intensity. Therefore, the intensity of the reflected light can be used to measure wash performance. Color measurements are made with digital color measurement with an average of the RGB values over the individual stain as results. The values for the detergent base were subtracted and a ratio of the performance of Amylase B vs. that of Amylase A was made and a value above 100% indicates higher performance than the performance by the Amylase A. In following Table 2 the average of at least 2 experiments is given.

| Amylase | 0 ppm | 0.02 ppm | 0.05 ppm | 0.1 ppm |
|---|---|---|---|---|
| A | 0 | 100% | 100% | 100% |
| B | 0 | 151% | 191% | 188% |

An identical wash trial was performed using a different detergent according to Formulation A of Table 1a above at room temperature at 0.02, 0.05, or 0.1 ppm of amylase added to the wash for 60 minutes before being rinsed for 5 min under running water, dried and measured for intensity of the reflected light as an average of the RGB values. The values for the detergent base were subtracted and a ratio of the performance of Amylase B vs. that of Amylase A was made and a value above 100% indicates higher performance than the performance by the Amylase A.
In following Table 3 the average of at least 2 experiments is given.

| Amylase | 0 ppm | 0.02 ppm | 0.05 ppm | 0.1 ppm |
|---|---|---|---|---|
| A | 0% | 100% | 100% | 100% |
| B | 0% | 153% | 158% | 213% |

For the Launderometer wash trials the Amylase A and B were used to wash different soiled stains (CS-129 Aged Tapioca, EMPA161 Cotton starch, CS-26 Corn starch, CS-28 Rice Starch, CS-28 Tapioca starch) in 3.5 g/L commercial Persil Non-Bio diluted with water (15°dH) at 40°C at 0.02, 0.05, or 0.1 ppm of amylase added to the wash for 40 minutes before being rinsed for 5 min under running water, dried and measured for intensity of the reflected light as an average of the RGB values. The values for the detergent base were subtracted and a ratio of the performance of Amylase B vs. that of Amylase A was made and a value above 100% indicates higher performance than the performance by the Amylase A.

In following Table 4 the average of at least 2 experiments is given.

| | 0 ppm | 0.01 ppm | 0.02 ppm | 0.04 ppm |
|---|---|---|---|---|
| EMPA161 | - | 182% | 159% | 165% |
| CS26 | - | 290% | 245% | 186% |
| CS28 | - | 289% | 206% | 147% |
| CS129 | - | 217% | 193% | 174% |
| CS29 | - | 284% | 277% | 257% |

From the tables above it becomes apparent that the Amylase B is superior over Amylase A in all tested detergents.

### Example 2

### Improved temperature stability of the alpha-amylase variants of the parent amylase

More stable variants were identified using a heat challenge assay. Herefore, the individual supernatants of *Bacillus licheniformis* expressing singular mutations of the alpha-amylase of Amylase B (tested in Example 1; SEQ ID NO: 1) were diluted on a 384 well plate using 100 mM HEPES (pH8) with Amylase B being the positive control. The dilutions were split into two plates with one plate being stored at 8°C representing the baseline activity and the other plate was incubated for 10 min at 92 °C representing the challenged values. For the activity measurement 3 µL of the samples are added to 27µL of a 1.1% Red starch solution (in 100 mM HEPES pH 8). The mix was incubated at room temperature for 10 min. The reaction was stopped by adding 60 µL ice cold ethanol (95%) and after centrifugation 40 µL of the supernatant was removed and added to a fresh 384 well plate. The absorbance at 510 nm was recorded. By dividing the absorbance value of the challenged sample with the absorbance value for the baseline sample the residual activity was calculated. The improvement factor is the ratio of the residual activity of the variant vs. the residual activity of the Amylase B (SEQ ID NO:1). In the table the amylase variants with elevated temperature stability towards the parent are given (numbering according to SEQ ID NO: 3).

The results are given in following Table 5:

| Mutation | IF | Mutation | IF | Mutation | IF | Mutation | IF |
|---|---|---|---|---|---|---|---|
| AmylaseB | 1 | A91H | 1.4 | R234C | 1.4 | V366I | 1.2 |
| H1G | 1.1 | A91T | 1.4 | I235M | 1.4 | V366L | 1.1 |
| H1V | 1.1 | A91I | 1.4 | I235V | 1.2 | V366T | 1.1 |
| H1R | 1.1 | A91W | 1.3 | I235L | 1.1 | F367S | 1.2 |
| H2S | 1.8 | A91N | 1.3 | D236Y | 1.1 | F367E | 1.1 |
| H2I | 1.2 | A91V | 1.3 | A237C | 1.5 | Y368N | 1.2 |
| N3Q | 1.3 | A91L | 1.2 | A237I | 1.1 | Y368L | 1.1 |
| N3K | 1.2 | A91C | 1.2 | V238T | 1.2 | Y368A | 1.1 |
| N3F | 1.2 | A91G | 1.1 | H240M | 1.1 | D370E | 1.2 |
| N3G | 1.1 | A91D | 1.1 | K242M | 1.2 | D370I | 1.2 |
| N3L | 1.1 | A91E | 1.1 | Y243F | 1.2 | Y372C | 1.6 |
| G4Q | 1.7 | A91M | 1.1 | F245E | 1.2 | Y372H | 1.2 |
| G4R | 1.2 | L92V | 1.5 | F245M | 1.1 | Y372A | 1.1 |
| G4S | 1.2 | L92M | 1.4 | F245H | 1.1 | Y372F | 1.1 |
| G4K | 1.1 | L92D | 1.1 | W249D | 1.1 | Y372M | 1.1 |
| G4M | 1.1 | K93K | 1.5 | W249I | 1.1 | Y372Q | 1.1 |
| G4A | 1.1 | K93Q | 1.2 | L250V | 1.1 | Y372N | 1.1 |
| G4C | 1.1 | S94M | 1.4 | T251E | 1.2 | Y372E | 1.1 |
| T5F | 3.8 | S94K | 1.2 | T251A | 1.1 | P375K | 1.8 |
| T5E | 1.9 | S94Y | 1.2 | T251L | 1.1 | P375A | 1.3 |
| T5M | 1.8 | S94D | 1.1 | T251S | 1.1 | P375D | 1.2 |
| T5Y | 1.7 | S94A | 1.1 | T251M | 1.1 | P375E | 1.1 |
| T5R | 1.5 | S94V | 1.1 | T251F | 1.1 | P375R | 1.1 |
| T5Q | 1.5 | N95V | 1.2 | H252C | 1.1 | P375T | 1.1 |
| T5K | 1.4 | N95I | 1.2 | H252I | 1.1 | P375I | 1.1 |
| T5L | 1.3 | N95E | 1.1 | H252S | 1.1 | P375W | 1.1 |
| T5A | 1.2 | N95L | 1.1 | V253Y | 1.2 | P375Y | 1.1 |
| T5V | 1.2 | G96N | 1.2 | V253G | 1.1 | P375Q | 1.1 |
| T5C | 1.2 | G96K | 1.1 | N255T | 1.1 | T376S | 1.1 |
| T5N | 1.2 | I97V | 1.1 | N255V | 1.1 | T376R | 1.1 |
| T5H | 1.2 | Q98G | 1.2 | N255D | 1.1 | T376M | 1.1 |
| T5D | 1.1 | Q98E | 1.1 | N255F | 1.1 | T376I | 1.1 |
| T5P | 1.1 | Q98N | 1.1 | N255I | 1.1 | T376V | 1.1 |
| T5I | 1.1 | V99K | 1.2 | T256C | 1.1 | T376Q | 1.1 |
| N6T | 3.4 | V99H | 1.1 | T257Y | 1.3 | T376L | 1.1 |
| N6E | 2.9 | V99N | 1.1 | T257L | 1.2 | G378E | 1.1 |
| N6A | 2.7 | Y100D | 1.3 | T257V | 1.1 | G378R | 1.1 |
| N6K | 2.5 | Y100K | 1.2 | K259L | 1.1 | G378D | 1.1 |
| N6Q | 2.3 | Y100L | 1.1 | M261R | 1.1 | G378C | 1.1 |
| N6V | 1.9 | Y100F | 1.1 | F262D | 1.4 | V379L | 1.8 |
| N6M | 1.9 | V103A | 1.1 | F262Y | 1.4 | V379A | 1.2 |
| N6G | 1.9 | N106D | 1.2 | F262C | 1.2 | V379S | 1.1 |
| N6L | 1.8 | K108A | 1.3 | F262P | 1.2 | A381V | 1.1 |
| N6H | 1.6 | G109A | 1.2 | F262E | 1.2 | A381E | 1.1 |
| N6W | 1.6 | A113Y | 2.3 | F262H | 1.2 | M382N | 1.4 |
| N6F | 1.6 | A113H | 2.1 | A263I | 1.3 | M382H | 1.2 |
| N6P | 1.5 | A113M | 1.7 | V264T | 1.2 | M382Q | 1.1 |
| N6S | 1.5 | A113V | 1.7 | V264W | 1.2 | M382K | 1.1 |
| N6C | 1.4 | A113F | 1.5 | V264H | 1.1 | M382A | 1.1 |
| N6Y | 1.4 | A113W | 1.2 | A265S | 1.4 | M382S | 1.1 |
| G7Y | 1.8 | A113R | 1.2 | W268G | 1.1 | K383R | 1.3 |
| G7S | 1.6 | T114D | 4.6 | N270A | 1.1 | K383I | 1.3 |
| G7H | 1.5 | T114H | 3.5 | N270Y | 1.1 | K383D | 1.3 |
| G7C | 1.5 | T114N | 3.2 | I272L | 1.2 | K383V | 1.2 |
| G7Q | 1.5 | T114W | 2.9 | I272G | 1.1 | K383M | 1.2 |
| G7F | 1.4 | T114E | 2.7 | G273H | 1.4 | K383E | 1.2 |
| G7P | 1.4 | T114C | 2.5 | G273V | 1.3 | K383N | 1.2 |
| G7W | 1.1 | T114G | 2.4 | G273L | 1.3 | K383S | 1.1 |
| G7R | 1.1 | T114L | 2.4 | G273M | 1.3 | K383Q | 1.1 |
| G7K | 1.1 | T114M | 2.3 | G273C | 1.3 | K383Y | 1.1 |
| G7E | 1.1 | T114Q | 2.1 | G273D | 1.3 | K383C | 1.1 |
| G7V | 1.1 | T114F | 2 | G273W | 1.3 | K383H | 1.1 |
| G7N | 1.1 | T114A | 1.9 | G273F | 1.2 | S384W | 1.3 |
| T8C | 2.1 | T114V | 1.7 | G273A | 1.2 | S384Y | 1.2 |
| T8V | 1.9 | T114K | 1.4 | G273E | 1.2 | S384V | 1.2 |
| T8F | 1.7 | T114S | 1.4 | G273P | 1.2 | S384R | 1.2 |
| T8Y | 1.6 | T114Y | 1.3 | G273Y | 1.1 | S384N | 1.2 |
| T8P | 1.4 | T114R | 1.2 | G273R | 1.1 | S384E | 1.2 |
| T8W | 1.4 | T114I | 1.1 | G273I | 1.1 | S384A | 1.1 |
| T8A | 1.3 | E115C | 5.5 | A274S | 1.4 | S384D | 1.1 |
| T8S | 1.3 | E115K | 5.3 | E276Y | 1.4 | S384T | 1.1 |
| T8M | 1.1 | E115N | 4.8 | E276K | 1.3 | S384C | 1.1 |
| T8I | 1.1 | E115T | 4.5 | E276L | 1.2 | S384F | 1.1 |
| M10W | 1.4 | E115M | 3.9 | E276D | 1.1 | S384L | 1.1 |
| M10Y | 1.3 | E115S | 3.7 | L279P | 1.7 | S384I | 1.1 |
| M10L | 1.2 | E115Q | 3.4 | S280I | 1.2 | S384M | 1.1 |
| M10C | 1.2 | E115R | 2.9 | S280V | 1.2 | S384Q | 1.1 |
| M10E | 1.2 | E115D | 1.7 | S280N | 1.2 | K385G | 1.6 |
| M10F | 1.2 | E115V | 1.3 | S280H | 1.1 | K385V | 1.5 |
| M10A | 1.1 | W116L | 1.1 | S280Y | 1.1 | K385T | 1.3 |
| Y12N | 2 | V117D | 2.7 | S280K | 1.1 | K385Y | 1.3 |
| Y12S | 1.1 | V117Y | 1.9 | S280R | 1.1 | K385I | 1.3 |
| F13A | 1.8 | V117N | 1.7 | W284Y | 1.4 | K385D | 1.2 |
| F13Y | 1.1 | V117C | 1.7 | W284A | 1.3 | K385C | 1.2 |
| E14N | 1.5 | V117R | 1.6 | W284L | 1.2 | K385Q | 1.2 |
| W15R | 1.2 | V117W | 1.5 | W284F | 1.2 | K385A | 1.2 |
| Y16F | 1.5 | V117A | 1.5 | W284H | 1.2 | K385W | 1.2 |
| Y16R | 1.1 | V117I | 1.4 | W284N | 1.1 | K385L | 1.2 |
| L17K | 1.2 | V117P | 1.3 | W284M | 1.1 | K385H | 1.2 |
| L17V | 1.1 | V117F | 1.3 | N285N | 1.5 | K385M | 1.1 |
| P18R | 1.7 | A119Y | 1.7 | N285L | 1.3 | K385N | 1.1 |
| P18M | 1.6 | A119R | 1.3 | N285G | 1.1 | K385S | 1.1 |
| P18F | 1.3 | A119P | 1.2 | N285P | 1.1 | K385F | 1.1 |
| P18K | 1.3 | A119H | 1.1 | H286Q | 1.1 | K385E | 1.1 |
| P18T | 1.1 | S125A | 2.9 | S287E | 1.1 | K385R | 1.1 |
| P18I | 1.1 | S125K | 2.5 | S287H | 1.1 | K385P | 1.1 |
| N19P | 3.3 | S125L | 2.4 | S287A | 1.1 | D387N | 1.2 |
| N19L | 2.7 | S125T | 2.4 | S287D | 1.1 | D387C | 1.1 |
| N19F | 2.6 | S125R | 2.3 | S287T | 1.1 | D387E | 1.1 |
| N19Y | 2.4 | S125F | 2.2 | V288K | 1.5 | P388R | 1.3 |
| N19T | 2.3 | S125W | 1.8 | V288P | 1.2 | P388F | 1.2 |
| N19I | 2.2 | S125Q | 1.8 | V288Y | 1.2 | P388H | 1.1 |
| N19Q | 2.1 | S125V | 1.7 | V288A | 1.1 | P388V | 1.1 |
| N19C | 2 | S125Y | 1.6 | F289T | 1.2 | P388E | 1.1 |
| N19H | 2 | S125H | 1.6 | F289R | 1.2 | P388M | 1.1 |
| N19S | 1.8 | S125N | 1.5 | F289G | 1.1 | P388I | 1.1 |
| N19M | 1.6 | S125E | 1.4 | F289F | 1.1 | I389G | 1.2 |
| N19K | 1.5 | S125G | 1.3 | D290Q | 3.5 | I389K | 1.2 |
| N19G | 1.4 | S125M | 1.3 | D290N | 2.1 | I389H | 1.1 |
| N19D | 1.3 | N126D | 1.5 | D290M | 1.6 | L390P | 1.3 |
| N19A | 1.1 | N128C | 2.6 | D290D | 1.3 | L390N | 1.3 |
| D20C | 3.8 | N128L | 2.2 | D290W | 1.1 | L390R | 1.2 |
| D20S | 2.8 | N128Y | 1.5 | V291D | 2.1 | L390F | 1.1 |
| D20L | 2.7 | N128E | 1.3 | V291Y | 1.8 | L390M | 1.1 |
| D20M | 2.5 | N128W | 1.3 | V291K | 1.3 | L390D | 1.1 |
| D20T | 2.5 | N128M | 1.2 | V291T | 1.3 | E391T | 1.4 |
| D20D | 2.4 | Q129E | 2.7 | P292W | 2.2 | E391F | 1.2 |
| D20Q | 2.3 | V131I | 2 | P292Y | 1.8 | E391S | 1.2 |
| D20F | 1.8 | V131C | 1.4 | P292F | 1.4 | E391A | 1.2 |
| D20P | 1.8 | S132T | 1.1 | P292D | 1.4 | E391G | 1.1 |
| D20G | 1.7 | G133E | 1.8 | P292L | 1.2 | E391M | 1.1 |
| D20V | 1.6 | G133P | 1.7 | P292I | 1.2 | E391N | 1.1 |
| D20W | 1.6 | G133C | 1.7 | P292T | 1.2 | E391Y | 1.1 |
| D20A | 1.6 | G133D | 1.5 | P292C | 1.1 | E391Q | 1.1 |
| D20Y | 1.4 | G133Q | 1.4 | L293D | 2.3 | E391K | 1.1 |
| D20H | 1.4 | G133S | 1.4 | L293R | 1.9 | A392C | 1.2 |
| D20E | 1.2 | G133H | 1.4 | L293K | 1.4 | A392V | 1.1 |
| G21S | 1.8 | G133A | 1.3 | L293E | 1.3 | R393V | 1.7 |
| G21E | 1.2 | G133N | 1.2 | L293F | 1.2 | R393E | 1.3 |
| N22E | 2 | G133K | 1.1 | H294T | 1.2 | R393H | 1.2 |
| N22F | 1.7 | D134Y | 2.7 | H294G | 1.1 | R393S | 1.1 |
| N22A | 1.7 | D134W | 2.1 | Y295F | 1.1 | R393P | 1.1 |
| N22D | 1.4 | D134F | 1.9 | N296Y | 1.2 | Q394C | 1.2 |
| N22M | 1.3 | D134P | 1.8 | N296L | 1.1 | Q394L | 1.2 |
| N22T | 1.3 | D134E | 1.8 | N296C | 1.1 | Q394R | 1.1 |
| N22K | 1.2 | D134V | 1.5 | N296A | 1.1 | Q394S | 1.1 |
| N22Y | 1.2 | D134L | 1.4 | L297M | 1.2 | Q394A | 1.1 |
| N22G | 1.2 | D134M | 1.3 | L297K | 1.1 | Q394E | 1.1 |
| N22R | 1.2 | D134T | 1.3 | L297S | 1.1 | Q394N | 1.1 |
| N22Q | 1.1 | D134C | 1.2 | L297H | 1.1 | Q394M | 1.1 |
| N22W | 1.1 | D134I | 1.1 | L297V | 1.1 | Q394H | 1.1 |
| N22L | 1.1 | Y135M | 1.2 | L297E | 1.1 | Q394I | 1.1 |
| H23N | 1.2 | T136D | 1.8 | L297F | 1.1 | K395V | 1.1 |
| H23Q | 1.1 | T136E | 1.6 | N299L | 1.5 | K395M | 1.1 |
| H23W | 1.1 | T136F | 1.3 | N299G | 1.3 | K395A | 1.1 |
| W24G | 1.2 | T136M | 1.3 | N299I | 1.2 | K395H | 1.1 |
| N25H | 3.1 | T136P | 1.2 | N299K | 1.2 | Y396P | 1.2 |
| N25Y | 3 | T136H | 1.2 | N299S | 1.1 | Y396H | 1.2 |
| N25K | 2.6 | T136C | 1.2 | N299Y | 1.1 | A397P | 1.4 |
| N25F | 2.2 | T136A | 1.1 | S301F | 1.3 | A397D | 1.3 |
| N25S | 1.9 | T136W | 1.1 | R302H | 1.4 | A397S | 1.1 |
| N25C | 1.7 | T136L | 1.1 | R302I | 1.3 | A397H | 1.1 |
| N25D | 1.6 | T136N | 1.1 | R302Q | 1.1 | Y398M | 1.1 |
| N25A | 1.6 | A139C | 1.5 | R302Y | 1.1 | G399P | 1.1 |
| N25G | 1.5 | T141V | 1.1 | R302V | 1.1 | T400L | 1.7 |
| N25W | 1.3 | K142C | 2.3 | S303P | 1.5 | T400P | 1.6 |
| N25Q | 1.2 | K142W | 1.8 | S303M | 1.4 | T400I | 1.5 |
| N25M | 1.2 | K142Y | 1.7 | S303E | 1.2 | T400S | 1.5 |
| N25L | 1.1 | K142L | 1.6 | S303I | 1.1 | T400A | 1.4 |
| R26S | 2.4 | K142M | 1.5 | S303R | 1.1 | T400R | 1.4 |
| R26V | 2.3 | K142Q | 1.5 | S303K | 1.1 | T400Q | 1.3 |
| R26M | 1.9 | K142F | 1.4 | S303N | 1.1 | T400D | 1.2 |
| R26A | 1.6 | K142E | 1.1 | S303L | 1.1 | T400K | 1.2 |
| R26D | 1.6 | K142R | 1.1 | S303H | 1.1 | T400N | 1.2 |
| R26E | 1.5 | F143F | 1.6 | S303T | 1.1 | T400V | 1.2 |
| R26F | 1.5 | D144Q | 1.5 | G304H | 1.4 | T400W | 1.1 |
| R26P | 1.3 | D144C | 1.5 | G304A | 1.4 | T400H | 1.1 |
| R26Y | 1.3 | D144V | 1.4 | G304R | 1.4 | T400E | 1.1 |
| R26L | 1.2 | D144K | 1.4 | G304Y | 1.2 | T400G | 1.1 |
| L27D | 4.6 | D144T | 1.4 | G304D | 1.2 | T400M | 1.1 |
| L27I | 4.6 | D144Y | 1.4 | G304N | 1.2 | Q401 M | 1.2 |
| L27F | 2.8 | D144E | 1.2 | G304M | 1.1 | Q401 K | 1.2 |
| L27R | 2.6 | D144G | 1.1 | G304E | 1.1 | Q401T | 1.1 |
| L27G | 2.5 | D144N | 1.1 | G304P | 1.1 | Q401I | 1.1 |
| L27Q | 2.4 | D144S | 1.1 | G304W | 1.1 | D403N | 1.1 |
| L27V | 2.2 | D144R | 1.1 | G304K | 1.1 | L405H | 1.2 |
| L27T | 2 | F145A | 1.1 | G304T | 1.1 | L405V | 1.1 |
| L27A | 1.6 | P146C | 2.2 | N306M | 1.3 | L405T | 1.1 |
| L27H | 1.4 | P146H | 1.7 | N306E | 1.3 | L405C | 1.1 |
| R28N | 3 | P146K | 1.6 | N306A | 1.3 | D406P | 1.1 |
| R28S | 2.6 | P146T | 1.6 | N306G | 1.2 | H407S | 1.1 |
| R28F | 2 | P146E | 1.3 | N306W | 1.2 | H407R | 1.1 |
| R28C | 1.7 | P146S | 1.3 | N306V | 1.2 | H407D | 1.1 |
| R28E | 1.6 | P146D | 1.3 | N306S | 1.2 | P408Q | 1.1 |
| R28T | 1.6 | P146W | 1.3 | N306I | 1.2 | P408E | 1.1 |
| R28G | 1.5 | P146F | 1.3 | N306H | 1.1 | P408I | 1.1 |
| R28K | 1.4 | P146L | 1.2 | N306T | 1.1 | V410R | 1.1 |
| R28D | 1.3 | P146G | 1.1 | N306D | 1.1 | V410K | 1.1 |
| R28V | | G147M | 1.5 | N306Q | 1.1 | V410I | 1.1 |
| R28I | 1.2 | G149E | 1.5 | N306Y | 1.1 | V410L | 1.1 |
| R28Q | 1.1 | N150Q | 1.6 | N306R | 1.1 | V410H | 1.1 |
| S29Y | 3.7 | N150E | 1.4 | Y307F | 1.4 | W413S | 1.3 |
| S29L | 3.5 | N150C | 1.4 | Y307M | 1.1 | T414E | 1.2 |
| S29P | 3.4 | T151C | 1.4 | D308S | 1.7 | T414C | 1.1 |
| S29W | 3.4 | T151E | 1.1 | M309H | 1.3 | T414S | 1.1 |
| S29K | 3.3 | N154Y | 1.4 | M309L | 1.2 | R415I | 1.1 |
| S29F | 2.7 | N154A | 1.1 | M309Q | 1.1 | R415E | 1.1 |
| S29N | 2.5 | F155Y | 1.1 | R310Q | 1.1 | E416S | 1.1 |
| S29H | 1.8 | K156V | 1.1 | R310A | 1.1 | D418C | 1.2 |
| S29D | 1.7 | Y160W | 1.2 | Q311G | 2 | D418P | 1.1 |
| S29V | 1.6 | H161T | 1.3 | Q311 E | 1.4 | D418N | 1.1 |
| S29G | 1.5 | F162V | 1.2 | Q311 H | 1.2 | H421P | 1.1 |
| S29Q | 1.3 | G164V | 1.6 | Q311A | 1.1 | H421N | 1.1 |
| S29I | 1.2 | V165W | 1.1 | Q311 K | 1.1 | | |
| D30F | 2.9 | Q169E | 1.2 | Q311T | 1.1 | | |
| D30I | 2.6 | Q169V | 1.1 | Q311 N | 1.1 | S424A | 1.2 |
| D30A | 2.3 | S170F | 1.2 | Q311 R | 1.1 | L426D | 1.3 |
| D30K | 2 | S170C | 1.1 | Q311Y | 1.1 | L426W | 1.2 |
| D30W | 1.8 | Q172C | 1.3 | I312M | 1.2 | A427Q | 2 |
| D30Y | 1.6 | Q172K | 1.2 | I312L | 1.1 | A427C | 1.9 |
| D30M | 1.5 | Q172A | 1.1 | F313V | 1.1 | A427F | 1.7 |
| D30L | 1.5 | L173I | 2.2 | N314C | 1.1 | A427T | 1.5 |
| D30H | 1.5 | L173Y | 1.1 | N314Q | 1.1 | A427V | 1.5 |
| D30Q | 1.4 | Q174P | 1.2 | G315C | 1.3 | A427G | 1.4 |
| D30G | 1.3 | Q174M | 1.1 | G315A | 1.2 | A427S | 1.3 |
| D30E | 1.2 | Q174E | 1.1 | G315H | 1.2 | A427R | 1.3 |
| D30T | 1.1 | Q174S | 1.1 | G315T | 1.1 | A427K | 1.2 |
| A31S | 1.8 | Q174H | 1.1 | G315E | 1.1 | L429M | 2.7 |
| A31V | 1.7 | N175G | 1.2 | G315K | 1.1 | L429D | 2.5 |
| A31Q | 1.6 | N175Q | 1.1 | V318T | 1.2 | L429E | 2.5 |
| A31T | 1.2 | N175H | 1.1 | V318S | 1.2 | L429N | 2.4 |
| A31N | 1.2 | R176K | 1.7 | V318I | 1.2 | L429T | 2.1 |
| A31W | 1.1 | G184G | 1.6 | Q319K | 1.3 | L429A | 2.1 |
| S32T | 3.3 | D188V | 1.1 | Q319H | 1.3 | L429P | 2.1 |
| S32H | 2.5 | D188H | 1.1 | Q319P | 1.2 | L429W | 2 |
| S32W | 2.4 | V191K | 1.3 | Q319A | 1.2 | L429Q | 1.9 |
| S32F | 2.3 | V191H | 1.2 | Q319I | 1.2 | L429S | 1.8 |
| S32Q | 2.2 | V191F | 1.2 | Q319D | 1.2 | L429F | 1.8 |
| S32I | 1.9 | V191W | 1.2 | Q319M | 1.1 | L429G | 1.4 |
| S32A | 1.9 | V191Y | 1.1 | Q319S | 1.1 | L429I | 1.4 |
| S32P | 1.8 | Y203E | 1.1 | Q319N | 1.1 | L429V | 1.1 |
| S32E | 1.6 | Y203R | 1.1 | Q319T | 1.1 | S431I | 1.2 |
| S32M | 1.6 | H210D | 2.8 | Q319W | 1.1 | G436E | 1.2 |
| S32N | 1.4 | H210C | 2.6 | R320G | 1.2 | K438P | 2.3 |
| S32D | 1.3 | H210E | 2 | R320Y | 1.2 | K438F | 1.4 |
| S32L | 1.2 | H210N | 1.6 | R320L | 1.2 | K438Y | 1.3 |
| N33Y | 2 | H210F | 1.5 | R320N | 1.1 | W439C | 1.2 |
| N33D | 1.3 | H210Y | 1.4 | R320A | 1.1 | W439P | 1.2 |
| N33M | 1.2 | H210M | 1.4 | R320D | 1.1 | M440V | 1.1 |
| N33Q | 1.2 | H210Q | 1.3 | R320K | 1.1 | V442Q | 1.5 |
| N33K | 1.2 | H210S | 1.3 | R320Q | 1.1 | G443T | 1.1 |
| N33R | 1.1 | H210A | 1.1 | R320E | 1.1 | G443H | 1.1 |
| L34H | 1.4 | P211 D | 1.7 | R320H | 1.1 | N445M | 1.3 |
| L34V | 1.3 | P211N | 1.6 | R320C | 1.1 | N445T | 1.3 |
| L34I | 1.2 | P211C | 1.6 | R320S | 1.1 | N445A | 1.3 |
| K35C | 3.9 | P211T | 1.5 | H321E | 1.3 | N445G | 1.2 |
| K35Y | 2.6 | P211E | 1.4 | H321W | 1.2 | N445S | 1.2 |
| K35P | 2.5 | P211G | 1.3 | H321T | 1.2 | N445F | 1.2 |
| K35T | 2.1 | P211V | 1.2 | H321A | 1.1 | N445R | 1.2 |
| K35M | 2 | P211Q | 1.2 | H321N | 1.1 | N445Q | 1.2 |
| K35H | 1.9 | P211A | 1.2 | H321V | 1.1 | N445C | 1.1 |
| K35A | 1.8 | P211H | 1.2 | H321K | 1.1 | N445D | 1.1 |
| K35V | 1.8 | P211L | 1.1 | T323K | 1.2 | N445K | 1.1 |
| K35I | 1.8 | P211S | 1.1 | T323G | 1.1 | N445H | 1.1 |
| K35G | 1.5 | E212W | 1.6 | H324W | 1.1 | N445V | 1.1 |
| K35Q | 1.4 | E212L | 1.2 | H324K | 1.1 | N446P | 1.4 |
| K35D | 1.4 | E212I | 1.2 | H324Y | 1.1 | N446I | 1.4 |
| K35N | 1.2 | E212D | 1.1 | V326Y | 1.3 | N446V | 1.3 |
| K35R | 1.2 | E212C | 1.1 | V326N | 1.2 | N446F | 1.2 |
| K35S | 1.1 | V213A | 1.5 | V326G | 1.1 | N446L | 1.2 |
| K35L | 1.1 | V213S | 1.4 | V326S | 1.1 | N446R | 1.2 |
| D36V | 1.3 | V213M | 1.3 | T327M | 1.2 | N446Q | 1.2 |
| D36I | 1.3 | V213R | 1.3 | T327C | 1.2 | N446W | 1.2 |
| D36T | 1.3 | V213C | 1.1 | T327L | 1.2 | A447V | 1.3 |
| D36K | 1.3 | V214P | 1.4 | D333I | 1.2 | G448E | 1.2 |
| D36A | 1.2 | V214E | 1.3 | P336K | 1.3 | G448H | 1.1 |
| D36S | 1.2 | N215E | 1.4 | E337N | 1.3 | G448N | 1.1 |
| D36R | 1.2 | N215D | 1.3 | E337K | 1.3 | G448D | 1.1 |
| D36M | 1.1 | N215H | 1.2 | E337M | 1.3 | E449V | 1.7 |
| D36E | 1.1 | N215W | 1.1 | E337T | 1.2 | E449Y | 1.6 |
| D36P | 1.1 | N215A | 1.1 | E337V | 1.2 | E449F | 1.5 |
| D36G | 1.1 | E216H | 1.2 | E337Q | 1.2 | E449L | 1.5 |
| D36N | 1.1 | E216G | 1.1 | E337S | 1.2 | E449P | 1.4 |
| D36Q | 1.1 | R218Q | 1.7 | E337R | 1.2 | E449G | 1.3 |
| K37P | 1.2 | R218E | 1.6 | E337I | 1.1 | E449W | 1.3 |
| K37V | 1.2 | R218C | 1.6 | E337Y | 1.1 | E449M | 1.2 |
| K37W | 1.2 | R218N | 1.5 | E337G | 1.1 | E449R | 1.2 |
| K37A | 1.1 | R218F | 1.5 | E337F | 1.1 | E449N | 1.2 |
| K37G | 1.1 | R218S | 1.4 | E337A | 1.1 | E449K | 1.2 |
| G38N | 1.2 | R218G | 1.4 | E337L | 1.1 | E449Q | 1.1 |
| I39E | 1.3 | R218K | 1.4 | E338G | 1.2 | E449S | 1.1 |
| I39K | 1.1 | R218I | 1.4 | E338T | 1.1 | E449A | 1.1 |
| T40I | 1.2 | R218T | 1.3 | E338S | 1.1 | W451L | 1.1 |
| T40S | 1.1 | R218D | 1.3 | E341V | 1.1 | D453G | 1.9 |
| A41G | 1.3 | R218Y | 1.3 | S342P | 1.1 | D453I | 1.3 |
| A41Q | 1.3 | R218H | 1.2 | F343W | 1.1 | D453P | 1.3 |
| A41C | 1.3 | R218L | 1.2 | F343Y | 1.1 | D453Y | 1.3 |
| A41D | 1.3 | R218V | 1.1 | V344I | 1.2 | D453N | 1.3 |
| A41E | 1.2 | R218A | 1.1 | V344Q | 1.2 | T455L | 1.1 |
| A41S | 1.1 | R218W | 1.1 | V344V | 1.1 | G456M | 1.3 |
| A41T | 1.1 | N219E | 1.4 | E345N | 1.3 | G456S | 1.3 |
| V42Q | 2.2 | N219Q | 1.4 | E345T | 1.3 | G456Y | 1.2 |
| V42V | 1.5 | N219K | 1.3 | E345M | 1.2 | G456V | 1.2 |
| V42I | 1.2 | N219T | 1.2 | E345G | 1.1 | G456L | 1.2 |
| V42C | 1.1 | N219M | 1.2 | E345S | 1.1 | G456W | 1.2 |
| P45N | 1.7 | N219D | 1.2 | E345D | 1.1 | G456R | 1.1 |
| P45G | 1.1 | N219F | 1.2 | E345Q | 1.1 | N457K | 1.6 |
| A47S | 1.2 | N219S | 1.1 | E346D | 1.3 | N457C | 1.6 |
| W48F | 1.5 | N219R | 1.1 | E346C | 1.1 | N457L | 1.5 |
| W48I | 1.1 | N219A | 1.1 | E346H | 1.1 | N457S | 1.5 |
| W48M | 1.1 | N219Y | 1.1 | E346A | 1.1 | N457R | 1.4 |
| A51T | 1.1 | N219H | 1.1 | E346G | 1.1 | N457F | 1.4 |
| G59T | 1.2 | N219W | 1.1 | E346Q | 1.1 | N457T | 1.3 |
| A60T | 1.5 | N219G | 1.1 | E346N | 1.1 | N457A | 1.3 |
| L63V | 1.8 | N219C | 1.1 | F348T | 1.1 | N457V | 1.3 |
| L63C | 1.4 | G221N | 1.1 | P350P | 1.2 | N457W | 1.3 |
| N70N | 1.2 | G221F | 1.1 | P350H | 1.1 | N457E | 1.3 |
| N70L | 1.1 | V222S | 1.1 | P350K | 1.1 | N457M | 1.2 |
| N70M | 1.1 | V222T | 1.1 | L351M | 1.1 | N457G | 1.1 |
| N70F | 1.1 | V222D | 1.1 | L351A | 1.1 | Q458V | 1.4 |
| N70Y | 1.1 | W223V | 1.1 | A352S | 1.1 | Q458W | 1.3 |
| Q71D | 1.5 | W223Y | 1.1 | Y353H | 1.2 | Q458I | 1.3 |
| K72T | 4.6 | W223L | 1.1 | A354Y | 2.5 | Q458K | 1.2 |
| K72E | 3 | W223C | 1.1 | A354N | 1.1 | T459Q | 1.2 |
| K72D | 1.8 | Y224V | 1.1 | A354T | 1.1 | T459D | 1.2 |
| K72N | 1.5 | Y224F | 1.1 | A354I | 1.1 | T459I | 1.2 |
| K72C | 1.3 | T225A | 1.5 | L355I | 1.1 | T459K | 1.2 |
| G73Q | 1.1 | T225R | 1.3 | L355M | 1.1 | T459C | 1.1 |
| G73L | 1.1 | T225N | 1.3 | L357A | 1.1 | T459V | 1.1 |
| G73N | 1.1 | T225E | 1.3 | T358P | 1.5 | T459E | 1.1 |
| V75A | 1.5 | T225H | 1.2 | R359E | 1.1 | T459Y | 1.1 |
| V75I | 1.3 | T225F | 1.1 | D360N | 1.5 | N460E | 1.2 |
| V75W | 1.1 | T225C | 1.1 | D360R | 1.5 | N460Q | 1.2 |
| V75P | 1.1 | T225Y | 1.1 | D360S | 1.2 | N460S | 1.2 |
| V75G | 1.1 | T225I | 1.1 | D360Y | 1.2 | N460R | 1.2 |
| V75T | 1.1 | N226C | 1.7 | D360V | 1.2 | N460A | 1.2 |
| R76G | 1.4 | N226W | 1.3 | D360L | 1.2 | N460T | 1.1 |
| R76T | 1.3 | N226I | 1.3 | D360A | 1.1 | N460D | 1.1 |
| R76C | 1.2 | N226R | 1.3 | D360I | 1.1 | N460V | 1.1 |
| R76V | 1.1 | N226Y | 1.3 | D360T | 1.1 | T461A | 1.3 |
| R76E | 1.1 | N226D | 1.2 | D360G | 1.1 | T461R | 1.3 |
| R76L | 1.1 | N226L | 1.2 | D360F | 1.1 | T461F | 1.2 |
| T81H | 1.1 | N226M | 1.2 | G362T | 1.5 | T461E | 1.2 |
| R82M | 1.1 | N226G | 1.2 | G362M | 1.3 | T461S | 1.2 |
| R82K | 1.1 | N226T | 1.1 | G362V | 1.2 | T461V | 1.1 |
| N83G | 1.2 | N226Q | 1.1 | G362N | 1.2 | T461M | 1.1 |
| N83R | 1.1 | N226S | 1.1 | G362Y | 1.1 | T461K | 1.1 |
| N83S | 1.1 | N226A | 1.1 | G362F | 1.1 | T461N | 1.1 |
| Q86K | 1.1 | N226V | 1.1 | G362K | 1.1 | T461Q | 1.1 |
| V89F | 1.6 | N226E | 1.1 | Y363M | 1.5 | V462K | 1.3 |
| V89A | 1.5 | N226H | 1.1 | Y363G | 1.4 | T463L | 1.1 |
| V89C | 1.4 | T227W | 1.5 | Y363S | 1.4 | N465P | 1.3 |
| V89L | 1.3 | T227C | 1.3 | Y363V | 1.4 | N465V | 1.1 |
| V89G | 1.1 | T227T | 1.3 | Y363C | 1.3 | N465H | 1.1 |
| V89M | 1.1 | T227R | 1.2 | Y363T | 1.3 | N465R | 1.1 |
| V89S | 1.1 | T227M | 1.2 | Y363H | 1.3 | D467A | 1.2 |
| V89R | 1.1 | T227K | 1.2 | Y363P | 1.3 | D467E | 1.1 |
| T90Q | 2.1 | T227Y | 1.2 | Y363D | 1.3 | D467H | 1.1 |
| T90F | 2.1 | T227V | 1.2 | Y363L | 1.2 | G468H | 1.4 |
| T90G | 1.9 | T227F | 1.2 | Y363R | 1.2 | G468D | 1.1 |
| T90E | 1.6 | T227I | 1.2 | Y363Q | 1.2 | G470Q | 1.6 |
| T90A | 1.5 | T227H | 1.1 | Y363E | 1.2 | G470T | 1.2 |
| T90N | 1.5 | T227L | 1.1 | Y363Y | 1.2 | G470A | 1.1 |
| T90I | 1.4 | T227G | 1.1 | Y363K | 1.2 | V474P | 1.8 |
| T90R | 1.4 | L230A | 1.5 | Y363W | 1.1 | V474H | 1.4 |
| T90Y | 1.4 | L230F | 1.2 | Y363A | 1.1 | V474F | 1.1 |
| T90V | 1.3 | D231N | 1.4 | P364G | 1.6 | S478A | 1.6 |
| T90S | 1.2 | D231C | 1.4 | P364T | 1.4 | S478E | 1.1 |
| T90M | 1.2 | F233T | 1.4 | P364S | 1.4 | S480Y | 1.9 |
| T90D | 1.1 | F233W | 1.4 | P364A | 1.3 | S480M | 1.3 |
| A91S | 1.8 | F233H | 1.2 | P364V | 1.3 | S480E | 1.3 |
| A91Y | 1.7 | F233C | 1.2 | P364C | 1.2 | S480D | 1.2 |
| A91K | 1.5 | F233I | 1.1 | P364K | 1.1 | S480R | 1.1 |
| A91Q | 1.4 | F233Y | 1.1 | P364L | 1.1 | Y482T | 1.8 |
| A91F | 1.4 | F233M | 1.1 | P364N | 1.1 | Y482C | 1.3 |

### Example 3

Storage stability of single point mutations of SEQ ID NO: 1 in a detergent according to Formulation A of Table 1a above at 40 °C relative to Amylase B (SEQ ID NO: 1).

To assess further if the improved heat stability is also connected to elevated stability in detergent, the variants of Amylase B (SEQ ID NO: 1) were formulated in a detergent and tested for their residual activity after 3 days of storage at 40°C.

Storage in liquid laundry detergent was done by incubating the amylases in the detergent according to Formulation A of Table 1a above. Therefore, 5 µL of diluted supernatant from *Bacillus licheniformis* expressing the amylases was added to 45 µL detergent. The mixture was incubated at 40°C and after indicated timepoints 5 µL of the detergent mix was removed and diluted using 45 µL MOPS buffer (50 mM, supplemented with 1 mM CaCl2 pH7.0). To 25 µL of this mix 25 µL of Infinity amylase reagent was added. The activity is the slope at 405nm calculated as the 5 point MaxV over 5 minutes. The residual activity was calculated by dividing the activity after storage time with the activity of the sample at time point zero. The residual activity was compared to the residual activity of the reference Amylase B (SEQ ID NO: 1).

In the Table 6 below the mutations with improved residual activity over Amylase B (SEQ ID NO: 1) are given (numbering according SEQ ID NO: 3).

**Table 6: Storage stability in detergent Formulation A at 40 °C relative to Amylase B (SEQ ID NO: 1). Residual activity after is given after 3 days of challenge.**

| Variant | Residual activity [%] | Variant | Residual activity [%] |
|---|---|---|---|
| V213C | 48 | N25Y | 11 |
| Y160W | 44 | T251E | 11 |
| N460G | 44 | N70H | 9 |
| P434S | 42 | Y398G | 7 |
| R176K | 40 | N25H | 6 |
| L405M | 40 | Q174H | 6 |
| E276R | 36 | S125K | 5 |
| T461L | 36 | D134F | 5 |
| H210C | 35 | H210D | 5 |
| Y368F | 34 | P292C | 5 |
| M382Q | 34 | S125R | 4 |
| Y372C | 33 | A139C | 4 |
| T323G | 32 | P364T | 4 |
| V131I | 31 | G73C | 3 |
| N6H | 28 | E115T | 3 |
| T459N | 28 | V291T | 3 |
| R28V | 27 | V318L | 3 |
| N33M | 27 | V326Y | 3 |
| G4Q | 26 | E338S | 3 |
| N6E | 26 | E338T | 3 |
| R359W | 26 | F343W | 3 |
| L27I | 24 | A352C | 3 |
| G38V | 21 | T356V | 3 |
| S94M | 21 | V379A | 3 |
| N175A | 19 | K385W | 3 |
| T81L | 18 | P388E | 3 |
| D134Y | 18 | R393Q | 3 |
| N299S | 17 | T400S | 3 |
| K72C | 15 | D418F | 3 |
| V75L | 15 | A447L | 3 |
| N128C | 15 | E449G | 3 |
| G96Q | 13 | N465H | 3 |
| T400A | 13 | SEQ ID NO: 1 | 2 |

### Example 4

Storage stability in ES1-C detergent at 40 °C for 7 days.

To assess further if the improved heat stability is also connected to elevated stability in detergent, the variants of Amylase B (SEQ ID NO: 1) were formulated in a detergent and tested for their residual activity after 7 days of storage at 40°C.
Storage in liquid laundry detergent was done by incubating the amylases in ES1-C (as described in Table 1b above) detergent. Therefore, 5 µL of diluted supernatant from *Bacillus licheniformis* expressing the amylases was added to 45 µL detergent. The mixture was incubated at 40°C and after indicated timepoints 5 µL of the detergent mix was removed and diluted using 45 µL MOPS buffer (50 mM, supplemented with 1 mM CaCl2 pH7.0). To 25 µL of this mix 25 µL of Infinity amylase reagent was added. The activity is the slope at 405nm calculated as the 5 point MaxV over 5 minutes. The residual activity was calculated by dividing the activity after storage time with the activity of the sample at time point zero. The residual activity was compared to the residual activity of the reference Amylase B (SEQ ID NO: 1). In the Table 7 below the mutations with improved residual activity over SEQ ID NO: 1 are given (Numbering according SEQ ID NO: 3).

**Table 7: Storage stability in detergent ES1-C at 40 °C relative to Amylase B (SEQ ID NO: 1). Residual activity after 3 days challenge.**

| Variant | Residual activity [%] | Variant | Residual activity [%] |
|---|---|---|---|
| V213C | 65 | H210D | 24 |
| N460G | 65 | N25H | 22 |
| Y160W | 59 | Q174H | 21 |
| P434S | 57 | D134F | 20 |
| R176K | 56 | P364T | 20 |
| T461L | 56 | S125K | 16 |
| E276R | 55 | S125R | 16 |
| L405M | 55 | P292C | 15 |
| T459N | 55 | V326Y | 15 |
| H210C | 54 | Y363M | 15 |
| Y368F | 54 | Y363S | 14 |
| M382Q | 52 | V318L | 13 |
| Y372C | 51 | A352C | 13 |
| T323G | 49 | T356V | 13 |
| N33M | 46 | G378E | 13 |
| S94M | 45 | K385W | 13 |
| D134Y | 45 | D387E | 13 |
| N6H | 43 | L390F | 13 |
| R28V | 43 | R393Q | 13 |
| L27I | 42 | D20K | 12 |
| G4Q | 41 | A41C | 12 |
| G38V | 41 | K93Q | 12 |
| N6E | 39 | L250I | 12 |
| T81L | 39 | V253C | 12 |
| N299S | 38 | N314Q | 12 |
| V75L | 37 | Q319K | 12 |
| G96Q | 35 | V379A | 12 |
| N175A | 34 | V379R | 12 |
| N126D | 31 | D387N | 12 |
| K72C | 30 | D387S | 12 |
| T400A | 30 | P388E | 12 |
| N25Y | 29 | P388K | 12 |
| R359W | 29 | P388Y | 12 |
| N128C | 28 | Y396S | 12 |
| T251E | 27 | P434R | 12 |
| N70H | 25 | T461A | 12 |
| | | SEQ ID NO: 1 | 11 |

### Example 5

### Combinatorial library of stabilizing mutations

Mutations in Amylase B (SEQ ID NO: 1) were combined to create amylases with multiple mutations over Amylase B (SEQ ID NO: 1). To assess further if the improved heat stability is also connected to elevated stability in detergent, the variants of Amylase B (SEQ ID NO: 1) were formulated in a detergent according to Formulation A of Table 1a above and tested for their residual activity after indicated timepoints. Storage occurred at 40° or 50°C with or without the addition of a protease (if added: 0.3 g/L of subtilisin protease from *Bacillus lentus* (BLAP) comprising R101E mutation (BPN' numbering)).
Storage in the liquid laundry detergent was done by incubating the amylases in detergent. Therefore, 25 µL of diluted supernatant from *Bacillus licheniformis* expressing the amylases was added to 475 µL detergent. The mixture was incubated at 40°C and after indicated timepoints 10 µL of the detergent mix was removed and diluted using 90 µL MOPS buffer (50 mM, supplemented with 1 mM CaCl2 pH7.0). To 50 µL of this mix 50 µL of Infinity amylase reagent was added. The activity is the slope at 405nm calculated as the 5 point MaxV over 5 minutes. The residual activity was calculated by dividing the activity after storage time with the activity of the sample at time point zero. The residual activity was compared to the residual activity of the reference Amylase B (SEQ ID NO: 1). The numbering of following tables is according to SEQ ID NO: 3.

In the Table 8 below results are shown for the residual activity after 28 days storage, stored at 40°C in presence of the protease.

| Variants over the parent amylase (SEQ ID NO: 1) | Residual activity [%] |
|---|---|
| Parent (SEQ ID NO:1) | 1% |
| N25H+R176K+G186E+I206Y+T251E+Y482W | 97% |
| G4Q+N25H+R176K+G186E+I206Y+T251 E+L405M+Y482W | 98% |
| N25H+R176K+G186E+I206Y+Y482W | 97% |
| N25H+G186E+I206Y+L405M+Y482W | 94% |
| N25H+G186E+I206Y+Y482W | 95% |
| N25H+R176K+G186E+T193E+I206Y+T251E+Y482W | 97% |
| G4Q+R176K+G186E+T193E+I206Y+T251 E+L405M+Y482W | 89% |
| N25H+R176K+G186E+Y482W | 78% |
| N25H+R176K+G186E+T193E+I206Y+Y482W | 93% |
| G4Q+N25H+R176K+G186E+T251E+L405M+Y482W | 74% |
| N25H+R176K+G186E+I206Y | 96% |
| G4Q+R176K+G186E+T193E+I206Y+T251E+L405M | 87% |
| G4Q+I206Y+N460G+Y482W | 16% |
| G4Q+N25H+R176K+I206Y+T251E+N460G+Y482W | 61% |
| G4Q+N25H+R176K+T193E+T251E+G186E+Y482W | 27% |
| G4Q+T193E+I206Y+E276R+G186E+Y482W | 76% |
| G186E+N25H+Y482W | 52% |
| N25H+T193E+I206Y+T251E+E276R+L405M+G186E+Y482W | 69% |
| N25H+T251E+G186E+Y482W | 53% |
| N25H+Y160W+R176K+G186E+T251E+L405M+Y482W | 70% |
| T193E+I206Y+L405M+G186E+Y482W | 75% |
| G4Q+G7W+R176K+G186E+ T193E+I206Y+T251 E+L405M | 55% |
| G4Q+K37M+R176K+G186E+T193E+I206Y+T251E+L405M | 52% |
| G4Q+N25D+R176K+G186E+T193E+I206Y+T251E+L405M | 63% |
| G4Q+N25H+R176K+G186E+I206Y+T251E+L405M+N460G | 87% |
| G4Q+N25H+R176K+G186E+I206Y+T251E+N460G | 85% |
| G4Q+N25Y+R176K+G186E+T193E+I206Y+T251E+L405M | 62% |
| G4Q+R118D+R176K+G186E+T193E+I206Y+T251E+L405M | 45% |
| G4Q+R176K+G182D+G186E+T193E+I206Y+T251E+L405M | 19% |
| G4Q+R176K+G186E+T193E+I206Y+T251E+G258Q+L405M | 46% |
| G4Q+R176K+G186E+T193E+I206Y+T251E+L405M | 49% |
| G4Q+R176K+G186E+T193E+I206Y+T251E+L405M+N460G | 47% |
| G4Q+R176K+G186E+T193E+I206Y+T251E+Y363E+L405M | 49% |
| G4Q+R176K+G186E+T193E+I206Y+T251E+Y363H+L405M | 48% |
| G4Q+R176K+G186E+T193E+I206Y+T251E+Y363N+L405M | 45% |
| G4Q+R176K+R181D+G186E+T193E+I206Y+T251 E+L405M | 56% |
| G4Q+R176K+R181F+G186E+T193E+I206Y+T251E+L405M | 52% |
| G4Q+R176K+R181N+G186E+T193E+I206Y+T251E+L405M | 50% |
| G4Q+R176K+R181Q+G186E+T193E+I206Y+T251E+L405M | 44% |
| G4Q+T136E+R176K+G186E+T193E+I206Y+T251E+L405M | 43% |
| G4Q+Y100W+R176K+G186E+T193E+I206Y+T251E+L405M | 50% |
| G4Q+Y135D+R176K+G186E+T193E+I206Y+T251E+L405M | 19% |
| G4Q+Y135E+R176K+G186E+T193E+I206Y+T251E+L405M | 12% |
| G4Q+Y135T+R176K+G186E+T193E+I206Y+T251E+L405M | 12% |
| G4Q+Y135W+R176K+G186E+T193E+I206Y+T251E+L405M | 6% |
| G4Q+Y160D+R176K+G186E+T193E+I206Y+T251E+L405M | 29% |
| G4Q+Y160E+R176K+G186E+T193E+I206Y+T251E+L405M | 29% |
| G7H+N25H+R176K+G186E+I206Y | 84% |
| G7W+N25H+R176K+G186E+I206Y | 82% |
| N25D+R176K+G186E+I206Y | 84% |
| N25H+G186E+I206Y+L405M+N460G | 77% |
| N25H+G186E+I206Y+N460G | 73% |
| N25H+K37M+R176K+G186E+I206Y | 82% |
| N25H+R118D+R176K+G186E+I206Y | 84% |
| N25H+R176K+G182D+G186E+I206Y | 77% |
| N25H+R176K+G186E+I206Y | 49% |
| N25H+R176K+G186E+I206Y+G258Q | 86% |
| N25H+R176K+G186E+I206Y+K281N | 32% |
| N25H+R176K+G186E+I206Y+N460G | 88% |
| N25H+R176K+G186E+I206Y+T251E+N460G | 86% |
| N25H+R176K+G186E+I206Y+Y363E | 85% |
| N25H+R176K+G186E+I206Y+Y363H | 87% |
| N25H+R176K+G186E+I206Y+Y363N | 87% |
| N25H+R176K+G186E+N460G | 34% |
| N25H+R176K+G186E+T193E+I206Y | 68% |
| N25H+R176K+G186E+T193E+I206Y+N460G | 61% |
| N25H+R176K+G186E+T193E+I206Y+T251E+N460G | 62% |
| N25H+R176K+R181D+G186E+I206Y | 87% |
| N25H+R176K+R181N+G186E+I206Y | 85% |
| N25H+R176K+R181Q+G186E+I206Y | 81% |
| N25H+T136E+R176K+G186E+I206Y | 82% |
| N25H+Y100W+R176K+G186E+I206Y | 84% |
| N25H+Y135E+R176K+G186E+I206Y | 72% |
| N25H+Y135T+R176K+G186E+I206Y | 74% |
| N25H+Y135W+R176K+G186E+I206Y | 70% |
| N25H+Y160D+R176K+G186E+I206Y | 70% |
| N25H+Y160E+R176K+G186E+I206Y | 78% |
| N25Y+R176K+G186E+I206Y | 85% |

In the Table 9 below results are shown for the residual activity after 7 days storage in the detergent, stored at 40°C in absence of the protease.

| Variants over the parent amylase (SEQ ID NO: 1) | Residual activity [%] |
|---|---|
| Parent | 21% |
| N3I+T356V | 23% |
| N3I+T356I | 24% |
| N83D+S94E | 24% |
| S94D+S125E | 25% |
| V131I+H377Q+V410H | 25% |
| W48F+S94D | 27% |
| W48Y+W116K+R218K | 32% |
| T5L+R218K+T225S | 33% |
| N83E+W116R+R158Y+R181E | 34% |
| A51V+R218K | 35% |
| IN83E+R181E | 43% |

In the Table 10 below results are shown for the residual activity after 7 days storage in the detergent, stored at 40°C in presence of the protease (0.3 g/L of protease in detergent).

| Variants over the parent amylase (SEQ ID NO: 1) | Residual activity [%] |
|---|---|
| Parent | 1% |
| A87D+G186E+G315K+V420K | 45% |
| A87D+G186E+N226D+N314E+V420E+T461E | 43% |
| A87D+G186E+N226D+V420E | 41% |
| A87D+G186E+N226D+V420E+T461E | 42% |
| A87D+G186E+N314E+Q471E | 41% |
| A87D+G186E+Q311K+N314E+V420K | 40% |
| A87D+G186E+Y160D+T461E | 30% |
| A87R+G186E+G315K+T461R | 43% |
| A87R+G186E+N226D+Q471E | 42% |
| A87R+G186E+N226R+N314K+V420K+T461R | 39% |
| A87R+G186E+N226R+Q311K+V420K | 45% |
| A87R+G186E+N314K+G315K | 22% |
| A87R+G186E+Q311K+N314E | 44% |
| A87R+G186E+V420E+V450R | 40% |
| A87R+G186E+V450R+Y452R | 41% |
| G186E+G315K+V420E+Y452R | 47% |
| G186E+N226D+N314E+T461E+Q471E | 39% |
| G186E+N226D+N314E+Y452R | 43% |
| G186E+N226D+N314K+N460D+Q471E+Q485R | 46% |
| G186E+N226D+Q311K+N460D | 42% |
| G186E+N226D+T461E+Q471E | 41% |
| G186E+N314E+N460D+T461E | 43% |
| G186E+N314E+V420E+Y452R+T461E | 40% |
| G186E+N314K+V450R+N460D | 39% |
| G186E+N460D+Q471E+Q485R | 41% |
| G186E+Q311K+N314K+Y452R | 32% |
| G186E+Q311K+T461E+Q485R | 40% |
| G186E+Q311K+V420E+Q471E | 40% |
| G186E+V420K+V450R+Q471E+Q485R | 42% |
| G186E+V420K+V450R+Q485R | 40% |
| G186E+Y452R+T461E+Q471E | 40% |
| G4Q+R176K+G186E+T193E+I206Y+T251E+L405M | 85% |
| N25H+R176K+G186E+1206Y | 95% |
| N83E+G186E+N219D+N226R | 38% |
| N83E+G186E+N219R+N226D+N314E+Y452R | 48% |
| N83E+G186E+N219R+N226R | 42% |
| N83E+G186E+Y160D+N219D+N226R+Y452R | 27% |
| N83R+G186E+N219R+N226D | 42% |
| Y160D+G186E+G315K+V450R | 37% |
| Y160D+G186E+N226D+N314K+N460D+T461E | 30% |
| Y160D+G186E+N226D+N314K+V420E | 31% |
| Y160D+G186E+N314K+Q485R | 32% |
| Y160D+G186E+V420E+Y452R | 26% |
| Y160D+G186E+V420K+N460D | 32% |

In the Table 11 below results are shown for the residual activity after 8 days storage in the detergent, stored at 50°C in absence of the protease.

| Variants over the parent amylase (SEQ ID NO: 1) | Residual activity [%] |
|---|---|
| Parent | 1% |
| G186E+E276R | 12% |
| G186E+I206Y | 64% |
| G186E+I206Y+E276R | 81% |
| G186E+I206Y+E276R+L405M | 78% |
| G186E+I206Y+L405M | 80% |
| G186E+I206Y+T251E | 81% |
| G186E+I206Y+T251E+E276R | 77% |
| G186E+I206Y+T251 E+E276R+L405M | 82% |
| G186E+I206Y+T251 E+L405M | 75% |
| G186E+I206Y+T251E+T323G+L405M | 80% |
| G186E+I206Y+T323G+L405M | 77% |
| G186E+L405M | 23% |
| G186E+T193E+I206Y+L405M | 60% |
| G186E+T193E+I206Y+T251E | 50% |
| G186E+T251E | 11% |
| G186E+T251E+L405M | 23% |
| G4Q+G186E | 12% |
| G4Q+G186E+E276R | 12% |
| G4Q+G186E+I206Y | 76% |
| G4Q+G186E+I206Y+E276R+L405M | 77% |
| G4Q+G186E+I206Y+L405M | 74% |
| G4Q+G186E+I206Y+T251E+E276R | 78% |
| G4Q+G186E+I206Y+T251E+E276R+L405M | 70% |
| G4Q+G186E+I206Y+T251 E+L405M | 78% |
| G4Q+G186E+I206Y+T251E+T323G+L405M | 78% |
| G4Q+G186E+L405M | 14% |
| G4Q+G186E+T193E+I206Y+E276R | 40% |
| G4Q+G186E+T193E+I206Y+L405M | 61% |
| G4Q+G186E+T193E+I206Y+T251E+L405M | 47% |
| G4Q+G186E+T251E+E276R+L405M | 14% |
| G4Q+N25H+G186E+I206Y | 83% |
| G4Q+N25H+G186E+I206Y+E276R | 85% |
| G4Q+N25H+G186E+I206Y+E276R+L405M | 79% |
| G4Q+N25H+G186E+I206Y+L405M | 79% |
| G4Q+N25H+G186E+I206Y+T251E+T323A+L405M | 85% |
| G4Q+N25H+G186E+I206Y+T323G+L405M | 78% |
| G4Q+N25H+G186E+L405M | 13% |
| G4Q+N25H+R176K+G186E+I206Y+E276R | 99% |
| G4Q+N25H+R176K+G186E+I206Y+E276R+L405M | 91% |
| G4Q+N25H+R176K+G186E+I206Y+T251E+E276R+L405M | 94% |
| G4Q+N25H+R176K+G186E+I206Y+T251E+L405M | 95% |
| G4Q+N25H+R176K+G186E+T251E | 58% |
| G4Q+N25H+R176K+G186E+T251E+L405M | 54% |
| G4Q+N25H+R176K+I206Y | 18% |
| G4Q+N25H+R176K+I206Y+L405M N460G | 19% |
| G4Q+N25H+R176K+I206Y+N460G | 18% |
| G4Q+N25H+R176K+I206Y+T251 E | 19% |
| G4Q+N25H+R176K+I206Y+T251 E+E276R+L405M | 14% |
| G4Q+N25H+R176K+I206Y+T251E+N460G | 19% |
| G4Q+N25H+R176K+I206Y+T323G | 18% |
| G4Q+N25H+Y160W+G186E+I206Y+L405M | 74% |
| G4Q+N25H+Y160W+G186E+I206Y+T251 E | 84% |
| G4Q+N25H+Y160W+G186E+I206Y+T251E+E276R+L405M | 82% |
| G4Q+N25H+Y160W+G186E+I206Y+T323G+L405M | 82% |
| G4Q+N25H+Y160W+G186E+L405M | 34% |
| G4Q+N25H+Y160W+G186E+T193E+I206Y+E276R | 51% |
| G4Q+N25H+Y160W+G186E+T323G | 28% |
| G4Q+N25H+Y160W+Q169R+G186E+I206Y+E276R | 82% |
| G4Q+N25H+Y160W+R176K+G186E+E276R+L405M | 55% |
| G4Q+N25H+Y160W+R176K+G186E+I206Y+E276R | 91% |
| G4Q+N25H+Y160W+R176K+G186E+I206Y+T251E | 91% |
| G4Q+N25H+Y160W+R176K+G186E+I206Y+T251 E+L405M | 95% |
| G4Q+N25H+Y160W+R176K+G186E+L405M | 59% |
| G4Q+N25H+Y160W+R176K+G186E+T251 E+E276R+L405M | 52% |
| G4Q+N25H+Y160W+R176K+G186E+T251 E+L405M | 60% |
| G4Q+N25H+Y160W+R176K+I206Y | 24% |
| G4Q+N25H+Y160W+R176K+I206Y+L405M | 23% |
| G4Q+N25H+Y160W+R176K+I206Y+N460G | 19% |
| G4Q+N25H+Y160W+R176K+I206Y+T251 E+N460G | 23% |
| G4Q+R176K+G186E+E276R+L405M | 53% |
| G4Q+R176K+G186E+I206Y+E276R | 88% |
| G4Q+R176K+G186E+I206Y+E276R+L405M | 88% |
| G4Q+R176K+G186E+I206Y+L405M | 84% |
| G4Q+R176K+G186E+I206Y+T251 E+E276R+L405M | 82% |
| G4Q+R176K+G186E+I206Y+T251 E+L405M | 88% |
| G4Q+R176K+G186E+L405M | 40% |
| G4Q+R176K+G186E+T193E+I206Y+E276R+L405M | 69% |
| G4Q+R176K+G186E+T193E+I206Y+T251E+L405M | 74% |
| G4Q+R176K+G186E+T251E+E276R+L405M | 50% |
| G4Q+R176K+G186E+T251E+L405M | 47% |
| G4Q+R176K+G186E+T323G+L405M | 49% |
| G4Q+R176K+I206Y | 18% |
| G4Q+R176K+I206Y+E276R | 14% |
| G4Q+R176K+I206Y+L405M | 18% |
| G4Q+R176K+I206Y+L405M N460G | 17% |
| G4Q+R176K+I206Y+N460G | 18% |
| G4Q+R176K+I206Y+T251 E+E276R+L405M | 16% |
| G4Q+R176K+I206Y+T251 E+E276R+L405M N460G | 18% |
| G4Q+R176K+I206Y+T251E+L405M | 14% |
| G4Q+R176K+I206Y+T251 E+L405M N460G | 16% |
| G4Q+R176K+I206Y+T251E+T323A+N460G | 17% |
| G4Q+Y160W+G186E+E276R+L405M | 16% |
| G4Q+Y160W+G186E+I206Y | 84% |
| G4Q+Y160W+G186E+I206Y+E276R+T323G | 73% |
| G4Q+Y160W+G186E+I206Y+L405M | 75% |
| G4Q+Y160W+G186E+I206Y+T251 E | 77% |
| G4Q+Y160W+G186E+I206Y+T251 E+E276R+L405M | 78% |
| G4Q+Y160W+G186E+L405M | 12% |
| G4Q+Y160W+G186E+T251 E+L405M | 28% |
| G4Q+Y160W+R176K+G186E | 14% |
| G4Q+Y160W+R176K+G186E+E276R+L405M | 65% |
| G4Q+Y160W+R176K+G186E+I206Y+T251E | 84% |
| G4Q+Y160W+R176K+G186E+T251 E+E276R+L405M | 54% |
| G4Q+Y160W+R176K+G186E+T251E+L405M | 47% |
| G4Q+Y160W+R176K+I206Y | 13% |
| G4Q+Y160W+R176K+I206Y+T251 E+E276R+N460G | 19% |
| G4Q+Y160W+R176K+I206Y+T251E+T323G+L405M+N460G | 22% |
| N25H+G186E | 17% |
| N25H+G186E+I206Y | 85% |
| N25H+G186E+I206Y+E276R+L405M | 84% |
| N25H+G186E+I206Y+E276R+T323G | 85% |
| N25H+G186E+I206Y+L405M | 82% |
| N25H+G186E+I206Y+T251E+E276R+T323G+L405M | 81% |
| N25H+G186E+I206Y+T251E+L405M | 82% |
| N25H+G186E+L405M | 28% |
| N25H+G186E+M202I+I206Y+L405M | 83% |
| N25H+G186E+T193E+I206Y+E276R+L405M | 44% |
| N25H+G186E+T193E+I206Y+L405M | 54% |
| N25H+G186E+T193E+I206Y+T251E+E276R+L405M | 35% |
| N25H+G186E+T251E | 18% |
| N25H+G186E+T251E+E276R | 17% |
| N25H+R176K+G186E | 61% |
| N25H+R176K+G186E+E276R+T323G+L405M | 55% |
| N25H+R176K+G186E+I206Y | 91% |
| N25H+R176K+G186E+I206Y+E276R | 94% |
| N25H+R176K+G186E+I206Y+T251E | 95% |
| N25H+R176K+G186E+I206Y+T251E+E276R+L405M | 93% |
| N25H+R176K+G186E+I206Y+T251E+L405M | 95% |
| N25H+R176K+G186E+L405M | 61% |
| N25H+R176K+G186E+T193E+I206Y | 76% |
| N25H+R176K+G186E+T193E+1206Y+T251E | 77% |
| N25H+R176K+G186E+T193E+I206Y+T251E+E276R+L405M | 77% |
| N25H+R176K+G186E+T251E+E276R | 58% |
| N25H+R176K+I206Y | 19% |
| N25H+R176K+I206Y+E276R | 17% |
| N25H+R176K+I206Y+E276R+L405M | 17% |
| N25H+R176K+I206Y+E276R+N460G | 15% |
| N25H+R176K+I206Y+L405M | 20% |
| N25H+R176K+I206Y+N460G | 20% |
| N25H+R 176K+I206Y+T251 E | 18% |
| N25H+R176K+I206Y+T251E+E276R+L405M+N460G | 17% |
| N25H+R176K+I206Y+T251E+E276R+N460G | 17% |
| N25H+R176K+I206Y+T251E+L405M | 18% |
| N25H+Y160W+G186E+I206Y | 83% |
| N25H+Y160W+G186E+I206Y+T251 E | 83% |
| N25H+Y160W+G186E+I206Y+T251 E+E276R | 81% |
| N25H+Y160W+G186E+I206Y+T251 E+E276R+T323G+L405M | 84% |
| N25H+Y160W+G186E+I206Y+T251 E+L405M | 85% |
| N25H+Y160W+G186E+I206Y+T323G+L405M | 82% |
| N25H+Y160W+G186E+T251E+E276R+L405M | 15% |
| N25H+Y160W+R176K+G186E+I206Y+E276R | 84% |
| N25H+Y160W+R176K+G186E+I206Y+E276R+L405M | 90% |
| N25H+Y160W+R176K+G186E+I206Y+T251E+E276R+T323G | 89% |
| N25H+Y160W+R176K+G186E+I206Y+T251E+L405M | 93% |
| N25H+Y160W+R176K+G186E+L405M | 59% |
| N25H+Y160W+R176K+G186E+T193E+I206Y | 76% |
| N25H+Y160W+R176K+G186E+T193E+I206Y+L405M | 80% |
| N25H+Y160W+R176K+G186E+T193E+I206Y+T251E+L405M | 80% |
| N25H+Y160W+R176K+G186E+T251E | 56% |
| N25H+Y160W+R176K+G186E+T251E+E276R | 59% |
| N25H+Y160W+R176K+G186E+T251E+L405M | 59% |
| N25H+Y160W+R176K+G186E+T323G+L405M | 45% |
| N25H+Y160W+R176K+I206Y+E276R+T323G+L405M | 18% |
| N25H+Y160W+R176K+I206Y+N460G | 23% |
| N25H+Y160W+R176K+I206Y+T251E | 26% |
| N25H+Y160W+R176K+I206Y+T251E+T323G+L405M | 23% |
| R176K+G186E+I206Y | 91% |
| R176K+G186E+I206Y+E276R | 90% |
| R176K+G186E+I206Y+E276R+L405M | 92% |
| R176K+G186E+I206Y+L405M | 89% |
| R176K+G186E+I206Y+T251E+L405M | 88% |
| R176K+G186E+L405M | 51% |
| R176K+G186E+T193E+I206Y+L405M | 74% |
| R176K+G186E+T193E+I206Y+T251E+L405M | 75% |
| R176K+G186E+T193E+L405M | 2% |
| R176K+G186E+T251E+E276R+L405M | 43% |
| R176K+G186E+T251E+L405M | 53% |
| R176K+I206Y | 19% |
| R176K+I206Y+E276R | 14% |
| R176K+I206Y+E276R+N460G | 14% |
| R176K+I206Y+L405M | 18% |
| R176K+I206Y+L405M N460G | 17% |
| R176K+I206Y+N460G | 18% |
| R176K+I206Y+T251E | 17% |
| R176K+I206Y+T251E+L405M | 19% |
| Y160W+G186E | 16% |
| Y160W+G186E+E276R+L405M | 18% |
| Y160W+G186E+I206Y+E276R | 81% |
| Y160W+G186E+I206Y+E276R+L405M | 81% |
| Y160W+G186E+I206Y+L405M | 79% |
| Y160W+G186E+I206Y+T251 E | 76% |
| Y160W+G186E+I206Y+T251E+L405M | 79% |
| Y160W+G186E+L405M | 69% |
| Y160W+G186E+ T193E+I206Y | 46% |
| Y160W+G186E+T193E+I206Y+L405M | 48% |
| Y160W+G186E+T251E+E276R | 21% |
| Y160W+G186E+T251E+E276R+L405M | 21% |
| Y160W+G186E+T323G+L405M | 22% |
| Y160W+R176K+G186E+E276R+L405M | 53% |
| Y160W+R176K+G186E+I206Y | 79% |
| Y160W+R176K+G186E+I206Y+T251E+L405M | 87% |
| Y160W+R176K+G186E+L405M | 53% |
| Y160W+R176K+G186E+T193E+I206Y | 76% |
| Y160W+R176K+G186E+T193E+I206Y+L405M | 72% |
| Y160W+R176K+G186E+T193E+I206Y+T251E | 77% |
| Y160W+R176K+I206Y+E276R+L405M N460G | 19% |
| Y160W+R176K+I206Y+E276R+T323G+N460G | 18% |
| Y160W+R176K+I206Y+L405M | 21% |
| Y160W+R176K+I206Y+L405M N460G | 21% |
| Y160W+R176K+I206Y+N460G | 17% |
| Y160W+R176K+I206Y+T251 E+E276R | 18% |
| Y160W+R176K+I206Y+T251 E+L405M+N460G | 24% |
| Y160W+ R176K+I206Y+T323G+L405M+N460G | 22% |

In the Table 12 below results are shown for the residual activity after 7 days storage in the detergent, stored at 40°C in absence of the protease.

| Variants over the parent amylase (SEQ ID NO: 1) | Residual activity [%] |
|---|---|
| Parent | 1% |
| G4Q+R176K+G186E+T193E+I206Y+T251E+L405M | 86% |
| G4Q+R176K+G186E+T193E+I206Y+T251E+L405M+G258Q | 85% |
| G4Q+R176K+G186E+T193E+I206Y+T251 E+L405M+G258Q+K281N | 30% |
| G4Q+R176K+G186E+T193E+I206Y+T251E+L405M+G258Q+K281N+Y363H | 31% |
| G4Q+R176K+G186E+T193E+I206Y+T251E+L405M+G258Q+Y363E | 87% |
| G4Q+R176K+G186E+T193E+I206Y+T251 E+L405M+K281N | 63% |
| G4Q+R176K+G186E+T193E+I206Y+T251E+L405M+K281N+Y363E | 38% |
| G4Q+R176K+G186E+T193E+I206Y+T251E+L405M+K281N+Y363H | 53% |
| G4Q+R176K+G186E+T193E+I206Y+T251 E+L405M+R181 Q | 86% |
| G4Q+R176K+G186E+T193E+I206Y+T251 E+L405M+R181 Q+G258Q | 87% |
| G4Q+R176K+G186E+T193E+I206Y+T251 E+L405M+R181 Q+G258Q+K281 N | 30% |
| G4Q+R176K+G186E+T193E+I206Y+T251 E+L405M+R181 Q+G258Q+K281 N+ Y363H | 35% |
| G4Q+R176K+G186E+T193E+I206Y+T251 E+L405M+R181 Q+K281 N | 35% |
| G4Q+R176K+G186E+T193E+I206Y+T251 E+L405M+R181 Q+K281 N+Y363E | 34% |
| G4Q+R176K+G186E+T193E+I206Y+T251 E+L405M+R181 Q+Y363E | 88% |
| G4Q+R176K+G186E+T193E+I206Y+T251 E+L405M+R181 Q+Y363H | 91% |
| G4Q+R176K+G186E+T193E+I206Y+T251E+L405M+Y100W | 81% |
| G4Q+R176K+G186E+T193E+I206Y+T251 E+L405M+Y100W+G258Q | 81% |
| G4Q+R176K+G186E+T193E+I206Y+T251 E+L405M+Y1 00W+G258Q+K281 N | 28% |
| G4Q+R176K+G186E+T193E+I206Y+T251 E+L405M+Y1 00W+G258Q+K281 N+ Y363E | 31% |
| G4Q+R176K+G186E+T193E+I206Y+T251 E+L405M+Y1 00W+G258Q+K281 N+ Y363H | 37% |
| G4Q+R176K+G186E+T193E+I206Y+T251 E+L405M+Y1 00W+G258Q+K281 N+ Y363H+I272V | 24% |
| G4Q+R176K+G186E+T193E+I206Y+T251 E+L405M+Y100W+G258Q+Y363E | 82% |
| G4Q+R176K+G186E+T193E+I206Y+T251 E+L405M+Y100W+G258Q+Y363H | 79% |
| G4Q+R176K+G186E+T193E+I206Y+T251 E+L405M+Y1 00W+K281 N | 29% |
| G4Q+R176K+G186E+T193E+I206Y+T251 E+L405M+Y1 00W+K281 N+Y363E | 29% |
| G4Q+R176K+G186E+T193E+I206Y+T251 E+L405M+Y1 00W+K281 N+Y363H | 35% |
| G4Q+R176K+G186E+T193E+I206Y+T251 E+L405M+Y1 00W+R181Q | 76% |
| G4Q+R176K+G186E+T193E+I206Y+T251 E+L405M+Y1 00W+R181Q+G258Q | 85% |
| G4Q+R176K+G186E+T193E+I206Y+T251 E+L405M+Y1 00W+R181Q+G258Q+ K281N | 37% |
| G4Q+R176K+G186E+T193E+I206Y+T251E+L405M+Y100W+R181Q+G258Q+ K281N+Y363E | 35% |
| G4Q+R176K+G186E+T193E+I206Y+T251 E+L405M+Y1 00W+R181Q+G258Q+ Y363E | 84% |
| G4Q+R176K+G186E+T193E+I206Y+T251 E+L405M+Y1 00W+R181Q+G258Q+ Y363H | 74% |
| G4Q+R176K+G186E+T193E+I206Y+T251 E+L405M+Y1 00W+R181Q+K281 N | 29% |
| G4Q+R176K+G186E+T193E+I206Y+T251 E+L405M+Y1 00W+R181Q+K281 N+ Y363H | 40% |
| G4Q+R176K+G186E+T193E+I206Y+T251 E+L405M+Y1 00W+R181Q+Y363H | 58% |
| G4Q+R176K+G186E+T193E+I206Y+T251 E+L405M+Y1 00W+R181Q+Y363H+ R254Q | 84% |
| G4Q+R176K+G186E+T193E+I206Y+T251E+L405M+Y100W+Y135T | 67% |
| G4Q+R176K+G186E+T193E+I206Y+T251E+L405M+Y100W+Y135T+G258Q | 70% |
| G4Q+R176K+G186E+T193E+I206Y+T251E+L405M+Y100W+Y135T+G258Q+ K281N+Y363H | 24% |
| G4Q+R176K+G186E+T193E+I206Y+T251E+L405M+Y100W+Y135T+G258Q+ Y363E | 70% |
| G4Q+R176K+G186E+T193E+I206Y+T251E+L405M+Y100W+Y135T+G258Q+ Y363H | 70% |
| G4Q+R176K+G186E+T193E+I206Y+T251 E+L405M+Y1 00W+Y135T +R181Q | 61% |
| G4Q+R176K+G186E+T193E+I206Y+T251 E+L405M+Y1 00W+Y135T +R181Q+ G258Q | 62% |
| G4Q+R176K+G186E+T193E+I206Y+T251 E+L405M+Y1 00W+Y135T+R181Q+ G258Q+K281 N | 26% |
| G4Q+R176K+G186E+T193E+I206Y+T251E+L405M+Y100W+Y135T+R181Q+ G258Q+Y363E | 64% |
| G4Q+R176K+G186E+T193E+I206Y+T251E+L405M+Y100W+Y135T+R181Q+ G258Q+Y363H | 81% |
| G4Q+R176K+G186E+T193E+I206Y+T251E+L405M+Y100W+Y135T+R181Q+ K281N+Y363H | 11% |
| G4Q+R176K+G186E+T193E+I206Y+T251 E+L405M+Y1 00W+Y135T+R181Q+ Y363E | 65% |
| G4Q+R176K+G186E+T193E+I206Y+T251 E+L405M+Y1 00W+Y135T+R181Q+ Y363H | 65% |
| G4Q+R176K+G186E+T193E+I206Y+T251E+L405M+Y100W+Y135T+Y363E | 73% |
| G4Q+R176K+G186E+T193E+I206Y+T251E+L405M+Y100W+Y135T+Y363H | 71% |
| G4Q+R176K+G186E+T193E+I206Y+T251E+L405M+Y100W+Y363E | 82% |
| G4Q+R176K+G186E+T193E+I206Y+T251E+L405M+Y100W+Y363H | 80% |
| G4Q+R176K+G186E+T193E+I206Y+T251E+L405M+Y135T | 67% |
| G4Q+R176K+G186E+T193E+I206Y+T251E+L405M+Y135T+G258Q | 71% |
| G4Q+R176K+G186E+T193E+I206Y+T251E+L405M+Y135T+G258Q+T8A | 74% |
| G4Q+R176K+G186E+T193E+I206Y+T251E+L405M+Y135T+G258Q+K281N+ Y363E | 21% |
| G4Q+R176K+G186E+T193E+I206Y+T251E+L405M+Y135T+G258Q+Y363H | 72% |
| G4Q+R176K+G186E+T193E+I206Y+T251E+L405M+Y135T+K281N | 5% |
| G4Q+R176K+G186E+T193E+I206Y+T251 E+L405M+Y135T+R181 Q+G258Q | 57% |
| G4Q+R176K+G186E+T193E+I206Y+T251E+L405M+Y135T+R181Q+G258Q+ K281N | 8% |
| G4Q+R176K+G186E+T193E+I206Y+T251E+L405M+Y135T+R181Q+G258Q+ Y363H | 60% |
| G4Q+R176K+G186E+T193E+I206Y+T251E+L405M+Y135T+Y363E | 73% |
| G4Q+R176K+G186E+T193E+I206Y+T251E+L405M+Y135T+Y363H | 75% |
| G4Q+R176K+G186E+T193E+I206Y+T251E+L405M+Y363E | 85% |
| G4Q+R176K+G186E+T193E+I206Y+T251E+L405M+Y363H | 85% |
| N25H+R176K+G186E+I206Y | 97% |
| N25H+R176K+G186E+I206Y+G258Q | 94% |
| N25H+R176K+G186E+I206Y+G258Q | 95% |
| N25H+R176K+G186E+I206Y+G258Q+K281N | 71% |
| N25H+R176K+G186E+I206Y+G258Q+K281N+Y363E | 70% |
| N25H+R176K+G186E+I206Y+G258Q+Y363E | 91% |
| N25H+R176K+G186E+I206Y+G258Q+Y363E+N123D | 89% |
| N25H+R176K+G186E+I206Y+K281N | 70% |
| N25H+R176K+G186E+I206Y+R181 Q | 95% |
| N25H+R176K+G186E+I206Y+R181 Q+T193E | 88% |
| N25H+R176K+G186E+I206Y+R181Q+Y363E | 101% |
| N25H+R176K+G186E+I206Y+T193E | 87% |
| N25H+R176K+G186E+I206Y+T193E+G258Q | 86% |
| N25H+R176K+G186E+I206Y+T193E+K281N+Y363E | 42% |
| N25H+R176K+G186E+I206Y+T193E+Y363E | 88% |
| N25H+R176K+G186E+I206Y+Y100W | 91% |
| N25H+R176K+G186E+I206Y+Y100W+G258Q | 95% |
| N25H+R176K+G186E+I206Y+Y100W+G258Q+K281N | 69% |
| N25H+R176K+G186E+I206Y+Y100W+G258Q+K281N+Y363E | 74% |
| N25H+R176K+G186E+I206Y+Y100W+G258Q+Y363E | 92% |
| N25H+R176K+G186E+I206Y+Y100W+G258Q+Y363E+Y135C | 89% |
| N25H+R176K+G186E+I206Y+Y100W+K281N | 73% |
| N25H+R176K+G186E+I206Y+Y100W+K281N+Y363E | 74% |
| N25H+R176K+G186E+I206Y+Y100W+Q361R | 93% |
| N25H+R176K+G186E+I206Y+Y1 00W+R181Q+K281 N+Y363E | 70% |
| N25H+R176K+G186E+I206Y+Y100W+R181Q+K281N+Y363E+N175D | 71% |
| N25H+R176K+G186E+I206Y+Y1 00W+R181Q+T193E | 89% |
| N25H+R176K+G186E+I206Y+Y1 00W+R181Q+T193E+G258Q+K281 N | 34% |
| N25H+R176K+G186E+I206Y+Y100W+R181Q+T193E+G258Q+Y363E | 78% |
| N25H+R176K+G186E+I206Y+Y1 00W+R181Q+T193E+K281 N+Y363E | 34% |
| N25H+R176K+G186E+I206Y+Y100W+R181Q+T193E+Y363E | 85% |
| N25H+R176K+G186E+I206Y+Y1 00W+R181Q+Y363E | 91% |
| N25H+R176K+G186E+I206Y+Y1 00W+93E | 87% |
| N25H+R176K+G186E+I206Y+Y100W+T193E+G258Q | 82% |
| N25H+R176K+G186E+I206Y+Y100W+T193E+G258Q+H473R | 84% |
| N25H+R176K+G186E+I206Y+Y100W+T193E+G258Q+K281N | 33% |
| N25H+R176K+G186E+I206Y+Y100W+T193E+G258Q+K281N+Y363E | 35% |
| N25H+R176K+G186E+I206Y+Y100W+T193E+G258Q+Y363E | 87% |
| N25H+R176K+G186E+I206Y+Y100W+T193E+Y363E | 85% |
| N25H+R176K+G186E+I206Y+Y100W+Y135T | 91% |
| N25H+R176K+G186E+I206Y+Y100W+Y135T+G258Q | 89% |
| N25H+R176K+G186E+I206Y+Y100W+Y135T+G258Q+K281N | 45% |
| N25H+R176K+G186E+I206Y+Y100W+Y135T+G258Q+K281N+Y363E | 47% |
| N25H+R176K+G186E+I206Y+Y100W+Y135T+G258Q+Y363E | 83% |
| N25H+R176K+G186E+I206Y+Y100W+Y135T+K281N | 53% |
| N25H+R176K+G186E+I206Y+Y100W+Y135T+K281N+Y363E | 48% |
| N25H+R176K+G186E+I206Y+Y1 00W+Y135T+R181Q | 90% |
| N25H+R176K+G186E+I206Y+Y100W+Y135T+R181Q+G258Q+K281N+Y363E | 42% |
| N25H+R176K+G186E+I206Y+Y1 00W+Y135T+R181Q+K281 N+Y363E | 37% |
| N25H+R176K+G186E+I206Y+Y100W+Y135T+R181Q+T193E | 74% |
| N25H+R176K+G186E+I206Y+Y100W+Y135T+R181Q+T193E+G258Q+Y363E | 61% |
| N25H+R176K+G186E+I206Y+Y100W+Y135T+R181Q+T193E+Y363E | 61% |
| N25H+R176K+G186E+I206Y+Y100W+Y135T+T193E | 80% |
| N25H+R176K+G186E+I206Y+Y100W+Y135T+T193E+G258Q+Y363E | 70% |
| N25H+R176K+G186E+I206Y+Y100W+Y135T+T193E+Y363E | 76% |
| N25H+R176K+G186E+I206Y+Y100W+Y135T+Y363E | 91% |
| N25H+R176K+G186E+I206Y+Y100W+Y363E | 91% |
| N25H+R176K+G186E+I206Y+Y135T | 94% |
| N25H+R176K+G186E+I206Y+Y135T +G258Q | 91% |
| N25H+R176K+G186E+I206Y+Y135T+G258Q+K281N | 55% |
| N25H+R176K+G186E+I206Y+Y135T+G258Q+K281N+Y363E | 50% |
| N25H+R176K+G186E+I206Y+Y135T +G258Q+Y363E | 92% |
| N25H+R176K+G186E+I206Y+Y135T+K281N | 52% |
| N25H+R176K+G186E+I206Y+Y135T+K281N+Y363E | 53% |
| N25H+R176K+G186E+I206Y+Y135T+K281N+Y363E+R415S | 47% |
| N25H+R176K+G186E+I206Y+Y135T+R181Q+G258Q | 90% |
| N25H+R176K+G186E+I206Y+Y135T+R181Q+G258Q+Y363E | 90% |
| N25H+R176K+G186E+I206Y+Y135T+T193E+G258Q | 74% |
| N25H+R176K+G186E+I206Y+Y135T+Y363E | 95% |
| N25H+R176K+G186E+I206Y+Y363E | 95% |
| Y160W+G184Q | 54% |

### Example 6

### Wash trials

For the microscale wash trials of the variants of Amylase B (SEQ ID NO: 1) containing several amino acid substitutions at positions of interest were evaluated for wash performance using methods described in Example 1 using CS-126 as fabric.

The following Table 13 describes the results for Amylase B variants containing single amino acid substitutions (Numbering according SEQ ID NO: 3).

| Amylase B variants with improved performance on CS-126 (reported as IF) | | | |
|---|---|---|---|
| Amylase variant | 0.02ppm | 0.05 ppm | 0.1 ppm |
| Amylase B (SEQ ID NO: 1) | 1 | 1 | 1 |
| A119S | 0.7 | 0.8 | 1.1 |
| A139S | 1.1 | 1.2 | 1.2 |
| A274F | 1 | 1.2 | 1.1 |
| A41G | 1 | 1.1 | 1 |
| D163A | 2 | 1.1 | 1.1 |
| D163Q | 1.3 | 1.2 | 1.1 |
| D163T | 2 | 1.1 | 1 |
| D188T | 1 | 1.1 | 1.2 |
| D20N | 0.8 | 1.2 | 1.2 |
| D231D | 1.8 | 0.9 | 0.8 |
| D271S | 2.1 | 1.5 | 1.2 |
| D360T | 1 | 1 | 1.1 |
| D370S | 1.4 | 1.4 | 1.2 |
| D36H | 1 | 1.2 | 1 |
| D36K | 1 | 1.1 | 1 |
| D387S | 1.2 | 1 | 1 |
| E276N | 1.2 | 1.2 | 1.1 |
| E439K | 0.7 | 0.9 | 1.1 |
| F180M | 1.1 | 1 | 1 |
| F180N | 1.2 | 1.2 | 1.1 |
| F180T | 1.1 | 1 | 1 |
| F180W | 1.7 | 1.2 | 1.2 |
| G182D | 1.5 | 1.7 | 1.2 |
| G182E | 1.4 | 1.5 | 1.2 |
| G182N | 1 | 1.2 | 1 |
| G182Q | 1 | 1.2 | 1.1 |
| G182S | 1.2 | 1.3 | 0.9 |
| G258F | 3.2 | 1.9 | 1.3 |
| G258Q | 1.9 | 1.4 | 1 |
| G258Q | 1.4 | 1.2 | 1.3 |
| G258R | 2.7 | 1.7 | 1.3 |
| G273Q | 1.1 | 1.4 | 1.2 |
| G362C | 0.5 | 1.2 | 1.1 |
| G362M | 1.4 | 1.7 | 1.4 |
| G73C | 0.7 | 1.1 | 1.4 |
| G73N | 0.5 | 1.1 | 1.1 |
| G7H | 1.4 | 1.2 | 1.1 |
| G7W | 1.3 | 1.5 | 1.4 |
| G96Q | 1.1 | 1.1 | 1 |
| H252S | 2.6 | 1.6 | 1.1 |
| H286Q | 1.2 | 1.2 | 1.1 |
| H324K | N/A | 1 | 1.1 |
| I177A | 0.9 | 1 | 1.2 |
| I177G | 1.4 | 1.4 | 1.1 |
| I177K | 1.1 | 1.1 | 1 |
| I177N | 1.6 | 1.1 | 1.2 |
| I177P | 2.6 | 1.8 | 1.2 |
| I177R | 1 | 1.2 | 1.1 |
| I177S | 1.4 | 1.2 | 1.1 |
| I177W | 1.2 | 1.2 | 1.1 |
| I272F | 2.2 | 1.4 | 1.1 |
| I272S | 1 | 1.1 | 1.2 |
| I275V | 0.9 | 1 | 1.1 |
| K156E | 0.6 | 0.8 | 1.1 |
| K185E | 1 | 1.4 | 1 |
| K185M | 2.2 | 1.7 | 1.3 |
| K185N | 1.3 | 1.5 | 1 |
| K242N | 1 | 1.1 | 1.1 |
| K269M | 1.5 | 1.3 | 1.2 |
| K281A | 0.9 | 1 | 1.1 |
| K281D | 2.2 | 1.7 | 1.2 |
| K281E | 0.9 | 1.1 | 1.1 |
| K281H | 3 | 2 | 1.3 |
| K281N | 2.9 | 1.7 | 1.2 |
| K281S | 1.2 | 1.1 | 1 |
| K37F | 2.1 | 1.7 | 1.3 |
| K37L | 3.5 | 1.9 | 1.5 |
| K37M | 2.6 | 2.7 | 1.8 |
| K37W | 2.3 | 2.2 | 1.6 |
| K37Y | 2.7 | 2 | 1.4 |
| K395C | 5.1 | 1.8 | 1.2 |
| K395M | 2.6 | 1.5 | 1.1 |
| L279A | 2.4 | 1.6 | 1.1 |
| L357C | 4.1 | 1.9 | 1.3 |
| L357F | 2.6 | 1.3 | 1.2 |
| L429P | 4.3 | 1.6 | 1.2 |
| M382Q | 0.9 | 1.1 | 1.2 |
| N224F | 2.9 | 1.7 | 1.2 |
| N260H | 5.1 | 2.3 | 1.4 |
| N25D | 4.9 | 3 | 1.8 |
| N25G | 0.9 | 0.9 | 1.2 |
| N25Y | 1.3 | 1.2 | 1.1 |
| N270Q | 0.8 | 1 | 1.1 |
| N277D | 1.2 | 1.4 | 1.1 |
| N277E | 1.8 | 1.4 | 1.1 |
| N277T | 0.7 | 1 | 1.1 |
| N299S | 1.7 | 1.5 | 1.2 |
| N314Q | 0.7 | 1.1 | 1.1 |
| N314S | 2.4 | 1.7 | 1.4 |
| N33L | 1.7 | 2 | 1.7 |
| N460G | 1.1 | 1.1 | 1.1 |
| N6P | 1.1 | 1.5 | 1.3 |
| N83E | 0.9 | 1 | 1.1 |
| N95A | 0.6 | 1.1 | 1.1 |
| P322K | 3.8 | 1.6 | 1.2 |
| P350P | 0.7 | 1 | 1.2 |
| P434E | 1.6 | 1.2 | 1.1 |
| P434H | 1.9 | 1.3 | 1 |
| P434S | 1.8 | 1.3 | 1.2 |
| Q169D | 1 | 1.3 | 1.4 |
| Q169S | 1.2 | 1.4 | 1.2 |
| Q174E | 1.1 | 1.3 | 1.2 |
| Q174G | 1.5 | 1 | 1.1 |
| Q174H | 1.8 | 1.2 | 1.2 |
| Q174M | 1.4 | 1.3 | 1.2 |
| Q174N | 1.9 | 1.6 | 1.4 |
| Q311W | 4.4 | 1.8 | 1.3 |
| Q98A | 1.1 | 1.2 | 1.1 |
| Q98N | 1.2 | 1.3 | 1.2 |
| Q98V | 1.7 | 1.4 | 1.3 |
| R118A | 0.6 | 1 | 1.1 |
| R118D | 1.5 | 1.4 | 1.3 |
| R118E | 2.4 | 1.5 | 1.2 |
| R176S | 1.4 | 1.2 | 1.2 |
| R176T | 1.5 | 1.5 | 1.2 |
| R359W | 1.3 | 1.1 | 1.1 |
| R359W | 1.3 | 1.3 | 1 |
| R82L | 0.8 | 0.9 | 1.1 |
| S280D | 2.2 | 1.6 | 1.1 |
| S280F | 1.6 | 1.3 | 1.1 |
| S280G | 2.1 | 1.4 | 1.1 |
| S280K | 2.5 | 1.3 | 1.1 |
| S280R | 1.5 | 1.2 | 1 |
| S365L | 0.4 | 0.8 | 1.1 |
| S365Q | 0.6 | 0.9 | 1.1 |
| S365Y | 0.6 | 0.9 | 1.1 |
| S94E | 1.6 | 1.2 | 1.1 |
| T136E | 1.7 | 1.7 | 1.2 |
| T193D | 0.9 | 1 | 1.1 |
| T193E | 1.5 | 1.3 | 1.1 |
| T193G | 1.3 | 1.2 | 1.1 |
| T193V | 0.7 | 1.1 | 1.3 |
| T251T | 1 | 1.1 | 1 |
| T282V | 1.3 | 1.3 | 1.2 |
| T356C | 2.4 | 1.4 | 1.3 |
| T356L | 1.5 | 1.3 | 1.2 |
| T356V | 2.9 | 1.6 | 1.3 |
| T358M | 2.4 | 1.5 | 1.1 |
| T459N | 1.1 | 1.1 | 1 |
| T461L | 0.7 | 0.8 | 1.1 |
| T463A | 1.4 | 1.2 | 1.1 |
| T463V | 1.8 | 1.4 | 1.2 |
| T5D | 0.9 | 1 | 1.1 |
| V120E | 1.5 | 1.4 | 1.3 |
| V120G | 0.6 | 0.9 | 1.2 |
| V120M | 2.1 | 1.5 | 1.2 |
| V120S | 2.2 | 1.5 | 1.2 |
| V120W | 1.5 | 1.4 | 1.3 |
| V120Y | 1.6 | 1.3 | 1.1 |
| V131Q | 1.5 | 1.2 | 1.3 |
| V165S | 2.6 | 1.5 | 1.2 |
| V165T | 1.3 | 1.3 | 1.2 |
| V191M | 0.9 | 0.9 | 1.1 |
| V238A | 1 | 1 | 1.1 |
| V238F | 1 | 1.1 | 1.1 |
| V317M | 1 | 1.3 | 1.3 |
| V318L | 1 | 1.3 | 1.3 |
| V344V | 0.4 | 1 | 1.2 |
| V75H | 2.3 | 1.7 | 1.4 |
| V75I | 0.6 | 1.2 | 1.4 |
| V75L | 0.7 | 1 | 1.2 |
| V75T | 0.5 | 1 | 1.3 |
| W116I | 2 | 1.6 | 1.3 |
| W116M | 1.8 | 1.3 | 1.3 |
| W187A | 3.6 | 2.3 | 1.4 |
| W187D | 2.4 | 1.7 | 0.9 |
| W187M | 1.2 | 1.4 | 1.2 |
| W187V | 2 | 1.2 | 0.8 |
| W189I | 0.9 | 1.3 | 1.2 |
| W268F | 2.2 | 1.4 | 1.2 |
| Y100W | 2.9 | 1.7 | 1.3 |
| Y135D | 2.4 | 1.7 | 1.3 |
| Y135E | 1.4 | 1.4 | 1.2 |
| Y135G | 1 | 1.3 | 1.1 |
| Y135N | 2.1 | 1.5 | 1.3 |
| Y135P | 1.3 | 1.4 | 1.2 |
| Y135S | 1.5 | 1.5 | 1.3 |
| Y135T | 1.6 | 1.6 | 1.3 |
| Y135W | 0.8 | 1.3 | 1.2 |
| Y160D | 1 | 1.8 | 1.3 |
| Y160E | 2.8 | 1.8 | 1.4 |
| Y178F | 1.1 | 1 | 0.9 |
| Y243D | 2.2 | 1.5 | 1 |
| Y298V | 1.4 | 1.4 | 1.2 |
| Y363E | 1.9 | 1.9 | 1.5 |
| Y363H | 1.3 | 1.6 | 1.4 |
| Y363N | 1.8 | 1.7 | 1.3 |
| Y363Y | 1.5 | 1.3 | 1.2 |
| Y368F | 0.4 | 0.8 | 1.2 |
| Y372M | 1.1 | 1.4 | 1.3 |
| Y372S | 0.8 | 1.1 | 1.1 |
| Y372T | 0.6 | 0.9 | 1.1 |
| Y396C | 1.7 | 1.3 | 1.1 |
| A41C | 2.3 | 2 | 1.3 |
| R393Q | 1.8 | 1.6 | 1.3 |
| G7F | 1.8 | 1.7 | 1.3 |
| Y363M | 1.7 | 1.7 | 1.4 |
| P434R | 1.5 | 1.6 | 1.3 |
| V318L | 1.5 | 1.5 | 1.3 |
| G456S | 1.5 | 1.4 | 1.3 |
| T193E | 1.4 | 1.1 | 0.9 |
| T459N | 1.4 | 1.2 | 1 |
| T461R | 1.4 | 1.6 | 1 |
| H321Y | 1.4 | 1.4 | 1.3 |
| H210D | 1.4 | 1.2 | 0.9 |
| N460G | 1.4 | 1.3 | 1.1 |
| V326Y | 1.4 | 1.3 | 1 |
| D387S | 1.4 | 1.2 | 1 |
| R320A | 1.4 | 1.6 | 1.4 |
| F343W | 1.3 | 1.3 | 1.1 |
| N314Q | 1.3 | 1.1 | 1.2 |
| P388H | 1.3 | 1.3 | 1.2 |
| L390F | 1.3 | 1.5 | 1.3 |
| Y372C | 1.3 | 1.5 | 1.2 |
| S365L | 1.3 | 1.4 | 1.3 |
| V253C | 1.3 | 1.1 | 1 |
| Y368F | 1.3 | 1.3 | 1.3 |
| N299S | 1.3 | 1 | 0.9 |
| N25Y | 1.3 | 0.9 | 1 |
| P388K | 1.2 | 1.3 | 1.2 |
| N126D | 1.2 | 1.1 | 1.1 |
| A397S | 1.2 | 0.8 | 0.7 |
| T400A | 1.2 | 1.3 | 1.2 |
| N465H | 1.2 | 1.2 | 0.8 |
| Y363S | 1.2 | 1.3 | 1.3 |
| D387N | 1.2 | 1.2 | 1.2 |
| Q174H | 1.2 | 1 | 1.1 |
| K72C | 1.2 | 1.1 | 0.9 |
| T400L | 1.2 | 1.2 | 1.2 |
| T323G | 1.2 | 1.2 | 1 |
| T356V | 1.2 | 1.6 | 1.4 |
| N6E | 1.2 | 1.1 | 1.2 |
| D387E | 1.2 | 1.1 | 1.1 |
| Q311G | 1.1 | 1.1 | 1 |
| A352C | 1.1 | 1.2 | 1 |
| G378E | 1.1 | 1.2 | 1.1 |
| T282V | 1.1 | 1 | 0.9 |
| Y372T | 1.1 | 1.4 | 1.4 |
| S365Q | 1.1 | 1.1 | 1.1 |
| P388E | 1.1 | 1.2 | 1.2 |
| T193V | 1.1 | 1 | 1.1 |
| Y396S | 1.1 | 1.1 | 1 |
| P364T | 1.1 | 1.3 | 1.2 |
| N270A | 1.1 | 1 | 0.9 |
| N6H | 1.1 | 1 | 1 |
| L405H | 1.1 | 1.4 | 0.8 |
| V379R | 1.1 | 1.1 | 1 |
| N277D | 1.1 | 1.1 | 1.1 |
| L297M | 1 | 0.9 | 1.1 |
| N219Q | 1 | 1 | 1.1 |
| T400S | 1 | 1.2 | 1.1 |
| T461A | 1 | 1.3 | 1.2 |
| E391K | 1 | 1.3 | 1.1 |
| K385W | 1 | 1.3 | 1.1 |
| L405M | 1 | 1.2 | 1 |
| T81L | 0.9 | 0.9 | 1.2 |
| R82C | 0.9 | 1.1 | 1.2 |
| M382Q | 0.9 | 1.4 | 1.2 |
| G4Q | 0.8 | 0.9 | 1.1 |
| V75L | 0.6 | 0.9 | 1.1 |

The following table 14 describes the results for Amylase B variants containing multiple amino acid substitutions in Amylase B (Numbering according SEQ ID NO: 3).

| Amylase B variants with improved performance on CS-126 (reported as IF) | | | |
|---|---|---|---|
| Amylase variant | 0.02p pm | 0.05 ppm | 0.1 ppm |
| Amylase B (SEQ ID NO: 1) | 1 | 1 | 1 |
| G4Q+G7W+R176K+G186E+T193E+I206Y+T251E+L405M | 1.1 | 1.2 | 1.1 |
| G4Q+K37M+R176K+G186E+T193E+I206Y+T251E+L405M | 1.6 | 1.5 | 1.2 |
| G4Q+N25H+R176K+G186E+I206Y+T251E+L405M+N460G | 1.4 | 1.2 | 1.2 |
| G4Q+N25H+R176K+G186E+I206Y+T251E+N460G+ | 2 | 1.4 | 1.2 |
| G4Q+N25Y+R176K+G186E+T193E+I206Y+T251E+L405M | 1.6 | 1.2 | 1 |
| G4Q+R176K+G182D+G186E+T193E+I206Y+T251E+L405M | 0.7 | 1.1 | 1.2 |
| G4Q+R176K+G186E+T193E+l206Y+T251E+G258Q+L405M | 0.8 | 1.1 | 1.2 |
| G4Q+R176K+G186E+T193E+I206Y+T251E+K281N+L405M | 0.8 | 1.1 | 1.2 |
| G4Q+R176K+G186E+T193E+I206Y+T251E+L405M+N460G | 1 | 1.1 | 1.1 |
| G4Q+R176K+G186E+T193E+I206Y+T251E+Y363E+L405M | 0.6 | 1.1 | 1.2 |
| G4Q+R176K+G186E+T193E+I206Y+T251E+Y363H+L405M | 0.7 | 1.1 | 1.2 |
| G4Q+R176K+G186E+T193E+I206Y+T251E+Y363N+L405M | 0.9 | 1.1 | 1.2 |
| G4Q+R176K+R181D+G186E+T193E+I206Y+T251 E+L405M | 1.3 | 1.3 | 1 |
| G4Q+R176K+R181F+G186E+T193E+I206Y+T251E+L405M | 0.6 | 1 | 1.2 |
| G4Q+R176K+R181N+G186E+T193E+I206Y+T251E+L405M | 0.9 | 1.1 | 1 |
| G4Q+R176K+R181Q+G186E+T193E+I206Y+T251E+L405M | 0.7 | 1.1 | 1.2 |
| G4Q+T136E+R176K+G186E+T193E+I206Y+T251E+L405M | 1.1 | 1.1 | 1 |
| G4Q+Y100W+R176K+G186E+T193E+I206Y+T251E+L405M | 1.5 | 1.3 | 1.2 |
| G4Q+Y135E+R176K+G186E+T193E+I206Y+T251E+L405M | 1.4 | 1.3 | 1 |
| G4Q+Y135T+R176K+G186E+T193E+I206Y+T251E+L405M | 1.4 | 1.3 | 1.1 |
| G4Q+Y135W+R176K+G186E+T193E+I206Y+T251E+L405M | 1.8 | 1.2 | 1.1 |
| G4Q+Y160D+R176K+G186E+T193E+I206Y+T251E+L405M | 0.7 | 0.9 | 1.1 |
| G4Q+Y160E+R176K+G186E+T193E+I206Y+T251E+L405M | 0.6 | 1 | 1.2 |
| G7H+N25H+R176K+G186E+I206Y | 1 | 1.5 | 1.2 |
| G7W+N25H+R176K+G186E+1206Y | 1.1 | 1.4 | 1.2 |
| N25D+R176K+G186E+I206Y | 0.9 | 1.2 | 1.1 |
| N25H+G186E+I206Y+L405M+N460G | 1.6 | 1.3 | 1.1 |
| N25H+G186E+1206Y+N460G | 2 | 1.3 | 1.1 |
| N25H+K37M+R176K+G186E+I206Y | 1.1 | 1.1 | 1.1 |
| N25H+R118D+R176K+G186E+I206Y | 1 | 1.3 | 1.1 |
| N25H+R176K+G186E+I206Y+G258Q | 1.3 | 1.3 | 1.2 |
| N25H+R176K+G186E+I206Y+K281N | 0.8 | 1.2 | 1.2 |
| N25H+R176K+G186E+I206Y+N460G | 1.7 | 1.2 | 1.2 |
| N25H+R176K+G186E+I206Y+T251E+N460G | 1.8 | 1.3 | 1.2 |
| N25H+R176K+G186E+I206Y+Y363E | 1.6 | 1.5 | 1.3 |
| N25H+R176K+G186E+I206Y+Y363H | 1.6 | 1.6 | 1.2 |
| N25H+R176K+G186E+I206Y+Y363N | 1.7 | 1.7 | 1.4 |
| N25H+R176K+G186E+N460G | 2.1 | 1.4 | 1.2 |
| N25H+R176K+G186E+T193E+I206Y | 1.3 | 1.1 | 1.1 |
| N25H+R176K+G186E+T193E+I206Y+N460G | 1.3 | 1.3 | 1 |
| N25H+R176K+G186E+T193E+I206Y+T251E+N460G | 2 | 1.3 | 1.1 |
| N25H+R176K+R181D+G186E+I206Y | 1.6 | 1.2 | 1.2 |
| N25H+R176K+R181N+G186E+1206Y | 1.4 | 1.2 | 1.2 |
| N25H+R176K+R181Q+G186E+I206Y | 1.4 | 1.4 | 1 |
| N25H+T136E+R176K+G186E+I206Y | 0.9 | 1.2 | 1.2 |
| N25H+Y100W+R176K+G186E+I206Y | 0.8 | 1.4 | 1.3 |
| N25H+Y135D+R176K+G186E+I206Y | 0.7 | 1.1 | 0.9 |
| N25H+Y135E+R176K+G186E+I206Y | 1.6 | 1.2 | 1.1 |
| N25H+Y135T+R176K+G186E+I206Y | 1.3 | 1.2 | 1.2 |
| N25H+Y135W+R176K+G186E+I206Y | 1.7 | 1.2 | 1.1 |
| N25H+Y160D+R176K+G186E+I206Y | 1.6 | 1.4 | 1.1 |
| N25H+Y160E+R176K+G186E+I206Y | 0.7 | 1.1 | 1.2 |
| N25Y+R176K+G186E+I206Y | 1.1 | 1.2 | 1.2 |
| G186E | 1.3 | 1.5 | 1.2 |
| G186E+I206Y | 1.4 | 1.4 | 1.2 |
| G186E+1206Y+L405M | 1.1 | 1.3 | 1.1 |
| G186E+I206Y+T251E | 1.5 | 1.4 | 1.1 |
| G186E+I206Y+T251 E+E276R+L405M | 1.8 | 0.8 | 0.8 |
| G186E+I206Y+T251 E+L405M | 0.9 | 0.9 | 1.1 |
| G186E+I206Y+T251E+T323G+L405M | 0.5 | 1.1 | 1.3 |
| G186E+I206Y+T323G+L405M | 0.7 | 0.9 | 1.3 |
| G186E+L405M | 3.8 | 1.5 | 1.1 |
| G186E+T193E+I206Y+T251E | 0.9 | 1.1 | 1.1 |
| G186E+T251E+L405M | 0.8 | 1.1 | 1.2 |
| G4Q+G186E+I206Y | 1.4 | 1.3 | 1.1 |
| G4Q+G186E+I206Y+L405M | 1.1 | 1.2 | 1.1 |
| G4Q+G186E+I206Y+T251E+E276R | 1.1 | 0.9 | 0.9 |
| G4Q+G186E+I206Y+T251 E+L405M | 1.2 | 1.2 | 1.1 |
| G4Q+G186E+I206Y+T251E+T323G+L405M | 1 | 1.3 | 1 |
| G4Q+G186E+T193E+I206Y+L405M | 1.3 | 1.2 | 1.1 |
| G4Q+G186E+T193E+I206Y+T251E+L405M | 1.2 | 1.2 | 1.1 |
| G4Q+N25H+G186E+I206Y | 0.8 | 1 | 1.1 |
| G4Q+N25H+G186E+I206Y+L405M | 1.4 | 1.2 | 0.9 |
| G4Q+N25H+G186E+I206Y+T251E+T323A+L405M | 1.4 | 1.3 | 1.2 |
| G4Q+N25H+G186E+I206Y+T323G+L405M | 1.3 | 1.2 | 1.1 |
| G4Q+N25H+R176K+G186E+I206Y+T251E+L405M | 1.4 | 1.2 | 1.2 |
| G4Q+N25H+R176K+G186E+T251E | 2.9 | 1.4 | 1.3 |
| G4Q+N25H+R176K+G186E+T251E+L405M | 3.3 | 1.5 | 1.3 |
| G4Q+N25H+R176K+I206Y+L405M+N460G | 1.1 | 0.8 | 0.8 |
| G4Q+N25H+R176K+I206Y+N460G | 2.3 | 1.2 | 1 |
| G4Q+N25H+R176K+I206Y+T251 E | 1.8 | 1.3 | 1.1 |
| G4Q+N25H+R176K+I206Y+T251E+N460G | 2 | 1.1 | 0.9 |
| G4Q+N25H+R176K+I206Y+T323G | 1.2 | 1 | 0.9 |
| G4Q+N25H+Y160W+G186E+I206Y+L405M | 0.8 | 1.1 | 1.1 |
| G4Q+N25H+Y160W+G186E+I206Y+T251 E | 1 | 1.3 | 1.1 |
| G4Q+N25H+Y160W+G186E+I206Y+T323G+L405M | 0.9 | 1.3 | 1.1 |
| G4Q+N25H+Y160W+G186E+L405M | 1 | 1.1 | 1.2 |
| G4Q+N25H+Y160W+G186E+T193E+I206Y+E276R | 1.1 | 0.9 | 0.9 |
| G4Q+N25H+Y160W+G186E+T323G | 1.1 | 1.3 | 1.3 |
| G4Q+N25H+Y160W+R176K+G186E+I206Y+T251E | 0.8 | 1.2 | 1 |
| G4Q+N25H+Y160W+R176K+G186E+I206Y+T251 E+L405M | 1.1 | 1.1 | 1.2 |
| G4Q+N25H+Y160W+R176K+G186E+L405M | 1.7 | 1.4 | 1 |
| G4Q+N25H+Y160W+R176K+G186E+T251 E+L405M | 1 | 1.3 | 1.2 |
| G4Q+N25H+Y160W+R176K+I206Y | 1.3 | 1.1 | 0.9 |
| G4Q+N25H+Y160W+R176K+I206Y+T251 E+N460G | 0.7 | 0.9 | 1.1 |
| G4Q+R176K+G186E+I206Y+L405M | 1.2 | 1.4 | 1.2 |
| G4Q+R176K+G186E+I206Y+T251 E+L405M | 1.8 | 1.1 | 1.1 |
| G4Q+R176K+G186E+L405M | 1.2 | 1.1 | 1.2 |
| G4Q+R176K+G186E+T193E+I206Y+E276R+L405M | 0.8 | 1 | 1.1 |
| G4Q+R176K+G186E+T193E+I206Y+T251E+L405M | 1.4 | 1.1 | 1.1 |
| G4Q+R176K+G186E+T251E+L405M | 0.9 | 1.1 | 1.1 |
| G4Q+R176K+G186E+T323G+L405M | 2.9 | 1.6 | 1.3 |
| G4Q+R176K+1206Y | 0.6 | 1.1 | 1.2 |
| G4Q+R176K+1206Y+L405M | 1.5 | 0.9 | 0.8 |
| G4Q+R176K+I206Y+L405M+N460G | 0.6 | 0.9 | 1.2 |
| G4Q+R176K+1206Y+N460G | 1.2 | 0.9 | 0.8 |
| G4Q+R176K+I206Y+T251E+L405M | 0.4 | 0.9 | 1.1 |
| G4Q+R176K+I206Y+T251 E+L405M+N460G | 1.1 | 1 | 0.9 |
| G4Q+R176K+I206Y+T251E+T323A+N460G | 1.8 | 1.4 | 1.1 |
| G4Q+Y160W+ R176K+G186E+I206Y+T251E | 1.2 | 1.1 | 0.9 |
| G4Q+Y160W+R176K+G186E+T251E+L405M | 0.9 | 1 | 1.2 |
| G4Q+Y160W+R176K+I206Y+T251E+T323G+L405M+N460G | 0.8 | 1.2 | 1.1 |
| N25H+G186E+1206Y | 1.1 | 1 | 1.1 |
| N25H+G186E+1206Y+L405M | 1.6 | 1.1 | 1.1 |
| N25H+G186E+I206Y+T251E+L405M | 1.2 | 1.2 | 1 |
| N25H+G186E+L405M | 1.3 | 1.2 | 1.1 |
| N25H+G186E+T193E+I206Y+L405M | 1 | 1.3 | 1.2 |
| N25H+R176K+G186E | 1.9 | 1.6 | 1.5 |
| N25H+R176K+G186E+E276R+T323G+L405M | 1.2 | 1 | 0.9 |
| N25H+R176K+G186E+I206Y | 1.5 | 1.1 | 1.1 |
| N25H+R176K+G186E+I206Y+E276R | 1.6 | 0.8 | 0.8 |
| N25H+R176K+G186E+I206Y+T251E | 1 | 1.1 | 1.1 |
| N25H+R176K+G186E+I206Y+T251E+L405M | 2 | 1.3 | 1.1 |
| N25H+R176K+G186E+L405M | 2 | 1.6 | 1.1 |
| N25H+R176K+G186E+T193E+I206Y | 1.2 | 1.3 | 1.1 |
| N25H+R176K+G186E+T193E+I206Y+T251E | 1.3 | 1.2 | 1 |
| N25H+R176K+G186E+T193E+I206Y+T251E+E276R+L405M | 2.2 | 1.1 | 1 |
| N25H+R176K+G186E+T251E+E276R | 3 | 1.2 | 1 |
| N25H+R176K+1206Y | 2.2 | 1.1 | 0.9 |
| N25H+R176K+1206Y+L405M | 1.1 | 1 | 0.9 |
| N25H+R176K+I206Y+N460G | 2.1 | 1.1 | 1 |
| N25H+R 176K+I206Y+T251 E | 0.9 | 1.2 | 1.1 |
| N25H+R176K+I206Y+T251E+L405M | 0.7 | 1 | 1.1 |
| N25H+Y160W+G186E+I206Y | 0.7 | 1.1 | 1.2 |
| N25H+Y160W+G186E+I206Y+T251 E | 1 | 1.3 | 1.1 |
| N25H+Y160W+G186E+I206Y+T251E+L405M | 0.6 | 0.8 | 1.1 |
| N25H+Y160W+G186E+I206Y+T323G+L405M | 1.5 | 1.4 | 1.1 |
| N25H+Y160W+R176K+G186E+I206Y+T251E+L405M | 0.8 | 1 | 1.1 |
| N25H+Y160W+R176K+G186E+L405M | 1 | 1.2 | 1.1 |
| N25H+Y160W+R176K+G186E+T193E+I206Y | 1.3 | 1 | 0.8 |
| N25H+Y160W+R176K+G186E+T193E+I206Y+L405M | 0.7 | 1.1 | 1.1 |
| N25H+Y160W+R176K+G186E+T193E+I206Y+T251E+L405M | 0.9 | 1.2 | 1 |
| N25H+Y160W+R176K+G186E+T251E | 1.7 | 1.4 | 1.1 |
| N25H+Y160W+R176K+G186E+T251E+L405M | 2.5 | 1.4 | 1.3 |
| N25H+Y160W+R176K+I206Y+T251E+T323G+L405M | 1.9 | 1.5 | 1.2 |
| R176K+G186E+I206Y | 0.7 | 1.1 | 1.2 |
| R176K+G186E+I206Y+L405M | 0.8 | 1.2 | 1.1 |
| R176K+G186E+I206Y+T251E+L405M | 0.8 | 1 | 1.1 |
| R176K+G186E+L405M | 1.4 | 1.3 | 1.1 |
| R176K+G186E+T193E+I206Y+L405M | 1.5 | 1.3 | 1.1 |
| R176K+G186E+T193E+I206Y+T251E+L405M | 1.5 | 1.4 | 1.2 |
| R176K+G186E+T251E+L405M | 2.9 | 1.5 | 1.3 |
| R176K+I206Y | 1.2 | 1 | 0.9 |
| R176K+I206Y+L405M | 1.1 | 1 | 0.9 |
| R176K+I206Y+L405M+N460G | 1.3 | 1.2 | 0.9 |
| R176K+I206Y+N460G | 0.6 | 0.9 | 1.1 |
| R176K+I206Y+T251E | 1 | 0.9 | 1.1 |
| Y160W+G186E+T193E+I206Y+L405M | 0.7 | 0.9 | 1.1 |
| Y160W+R176K+G186E+I206Y | 1.1 | 1.2 | 1.1 |
| Y160W+R176K+G186E+L405M | 0.7 | 1 | 1.1 |
| Y160W+R176K+G186E+T193E+I206Y | 1.8 | 1.2 | 1.1 |
| Y160W+R176K+G186E+T193E+1206Y+L405M | 1.2 | 1.1 | 1.1 |
| Y160W+R176K+G186E+T193E+I206Y+T251E | 0.8 | 0.9 | 1.1 |
| Y160W+R176K+I206Y+L405M | 0.5 | 0.7 | 1.1 |
| Y160W+R176K+I206Y+T323G+L405M+N460G | 0.8 | 1 | 1.2 |
| G4Q+R176K+G186E+T193E+I206Y+T251E+G258Q+K281N+L405M | 0.4 | 1.1 | 1.1 |
| G4Q+R176K+G186E+T193E+I206Y+T251E+G258Q+L405M | 1.4 | 1.3 | 1.2 |
| G4Q+R176K+G186E+T193E+I206Y+T251E+G258Q+Y363E+L405M | 0.5 | 1.1 | 1 |
| G4Q+R176K+G186E+T193E+I206Y+T251E+K281N+Y363E+L405M | 0.1 | 1 | 1.1 |
| G4Q+R176K+G186E+T193E+I206Y+T251E+L405M | 1 | 1.4 | 1.2 |
| G4Q+R176K+G186E+T193E+I206Y+T251E+Y363E+L405M | 0.6 | 1 | 1.1 |
| G4Q+R176K+G186E+T193E+I206Y+T251E+Y363H+L405M | 0.6 | 1 | 1.1 |
| G4Q+R176K+R181Q+G186E+T193E+I206Y+T251E+G258Q+L405M | 1 | 1.1 | 0.9 |
| G4Q+R176K+R181Q+G186E+T193E+I206Y+T251E+L405M | 1.1 | 1.6 | 1.2 |
| G4Q+R176K+R181Q+G186E+T193E+I206Y+T251E+Y363E+L405M | 0.3 | 0.5 | 1.1 |
| G4Q+T8A+Y135T+R176K+G186E+T193E+I206Y+T251E+G258Q+L4 05M | 0.2 | 1 | 1.2 |
| G4Q+Y100W+R176K+G186E+T193E+I206Y+T251 E+G258Q+K281N+ L405M | 0.4 | 1 | 1.1 |
| G4Q+Y100W+R176K+G186E+T193E+I206Y+T251E+G258Q+K281N+ Y363E+L405M | 0.4 | 0.7 | 1.1 |
| G4Q+Y100W+R176K+G186E+T193E+I206Y+T251E+G258Q+K281N+ Y363H+L405M | 1.7 | 1.3 | 1.1 |
| G4Q+Y100W+R176K+G186E+T193E+I206Y+T251E+G258Q+Y363E+ L405M | 1.3 | 1.4 | 1.1 |
| G4Q+Y100W+R176K+G186E+T193E+I206Y+T251E+G258Q+Y363H+ L405M | 1.7 | 1.4 | 1.1 |
| G4Q+Y100W+R176K+G186E+T193E+I206Y+T251E+K281N+L405M | 0.6 | 0.9 | 1.1 |
| G4Q+Y100W+R176K+G186E+T193E+I206Y+T251 E+K281N+Y363H+ L405M | 0.8 | 1.3 | 1 |
| G4Q+Y100W+R176K+G186E+T193E+I206Y+T251E+L405M | 1.3 | 1.4 | 1.3 |
| G4Q+Y100W+R176K+G186E+T193E+I206Y+T251E+Y363E+L405M | 0.5 | 1.1 | 1 |
| G4Q+Y100W+R176K+G186E+T193E+I206Y+T251E+Y363H+L405M | 1.3 | 1.3 | 1.2 |
| G4Q+Y100W+R176K+R181Q+G186E+T193E+I206Y+T251E+G258Q +K281N+Y363E+L405M | 2.5 | 1.2 | 0.9 |
| G4Q+Y100W+R176K+R181Q+G186E+T193E+I206Y+T251E+Y363H+ L405M | 0.6 | 1.2 | 1 |
| G4Q+Y100W+Y135T+R176K+G186E+T193E+I206Y+T251E+G258Q+ K281N+L405M | 0.3 | 0.8 | 1.1 |
| G4Q+Y100W+Y135T+R176K+G186E+T193E+I206Y+T251E+G258Q+ L405M | 0.8 | 1.4 | 0.9 |
| G4Q+Y100W+Y135T+R176K+G186E+T193E+I206Y+T251E+G258Q+ Y363H+L405M | 1.6 | 1.3 | 1 |
| G4Q+Y100W+Y135T+R176K+G186E+T193E+I206Y+T251E+K281N+ L405M | 0.7 | 0.8 | 1.1 |
| G4Q+Y100W+Y135T+R176K+G186E+T193E+I206Y+T251E+L405M | 1.7 | 1.4 | 1.1 |
| G4Q+Y100W+Y135T+R176K+G186E+T193E+I206Y+T251E+Y363E+ L405M | 0.9 | 1.2 | 1.1 |
| G4Q+Y100W+Y135T+R176K+G186E+T193E+I206Y+T251E+Y363E+ L405M | 0.6 | 0.9 | 1.1 |
| G4Q+Y100W+Y135T+R176K+R181Q+G186E+T193 E+I206Y+T251E+ G258Q+L405M | 0.4 | 0.9 | 1.1 |
| G4Q+Y100W+Y135T+R176K+R181Q+G186E+T193E+I206Y+T251E+ L405M | N/A | 0.9 | 1.1 |
| G4Q+Y135T+R176K+G186E+T193E+I206Y+T251E+G258Q+K281N+ L405M | 1.1 | 1.2 | 1.1 |
| G4Q+Y135T+R176K+G186E+T193E+I206Y+T251E+G258Q+K281N+ Y363E+L405M | 0.8 | 1.2 | 1 |
| G4Q+Y135T+R176K+G186E+T193E+I206Y+T251E+G258Q+L405M | 1.3 | 1.6 | 1.2 |
| G4Q+Y135T+R176K+G186E+T193E+I206Y+T251E+G258Q+Y363H+ L405M | 0.7 | 1.1 | 1.1 |
| G4Q+Y135T+R176K+G186E+T193E+I206Y+T251E+K281N+L405M | 2.4 | 1.7 | 1.2 |
| G4Q+Y135T+R176K+G186E+T193E+I206Y+T251E+L405M | 1.8 | 1.5 | 1.1 |
| G4Q+Y135T+R176K+G186E+T193E+I206Y+T251E+Y363E+L405M | 0.8 | 1.2 | 1.4 |
| G4Q+Y135T+R176K+G186E+T193E+I206Y+T251E+Y363H+L405M | 0.9 | 1.4 | 1.2 |
| G4Q+Y135T+R176K+R181Q+G186E+T193E+I206Y+T251E+G258Q+ K281N+L405M | 0.9 | 1 | 1.2 |
| G4Q+Y135T+R176K+R181Q+G186E+T193E+I206Y+T251 E+K281N+ Y363H+L405M | 0.5 | 0.9 | 1.1 |
| N25H+R176K+G186E+I206Y | 0.9 | 1.2 | 1.1 |
| N25H+R176K+G186E+I206Y+G258Q | 1.2 | 1.2 | 1 |
| N25H+R176K+G186E+I206Y+G258Q+K281N+Y363E | 1.8 | 1.7 | 1.5 |
| N25H+R176K+G186E+I206Y+G258Q+Y363E | 0.8 | 1.4 | 1.4 |
| N25H+R176K+G186E+I206Y+G258Q+Y363E+N123D | 1 | 1.2 | 1 |
| N25H+R176K+G186E+I206Y+K281 N | 1.1 | 1.2 | 1.2 |
| N25H+R176K+G186E+I206Y+Y363E | 4.5 | 2 | 1.5 |
| N25H+R176K+G186E+T193E+I206Y | 0.9 | 1.1 | 1 |
| N25H+R176K+G186E+T193E+I206Y+G258Q | N/A | 2.1 | 1.6 |
| N25H+R176K+G186E+T193E+I206Y+K281N+Y363E | 1.2 | 1.4 | 1.4 |
| N25H+R176K+G186E+T193E+I206Y+Y363E | 2.3 | 1.9 | 1.3 |
| N25H+R176K+R181Q+G186E+I206Y | 1.5 | 1.4 | 1.6 |
| N25H+R176K+R181Q+G186E+I206Y+Y363E | 1 | 1.2 | 0.9 |
| N25H+R176K+R181Q+G186E+T193E+I206Y | 2.4 | 1.7 | 1.3 |
| N25H+Y100W+R176K+G186E+I206Y | 3.8 | 1.9 | 1.5 |
| N25H+Y100W+R176K+G186E+I206Y+G258Q | 1.3 | 1.6 | 1.3 |
| N25H+Y100W+R176K+G186E+I206Y+G258Q+K281N | 0.7 | 1.2 | 1.3 |
| N25H+Y100W+R176K+G186E+I206Y+G258Q+K281N+Y363E | 1.5 | 1.7 | 1.6 |
| N25H+Y100W+R176K+G186E+I206Y+G258Q+Y363E | 1.4 | 1.6 | 1.5 |
| N25H+Y100W+R176K+G186E+I206Y+K281N | 1.5 | 1.7 | 1.6 |
| N25H+Y100W+R176K+G186E+1206Y+K281N+Y363E | 1.5 | 1.5 | 1.5 |
| N25H+Y100W+R176K+G186E+I206Y+Q361R | 1.3 | 1.5 | 1.3 |
| N25H+Y100W+R176K+G186E+I206Y+Y363E | 1.5 | 1.6 | 1.6 |
| N25H+Y100W+R176K+G186E+T193E+I206Y | 3 | 1.5 | 1.6 |
| N25H+Y100W+R176K+G186E+T193E+I206Y+G258Q | 1.6 | 1.3 | 1.5 |
| N25H+Y100W+R176K+G186E+T193E+I206Y+G258Q+H473R | 1.5 | 1.8 | 1.8 |
| N25H+Y100W+R176K+G186E+T193E+I206Y+G258Q+K281N | N/A | 0.7 | 1.3 |
| N25H+Y100W+R176K+G186E+T193E+I206Y+G258Q+K281N+Y363E | 0.8 | 1.3 | 1.3 |
| N25H+Y100W+R176K+G186E+T193E+I206Y+G258Q+Y363E | 1.3 | 1.5 | 1.4 |
| N25H+Y100W+R176K+G186E+T193E+I206Y+Y363E | 0.9 | 1.2 | 1.2 |
| N25H+Y100W+R176K+R181Q+G186E+I206Y+Y363E | 0.7 | 1.1 | 1.1 |
| N25H+Y100W+R176K+R181Q+G186E+T193E+I206Y | 0.9 | 1 | 1.1 |
| N25H+Y100W+R176K+R181Q+G186E+T193E+I206Y+G258Q+K281 N | 0.4 | 0.7 | 1.2 |
| N25H+Y100W+R176K+R181Q+G186E+T193E+I206Y+G258Q+Y363 E | 0.4 | 0.8 | 1.2 |
| N25H+Y100W+Y135C+R176K+G186E+I206Y+G258Q+Y363E | 0.5 | 0.7 | 1.1 |
| N25H+Y100W+Y135T+R176K+G186E+I206Y | 3.3 | 2.1 | 1.4 |
| N25H+Y100W+Y135T+R176K+G186E+I206Y+G258Q | 1.6 | 2 | 1.9 |
| N25H+Y100W+Y135T+R176K+G186E+I206Y+G258Q+K281N | 0.4 | 1.2 | 1.4 |
| N25H+Y100W+Y135T+R176K+G186E+I206Y+G258Q+K281N+Y363E | 1.2 | 1.5 | 1.5 |
| N25H+Y100W+Y135T+R176K+G186E+I206Y+G258Q+Y363E | 0.9 | 1.2 | 1.1 |
| N25H+Y100W+Y135T+R176K+G186E+I206Y+K281N | 1.1 | 1.5 | 1.3 |
| N25H+Y100W+Y135T+R176K+G186E+I206Y+K281N+Y363E | 0.7 | 1.1 | 1.1 |
| N25H+Y100W+Y135T+R176K+G186E+I206Y+Y363E | 2.7 | 1.6 | 1.4 |
| N25H+Y100W+Y135T+R176K+G186E+T193E+I206Y | 1.1 | 1 | 1 |
| N25H+Y100W+Y135T+R176K+G186E+T193E+I206Y+G258Q+Y363E | 0.6 | 1.2 | 1.3 |
| N25H+Y100W+Y135T+R176K+G186E+T193E+I206Y+Y363E | 2.2 | 1.7 | 1.2 |
| N25H+Y100W+Y135T+R176K+R181Q+G186E+I206Y | 1 | 1.1 | 1.2 |
| N25H+Y100W+Y135T+R176K+R181Q+G186E+1206Y+G258Q+K281 N | 1.9 | 1.3 | 1.2 |
| N25H+Y100W+Y135T+R176K+R181Q+G186E+T193E+I206Y | 0.7 | 1.1 | 1 |
| N25H+Y100W+Y135T+R176K+R181Q+G186E+T193E+I206Y+G258Q | 0.3 | 0.7 | 1.1 |
| N25H+Y100W+Y135T+R176K+R181Q+G186E+T193E+I206Y+Y363E | 0.3 | 1 | 1.1 |
| N25H+Y135T+R176K+G186E+I206Y | 1.2 | 1.2 | 1.2 |
| N25H+Y135T+R176K+G186E+I206Y+G258Q | 1.1 | 1.5 | 1.3 |
| N25H+Y135T+R176K+G186E+I206Y+G258Q+K281N | 1.2 | 1.3 | 1.4 |
| N25H+Y135T+R176K+G186E+I206Y+G258Q+K281N+Y363E | 1.3 | 1.7 | 1.6 |
| N25H+Y135T+R176K+G186E+I206Y+G258Q+Y363E | 1.7 | 1.5 | 1.2 |
| N25H+Y135T+R176K+G186E+I206Y+K281N | 1.4 | 1.4 | 1.3 |
| N25H+Y135T+R176K+G186E+I206Y+K281N+Y363E | 0.9 | 1.4 | 1.2 |
| N25H+Y135T+R176K+G186E+I206Y+K281N+Y363E+R415S | 0.7 | 1.1 | 1 |
| N25H+Y135T+R176K+G186E+I206Y+Y363E | 1.4 | 1.7 | 1.5 |
| N25H+Y135T+R176K+G186E+T193E+I206Y+G258Q | 1.1 | 1.4 | 1.4 |
| N25H+Y135T+R176K+R181Q+G186E+I206Y+G258Q | 1.3 | 1.3 | 1.2 |
| N25H+Y135T+R176K+R181Q+G186E+I206Y+G258Q+Y363E | 0.5 | 1.1 | 1.1 |
| N25H+Y135T+R176K+R181Q+G186E+T193E+I206Y+G258Q+K281N | 0.9 | 1.2 | 1.3 |

## Claims

**1.** An alpha-amylase variant of a parent alpha-amylase, wherein said variant comprises
(i) amino acid alteration at one or more positions corresponding to positions selected from the group consisting of 1, 2, 3, 4, 5, 6, 7, 8, 10, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 45, 47, 48, 51, 54, 59, 60, 63, 70, 71, 72, 73, 75, 76, 81, 82, 83, 86, 87, 89, 90, 91, 92, 93, 94, 95, 96, 97, 98, 99, 100, 103, 106, 108, 109, 113, 114, 115, 116, 117, 119, 120, 123, 125, 126, 128, 129, 131, 132, 133, 134, 135, 136, 139, 141, 142, 143, 144, 145, 146, 147, 149, 150, 151, 154, 155, 156, 158, 160, 161, 162, 163, 164, 165, 169, 170, 172, 173, 174, 175, 176, 177, 178, 180, 182, 184, 185, 187, 188, 189, 191, 192, 193, 203, 208, 210, 211, 212, 213, 214, 215, 216, 218, 219, 221, 222, 223, 224, 225, 226, 227, 230, 231, 233, 234, 235, 236, 237, 238, 240, 242, 243, 245, 249, 250, 251, 252, 253, 255, 256, 257, 258, 259, 260, 261, 262, 263, 264, 265, 268, 269, 270, 271, 272, 273, 274, 275, 276, 277, 279, 280, 281, 282, 284, 285, 286, 287, 288, 289, 290, 291, 292, 293, 294, 295, 296, 297, 298, 299, 301, 302, 303, 304, 306, 307, 308, 309, 310, 311, 312, 313, 314, 315, 317, 318, 319, 320, 321, 322, 323, 324, 326, 327, 333, 334, 336, 337, 338, 341, 342, 343, 344, 345, 346, 348, 350, 351, 352, 353, 354, 355, 356, 357, 358, 359, 360, 362, 363, 364, 365, 366, 367, 368, 370, 372, 375, 376, 378, 379, 381, 382, 383, 384, 385, 387, 388, 389, 390, 391, 392, 393, 394, 395, 396, 397, 398, 399, 400, 401, 403, 405, 406, 407, 408, 410, 413, 414, 415, 416, 418, 421, 424, 426, 427, 429, 431, 432, 434, 436, 438, 439, 440, 442, 443, 445, 446, 447, 448, 449, 451, 453, 455, 456, 457, 458, 459, 460, 461, 462, 463, 465, 467, 468, 470, 471, 474, 478, 480, and 482 according to the numbering of the amino acid sequence set forth in SEQ ID NO: 3,
(ii) said variant has at least 60% but less than 100% sequence identity, preferably at least 91% identity, with the amino acid sequence set forth in SEQ ID NO: 1, and
(iii) said variant has alpha-amylase activity, preferably wherein the parent alpha-amylase for the alpha-amylase variant is an amylase according to SEQ ID NO: 1.

**2.** The alpha-amylase variant according to claim 1, wherein said variant comprises one or more amino acid substitution selected from the group of amino acid substitutions consisting of X100D, X100F, X100K, X100L, X103A, X106D, X108A, X109A, X10A, X10C, X10E, X10F, X10L, X10W, X10Y, X113F, X113H, X113M, X113R, X113V, X113W, X113Y, X114A, X114C, X114D, X114E, X114F, X114G, X114H, X114I, X114K, X114L, X114M, X114N, X114Q, X114R, X114S, X114V, X114W, X114Y, X115C, X115D, X115K, X115M, X115N, X115Q, X115R, X115S, X115T, X115V, X116I, X116K, X116L, X116M, X116R, X116T, X117A, X117C, X117D, X117F, X117I, X117N, X117P, X117R, X117W, X117Y, X118A, X118D, X118E, X119H, X119P, X119R, X119S, X119Y, X120E, X120G, X120M, X120S, X120W, X120Y, X123D, X123S, X125A, X125D, X125E, X125F, X125G, X125H, X125K, X125L, X125M, X125N, X125Q, X125R, X125T, X125V, X125W, X125Y, X126D, X128C, X128E, X128L, X128M, X128W, X128Y, X129E, X12N, X12S, X131C, X131I, X131L, X131Q, X131W, X132T, X133A, X133C, X133D, X133E, X133H, X133K, X133N, X133P, X133Q, X133S, X134C, X134E, X134F, X134I, X134L, X134M, X134P, X134T, X134V, X134W, X134Y, X135D, X135E, X135G, X135M, X135N, X135P, X135S, X135T, X135W, X136A, X136C, X136D, X136E, X136F, X136H, X136L, X136M, X136N, X136P, X136W, X139C, X139S, X13A, X13Y, X141V, X142C, X142E, X142F, X142L, X142M, X142Q, X142R, X142W, X142Y, X143F, X144C, X144E, X144G, X144K, X144N, X144Q, X144R, X144S, X144T, X144V, X144Y, X145A, X146C, X146D, X146E, X146F, X146G, X146H, X146K, X146L, X146S, X146T, X146W, X147M, X149E, X14N, X150C, X150E, X150Q, X151C, X151E, X154A, X154Y, X155Y, X156E, X156V, X158H, X158N, X158Y, X15R, X160D, X160E, X160W, X161T, X162V, X163A, X163Q, X163T, X164V, X165S, X165T, X165W, X169A, X169D, X169E, X169S, X169V, X16F, X16R, X170C, X170F, X172A, X172C, X172D, X172K, X172N, X173I, X173Y, X174D, X174E, X174G, X174H, X174M, X174N, X174P, X174S, X174T, X175A, X175G, X175H, X175Q, X176K, X176S, X176T, X177A, X177G, X177K, X177N, X177P, X177R, X177S, X177W, X178F, X17K, X17V, X180M, X180N, X180T, X180W, X182D, X182E, X182N, X182Q, X182S, X184G, X185E, X185M, X185N, X187A, X187D, X187M, X187V, X188H, X188T, X188V, X189I, X18F, X18l, X18K, X18M, X18R, X18T, X191F, X191H, X191K, X191M, X191W, X191Y, X192A, X192T, X193D, X193E, X193G, X193V, X19A, X19C, X19D, X19F, X19G, X19H, X19I, X19K, X19L, X19M, X19P, X19Q, X19S, X19T, X19Y, X1R, X1V, X203E, X203R, X208F, X208I, X208Y, X20A, X20C, X20D, X20E, X20F, X20G, X20H, X20K, X20L, X20M, X20N, X20P, X20Q, X20S, X20T, X20V, X20W, X20Y, X210A, X210C, X210D, X210E, X210F, X210M, X210N, X210Q, X210S, X210Y, X211A, X211C, X211D, X211E, X211G, X211H, X211L, X211N, X211Q, X211S, X211T, X211V, X212C, X212D, X212I, X212L, X212W, X213A, X213C, X213M, X213R, X213S, X214E, X214P, X215A, X215D, X215E, X215H, X215W, X216G, X216H, X218A, X218C, X218D, X218E, X218F, X218G, X218H, X218I, X218K, X218L, X218N, X218Q, X218S, X218T, X218V, X218W, X218Y, X219A, X219C, X219D, X219E, X219F, X219G, X219H, X219K, X219M, X219Q, X219R, X219S, X219T, X219W, X219Y, X21E, X21S, X221F, X221N, X222D, X222K, X222S, X222T, X223C, X223L, X223V, X223Y, X224F, X224V, X225A, X225C, X225E, X225F, X225H, X225I, X225N, X225R, X225S, X225Y, X226A, X226C, X226D, X226E, X226G, X226H, X226I, X226L, X226M, X226Q, X226R, X226S, X226T, X226V, X226W, X226Y, X227C, X227F, X227G, X227H, X227I, X227K, X227L, X227M, X227R, X227T, X227V, X227W, X227Y, X22A, X22D, X22E, X22F, X22G, X22K, X22L, X22M, X22Q, X22R, X22T, X22W, X22Y, X230A, X230F, X231C, X231D, X231N, X233C, X233H, X233I, X233M, X233T, X233W, X233Y, X234C, X235L, X235M, X235V, X236Y, X237C, X237I, X238A, X238F, X238T, X23N, X23Q, X23W, X240M, X242M, X242N, X243D, X243F, X245E, X245H, X245M, X249D, X249I, X24G, X250V, X251A, X251E, X251F, X251L, X251M, X251S, X251T, X252C, X252I, X252S, X253C, X253G, X253Y, X255D, X255F, X255I, X255T, X255V, X256C, X257L, X257V, X257Y, X258F, X258Q, X258R, X259L, X25A, X25C, X25D, X25F, X25G, X25H, X25K, X25L, X25M, X25Q, X25S, X25W, X25Y, X260H, X261R, X262C, X262D, X262E, X262H, X262P, X262Y, X263I, X264H, X264T, X264W, X265S, X268F, X268G, X269M, X26A, X26D, X26E, X26F, X26L, X26M, X26P, X26S, X26V, X26Y, X270A, X270Q, X270Y, X271S, X272F, X272G, X272L, X272S, X273A, X273C, X273D, X273E, X273F, X273H, X273I, X273L, X273M, X273P, X273Q, X273R, X273V, X273W, X273Y, X274F, X274S, X275V, X276D, X276K, X276L, X276N, X276R, X276Y, X277D, X277E, X277T, X279A, X279P, X27A, X27D, X27F, X27G, X27H, X27I, X27Q, X27R, X27T, X27V, X280D, X280F, X280G, X280H, X280I, X280K, X280N, X280R, X280V, X280Y, X281A, X281D, X281E, X281H, X284A, X284F, X284H, X284L, X284M, X284N, X284Y, X285G, X285L, X285N, X285P, X286Q, X287A, X287D, X287E, X287H, X287T, X288A, X288K, X288P, X288Y, X289F, X289G, X289R, X289T, X28C, X28D, X28E, X28F, X28G, X28I, X28K, X28N, X28Q, X28S, X28T, X28V, X290D, X290M, X290N, X290Q, X290W, X291D, X291K, X291T, X291Y, X292C, X292D, X292F, X292I, X292L, X292T, X292W, X292Y, X293D, X293E, X293F, X293K, X293R, X294G, X294T, X295F, X296A, X296C, X296L, X296Y, X297E, X297F, X297H, X297K, X297M, X297S, X297V, X299G, X299I, X299K, X299L, X299S, X299Y, X29D, X29E, X29F, X29G, X29H, X29I, X29K, X29L, X29N, X29P, X29Q, X29V, X29W, X29Y, X2I, X2S, X301F, X302H, X302I, X302Q, X302V, X302Y, X303E, X303H, X303I, X303K, X303L, X303M, X303N, X303P, X303R, X303T, X304A, X304D, X304E, X304H, X304K, X304M, X304N, X304P, X304R, X304T, X304W, X304Y, X306A, X306D, X306E, X306G, X306H, X306I, X306M, X306Q, X306R, X306S, X306T, X306V, X306W, X306Y, X307F, X307M, X308S, X309H, X309L, X309Q, X30A, X30E, X30F, X30G, X30H, X30I, X30K, X30L, X30M, X30Q, X30T, X30W, X30Y, X310A, X310Q, X311A, X311E, X311G, X311H, X311K, X311N, X311R, X311T, X311Y, X312L, X312M, X313V, X314C, X314E, X314K, X314Q, X315A, X315C, X315E, X315H, X315K, X315T, X318I, X318S, X318T, X319A, X319D, X319H, X319I, X319K, X319M, X319N, X319P, X319S, X319T, X319W, X31N, X31Q, X31S, X31T, X31V, X31W, X320A, X320C, X320D, X320E, X320G, X320H, X320K, X320L, X320N, X320Q, X320S, X320Y, X321A, X321E, X321K, X321N, X321T, X321V, X321W, X323A, X323G, X323K, X323L, X323V, X324K, X324L, X324M, X324W, X324Y, X326G, X326N, X326S, X326Y, X327C, X327L, X327M, X32A, X32D, X32E, X32F, X32H, X32I, X32L, X32M, X32N, X32P, X32Q, X32T, X32W, X333I, X334T, X336K, X337A, X337C, X337F, X337G, X337I, X337K, X337L, X337M, X337N, X337Q, X337R, X337S, X337T, X337V, X337Y, X338G, X338S, X338T, X33D, X33E, X33H, X33K, X33M, X33Q, X33R, X33Y, X341V, X342P, X343L, X343T, X343W, X343Y, X344I, X344Q, X344V, X345D, X345G, X345M, X345N, X345Q, X345S, X345T, X346A, X346C, X346D, X346G, X346H, X346N, X346Q, X348T, X34H, X34I, X34V, X350H, X350K, X350P, X351A, X351M, X352S, X353H, X354I, X354N, X354T, X354Y, X355I, X355M, X356I, X356V, X357A, X358I, X358L, X358N, X358P, X358V, X359E, X35A, X35C, X35D, X35G, X35H, X35I, X35L, X35M, X35N, X35P, X35Q, X35R, X35S, X35T, X35V, X35Y, X360A, X360F, X360G, X360I, X360L, X360N, X360Q, X360R, X360S, X360T, X360V, X360Y, X362F, X362K, X362M, X362N, X362T, X362V, X362Y, X363A, X363C, X363D, X363E, X363G, X363H, X363K, X363L, X363M, X363P, X363Q, X363R, X363S, X363T, X363V, X363W, X363Y, X364A, X364C, X364G, X364K, X364L, X364N, X364S, X364T, X364V, X366I, X366L, X366T, X367E, X367S, X368A, X368F, X368L, X368N, X36A, X36E, X36G, X36I, X36K, X36M, X36N, X36P, X36Q, X36R, X36S, X36T, X36V, X370E, X370I, X372A, X372C, X372E, X372F, X372H, X372M, X372N, X372Q, X375A, X375D, X375E, X375I, X375K, X375Q, X375R, X375T, X375W, X375Y, X376G, X376I, X376K, X376L, X376M, X376Q, X376R, X376S, X376V, X377Q, X378C, X378D, X378E, X378R, X379A, X379L, X379S, X37A, X37G, X37M, X37P, X37T, X37V, X37W, X381E, X381V, X382A, X382H, X382K, X382L, X382N, X382Q, X382S, X383C, X383D, X383E, X383H, X383I, X383M, X383N, X383Q, X383R, X383S, X383V, X383Y, X384A, X384C, X384D, X384E, X384F, X384I, X384L, X384M, X384N, X384Q, X384R, X384T, X384V, X384W, X384Y, X385A, X385C, X385D, X385E, X385F, X385G, X385H, X385I, X385L, X385M, X385N, X385P, X385Q, X385R, X385S, X385T, X385V, X385W, X385Y, X387C, X387E, X387N, X388E, X388F, X388H, X388I, X388M, X388R, X388V, X389G, X389H, X389K, X38N, X390D, X390F, X390M, X390N, X390P, X390R, X391A, X391F, X391G, X391K, X391M, X391N, X391Q, X391S, X391T, X391Y, X392C, X392V, X393E, X393H, X393P, X393S, X393V, X394A, X394C, X394E, X394H, X394I, X394L, X394M, X394N, X394R, X394S, X395A, X395H, X395M, X395V, X396H, X396P, X397D, X397H, X397P, X397S, X398M, X399P, X39E, X39K, X3A, X3F, X3G, X3I, X3K, X3L, X3Q, X3V, X400A, X400D, X400E, X400G, X400H, X400I, X400K, X400L, X400M, X400N, X400P, X400Q, X400R, X400S, X400V, X400W, X401I, X401K, X401M, X401T, X403N, X405C, X405H, X405M, X405T, X405V, X406P, X407D, X407R, X407S, X408E, X408I, X408Q, X40I, X40S, X410H, X410I, X410K, X410L, X410P, X410R, X410Y, X413S, X414C, X414E, X414S, X415E, X415I, X416S, X418C, X418N, X418P, X41C, X41D, X41E, X41G, X41Q, X41S, X41T, X421N, X421P, X424A, X426D, X426W, X427C, X427F,
X427G, X427K, X427Q, X427R, X427S, X427T, X427V, X429A, X429D, X429E, X429F, X429G, X429I, X429M, X429N, X429P, X429Q, X429S, X429T, X429V, X429W, X42C, X42I, X42Q, X42V, X431I, X434S, X436E, X438F, X438P, X438Y, X439C, X439P, X440V, X442Q, X443H, X443T, X445A, X445C, X445D, X445F, X445G, X445H, X445K, X445M, X445Q, X445R, X445S, X445T, X445V, X446F, X446I, X446L, X446P, X446Q, X446R, X446V, X446W, X447V, X448D, X448E, X448H, X448N, X449A, X449F, X449G, X449K, X449L, X449M, X449N, X449P, X449Q, X449R, X449S, X449V, X449W, X449Y, X451L, X453G, X453I, X453N, X453P, X453Y, X455L, X456L, X456M, X456R, X456S, X456V, X456W, X456Y, X457A, X457C, X457E, X457F, X457G, X457K, X457L, X457M, X457R, X457S, X457T, X457V, X457W, X458I, X458K, X458V, X458W, X459C, X459D, X459E, X459I, X459K, X459N, X459Q, X459R, X459V, X459Y, X45G, X45N, X460A, X460D, X460E, X460G, X460Q, X460R, X460S, X460T, X460V, X461A, X461E, X461F, X461K, X461L, X461M, X461N, X461Q, X461R, X461S, X461V, X462K, X463L, X465H, X465P, X465R, X465V, X467A, X467E, X467H, X468D, X468H, X470A, X470Q, X470T, X471E, X474F, X474H, X474P, X478A, X478E, X47S, X480D, X480E, X480M, X480R, X480Y, X482C, X482T, X482W, X48F, X48I, X48M, X48Y, X4A, X4C, X4K, X4M, X4Q, X4R, X4S, X51Q, X51T, X51V, X54D, X54G, X54Q, X59T, X5A, X5C, X5D, X5E, X5F, X5H, X5I, X5K, X5L, X5M, X5N, X5P, X5Q, X5R, X5V, X5Y, X60T, X63C, X63V, X6A, X6C, X6E, X6F, X6G, X6H, X6K, X6L, X6M, X6P, X6Q, X6S, X6T, X6V, X6W, X6Y, X70F, X70H, X70L, X70M, X70N, X70Y, X71D, X72C, X72D, X72E, X72N, X72T, X73L, X73N, X73Q, X75A, X75G, X75I, X75L, X75P, X75T, X75W, X76C, X76E, X76G, X76L, X76T, X76V, X7C, X7E, X7F, X7H, X7K, X7N, X7P, X7Q, X7R, X7S, X7V, X7W, X7Y, X81H, X81L, X82K, X82M, X83A, X83D, X83E, X83G, X83R, X83S, X86K, X87D, X87R, X89A, X89C, X89F, X89G, X89L, X89M, X89R, X89S, X8A, X8C, X8F, X8I, X8M, X8P, X8S, X8V, X8W, X8Y, X90A, X90D, X90E, X90F, X90G, X90I, X90M, X90N, X90Q, X90R, X90S, X90V, X90Y, X91C, X91D, X91E, X91F, X91G, X91H, X91I, X91K, X91L, X91M, X91N, X91Q, X91S, X91T, X91V, X91W, X91Y, X92D, X92M, X92V, X93K, X93Q, X94A, X94D, X94E, X94K, X94M, X94V, X94Y, X95E, X95I, X95L, X95V, X96K, X96N, X96Q, X97V, X98E, X98G, X98N, X99H, X99K, X99N, X100W, and X1G, preferably selected from X4Q, X25H, X100W, X135E, X135T, X135W, X135D, X160W, X176K, X193E, X251E, X258Q, X276R, X299S, X323G, X363E, X363H, X382Q, X405M, X460G, and X482W, according to the numbering of the amino acid sequence set forth in SEQ ID NO: 3 and wherein said variant has alpha-amylase activity.

**3.** The alpha-amylase variant according to any one of the preceding claims, wherein said variant comprises a combination of substitutions selected from the group consisting of X25H+X176K+X186E+X206Y+X251E+X482W, X4Q+X25H+X176K+X186E+X206Y+X251E+X405M+X482W, X25H+X176K+X186E+X206Y+X482W, X25H+X186E+X206Y+X405M+X482W, X25H+X186E+X206Y+X482W,X25H+X176K+X186E+X193E+X206Y+X251E+X482W, X4Q+X176K+X186E+X193E+X206Y+X251E+X405M+X482W, X25H+X176K+X186E+X482W, X25H+X176K+X186E+X193E+X206Y+X482W, X4Q+X25H+X176K+X186E+X251E+X405M+X482W, X25H+X176K+X186E+X206Y, X4Q+X176K+X186E+X193E+X206Y+X251E+X405M, X4Q+X206Y+X460G+X482W, X4Q+X25H+X176K+X206Y+X251E+X460G+X482W, X4Q+X25H+X176K+X193E+X251E+X186E+X482W, X4Q+X193E+X206Y+X276R+X186E+X482W, X186E+X25H+X482W, X25H+X193E+X206Y+X251E+X276R+X405M+X186E+X482W, X25H+X251 E+X186E+X482W, X25H+X160W+X176K+X186E+X251E+X405M+X482W, X193E+X206Y+X405M+X186E+X482W, X3I+X356V, X3I+X356I, X83D+X94E, X94D+X125E, X131I+X377Q+X410H, X48F+X94D, X48Y+X116K+X218K, X5L+X218K+X225S, X83E+X116R+X158Y+X181E, X51V+X218K, X83E+X181E, X4Q+X7W+X176K+X186E+X193E+X206Y+X251E+X405M, X4Q+X37M+X176K+X186E+X193E+X206Y+X251E+X405M, X4Q+X25D+X176K+X186E+X193E+X206Y+X251E+X405M, X4Q+X25H+X176K+X186E+X206Y+X251E+X405M+X460G, X4Q+X25H+X176K+X186E+X206Y+X251E+X460G, X4Q+X25Y+X176K+X186E+X193E+X206Y+X251E+X405M, X4Q+X118D+X176K+X186E+X193E+X206Y+X251E+X405M, X4Q+X176K+X182D+X186E+X193E+X206Y+X251E+X405M, X4Q+X176K+X186E+X193E+X206Y+X251E+X258Q+X405M, X4Q+X176K+X186E+X193E+X206Y+X251E+X405M, X4Q+X176K+X186E+X193E+X206Y+X251E+X405M+X460G, X4Q+X176K+X186E+X193E+X206Y+X251E+X363E+X405M, X4Q+X176K+X186E+X193E+X206Y+X251E+X363H+X405M, X4Q+X176K+X186E+X193E+X206Y+X251E+X363N+X405M, X4Q+X176K+X181D+X186E+X193E+X206Y+X251E+X405M, X4Q+X176K+X181F+X186E+X193E+X206Y+X251E+X405M, X4Q+X176K+X181N+X186E+X193E+X206Y+X251E+X405M, X4Q+X176K+X181Q+X186E+X193E+X206Y+X251E+X405M, X4Q+X136E+X176K+X186E+X193E+X206Y+X251E+X405M, X4Q+X100W+X176K+X186E+X193E+X206Y+X251E+X405M, X4Q+X135D+X176K+X186E+X193E+X206Y+X251E+X405M, X4Q+X135E+X176K+X186E+X193E+X206Y+X251E+X405M, X4Q+X135T+X176K+X186E+X193E+X206Y+X251E+X405M, X4Q+X135W+X176K+X186E+X193E+X206Y+X251E+X405M, X4Q+X160D+X176K+X186E+X193E+X206Y+X251E+X405M, X4Q+X160E+X176K+X186E+X193E+X206Y+X251E+X405M, X7H+X25H+X176K+X186E+X206Y,X7W+X25H+X176K+X186E+X206Y, X25D+X176K+X186E+X206Y, X25H+X186E+X206Y+X405M+X460G, X25H+X186E+X206Y+X460G, X25H+X37M+X176K+X186E+X206Y, X25H+X118D+X176K+X186E+X206Y, X25H+X176K+X182D+X186E+X206Y, X25H+X176K+X186E+X206Y,X25H+X176K+X186E+X206Y+X258Q, X25H+X176K+X186E+X206Y+X281N,X25H+X176K+X186E+X206Y+X460G, X25H+X176K+X186E+X206Y+X251E+X460G,X25H+X176K+X186E+X206Y+X363E, X25H+X176K+X186E+X206Y+X363H,X25H+X176K+X186E+X206Y+X363N, X25H+X176K+X186E+X460G, X25H+X176K+X186E+X193E+X206Y, X25H+X176K+X186E+X193E+X206Y+X460G, X25H+X176K+X186E+X193E+X206Y+X251E+X460G, X25H+X176K+X181D+X186E+X206Y,X25H+X176K+X181N+X186E+X206Y, X25H+X176K+X181Q+X186E+X206Y,X25H+X136E+X176K+X186E+X206Y, X25H+X100W+X176K+X186E+X206Y, X25H+X135E+X176K+X186E+X206Y, X25H+X135T+X176K+X186E+X206Y, X25H+X135W+X176K+X186E+X206Y, X25H+X160D+X176K+X186E+X206Y, X25H+X160E+X176K+X186E+X206Y, X25Y+X176K+X186E+X206Y, X87D+X186E+X315K+X420K, X87D+X186E+X226D+X314E+X420E+X461E,X87D+X186E+X226D+X420E, X87D+X186E+X226D+X420E+X461E, X87D+X186E+X314E+X471E, X87D+X186E+X311K+X314E+X420K, X87D+X186E+X160D+X461E, X87R+X186E+X315K+X461R, X87R+X186E+X226D+X471E, X87R+X186E+X226R+X314K+X420K+X461R,X87R+X186E+X226R+X311K+X420K, X87R+X186E+X314K+X315K, X87R+X186E+X311K+X314E, X87R+X186E+X420E+X450R, X87R+X186E+X450R+X452R, X186E+X315K+X420E+X452R, X186E+X226D+X314E+X461E+X471E, X186E+X226D+X314E+X452R, X186E+X226D+X314K+X460D+X471E+X485R, X186E+X226D+X311K+X460D, X186E+X226D+X461E+X471E, X186E+X314E+X460D+X461E, X186E+X314E+X420E+X452R+X461E, X186E+X314K+X450R+X460D, X186E+X460D+X471E+X485R, X186E+X311K+X314K+X452R, X186E+X311K+X461E+X485R, X186E+X311K+X420E+X471E, X186E+X420K+X450R+X471E+X485R, X186E+X420K+X450R+X485R, X186E+X452R+X461E+X471E, X4Q+X176K+X186E+X193E+X206Y+X251E+X405M, X25H+X176K+X186E+X206Y, X83E+X186E+X219D+X226R, X83E+X186E+X219R+X226D+X314E+X452R, X83E+X186E+X219R+X226R, X83E+X186E+X160D+X219D+X226R+X452R, X83R+X186E+X219R+X226D, X160D+X186E+X315K+X450R, X160D+X186E+X226D+X314K+X460D+X461E,X160D+X186E+X226D+X314K+X420E, X160D+X186E+X314K+X485R, X160D+X186E+X420E+X452R, X160D+X186E+X420K+X460D, X186E+X276R,X186E+X206Y, X186E+X206Y+X276R, X186E+X206Y+X276R+X405M, X186E+X206Y+X405M, X186E+X206Y+X251E, X186E+X206Y+X251E+X276R, X186E+X206Y+X251E+X276R+X405M, X186E+X206Y+X251E+X405M, X186E+X206Y+X251E+X323G+X405M, X186E+X206Y+X323G+X405M, X186E+X405M, X186E+X193E+X206Y+X405M, X186E+X193E+X206Y+X251E, X186E+X251E, X186E+X251E+X405M, X4Q+X186E, X4Q+X186E+X276R, X4Q+X186E+X206Y, X4Q+X186E+X206Y+X276R+X405M, X4Q+X186E+X206Y+X405M, X4Q+X186E+X206Y+X251E+X276R, X4Q+X186E+X206Y+X251E+X276R+X405M, X4Q+X186E+X206Y+X251E+X405M, X4Q+X186E+X206Y+X251E+X323G+X405M, X4Q+X186E+X405M, X4Q+X186E+X193E+X206Y+X276R, X4Q+X186E+X193E+X206Y+X405M, X4Q+X186E+X193E+X206Y+X251E+X405M, X4Q+X186E+X251E+X276R+X405M, X4Q+X25H+X186E+X206Y, X4Q+X25H+X186E+X206Y+X276R, X4Q+X25H+X186E+X206Y+X276R+X405M, X4Q+X25H+X186E+X206Y+X405M, X4Q+X25H+X186E+X206Y+X251E+X323A+X405M, X4Q+X25H+X186E+X206Y+X323G+X405M, X4Q+X25H+X186E+X405M, X4Q+X25H+X176K+X186E+X206Y+X276R, X4Q+X25H+X176K+X186E+X206Y+X276R+X405M, X4Q+X25H+X176K+X186E+X206Y+X251E+X276R+X405M, X4Q+X25H+X176K+X186E+X206Y+X251E+X405M, X4Q+X25H+X176K+X186E+X251E, X4Q+X25H+X176K+X186E+X251E+X405M, X4Q+X25H+X176K+X206Y, X4Q+X25H+X176K+X206Y+X405M N460G, X4Q+X25H+X176K+X206Y+X460G, X4Q+X25H+X176K+X206Y+X251E, X4Q+X25H+X176K+X206Y+X251E+X276R+X405M, X4Q+X25H+X176K+X206Y+X251E+X460G, X4Q+X25H+X176K+X206Y+X323G, X4Q+X25H+X160W+X186E+X206Y+X405M, X4Q+X25H+X160W+X186E+X206Y+X251E, X4Q+X25H+X160W+X186E+X206Y+X251E+X276R+X405M, X4Q+X25H+X160W+X186E+X206Y+X323G+X405M, X4Q+X25H+X160W+X186E+X405M, X4Q+X25H+X160W+X186E+X193E+X206Y+X276R, X4Q+X25H+X160W+X186E+X323G, X4Q+X25H+X160W+X169R+X186E+X206Y+X276R, X4Q+X25H+X160W+X176K+X186E+X276R+X405M, X4Q+X25H+X160W+X176K+X186E+X206Y+X276R, X4Q+X25H+X160W+X176K+X186E+X206Y+X251E, X4Q+X25H+X160W+X176K+X186E+X206Y+X251E+X405M, X4Q+X25H+X160W+X176K+X186E+X405M, X4Q+X25H+X160W+X176K+X186E+X251E+X276R+X405M, X4Q+X25H+X160W+X176K+X186E+X251E+X405M, X4Q+X25H+X160W+X176K+X206Y, X4Q+X25H+X160W+X176K+X206Y+X405M, X4Q+X25H+X160W+X176K+X206Y+X460G, X4Q+X25H+X160W+X176K+X206Y+X251E+X460G, X4Q+X176K+X186E+X276R+X405M, X4Q+X176K+X186E+X206Y+X276R, X4Q+X176K+X186E+X206Y+X276R+X405M, X4Q+X176K+X186E+X206Y+X405M, X4Q+X176K+X186E+X206Y+X251E+X276R+X405M, X4Q+X176K+X186E+X206Y+X251E+X405M, X4Q+X176K+X186E+X405M, X4Q+X176K+X186E+X193E+X206Y+X276R+X405M, X4Q+X176K+X186E+X193E+X206Y+X251E+X405M, X4Q+X176K+X186E+X251E+X276R+X405M, X4Q+X176K+X186E+X251E+X405M, X4Q+X176K+X186E+X323G+X405M, X4Q+X176K+X206Y, X4Q+X176K+X206Y+X276R, X4Q+X176K+X206Y+X405M, X4Q+X176K+X206Y+X405M N460G, X4Q+X176K+X206Y+X460G, X4Q+X176K+X206Y+X251E+X276R+X405M, X4Q+X176K+X206Y+X251E+X276R+X405M N460G, X4Q+X176K+X206Y+X251E+X405M, X4Q+X176K+X206Y+X251E+X405M N460G, X4Q+X176K+X206Y+X251E+X323A+X460G, X4Q+X160W+X186E+X276R+X405M, X4Q+X160W+X186E+X206Y, X4Q+X160W+X186E+X206Y+X276R+X323G, X4Q+X160W+X186E+X206Y+X405M, X4Q+X160W+X186E+X206Y+X251E, X4Q+X160W+X186E+X206Y+X251E+X276R+X405M, X4Q+X160W+X186E+X405M, X4Q+X160W+X186E+X251E+X405M, X4Q+X160W+X176K+X186E, X4Q+X160W+X176K+X186E+X276R+X405M, X4Q+X160W+X176K+X186E+X206Y+X251E, X4Q+X160W+X176K+X186E+X251E+X276R+X405M, X4Q+X160W+X176K+X186E+X251E+X405M, X4Q+X160W+X176K+X206Y, X4Q+X160W+X176K+X206Y+X251E+X276R+X460G, X4Q+X160W+X176K+X206Y+X251E+X323G+X405M+X460G, X25H+X186E, X25H+X186E+X206Y,X25H+X186E+X206Y+X276R+X405M, X25H+X186E+X206Y+X276R+X323G, X25H+X186E+X206Y+X405M, X25H+X186E+X206Y+X251E+X276R+X323G+X405M, X25H+X186E+X206Y+X251E+X405M, X25H+X186E+X405M, X25H+X186E+X202I+X206Y+X405M, X25H+X186E+X193E+X206Y+X276R+X405M, X25H+X186E+X193E+X206Y+X405M, X25H+X186E+X193E+X206Y+X251E+X276R+X405M, X25H+X186E+X251E, X25H+X186E+X251E+X276R,X25H+X176K+X186E, X25H+X176K+X186E+X276R+X323G+X405M, X25H+X176K+X186E+X206Y, X25H+X176K+X186E+X206Y+X276R, X25H+X176K+X186E+X206Y+X251E, X25H+X176K+X186E+X206Y+X251E+X276R+X405M, X25H+X176K+X186E+X206Y+X251E+X405M, X25H+X176K+X186E+X405M, X25H+X176K+X186E+X193E+X206Y, X25H+X176K+X186E+X193E+X206Y+X251E, X25H+X176K+X186E+X193E+X206Y+X251E+X276R+X405M, X25H+X176K+X186E+X251E+X276R, X25H+X176K+X206Y, X25H+X176K+X206Y+X276R,X25H+X176K+X206Y+X276R+X405M, X25H+X176K+X206Y+X276R+X460G, X25H+X176K+X206Y+X405M, X25H+X176K+X206Y+X460G, X25H+X176K+X206Y+X251E, X25H+X176K+X206Y+X251E+X276R+X405M+X460G, X25H+X176K+X206Y+X251E+X276R+X460G, X25H+X176K+X206Y+X251E+X405M, X25H+X160W+X186E+X206Y, X25H+X160W+X186E+X206Y+X251E, X25H+X160W+X186E+X206Y+X251E+X276R, X25H+X160W+X186E+X206Y+X251E+X276R+X323G+X405M, X25H+X160W+X186E+X206Y+X251E+X405M, X25H+X160W+X186E+X206Y+X323G+X405M, X25H+X160W+X186E+X251E+X276R+X405M, X25H+X160W+X176K+X186E+X206Y+X276R, X25H+X160W+X176K+X186E+X206Y+X276R+X405M, X25H+X160W+X176K+X186E+X206Y+X251E+X276R+X323G, X25H+X160W+X176K+X186E+X206Y+X251E+X405M, X25H+X160W+X176K+X186E+X405M, X25H+X160W+X176K+X186E+X193E+X206Y, X25H+X160W+X176K+X186E+X193E+X206Y+X405M, X25H+X160W+X176K+X186E+X193E+X206Y+X251E+X405M, X25H+X160W+X176K+X186E+X251E, X25H+X160W+X176K+X186E+X251E+X276R, X25H+X160W+X176K+X186E+X251E+X405M, X25H+X160W+X176K+X186E+X323G+X405M, X25H+X160W+X176K+X206Y+X276R+X323G+X405M, X25H+X160W+X176K+X206Y+X460G, X25H+X160W+X176K+X206Y+X251E, X25H+X160W+X176K+X206Y+X251E+X323G+X405M, X176K+X186E+X206Y, X176K+X186E+X206Y+X276R, X176K+X186E+X206Y+X276R+X405M, X176K+X186E+X206Y+X405M, X176K+X186E+X206Y+X251E+X405M, X176K+X186E+X405M,X176K+X186E+X193E+X206Y+X405M, X176K+X186E+X193E+X206Y+X251E+X405M, X176K+X186E+X193E+X405M, X176K+X186E+X251E+X276R+X405M, X176K+X186E+X251E+X405M, X176K+X206Y, X176K+X206Y+X276R, X176K+X206Y+X276R+X460G, X176K+X206Y+X405M, X176K+X206Y+X405M N460G, X176K+X206Y+X460G, X176K+X206Y+X251E, X176K+X206Y+X251E+X405M, X160W+X186E, X160W+X186E+X276R+X405M, X160W+X186E+X206Y+X276R, X160W+X186E+X206Y+X276R+X405M, X160W+X186E+X206Y+X405M, X160W+X186E+X206Y+X251 E, X160W+X186E+X206Y+X251E+X405M, X160W+X186E+X405M, X160W+X186E+X193E+X206Y, X160W+X186E+X193E+X206Y+X405M, X160W+X186E+X251E+X276R, X160W+X186E+X251E+X276R+X405M, X160W+X186E+X323G+X405M, X160W+X176K+X186E+X276R+X405M, X160W+X176K+X186E+X206Y, X160W+X176K+X186E+X206Y+X251 E+X405M, X160W+X176K+X186E+X405M, X160W+X176K+X186E+X193E+X206Y, X160W+X176K+X186E+X193E+X206Y+X405M, X160W+X176K+X186E+X193E+X206Y+X251E, X160W+X176K+X206Y+X276R+X405M N460G, X160W+X176K+X206Y+X276R+X323G+X460G, X160W+X176K+X206Y+X405M, X160W+X176K+X206Y+X405M N460G, X160W+X176K+X206Y+X460G, X160W+X176K+X206Y+X251E+X276R, X160W+X176K+X206Y+X251E+X405M+X460G, X160W+X176K+X206Y+X323G+X405M+X460G, X4Q+X176K+X186E+X193E+X206Y+X251E+X405M, X4Q+X176K+X186E+X193E+X206Y+X251E+X405M+X258Q, X4Q+X176K+X186E+X193E+X206Y+X251E+X405M+X258Q+X281N, X4Q+X176K+X186E+X193E+X206Y+X251E+X405M+X258Q+X281N+X363H, X4Q+X176K+X186E+X193E+X206Y+X251E+X405M+X258Q+X363E, X4Q+X176K+X186E+X193E+X206Y+X251E+X405M+X281N, X4Q+X176K+X186E+X193E+X206Y+X251E+X405M+X281N+X363E, X4Q+X176K+X186E+X193E+X206Y+X251E+X405M+X281N+X363H, X4Q+X176K+X186E+X193E+X206Y+X251E+X405M+X181Q, X4Q+X176K+X186E+X193E+X206Y+X251E+X405M+X181Q+X258Q, X4Q+X176K+X186E+X193E+X206Y+X251E+X405M+X181Q+X258Q+X281N, X4Q+X176K+X186E+X193E+X206Y+X251E+X405M+X181Q+X258Q+X281N+X363H, X4Q+X176K+X186E+X193E+X206Y+X251E+X405M+X181Q+X281N, X4Q+X176K+X186E+X193E+X206Y+X251E+X405M+X181Q+X281N+X363E, X4Q+X176K+X186E+X193E+X206Y+X251E+X405M+X181Q+X363E, X4Q+X176K+X186E+X193E+X206Y+X251E+X405M+X181Q+X363H, X4Q+X176K+X186E+X193E+X206Y+X251E+X405M+X100W, X4Q+X176K+X186E+X193E+X206Y+X251E+X405M+X100W+X258Q, X4Q+X176K+X186E+X193E+X206Y+X251E+X405M+X100W+X258Q+X281N, X4Q+X176K+X186E+X193E+X206Y+X251E+X405M+X100W+X258Q+X281N+X363E, X4Q+X176K+X186E+X193E+X206Y+X251E+X405M+X100W+X258Q+X281N+X363H, X4Q+X176K+X186E+X193E+X206Y+X251E+X405M+X100W+X258Q+X281N+X363H+X2 72V, X4Q+X176K+X186E+X193E+X206Y+X251 E+X405M+X100W+X258Q+X363E, X4Q+X176K+X186E+X193E+X206Y+X251E+X405M+X100W+X258Q+X363H, X4Q+X176K+X186E+X193E+X206Y+X251E+X405M+X100W+X281N, X4Q+X176K+X186E+X193E+X206Y+X251E+X405M+X100W+X281N+X363E, X4Q+X176K+X186E+X193E+X206Y+X251E+X405M+X100W+X281N+X363H, X4Q+X176K+X186E+X193E+X206Y+X251E+X405M+X100W+X181Q, X4Q+X176K+X186E+X193E+X206Y+X251E+X405M+X100W+X181Q+X258Q, X4Q+X176K+X186E+X193E+X206Y+X251E+X405M+X100W+X181Q+X258Q+X281N, X4Q+X176K+X186E+X193E+X206Y+X251E+X405M+X100W+X181Q+X258Q+X281N+X3 63E, X4Q+X176K+X186E+X193E+X206Y+X251E+X405M+X100W+X181Q+X258Q+X363E, X4Q+X176K+X186E+X193E+X206Y+X251E+X405M+X100W+X181Q+X258Q+X363H, X4Q+X176K+X186E+X193E+X206Y+X251E+X405M+X100W+X181Q+X281N, X4Q+X176K+X186E+X193E+X206Y+X251E+X405M+X100W+X181Q+X281N+X363H, X4Q+X176K+X186E+X193E+X206Y+X251E+X405M+X100W+X181Q+X363H, X4Q+X176K+X186E+X193E+X206Y+X251E+X405M+X100W+X181Q+X363H+X254Q, X4Q+X176K+X186E+X193E+X206Y+X251E+X405M+X100W+X135T, X4Q+X176K+X186E+X193E+X206Y+X251E+X405M+X100W+X135T+X258Q, X4Q+X176K+X186E+X193E+X206Y+X251E+X405M+X100W+X135T+X258Q+X281N+X3 63H, X4Q+X176K+X186E+X193E+X206Y+X251E+X405M+X100W+X135T+X258Q+X363E, X4Q+X176K+X186E+X193E+X206Y+X251E+X405M+X100W+X135T+X258Q+X363H, X4Q+X176K+X186E+X193E+X206Y+X251E+X405M+X100W+X135T+X181Q, X4Q+X176K+X186E+X193E+X206Y+X251E+X405M+X100W+X135T+X181Q+X258Q, X4Q+X176K+X186E+X193E+X206Y+X251E+X405M+X100W+X135T+X181Q+X258Q+X2 81N, X4Q+X176K+X186E+X193E+X206Y+X251E+X405M+X100W+X135T+X181Q+X258Q+X3 63E, X4Q+X176K+X186E+X193E+X206Y+X251E+X405M+X100W+X135T+X181Q+X258Q+X3 63H, X4Q+X176K+X186E+X193E+X206Y+X251E+X405M+X100W+X135T+X181Q+X281N+X3 63H, X4Q+X176K+X186E+X193E+X206Y+X251E+X405M+X100W+X135T+X181Q+X363E, X4Q+X176K+X186E+X193E+X206Y+X251E+X405M+X100W+X135T+X181Q+X363H, X4Q+X176K+X186E+X193E+X206Y+X251E+X405M+X100W+X135T+X363E, X4Q+X176K+X186E+X193E+X206Y+X251E+X405M+X100W+X135T+X363H, X4Q+X176K+X186E+X193E+X206Y+X251E+X405M+X100W+X363E, X4Q+X176K+X186E+X193E+X206Y+X251E+X405M+X100W+X363H, X4Q+X176K+X186E+X193E+X206Y+X251E+X405M+X135T, X4Q+X176K+X186E+X193E+X206Y+X251E+X405M+X135T+X258Q, X4Q+X176K+X186E+X193E+X206Y+X251E+X405M+X135T+X258Q+X8A, X4Q+X176K+X186E+X193E+X206Y+X251E+X405M+X135T+X258Q+X281N+X363E, X4Q+X176K+X186E+X193E+X206Y+X251E+X405M+X135T+X258Q+X363H, X4Q+X176K+X186E+X193E+X206Y+X251E+X405M+X135T+X281N, X4Q+X176K+X186E+X193E+X206Y+X251E+X405M+X135T+X181Q+X258Q, X4Q+X176K+X186E+X193E+X206Y+X251E+X405M+X135T+X181Q+X258Q+X281N, X4Q+X176K+X186E+X193E+X206Y+X251E+X405M+X135T+X181Q+X258Q+X363H, X4Q+X176K+X186E+X193E+X206Y+X251E+X405M+X135T+X363E, X4Q+X176K+X186E+X193E+X206Y+X251E+X405M+X135T+X363H, X4Q+X176K+X186E+X193E+X206Y+X251E+X405M+X363E, X4Q+X176K+X186E+X193E+X206Y+X251E+X405M+X363H, X25H+X176K+X186E+X206Y, X25H+X176K+X186E+X206Y+X258Q, X25H+X176K+X186E+X206Y+X258Q, X25H+X176K+X186E+X206Y+X258Q+X281N, X25H+X176K+X186E+X206Y+X258Q+X281N+X363E, X25H+X176K+X186E+X206Y+X258Q+X363E, X25H+X176K+X186E+X206Y+X258Q+X363E+X123D, X25H+X176K+X186E+X206Y+X281N, X25H+X176K+X186E+X206Y+X181Q, X25H+X176K+X186E+X206Y+X181Q+X193E, X25H+X176K+X186E+X206Y+X181Q+X363E,X25H+X176K+X186E+X206Y+X193E, X25H+X176K+X186E+X206Y+X193E+X258Q, X25H+X176K+X186E+X206Y+X193E+X281N+X363E, X25H+X176K+X186E+X206Y+X193E+X363E, X25H+X176K+X186E+X206Y+X100W, X25H+X176K+X186E+X206Y+X100W+X258Q, X25H+X176K+X186E+X206Y+X100W+X258Q+X281N, X25H+X176K+X186E+X206Y+X100W+X258Q+X281N+X363E, X25H+X176K+X186E+X206Y+X100W+X258Q+X363E, X25H+X176K+X186E+X206Y+X100W+X258Q+X363E+X135C, X25H+X176K+X186E+X206Y+X100W+X281N, X25H+X176K+X186E+X206Y+X100W+X281N+X363E, X25H+X176K+X186E+X206Y+X100W+X361R, X25H+X176K+X186E+X206Y+X100W+X181Q+X281N+X363E, X25H+X176K+X186E+X206Y+X100W+X181Q+X281N+X363E+X175D, X25H+X176K+X186E+X206Y+X100W+X181Q+X193E, X25H+X176K+X186E+X206Y+X100W+X181Q+X193E+X258Q+X281N, X25H+X176K+X186E+X206Y+X100W+X181Q+X193E+X258Q+X363E, X25H+X176K+X186E+X206Y+X100W+X181Q+X193E+X281N+X363E, X25H+X176K+X186E+X206Y+X100W+X181Q+X193E+X363E, X25H+X176K+X186E+X206Y+X100W+X181Q+X363E, X25H+X176K+X186E+X206Y+X100W+X193E, X25H+X176K+X186E+X206Y+X100W+X193E+X258Q, X25H+X176K+X186E+X206Y+X100W+X193E+X258Q+X473R, X25H+X176K+X186E+X206Y+X100W+X193E+X258Q+X281N, X25H+X176K+X186E+X206Y+X100W+X193E+X258Q+X281N+X363E, X25H+X176K+X186E+X206Y+X100W+X193E+X258Q+X363E, X25H+X176K+X186E+X206Y+X100W+X193E+X363E, X25H+X176K+X186E+X206Y+X100W+X135T, X25H+X176K+X186E+X206Y+X100W+X135T+X258Q, X25H+X176K+X186E+X206Y+X100W+X135T+X258Q+X281N, X25H+X176K+X186E+X206Y+X100W+X135T+X258Q+X281N+X363E, X25H+X176K+X186E+X206Y+X100W+X135T+X258Q+X363E, X25H+X176K+X186E+X206Y+X100W+X135T+X281N, X25H+X176K+X186E+X206Y+X100W+X135T+X281N+X363E, X25H+X176K+X186E+X206Y+X100W+X135T+X181Q, X25H+X176K+X186E+X206Y+X100W+X135T+X181Q+X258Q+X281N+X363E, X25H+X176K+X186E+X206Y+X100W+X135T+X181Q+X281N+X363E, X25H+X176K+X186E+X206Y+X100W+X135T+X181Q+X193E, X25H+X176K+X186E+X206Y+X100W+X135T+X181Q+X193E+X258Q+X363E, X25H+X176K+X186E+X206Y+X100W+X135T+X181Q+X193E+X363E, X25H+X176K+X186E+X206Y+X100W+X135T+X193E, X25H+X176K+X186E+X206Y+X100W+X135T+X193E+X258Q+X363E, X25H+X176K+X186E+X206Y+X100W+X135T+X193E+X363E, X25H+X176K+X186E+X206Y+X100W+X135T+X363E, X25H+X176K+X186E+X206Y+X100W+X363E,X25H+X176K+X186E+X206Y+X135T, X25H+X176K+X186E+X206Y+X135T+X258Q, X25H+X176K+X186E+X206Y+X135T+X258Q+X281N, X25H+X176K+X186E+X206Y+X135T+X258Q+X281N+X363E,and X25H+X176K+X186E+X206Y+X135T+X258Q+X363E
wherein the numbering is according to the amino acid sequence set forth in SEQ ID NO: 3 and wherein said variant has alpha-amylase activity.

**4.** The alpha-amylase variant according to any one of the preceding claims, wherein the variant has at least 85%, at least 90%, at least 91%, at least 92%, at least 93%, at least 94%, at least 95%, at least 96%, at least 97%, at least 98%, or at least 99%, but less than 100%, sequence identity to the amino acid sequence set forth in SEQ ID NO: 1.

**5.** The alpha-amylase variant according to any one of the preceding claims, wherein the variant comprises an A and B domain and a C domain wherein the amino acid sequence of the A and B domain is at least 75%, but less than 100%, identical to the amino acid sequence of SEQ ID NO: 6 and the amino acid sequence of the C domain is at least 75%, but less than 100%, identical to the amino acid sequence of SEQ ID NO: 8.

**6.** The alpha-amylase variant according to any one of the preceding claims, wherein the variant exhibits one or more improved property relative to said parent alpha-amylase, preferably relative to the parent alpha-amylase set forth in SEQ ID NO: 1 and/or SEQ ID NO: 3, preferably relative to the parent alpha-amylase set forth in SEQ ID NO: 1, preferably the alpha-amylase variant has an increase in expression, activity, stability, thermostability, specific activity, substrate specificity, pH-dependent activity, pH stability, oxidative stability, catalytic efficiency, catalytic rate, chemical stability, pH activity, stability under storage conditions, substrate binding, substrate cleavage, substrate stability, surface properties, thermal activity, Ca2+ dependency, wash performance, wash performance in a laundry detergent, and/or wash performance in an ADW detergent, preferably, the improved property is improved thermostability, improved stability under storage in a detergent composition, and/or an improved wash performance, most preferably improved thermostability, preferably improved thermostability in a detergent composition, preferably in laundry or dishwash detergent composition, preferably laundry detergent composition.

**7.** The alpha-amylase variant according to claim 7, wherein said improved property is expressed as an Improvement Factor (IF) of >1.0 and wherein preferably the Improvement Factor is equal or greater than 1.1, preferably, equal or greater than 1.2, more preferably, equal or greater than 1.3.

**8.** The alpha-amylase variant according to any one of the preceding claims, further comprising a deletion at one or more amino acids corresponding to positions selected from the group consisting of 181, 182, 183 and 184, preferably a deletion of two or more amino acids corresponding to positions selected from the group consisting of 181, 182, 183 and 184, preferably a deletion of amino acids corresponding to positions 181 and 182, 182 and 183, or 183 and 184, wherein the numbering is according to the amino acid sequence set forth in SEQ ID NO: 3.

**9.** The alpha-amylase variant according to any one of the preceding claims, wherein the number of substitutions compared to SEQ ID NO: 1 is 1 to 30, preferably 1 to 25, 1 to 20, 1 to 15, 1 to 10, 2 to 10, 3 to 10, or 1 to 5, such as 1, 2, 3, 4, 5, 6, 7, 8, 9, 10, 11, 12, 13, 14, 15, 15, 16, 17, 18, 19, 20, 21, 22, 23, 24, or 25 substitutions.

**10.** The alpha-amylase variant according to any one of the preceding claims, wherein said variant comprises or consists of the amino acid sequence set forth in SEQ ID NO: 1 with one or more, preferably 1-30, of the amino acid substitutions selected from the group of amino acid substitutions consisting of X100D, X100F, X100K, X100L, X103A, X106D, X108A, X109A, X10A, X10C, X10E, X10F, X10L, X10W, X10Y, X113F, X113H, X113M, X113R, X113V, X113W, X113Y, X114A, X114C, X114D, X114E, X114F, X114G, X114H, X114I, X114K, X114L, X114M, X114N, X114Q, X114R, X114S, X114V, X114W, X114Y, X115C, X115D, X115K, X115M, X115N, X115Q, X115R, X115S, X115T, X115V, X116I, X116K, X116L, X116M, X116R, X116T, X117A, X117C, X117D, X117F, X117I, X117N, X117P, X117R, X117W, X117Y, X118A, X118D, X118E, X119H, X119P, X119R, X119S, X119Y, X120E, X120G, X120M, X120S, X120W, X120Y, X123D, X123S, X125A, X125D, X125E, X125F, X125G, X125H, X125K, X125L, X125M, X125N, X125Q, X125R, X125T, X125V, X125W, X125Y, X126D, X128C, X128E, X128L, X128M, X128W, X128Y, X129E, X12N, X12S, X131C, X131I, X131L, X131Q, X131W, X132T, X133A, X133C, X133D, X133E, X133H, X133K, X133N, X133P, X133Q, X133S, X134C, X134E, X134F, X134I, X134L, X134M, X134P, X134T, X134V, X134W, X134Y, X135D, X135E, X135G, X135M, X135N, X135P, X135S, X135T, X135W, X136A, X136C, X136D, X136E, X136F, X136H, X136L, X136M, X136N, X136P, X136W, X139C, X139S, X13A, X13Y, X141V, X142C, X142E, X142F, X142L, X142M, X142Q, X142R, X142W, X142Y, X143F, X144C, X144E, X144G, X144K, X144N, X144Q, X144R, X144S, X144T, X144V, X144Y, X145A, X146C, X146D, X146E, X146F, X146G, X146H, X146K, X146L, X146S, X146T, X146W, X147M, X149E, X14N, X150C, X150E, X150Q, X151C, X151E, X154A, X154Y, X155Y, X156E, X156V, X158H, X158N, X158Y, X15R, X160D, X160E, X160W, X161T, X162V, X163A, X163Q, X163T, X164V, X165S, X165T, X165W, X169A, X169D, X169E, X169S, X169V, X16F, X16R, X170C, X170F, X172A, X172C, X172D, X172K, X172N, X173I, X173Y, X174D, X174E, X174G, X174H, X174M, X174N, X174P, X174S, X174T, X175A, X175G, X175H, X175Q, X176K, X176S, X176T, X177A, X177G, X177K, X177N, X177P, X177R, X177S, X177W, X178F, X17K, X17V, X180M, X180N, X180T, X180W, X182D, X182E, X182N, X182Q, X182S, X184G, X185E, X185M, X185N, X187A, X187D, X187M, X187V, X188H, X188T, X188V, X189I, X18F, X18I, X18K, X18M, X18R, X18T, X191F, X191H, X191K, X191M, X191W, X191Y, X192A, X192T, X193D, X193E, X193G, X193V, X19A, X19C, X19D, X19F, X19G, X19H, X19I, X19K, X19L, X19M, X19P, X19Q, X19S, X19T, X19Y, X1R, X1V, X203E, X203R, X208F, X208I, X208Y, X20A, X20C, X20D, X20E, X20F, X20G, X20H, X20K, X20L, X20M, X20N, X20P, X20Q, X20S, X20T, X20V, X20W, X20Y, X210A, X210C, X210D, X210E, X210F, X210M, X210N, X210Q, X210S, X210Y, X211A, X211C, X211D, X211E, X211G, X211H, X211L, X211N, X211Q, X211S, X211T, X211V, X212C, X212D, X212I, X212L, X212W, X213A, X213C, X213M, X213R, X213S, X214E, X214P, X215A, X215D, X215E, X215H, X215W, X216G, X216H, X218A, X218C, X218D, X218E, X218F, X218G, X218H, X218I, X218K, X218L, X218N, X218Q, X218S, X218T, X218V, X218W, X218Y, X219A, X219C, X219D, X219E, X219F, X219G, X219H, X219K, X219M, X219Q, X219R, X219S, X219T, X219W, X219Y, X21E, X21S, X221F, X221N, X222D, X222K, X222S, X222T, X223C, X223L, X223V, X223Y, X224F, X224V, X225A, X225C, X225E, X225F, X225H, X225I, X225N, X225R, X225S, X225Y, X226A, X226C, X226D, X226E, X226G, X226H, X226I, X226L, X226M, X226Q, X226R, X226S, X226T, X226V, X226W, X226Y, X227C, X227F, X227G, X227H, X227I, X227K, X227L, X227M, X227R, X227T, X227V, X227W, X227Y, X22A, X22D, X22E, X22F, X22G, X22K, X22L, X22M, X22Q, X22R, X22T, X22W, X22Y, X230A, X230F, X231C, X231D, X231N, X233C, X233H, X233I, X233M, X233T, X233W, X233Y, X234C, X235L, X235M, X235V, X236Y, X237C, X237I, X238A, X238F, X238T, X23N, X23Q, X23W, X240M, X242M, X242N, X243D, X243F, X245E, X245H, X245M, X249D, X249I, X24G, X250V, X251A, X251E, X251F, X251L, X251M, X251S, X251T, X252C, X252I, X252S, X253C, X253G, X253Y, X255D, X255F, X255I, X255T, X255V, X256C, X257L, X257V, X257Y, X258F, X258Q, X258R, X259L, X25A, X25C, X25D, X25F, X25G, X25H, X25K, X25L, X25M, X25Q, X25S, X25W, X25Y, X260H, X261R, X262C, X262D, X262E, X262H, X262P, X262Y, X263I, X264H, X264T, X264W, X265S, X268F, X268G, X269M, X26A, X26D, X26E, X26F, X26L, X26M, X26P, X26S, X26V, X26Y, X270A, X270Q, X270Y, X271S, X272F, X272G, X272L, X272S, X273A, X273C, X273D, X273E, X273F, X273H, X273I, X273L, X273M, X273P, X273Q, X273R, X273V, X273W, X273Y, X274F, X274S, X275V, X276D, X276K, X276L, X276N, X276R, X276Y, X277D, X277E, X277T, X279A, X279P, X27A, X27D, X27F, X27G, X27H, X27I, X27Q, X27R, X27T, X27V, X280D, X280F, X280G, X280H, X280I, X280K, X280N, X280R, X280V, X280Y, X281A, X281D, X281E, X281H, X284A, X284F, X284H, X284L, X284M, X284N, X284Y, X285G, X285L, X285N, X285P, X286Q, X287A, X287D, X287E, X287H, X287T, X288A, X288K, X288P, X288Y, X289F, X289G, X289R, X289T, X28C, X28D, X28E, X28F, X28G, X28I, X28K, X28N, X28Q, X28S, X28T, X28V, X290D, X290M, X290N, X290Q, X290W, X291D, X291K, X291T, X291Y, X292C, X292D, X292F, X292I, X292L, X292T, X292W, X292Y, X293D, X293E, X293F, X293K, X293R, X294G, X294T, X295F, X296A, X296C, X296L, X296Y, X297E, X297F, X297H, X297K, X297M, X297S, X297V, X299G, X299I, X299K, X299L, X299S, X299Y, X29D, X29E, X29F, X29G, X29H, X29I, X29K, X29L, X29N, X29P, X29Q, X29V, X29W, X29Y, X2I, X2S, X301F, X302H, X302I, X302Q, X302V, X302Y, X303E, X303H, X303I, X303K, X303L, X303M, X303N, X303P, X303R, X303T, X304A, X304D, X304E, X304H, X304K, X304M, X304N, X304P, X304R, X304T, X304W, X304Y, X306A, X306D, X306E, X306G, X306H, X306I, X306M, X306Q, X306R, X306S, X306T, X306V, X306W, X306Y, X307F, X307M, X308S, X309H, X309L, X309Q, X30A, X30E, X30F, X30G, X30H, X30I, X30K, X30L, X30M, X30Q, X30T, X30W, X30Y, X310A, X310Q, X311A, X311E, X311G, X311H, X311K, X311N, X311R, X311T, X311Y, X312L, X312M, X313V, X314C, X314E, X314K, X314Q, X315A, X315C, X315E, X315H, X315K, X315T, X318I, X318S, X318T, X319A, X319D, X319H, X319I, X319K, X319M, X319N, X319P, X319S, X319T, X319W, X31N, X31Q, X31S, X31T, X31V, X31W, X320A, X320C, X320D, X320E, X320G, X320H, X320K, X320L, X320N, X320Q, X320S, X320Y, X321A, X321E, X321K, X321N, X321T, X321V, X321W, X323A, X323G, X323K, X323L, X323V, X324K, X324L, X324M, X324W, X324Y, X326G, X326N, X326S, X326Y, X327C, X327L, X327M, X32A, X32D, X32E, X32F, X32H, X32I, X32L, X32M, X32N, X32P, X32Q, X32T, X32W, X333I, X334T, X336K, X337A, X337C, X337F, X337G, X337I, X337K, X337L, X337M, X337N, X337Q, X337R, X337S, X337T, X337V, X337Y, X338G, X338S, X338T, X33D, X33E, X33H, X33K, X33M, X33Q, X33R, X33Y, X341V, X342P, X343L, X343T, X343W, X343Y, X344I, X344Q, X344V, X345D, X345G, X345M, X345N, X345Q, X345S, X345T, X346A, X346C, X346D, X346G, X346H, X346N, X346Q, X348T, X34H, X34I, X34V, X350H, X350K, X350P, X351A, X351M, X352S, X353H, X354I, X354N, X354T, X354Y, X355I, X355M, X356I, X356V, X357A, X358I, X358L, X358N, X358P, X358V, X359E, X35A, X35C, X35D, X35G, X35H, X35I, X35L, X35M, X35N, X35P, X35Q, X35R, X35S, X35T, X35V, X35Y, X360A, X360F, X360G, X360I, X360L, X360N, X360Q, X360R, X360S, X360T, X360V, X360Y, X362F, X362K, X362M, X362N, X362T, X362V, X362Y, X363A, X363C, X363D, X363E, X363G, X363H, X363K, X363L, X363M, X363P, X363Q, X363R, X363S, X363T, X363V, X363W, X363Y, X364A, X364C, X364G, X364K, X364L, X364N, X364S, X364T, X364V, X366I, X366L, X366T, X367E, X367S, X368A, X368F, X368L, X368N, X36A, X36E, X36G, X36I, X36K, X36M, X36N, X36P, X36Q, X36R, X36S, X36T, X36V, X370E, X370I, X372A, X372C, X372E, X372F, X372H, X372M, X372N, X372Q, X375A, X375D, X375E, X375I, X375K, X375Q, X375R, X375T, X375W, X375Y, X376G, X376I, X376K, X376L, X376M, X376Q, X376R, X376S, X376V, X377Q, X378C, X378D, X378E, X378R, X379A, X379L, X379S, X37A, X37G, X37M, X37P, X37T, X37V, X37W, X381E, X381V, X382A, X382H, X382K, X382L, X382N, X382Q, X382S, X383C, X383D, X383E, X383H, X383I, X383M, X383N, X383Q, X383R, X383S, X383V, X383Y, X384A, X384C, X384D, X384E, X384F, X384I, X384L, X384M, X384N, X384Q, X384R, X384T, X384V, X384W, X384Y, X385A, X385C, X385D, X385E, X385F, X385G, X385H, X385I, X385L, X385M, X385N, X385P, X385Q, X385R, X385S, X385T, X385V, X385W, X385Y, X387C, X387E, X387N, X388E, X388F, X388H, X388I, X388M, X388R, X388V, X389G, X389H, X389K, X38N, X390D, X390F, X390M, X390N, X390P, X390R, X391A, X391F, X391G, X391K, X391M, X391N, X391Q, X391S, X391T, X391Y, X392C, X392V, X393E, X393H, X393P, X393S, X393V, X394A, X394C, X394E, X394H, X394I, X394L, X394M, X394N, X394R, X394S, X395A, X395H, X395M, X395V, X396H, X396P, X397D, X397H, X397P, X397S, X398M, X399P, X39E, X39K, X3A, X3F, X3G, X3I, X3K, X3L, X3Q, X3V, X400A, X400D, X400E, X400G, X400H, X400I, X400K, X400L, X400M, X400N, X400P, X400Q, X400R, X400S, X400V, X400W, X401I, X401K, X401M, X401T, X403N, X405C, X405H, X405M, X405T, X405V, X406P, X407D, X407R, X407S, X408E, X408I, X408Q, X40I, X40S, X410H, X410I, X410K, X410L, X410P, X410R, X410Y, X413S, X414C, X414E, X414S, X415E, X415I, X416S, X418C, X418N, X418P, X41C, X41D, X41E, X41G, X41Q, X41S, X41T, X421N, X421P, X424A, X426D, X426W, X427C, X427F, X427G, X427K, X427Q, X427R, X427S, X427T, X427V, X429A, X429D, X429E, X429F, X429G, X429I, X429M, X429N, X429P, X429Q, X429S, X429T, X429V, X429W, X42C, X42I, X42Q, X42V, X431I, X434S, X436E, X438F, X438P, X438Y, X439C, X439P, X440V, X442Q, X443H, X443T, X445A, X445C, X445D, X445F, X445G, X445H, X445K, X445M, X445Q, X445R, X445S, X445T, X445V, X446F, X446I, X446L, X446P, X446Q, X446R, X446V, X446W, X447V, X448D, X448E, X448H, X448N, X449A, X449F, X449G, X449K, X449L, X449M, X449N, X449P, X449Q, X449R, X449S, X449V, X449W, X449Y, X451L, X453G, X453I, X453N, X453P, X453Y, X455L, X456L, X456M, X456R, X456S, X456V, X456W, X456Y, X457A, X457C, X457E, X457F, X457G, X457K, X457L, X457M, X457R, X457S, X457T, X457V, X457W, X458I, X458K, X458V, X458W, X459C, X459D, X459E, X459I, X459K, X459N, X459Q, X459R, X459V, X459Y, X45G, X45N, X460A, X460D, X460E, X460G, X460Q, X460R, X460S, X460T, X460V, X461A, X461E, X461F, X461K, X461L, X461M, X461N, X461Q, X461R, X461S, X461V, X462K, X463L, X465H, X465P, X465R, X465V, X467A, X467E, X467H, X468D, X468H, X470A, X470Q, X470T, X471E, X474F, X474H, X474P, X478A, X478E, X47S, X480D, X480E, X480M, X480R, X480Y, X482C, X482T, X482W, X48F, X48I, X48M, X48Y, X4A, X4C, X4K, X4M, X4Q, X4R, X4S, X51Q, X51T, X51V, X54D, X54G, X54Q, X59T, X5A, X5C, X5D, X5E, X5F, X5H, X5I, X5K, X5L, X5M, X5N, X5P, X5Q, X5R, X5V, X5Y, X60T, X63C, X63V, X6A, X6C, X6E, X6F, X6G, X6H, X6K, X6L, X6M, X6P, X6Q, X6S, X6T, X6V, X6W, X6Y, X70F, X70H, X70L, X70M, X70N, X70Y, X71D, X72C, X72D, X72E, X72N, X72T, X73L, X73N, X73Q, X75A, X75G, X75I, X75L, X75P, X75T, X75W, X76C, X76E, X76G, X76L, X76T, X76V, X7C, X7E, X7F, X7H, X7K, X7N, X7P, X7Q, X7R, X7S, X7V, X7W, X7Y, X81H, X81L, X82K, X82M, X83A, X83D, X83E, X83G, X83R, X83S, X86K, X87D, X87R, X89A, X89C, X89F, X89G, X89L, X89M, X89R, X89S, X8A, X8C, X8F, X8I, X8M, X8P, X8S, X8V, X8W, X8Y, X90A, X90D, X90E, X90F, X90G, X90I, X90M, X90N, X90Q, X90R, X90S, X90V, X90Y, X91C, X91D, X91E, X91F, X91G, X91H, X91I, X91K, X91L, X91M, X91N, X91Q, X91S, X91T, X91V, X91W, X91Y, X92D, X92M, X92V, X93K, X93Q, X94A, X94D, X94E, X94K, X94M, X94V, X94Y, X95E, X95I, X95L, X95V, X96K, X96N, X96Q, X97V, X98E, X98G, X98N, X99H, X99K, X99N, X100W, and X1G.

**11.** The alpha-amylase variant according to any one of the preceding claims, further comprising additional amino acid alterations, preferably additional substitutions, at one or more positions corresponding to positions selected from the group consisting of 9, 130, 179, 181, 186, 190, 195, 202, 206, 244, 402, 419, 420, 422, 423, 428, 430, 435, 441, 444, 450, 452, 454, 466, 469, 473, 475, 476, 479, 483, and 485, preferably one or more additional amino acid substitutions selected from the group of amino acid substitutions consisting of X9D, X9F, X9K, X9L, X9N, X9P, X9Q, X9S, X9T, X9Y, X130V, X179G, X179L, X181D, X181E, X181F, X181H, X181I, X181N, X181Q, X181S, X181T, X181V, X181W, X181Y, X186A, X186C, X186D, X186E, X186F, X186H, X186I, X186K, X186L, X186M, X186N, X186Q, X186R, X186S, X186V, X186W, X186Y, X190H, X195F, X195H, X195K, X195L, X195W, X195Y, X202L X206C, X206H, X206L, X206M, X206Y, X244A, X244C, X244D, X244E, X244F, X244G, X244H, X244M, X244N, X244Q, X244V, X402T, X419C, X420D, X420E, X420G, X420H, X420K, X420L, X420Q, X422C, X422N, X422H, X423F, X423M, X423Q, X423S, X428A, X428C, X428D, X428E, X428G, X428I, X428K, X428L, X428M, X428N, X428R, X428S, X428V, X428W, X428Y, X430A, X430C, X430D, X430E, X430F, X430G, X430I, X430L, X430P, X430Q, X430S, X430T, X430V, X435E, X435K, X435P, X435R, X435S, X435A, X435D, X437A, X437L, X437T, X437W, X441C, X441D, X441K, X441L, X441M, X441N, X441S, X444E, X444H, X444K, X444M, X444N, X444R, X444T, X450C, X450D, X450E, X450H, X450I, X450L, X450M, X450P, X450Q, X450R, X450T, X452A, X452C, X452E, X452F, X452I, X452K, X452M, X452N, X452R, X452T, X454A, X454E, X454K, X454L, X454M, X454P, X454S, X454T, X466E, X466W, X469F, X469L, X469Y, X473H, X473Q, X473R, X475A, X475K, X475N, X475E, X475L, X476G, X476E, X479A, X479I, X479K, X479M, X483F, X483I, X483L, X483Q, X483R, X485K, and X485R wherein the numbering is according to the amino acid sequence set forth in SEQ ID NO: 3.

**12.** A polynucleotide encoding the alpha-amylase variant according to any one of the preceding claims.

**13.** A nucleic acid construct or an expression vector comprising the polynucleotide according to claim 12.

**14.** A host cell comprising the polynucleotide according to claim 12, the nucleic acid construct according to claim 13, or the expression vector according to claim 13.

**15.** A composition comprising the alpha-amylase variant according to any one of claims 1 to 11 and at least one additional component.

**16.** The composition according to claim 15, which is a detergent composition, preferably a laundry detergent composition or an Automatic Dish Wash (ADW) detergent composition.

**17.** The composition according to claim 16, wherein the composition comprises one or more surfactants and/or one or more builder, preferably strong sequestering builder.

**18.** The composition according to any one of claims 15-17, wherein the composition comprises one or more second enzyme different from the alpha-amylase variant referred to in any of the preceding claims, preferably one or more second enzyme selected from the group consisting of a protease, a second amylase, a lipase, a cellulase, a laccase, a mannanase, a pectinase, xylanase, and a nuclease, particularly preferred selected from protease, mannanase, and lipase, most preferably a protease.

**19.** A method of producing an alpha-amylase variant, comprising:
a. cultivating said host cell according to claim 14 under conditions suitable for expression of said alpha-amylase variant; and
b. recovering said alpha-amylase variant.

**21.** Use of an alpha-amylase variant according to any one of claims 1 to 11 in a cleaning process such as laundry or hard surface cleaning.
